# FASCICULE DE BREVET EUROPEEN

(11) **EP 1 773 796 B1**
(45) Date de publication et mention de la délivrance du brevet: **03.03.2010**
(21) Numéro de dépôt: 05790990.5
(22) Date de dépôt: 20.07.2005
(51) Int. Cl.: C07D 295/108, C07D 451/04, A61K 31/445, A61P 15/00

(54) **DERIVES D'OXOPIPERIDINE, LEUR PREPARATION ET LEUR APPLICATION EN THERAPEUTIQUE**
OXOPIPERIDINDERIVATE, IHRE HERSTELLUNG UND THERAPEUTISCHE VERWENDUNG
OXOPIPERIDINE DERIVATIVES, PREPARATION AND THERAPEUTIC USE THEREOF

(30) Priorité: 29.07.2004 FR 0408369
(43) Date de publication de la demande: 18.04.2007
(73) Titulaire: Sanofi-Aventis, 75013 Paris (FR)
(72) Inventeur: BRAUN, Alain, F-92100 Boulogne Billancourt (FR); COURTEMANCHE, Gilles, F-31600 Saubens (FR); CRESPIN, Olivier, F-95000 Cergy (FR); FETT, Eykmar, F-75016 Paris (FR); PASCAL, Cécile, F-92500 Rueil Malmaison (FR)
(74) Mandataire: Morel-Pécheux, Muriel
(86) Numéro de dépôt international: PCT/FR2005/001854
(87) Numéro de publication internationale: WO 2006/021655

(56) Documents cités:
- WO-A-00/74679
- WO-A-01/70708
- WO-A-02/15909
- WO-A-02/079146
- WO-A-03/007949
- WO-A-03/092690

## Description

La présente invention se rapporte à des composés agonistes des récepteurs aux mélanocortines, à leur préparation et à leur application en thérapeutique.

Les récepteurs aux mélanocortines (MC-Rs) appartiennent à la super-famille des récepteurs couplés aux protéines G présentant sept domaines transmembranaires. Leur voie de transduction passe par la production d'AMPc (Cone, R. D., Recent Prog. Horm. Res., 1996, 51, 287). Cinq sous types de MC-Rs sont actuellement décrits, MC1-R, MC2-R, MC3-R, MC4-R et MC5-R, et sont exprimés dans différents tissus tels que le cerveau (MC3, 4, 5-R), les glandes exocrines (MC5-R), les surrénales (MC2-R) et la peau (MC1-R) pour les principaux. Les ligands naturels des MC-Rs sont, pour les agonistes, l'ACTH, l'α, β et γ-MSH, et pour les antagonistes, l'agouti protéine et l'agouti-related protéine. Aucun des ligands naturels n'est très sélectif pour l'un des sous-types, à l'exception du γ-MSH, qui possède une certaine sélectivité pour le MC3-R.

Le système mélanocortine est impliqué dans de nombreux processus physiologiques, incluant la pigmentation, l'inflammation, le comportement alimentaire et sexuel (notamment la fonction érectile), la balance énergétique (régulation du poids corporel et stockage lipidique), les fonctions exocrines, la protection et régénération neuronale, l'immunomodulation, l'analgésie, etc ...

Notamment, il a été démontré que le MC4-R est impliqué dans le comportement sexuel (Van der Ploeg, L. H., Proc. Natl. Acad. Sci. USA, 2002, 99, 11381*;* Martin, W. J., Eur. J. Pharmacol., 2002, 454, 71). Il a également été démontré, par l'intermédiaire de modèles de souris spécifiquement dépourvues en certains MC-Rs (souris knockout), que les MC-Rs centraux (MC3 et 4-R) étaient impliqués dans le comportement alimentaire, l'obésité, le métabolisme et la balance énergétique (Huszar, D., Cell, 1997, 88(1), 131*;* Chen, A. S., Nat. Genet., 2000, 26(1), 97 *;* Butler, A.A., Trends Genet., 2001, 17, S50-S54). Ainsi, les souris knockout MC4-R sont hyperphagiques et obèses. Parallèlement, les antagonistes aux MC3 et/ou 4R favorisent la prise de nourriture, tandis que la stimulation des MC4-R par un agoniste endogène, tel que l'α-MSH, produit un signal de satiété.

Ces observations laissent penser que la stimulation du MC3-R et/ou du MC4-R central, en réduisant la prise de nourriture et le poids corporel, est une approche prometteuse pour traiter l'obésité, risque aggravant de nombreuses autres pathologies (hypertension, diabète, ...). Ainsi, les recherches ont permis d'identifier dans un premier temps des peptides, pseudo-peptides ou peptides cycliques, capables d'interagir avec les MC-Rs et de moduler ainsi la prise de nourriture.

Afin de maintenir sur le long terme une perte de poids efficace et limiter ainsi les co-morbidités, un traitement quotidien à long terme doit être envisagé. Ceci implique qu'un médicament, pour cette indication thérapeutique, doit pouvoir être administré par une voie simple pour le patient. La voie orale doit donc être privilégiée. Or, les composés peptidiques ne sont généralement pas les plus appropriés pour répondre à cette obligation. C'est pourquoi il est important de développer des petites molécules non peptidiques.

Dans cette optique, les demandes internationales PCT publiées sous les numéros WO 02/059095, WO 02/059108, WO 03/009850 et WO 03/061660 décrivent des dérivés de type pipérazine. D'autres demandes décrivent des dérivés de type pipéridine, telles que les WO 03/092690 et WO 03/093234. Les demandes WO 99/64002 et WO 01/70337 décrivent des dérivés de type spiro-pipéridine. La demande WO 01/91752 décrit des dérivés comportant un motif pipéridine fusionné avec un noyau pyrazolyle. La demande WO 02/059107 décrit des dérivés de type pipéridine et pipérazine substitués par une structure bicyclique. Les demandes WO 02/059117, WO 02/068388 et WO 03/009847 décrivent des dérivés de type pipéridine et/ou pipérazine substitués par un noyau phényle. Quant à la demande WO 03/094918, elle décrit des dérivés de type pipérazine substitués par un noyau phényle ou pyridinyle. On peut également citer les demandes WO 00/74679, WO 01/70708, WO 02/15909, WO 02/079146, WO 03/007949 et WO 04/024720, qui décrivent des dérivés de type pipéridine substituée, ou encore la demande WO 04/037797 ; les composés décrits dans ces demandes de brevets comportent toujours une fonction amide, mimant les structures peptidiques antérieurement connues.

On peut citer aussi WO2005/047253 qui décrit composés agonistes des récepteurs aux mélanocortines de formule générale :

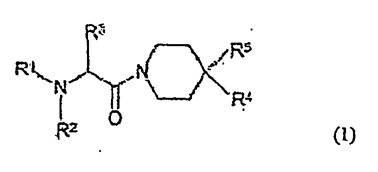

Face au besoin constant d'améliorer les thérapies existantes pour les pathologies évoquées précédemment, les Inventeurs se sont donnés pour but de pourvoir à de nouveaux composés agonistes des récepteurs aux mélanocortines.

La présente invention a pour objet des composés répondant à la formule (I) 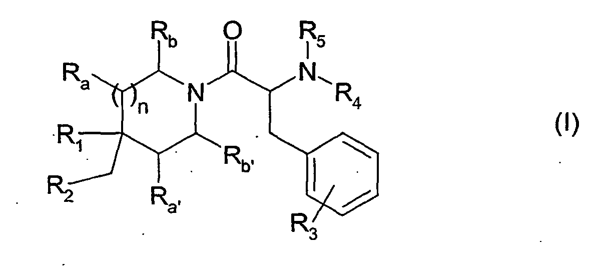 dans laquelle :
n est égal à 1,
Rₐ, R_{a'}, R_{b} et R_{b'}, sont identiques ou différents les uns des autres et représentent un atome d'hydrogène ou un groupe alkyle ou cycloalkyle, R_{b} et R_{b'}, pouvant former ensemble, avec les atomes de carbone du cycle auquel ils sont rattachés, un pont carboné comprenant de 4 à 5 chaînons,
**R**₁ représente un groupe alkyle ou cycloalkyle,
**R**₂ représente un groupe hétéroaryle,
**R**₃ représente 1 à 3 groupes, identiques ou différents les uns des autres, situés en des positions quelconques du noyau auquel ils sont rattachés et choisis parmi les atomes d'halogène et les groupes alkyles, cycloalkyles, -OR, -NRR', -CO-NRR', -NR-CO-R', -NR-CO-NRR', -NR-COOR', -NO₂, -CN et -COOR,
**R**₅ représente un atome d'hydrogène ou un groupe alkyle,
**R**₄ est choisi parmi les groupes de formules (a), (b) et (c), ci-dessous, éventuellement substitués par un groupe oxo, ou mono ou polysubstitué par un groupe aryle ou hétéroaryle: 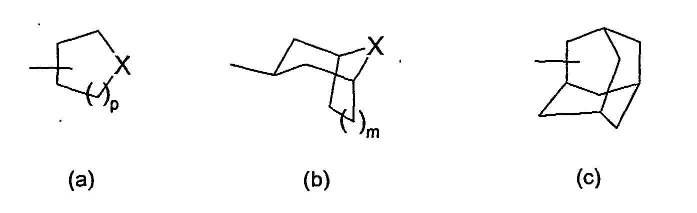
dans lesquelles:
p = 0, 1, 2 ou 3,
m = 0, 1 ou 2,
et soit
a) X représente un chaînon -N(R₁₀)-, où
   **R**₁₀ est choisi parmi :
      un groupe -(CH₂)ₓ-OR₈, -(CH₂)ₓ-COOR₈, -(CH₂)ₓ-NR₈R₉, -(CH₂)ₓ-CO-NR₈R₉ ou -(CH₂)ₓ-NR₈-COR₉, -(CH₂)ₓ-COR₈ dans lesquels x = 1, 2, 3 ou 4,
      un groupe cycloalkyle ou hétérocycloalkyle fusionné avec un groupe aryle ou hétéroaryle,
      un groupe cycloalkyle, hétérocycloalkyle, aryle, hétéroaryle, alkylaryle, alkylhétéroaryle, -CO-alkyle, -CO-cycloalkyle, -CO-hétérocycloalkyle, -CO-aryle, -CO-hétéroaryle, -CO-alkylaryle, -CO-alkylhétéroaryle, -CS-alkyle, -CS-cycloalkyle, -CS-hétérocycloalkyle, -CS-aryle, -CS-hétéroaryle, -CS-alkylaryle, -CS-alkylhétéroaryle, -CS-NR₈R₉, -C(=NH)-NP₈R₉, -SO₂-alkyle, -SO₂-cycloalkyle, -SO₂-hétérocycloalkyle, -SO₂-aryle, -SO₂-hétéroaryle, -SO₂-alkylaryle, -SO₂-alkylhétéroaryle ou -SO₂-NR₈R₉,
      les groupes alkyles, cycloalkyles, hétérocycloalkyles, aryles ou hétéroaryles étant éventuellement substitués par 1 ou plusieurs groupes choisis parmi les atomes d'halogène et les groupes R , R', OR, NRR', -CO-NRR', -NRCOR', NRCONRR', -NO₂, CN, -COOR, OCOR, COR, OCONRR', NRCOOR' ;
      les groupes cycloalkyles ou hétérocycloalkyles étant éventuellement fusionnés avec un groupe aryle ou hétéroaryle ;
      ou bien **R**₁₀ forme, avec l'atome d'azote auquel il est rattaché et un atome de carbone situé en une position quelconque de la structure cyclique de la formule (a), mais non adjacent audit atome d'azote, un pont comprenant de 3 à 5 chaînons,
   **R**₈ et **R**₉ sont choisis indépendamment l'un de l'autre parmi un atome d'hydrogène et les groupes alkyles, cycloalkyles, hétérocycloalkyles, aryles, hétéroaryles, alkylaryles, alkylhétéroaryles, -CO-alkyles, -CO-cycloalkyles, -CO-hétérocycloalkyles, -CO-aryles, -CO-hétéroaryles, -CO-alkylaryles, -CO-alkylhétéroaryles, -SO₂-alkyles, -SO₂-cycloalkyles, -SO₂-hétérocycloalkyles, -SO₂-aryles, -SO₂-hétéroaryles, -SO₂-alkylaryles, -SO₂-alkylhétéroaryles, -C(=NH)-NRR', -COOR, -CO-NRR', -CS-NRR' et -(CH₂)ₓ-OR, où x = 0, 1, 2, 3 ou 4, les groupes alkyles, cycloalkyles, hétérocycloalkyles, aryles et hétéroaryles étant éventuellement substitués par un ou plusieurs groupes choisis parmi les atomes d'halogène et les groupes R , R', OR, NRR', - CO-NRR', -NRCOR', NRCONRR', -NO₂, CN, -COOR, OCOR,COR,, OCONRR', NRCOOR' ;
   ou bien **R**₈ et **R**₉ forment ensemble un cycloalkyle ou un hétérocycloalkyle ;
   **R** et **R**' représentent indépendamment l'un de l'autre un atome d'hydrogène ou un groupe alkyle, cycloalkyle, hétérocycloalkyle, aryle, hétéroaryle, alkylaryle ou alkylhétéroaryle, ou peuvent former ensemble un cycloalkyle ou un hétérocycloalkyle.
   Soit,
b) X représente un chaînon -C(R₆)(R₇)- où
   **R**₆ est choisi parmi :
      un atome d'hydrogène, un atome d'halogène,
      un groupe -(CH₂)ₓ-OR₈, -(CH₂)ₓ-COOR₈, -(CH₂)ₓ-NR₈R₉, -(CH₂)ₓ-CO-NR₈R₉ ou -(CH₂)ₓ-NR₈-COR₉, dans lesquels x = 0, 1, 2, 3 ou 4,
      un groupe alkyle, cycloalkyle, hétérocycloalkyle, aryle, hétéroaryle, alkylaryle, alkylhétéroaryle, -CO-alkyle, -CO-cycloalkyle, -CO-hétérocycloalkyle, -CO-aryle, -CO-hétéroaryle, -CO-alkylaryle ou -CO-alkylhétéroaryle, -CS-alkyle, -CS-cycloalkyle, -CS-hétérocycloalkyle, -CS-aryle, -CS-hétéroaryle, -CS-alkylaryle, -CS-alkylhétéroaryle, -CS-NR₈R₉, -C(=NH)-NR₈R₉,
      un groupe cycloalkyle ou hétérocycloalkyle fusionné ou non situé en position spiro sur le cycle de.formule (a) auquel il est rattaché,
      un groupe cycloalkyle ou hétérocycloalkyle fusionné avec un groupe aryle ou hétéroaryle,
      les groupe alkyles, cycloalkyles, hétérocycloalkyles, aryles ou hétéroaryles étant éventuellement substitués par 1 ou plusieurs groupes choisis parmi les atomes d'halogène et les groupes R , R', OR, NRR', -CO-NRR', -NRCOR', NRCONRR', -NO₂, CN, -COOR, OCOR,COR,, OCONRR', NRCOOR' ;
      les groupes cycloalkyles ou hétérocycloalkyles étant éventuellement fusionnés avec un groupe aryle ou hétéroaryle,
   **R**₇ est choisi parmi les atomes d'hydrogène et d'halogène et les groupes alkyles, cycloalkyles, aryles, hétéroaryles, alkylaryles, alkylhétéroaryles, -OR, -O-aryles, -O-hétéroaryles, -O-alkylaryles, -O-alkylhétéroaryles, -NRR', -CO-NRR', -NR-CO-R', -NR-CO-NRR', -NR-COOR', -NO₂, -CN et -COOR,
   **R**₈ et **R**₉ sont choisis indépendamment l'un de l'autre parmi un atome d'hydrogène et les groupes alkyles, cycloalkyles, hétérocycloalkyles, aryles, hétéroaryles, alkylaryles, alkylhétéroaryles, -CO-alkyles, -CO-cycloalkyles, -CO-hétérocycloalkyles, -CO-aryles, -CO-hétéroaryles, -CO-alkylaryles, -CO-alkylhétéroaryles, -SO₂-alkyles, -SO₂-cycloalkyles, -SO₂-hétérocycloalkyles, -SO₂-aryles, -SO₂-hétéroaryles, -SO₂-alkylaryles, -SO₂-alkylhétéroaryles, -C(=NH)-NRR', -COOR, -CO-NRR', -CS-NRR' et -(CH₂)ₓ-OR, où x = 0, 1, 2, 3 ou 4, les groupes alkyles, cycloalkyles, hétérocycloalkyles, aryles et hétéroaryles étant éventuellement substitués par un ou plusieurs groupes choisis parmi les atomes d'halogène et les groupes R , R', OR, NRR', - CO-NRR', -NRCOR', NRCONRR', -NO₂, CN, -COOR, OCOR, COR, OCONRR', NRCOOR' ;
   ou bien **R**₈ et **R**₉ forment ensemble un cycloalkyle ou un hétérocycloalkyle ;
   **R** et **R**' représentent indépendamment l'un de l'autre un atome d'hydrogène ou un groupe alkyle, cycloalkyle, hétérocycloalkyle, aryle, hétéroaryle, alkylaryle ou alkylhétéroaryle, ou peuvent former ensemble un cycloalkyle ou un hétérocycloalkyle.
   à l'état de base ou de sel d'addition à un acide, ainsi qu'à l'état d'hydrate ou de solvat.

Parmi les composés de formule (I) objets de l'invention, on préfère ceux dans lesquels **R**₄ est choisi parmi les groupes de formules (a), (b) et (c) éventuellement mono ou polysubstitué (di-tri tétra substitué) par un groupe aryle ou hétéroaryle où X représente un chaînon -C(R₆)(R₇)-, dans lequel
**R**₆ est choisi parmi :
   un atome d'hydrogène,
   un groupe -(CH₂)ₓ-OR₈, -(CH₂)ₓ₋COOR₈, -(CH₂)ₓ₋NR₈R₉, -(CH₂)ₓ₋CO-NR₈R₉ ou -(CH₂)ₓ-NR₈-COR₉, dans lesquels x = 0, 1, 2, 3 ou 4,
   un groupe alkyle, cycloalkyl, hétérocycloalkyle, aryle, hétéroaryle, alkylaryle, alkylhétéroaryle, -CO-alkyle, -CO-cycloalkyle, -CO-hétérocycloalkyle, -CO-aryle, -CO-hétéroaryle, -CO-alkylaryle ou -CO-alkylhétéroaryle,
   un groupe cycloalkyle ou hétérocycloalkyle situé en position spiro sur le cycle de formule (a) auquel il est rattaché,
   un groupe cycloalkyle ou hétérocycloalkyle fusionné avec un groupe aryle ou hétéroaryle,
**R**₇ est choisi parmi les atomes d'hydrogène et d'halogène et les groupes alkyles, cycloalkyles, aryles, hétéroaryles, alkylaryles, alkylhétéroaryles, -OR, -O-aryles, -O-hétéroaryles, -O-alkylaryles, -O-alkylhétéroaryles, -NRR', -CO-NRR', -NR-CO-R', -NR-CO-NRR', -NR-COOR', -NO₂, -CN et -COOR,
**R**₈ et **R**₉ sont choisis indépendamment l'un de l'autre parmi un atome d'hydrogène et les groupes alkyles, cycloalkyles, hétérocycloalkyles, aryles, hétéroaryles, alkylaryles, alkylhétéroaryles, -CO-alkyles, -CO-cycloalkyles, -CO-hétérocycloalkyles, -CO-aryles, -CO-hétéroaryles, -CO-alkylaryles, -CO-alkylhétéroaryles, -SO₂-alkyles, -SO₂-cycloalkyles, -SO₂-hétérocycloalkyles, -SO₂-aryles, -SO₂-hétéroarytes, -SO₂-alkylaryles, -SO₂-alkylhétéroaryles, -C(=NH)-NRR', -COOR, -CO-NRR', -CS-NRR' et -(CH₂)ₓ-OR, où x = 0, 1, 2, 3 ou 4 ;
**R** et **R**' représentent indépendamment l'un de l'autre un atome d'hydrogène ou un groupe alkyle, cycloalkyle, hétérocycloalkyle, aryle, hétéroaryle, alkylaryle ou alkylhétéroaryle.

Parmi les composés de formule (I) objets de l'invention, on préfère encore ceux dans lesquels **R**₄ est choisi parmi les groupes de formules (a), (b) et (c) où X représente un chaînon -C(R₆)(R₇)-, dans lequel R₆ est choisi parmi un atome d'halogène ou un groupe cycloalkyle ou hétérocycloalkyle fusionné ou non situé en position spiro sur le cycle de formule (a) auquel il est rattaché.

On préfère encore ceux dans lesquels **R**₄ est choisi parmi les groupes de formules (a), (b) et (c) où X représente un chaînon -C(R₆)(R₇)-, dans lequel R6 est choisi parmi - CS-alkyle, -CS-cycloalkyle, -CS-hétérocycloalkyle, -CS-aryle, -CS-hétéroaryle, -CS-alkylaryle, -CS-alkylhétéroaryle, -CS-NR₈R₉, -C(=NH)-NR₈R₉.

On préfère aussi ceux dans lesquels **R**₄ est choisi parmi les groupes de formules (a), (b) et (c) où X représente un chaînon -C(R₆)(R₇)-, dans lequel les groupe alkyles, cycloalkyles, hétérocycloalkyles, aryles ou hétéroaryles sont éventuellement substitués par 1 ou plusieurs groupes choisis parmi R ou R', OCOR, COR, OCONRR', NRCOOR'.

On préfère encore ceux dans lesquels **R**₄ est choisi parmi les groupes de formules (a), (b) et (c) où X représente un chaînon -C(R₆)(R₇)-, dans lequel les groupes cycloalkyles ou hétérocycloalkyles sont éventuellement fusionnés avec un groupe aryle ou hétéroaryle.

On préfère encore ceux dans lesquels **R**₄ est choisi parmi les groupes de formules (a), (b) et (c) où X représente un chaînon -C(R₆)(R₇)-, dans lequel **R**₈ et **R**₉ choisis indépendamment l'un de l'autre représentent des groupes alkyles, cycloalkyles, hétérocycloalkyles, aryles et hétéroaryles étant éventuellement substitués par un ou plusieurs groupes choisis parmi les groupes R , R', COR, OCOR, OCONRR', NRCOOR' ;
ou bien **R**₈ et **R**₉ forment ensemble un cycloalkyle ou un hétérocycloalkyle.

On préfère encore ceux dans lesquels **R**₄ est choisi parmi les groupes de formules (a), (b) et (c) où X représente un chaînon -C(R₆)(R₇)-, dans lequel **R** et **R**' peuvent former ensemble un cycloalkyle ou un hétérocycloalkyle.

Parmi les composés de formule (I) objets de l'invention, on préfère encore ceux dans lesquels **R**₇ est l'hydrogène.

Parmi les composés de formule (I) objets de l'invention, on préfère encore ceux dans lesquels **R**₄ représente le groupe de formule a) où p=2 tel que défini ci-dessous :

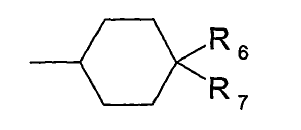

Parmi les composés de formule (I) objets de l'invention, on préfère encore ceux dans lesquels **R**₄ est choisi parmi les groupes de formules (a), (b) et (c)) éventuellement mono ou polysubstitué (di-tri tétra substitué) par un groupe aryle ou hétéroaryle où X représente un chaînon -N(R₁₀)- dans lequel
**R**₁₀ est choisi parmi :
   un groupe -CO-NR₈R₉, -COOR₈
   un groupe -(CH₂)ₓ-OR₈, -(CH₂)ₓ-COOR₈, -(CH₂)ₓ-NR₈R₉, -(CH₂)ₓ-CO-NR₈R₉ ou -(CH₂)ₓ-NR₈-COR₉, dans lesquels x = 1, 2, 3 ou 4,
   un groupe cycloalkyle ou hétérocycloalkyle fusionné avec un groupe aryle ou hétéroaryle,
   un groupe cycloalkyle, hétérocycloalkyle, aryle, hétéroaryle, alkylaryle, alkylhétéroaryle, , -CO-cycloalkyle, -CO-hétérocycloalkyle, , -CO-hétéroaryle, -CO-alkylaryle, -CO-alkylhétéroaryle, -CS-alkyle, -CS-cycloalkyle, -CS-hétérocycloalkyle, -CS-aryle, -CS-hétéroaryle, -CS-alkylaryle, -CS-alkylhétéroaryle, -CS-NR₈R₉, -C(=NH)-NR₈R₉, , -SO₂-cycloalkyle, -SO₂-hétérocycloalkyle, , -SO₂-hétéroaryle, -SO₂-alkylaryle, -SO₂-alkylhétéroaryle ou -SO₂-NR₈R₉;
ou bien **R**₁₀ forme, avec l'atome d'azote auquel il est rattaché et un atome de carbone situé en une position quelconque de la structure cyclique de la formule (a), mais non adjacent audit atome d'azote, un pont comprenant de 3 à 5 chaînons.
**R**₈ et **R**₉ sont choisis indépendamment l'un de l'autre parmi un atome d'hydrogène et les groupes alkyles, cycloalkyles, hétérocycloalkyles, aryles, hétéroaryles, alkylaryles, alkylhétéroaryles, -CO-alkyles, -CO-cycloalkyles, -CO-hétérocycloalkyles, -CO-aryles, -CO-hétéroaryles, -CO-alkylaryles, -CO-alkylhétéroaryles, -SO₂-alkyles, -SO₂-cycloalkyles, -SO₂-hétérocycloalkyles, -SO₂-aryles, -SO₂-hétéroaryles, -SO₂-alkylaryles, -SO₂-alkylhétéroaryles, -C(=NH)-NRR', -COOR, -CO-NRR', -CS-NRR' et -(CH₂)ₓ-OR, où x = 0, 1, 2, 3 ou 4 ;
**R** et **R**' représentent indépendamment l'un de l'autre un atome d'hydrogène ou un groupe alkyle, cycloalkyle, hétérocycloalkyle, aryle, hétéroaryle, alkylaryle ou alkylhétéroaryle.

On préfère aussi ceux dans lesquels **R**₄ est choisi parmi les groupes de formules (a), (b) et (c) éventuellement substitué par un groupe oxo où X représente un chaînon -N(R₁₀).

On préfère aussi ceux dans lesquels **R**₄ est choisi parmi les groupes de formules (a), (b) et (c) où X représente un chaînon -N(R₁₀)- dans lequel **R**₈ et **R**₉ choisis indépendamment l'un de l'autre représentent des groupes alkyles, cycloalkyles, hétérocycloalkyles, aryles et hétéroaryles étant éventuellement substitués par un ou plusieurs groupes choisis parmi les atomes d'halogène et les groupes R , R', OR, NRR', - CO-NRR', -OCOR, NRCOR', NRCONRR', COR, -NO₂, CN, -COOR, OCONRR', NRCOOR' ;
ou bien **R**₈ et **R**₉ forment ensemble un cycloalkyle ou un hétérocycloalkyle.

On préfère encore ceux dans lesquels **R**₄ est choisi parmi les groupes de formules (a), (b) et (c) où X représente un chaînon -N(R₁₀)- dans lequel **R**₁₀ est, -(CH₂)ₓCOR₈ dans lequel x = 1, 2, 3 ou 4.

On préfère aussi ceux dans lesquels **R**₄ est choisi parmi les groupes de formules (a), (b) et (c) où X représente un chaînon -N(R₁₀)- dans lequel les groupes alkyles, cycloalkyles, hétérocycloalkyles, aryles ou hétéroaryles sont éventuellement substitués par 1 ou plusieurs groupes choisis parmi R, R' OCOR, COR, OCONRR' ou NRCOOR'.

On préfère aussi ceux dans lesquels **R**₄ est choisi parmi les groupes de formules (a), (b) et (c) où X représente un chaînon -N(R₁₀)- dans lequel les groupes cycloalkyles ou hétérocycloalkyles sont éventuellement fusionnés avec un groupe aryle ou hétéroaryle.

On préfère aussi ceux dans lesquels **R**₄ représente le groupe de formule a) où p=2 tel que défini ci-dessous :

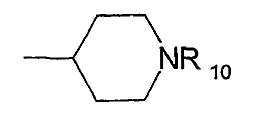

Les composés de formule (I) comportent au moins un atome de carbone asymétrique. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Parmi les composés de formule (I) objets de l'invention, on préfère ceux dans lesquels l'atome de carbone, identifié par l'astérisque * dans la formule suivante, présente une configuration (R) :

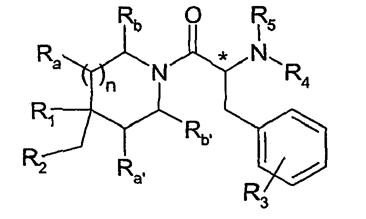

Les composés de formule (I) selon l'invention peuvent également exister sous forme de mélanges de conformères, qui font partie de l'invention. Ils peuvent en outre exister sous forme d'isomères cis ou trans, ou d'isomères endo ou exo. Ces isomères, ainsi que leurs mélanges, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, et sauf mention différente dans le texte, on entend par :
- un atome d'halogène : un fluor, un chlore, un brome ou un iode ;
- un groupe alkyle : un groupe aliphatique saturé ou insaturé (c'est-à-dire comprenant entre 1 et 3 insaturations de type éthylénique ou acétylénique), comprenant de 1 à 6 atomes de carbone, linéaire, cyclique ou ramifié. A titre d'exemples, on peut citer les groupes méthyle, éthyle, propyle, isopropyle, butyle, isobutyle, tertbutyle, pentyle, neo-pentyle, etc... et les groupes cycloalkyles définis ci-après, ainsi que les groupes alkyles cyclisés seulement en partie, tel que le groupe méthyl-cyclopropyle. Un tel groupe alkyle peut être substitué par 1 ou plusieurs groupes (par exemple par 1 à 6 groupes) choisis parmi les atomes d'halogène, (résultant par exemple en un groupe -CF₃) et les groupes R, R', -OR, -NRR', -CO-NRR', -NR-CO-R', -NR-CO-NRR', -NO₂, -CN et -COOR, OCOR, COR, OCONRR', NRCOOR' ; où R et R' représentent indépendemment l'un de l'autre un atome d'hydrogène ou un groupe alkyle, cycloalkyle, hétérocycloalkyle, aryle, hétéroaryle, alkylaryle ou alkylhétéroaryle, ou peuvent former ensemble un cycloalkyle ou un hétérocycloalkyle;
- un groupe cycloalkyle : un groupe alkyle cyclique comprenant entre 3 et 8 atomes de carbone, tous les atomes de carbone étant engagés dans la structure cyclique. A titre d'exemples, on peut citer les groupes cyclopropyle, cyclobutyle, cyclopentyle, cyclohexyle, etc ... Un tel groupe cycloalkyle peut être substitué comme décrit ci-dessus pour le groupe alkyle ;
- un groupe hétérocycloalkyle : un groupe cycloalkyle tel que défini ci-dessus, comprenant en outre entre 1 et 4 hétéroatomes, tels que l'azote, l'oxygène et/ou le soufre. Un tel groupe hétérocycloalkyle peut-être substitué comme décrit ci-dessus pour le groupe cycloalkyle et peut comprendre une ou plusieurs, par exemple 1 ou 2, insaturations éthyléniques ou acétyléniques, pour former par exemple un groupe 2,5-dihydro-1H-pyrrolyle ;
- un groupe alcoxy : un radical -O-alkyle, où le groupe alkyle est tel que précédemment défini ;
- un groupe aryle : un groupe aromatique cyclique comprenant entre 5 et 10 chaînons, par exemple un groupe phényle. Un tel groupe aryle peut être substitué par 1 ou plusieurs groupes (par exemple par 1 à 6 groupes) choisis parmi les atomes d'halogène (résultant par exemple en un groupe -CF₃) et les groupes R, R', -OR, -NRR', - CO-NRR', -NR-CO-R', -NR-CO-NRR', -NO₂, -CN et -COOR, OCOR, COR, OCONRR', NRCOOR' ; où R et R' représentent indépendemment l'un de l'autre un atome d'hydrogène ou un groupe alkyle, cycloalkyle, hétérocycloalkyle, aryle, hétéroaryle, alkylaryle ou alkylhétéroaryle, ou peuvent former ensemble un cycloalkyle ou un hétérocycloalkyle;
- un groupe alkylaryle : un groupe alkyle tel que défini ci-dessus, lui-même substitué par un groupe aryle tel que défini ci-dessus. Un tel groupe alkylaryle est par exemple un groupe benzyle ;
- un groupe hétéroaryle : un groupe aromatique cyclique comprenant entre 5 et 10 chaînons et comprenant entre 1 et 6 hétéroatomes, tels que l'azote, l'oxygène et/ou le soufre. A titre d'exemple on peut citer le groupe pyridinyle. Un tel groupe hétéroaryle peut-être substitué comme décrit ci-dessus pour le groupe aryle ;
- un groupe alkylhétéroaryle : un groupe alkyle tel que défini ci-dessus, lui-même substitué par un groupe hétéroaryle tel que défini ci-dessus.

Parmi les composés de formule (I) objets de l'invention, on peut citer ceux dans lesquels n, Rₐ, R_{a'}, R_{b}, R_{b'}, R₂, R₃, R₄ et R₅ sont tels que définis ci-dessus et R₁ représente un groupe cycloalkyle, tel qu'un groupe cyclohexyle.

Parmi les composés de formule (I) objets de l'invention, on peut également citer ceux dans lesquels n, Rₐ, R_{a'}, R_{b}, R_{b'}, R₁, R₃, R₄ et R₅ sont tels que définis ci-dessus et R₂ représente un groupe triazolyle (avantageusement, le groupe 1,2,4-triazolyle).

Parmi les composés de formule (I) objets de l'invention, on peut également citer ceux dans lesquels n, Rₐ, R_{a'}, R_{b}, R_{b'}, R₁, R₂, R₄ et R₅ sont tels que définis ci-dessus et R₃ représente 1 à 3 groupes, identiques ou différents les uns des autres, choisis parmi les atomes d'halogène. Avantageusement, R₃ représente un seul groupe, de préférence un atome de chlore.

Parmi les composés de formule (I) objets de l'invention, on peut également citer ceux dans lesquels n, Rₐ, R_{a'}, R_{b}, R_{b'}, R₁, R₂, R₃ et R₄ sont tels que définis ci-dessus et R₅ représente un atome d'hydrogène ou un groupe alkyle comprenant de 1 à 4 atomes de carbone. R₅ représente de préférence un atome d'hydrogène.

On peut encore citer d'autres sous-groupes des composés de formule (I) objets de l'invention, dans lesquels R₁, R₂, R₃, R₄ et R₅ présentent l'une quelconque des significations décrites auparavant et dans lesquels :
- n=1 et Rₐ = R_{a'} = R_{b} = R_{b'} = H,
- n = 1, Rₐ = R_{a'} = H et R_{b} et R_{b'} forment ensemble, avec les atomes de carbone du cycle auquel ils sont rattachés, un pont carboné comprenant 4 chaînons,

Chacune des définitions données ci-avant pour les groupes Rₐ, R_{a'}, R_{b}, R_{b'}, R₁, R₂, R₃, R₄, R₅, R₆, R₇, R₈, R₉, R₁₀, R et R' peuvent se combiner les unes avec les autres de façon à obtenir différents sous-groupes de composés de formule (I) selon la présente invention.

Selon un autre objet l'invention se rapporte aux composés dont les noms suivent :

Dans les listes qui suivent les chiffres devant les nomenclatures des produits correpondent aux n° d'exemple des composés du tableau
5: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(2-phenylethyl)piperidin-4-amine
9: 4-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]phenol
12: *cis*-*N*-{(1*R*)-1-(4-chlorobenzyl)-2-[4-cyclohexyl-4-(1*H*-1,2,4-triazot-1-ylmethyl)piperidin-1-yl]-2-oxoethyl}cyclohexane-1,4-diamine
13: *trans-N-*{(1*R*)-1-(4-chlorobenzyl)-2-[4-cyclohexyl-4-(1*H*-1,2,4-triazol-1-ylmethyl)piperidin-1-yl]-2-oxoethyl}cyclohexane-1,4-diamine
15: *N*-{(1*R*)-1-(4-chlorobenzyl)-2-[4-cyclohexyl-4-(1*H*-1,2,4-triazol-1-ylmethyl)piperidin-1-yl]-2-oxoethyl}-8-methyl-8-azabicyclo[3.2.1]octan-3-amine
16: *N*-{(1*R*)-1-(4-chlorobenzyl)-2-[4-cyclohexyl-4-(1*H*-1,2,4-triazol-1-ylmethyl)piperidin-1-yl]-2-oxoethyl}-9-methyl-9-azabicyclo[3.3.1]nonan-3-amine
20: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-phenylpiperidin-4-amine
28: 1-benzoyl-*N*{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
29: 1-acetyl-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
32: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1,4-dioxaspiro[4.5]decan-8-amine
33: *N'*-{(1*R*)-1-(4-chlorobenzyl)-2-[4-cyclohexyl-4-(1*H*-1,2,4-triazol-1-ylmethyl)piperidin-1-yl]-2-oxoethyl}-*N*,*N*-dimethylcyclohexane-1,4-diamine
34: 4-(aminomethyl)-*N*-{(1*R*)-1-(4-chlorobenzyl)-2-[4-cyclohexyl-4-(1*H*-1,2,4-triazol-1-ylmethyl)piperidin-1-yl]-2-oxoethyl}cyclohexanamine
35: 3-({(1*R*)-1-(4-chlorobenzyl)-2-[4-cyclohexyl-4-(1*H*-1,2,4-triazol-1-ylmethyl)piperidin-1-yl]-2-oxoethyl}amino)-8-methyl-8-azabicyclo[3.2.1]octan-6-ol
36: *cis-* 4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanol
   *trans-*4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanol
37: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(trifluoroacetyl)piperidin-4-amine
39: 4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidine-1-carboxamide
40: *cis-* 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-1-phenylcyclohexanol
   *trans-*4-({(1*R*)-1-(4-chlorobenzyl)-2-[4-cyclohexyl-4-(1*H*-1,2,4-triazol-1-ylmethyl)piperidin-1-yl]-2-oxoethyl}amino)-1-phenylcyclohexanol
44: *cis* -*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-fluorophenyl)cyclohexane-1,4-diamine
   *trans-N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-fluorophenyl)cyclohexane-1,4-diamine
45: *N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2,2,2-trifluoroacetamide
   *N*-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2,2,2-trifluoroacetamide
46: *N*-[*cis-*4-({(1*R*)-1*-*(4-chlorobenzyl)-2-[4-cyclohexyl-4-(1*H*-1,2,4-triazol-1-ylmethyl)piperidin-1-yl]-2-oxoethyl}amino)cyclohexyl]acetamide
   *N*-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]acetamide
47: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(4-fluorobenzoyl)piperidin-4-amine
48: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(cyclopentylcarbonyl)piperidin-4-amine
49: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(cyclobutylcarbonyl)piperidin-4-amine
50: *cis-N*-{(1*R*)-1-(4-chlorobenzyl)-2-[4-cyclohexyl-4-(1*H*-1,2,4-triazol-1-ylmethyl)piperidin-1-yl]-2-oxoethyl}-*N'*-methylcyclohexane-1,4-diamine
52: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(pyridin-2-ylcarbonyl)piperidin-4-amine
53: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(phenylacetyl)piperidin-4-amine
54: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(methylsulfonyl)piperidin-4-amine
55: *N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-*N*-methylacetamide
56: *N*{(1*R*)-1-(4-chlorobenzyl)-2-[4-cyclohexyl-4-(1*H*-1,2,4-triazol-1-ylmethyl)piperidin-1-yl]-2-oxoethyl}-4-(1,3-dihydro-2*H*-isoindol-2-yl)cyclohexanamine
57: *N*-[*cis*-4-({(1*R*)-1-(4-chlorobenzyl)-2-[4-cyclohexyl-4-(1*H*-1,2,4-triazol-1-ylmethyl)piperidin-1-yl]-2-exoethyl}amino)cyclohexyl]-*N*-methylbenzamide
58: ethyl *cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarboxylate
   ethyl *trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarboxylate
62: *trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-phenylcyclohexane-1,4-diamine
63: *cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-phenylcyclohexane-1,4-diamine
69: *N*'-{(1*R*)-1-(4-chlorobenzyl)-2-[4-cyclohexyl-4-(1*H*-1,2,4-triazol-1-ylmethyl)piperidin-1-yl]-2-oxoethyl}-*N*-methyl-*N*-phenylcyclohexane-1,4-diamine
70: *N*'-{(1*R*)-1-(4-chlorobenzyl)-2-[4-cyclohexyl-4-(1*H*-1,2,4-triazol-1-ylmethyl)piperidin-1-yl]-2-oxoethyl}-*N*-(4-fluorophenyl)-*N*-methylcyclohexane-1,4-diamine
73 : *cis ou trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*,*N*-diethylcyclohexanecarboxamide
74: *cis ou trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*,*N*-diethylcyclohexanecarboxamide
75: *cis ou trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*,*N*-dimethylcyclohexanecarboxamide
76: *cis ou trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*,*N*-dimethylcyclohexanecarboxamide
78: *cis*-*N*-benzyl-4-({(1R)-1-(4-chlorobenzyl)-2-[4-cyclohexyl-4-(1*H*-1,2,4-triazol-1-ylmethyl)piperidin-1-yl]-2-oxoethyl}amino)-*N*-methylcyclohexanecarboxamide
   *trans*-*N*-benzyl-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-methylcyclohexanecarboxamide
79: *cis-N*-{(1*R*)-1*-*(4-chlorobenzyl)-2-[4-cyclohexyl-4-(1*H*-1,2,4-triazol-1-ylmethyl)piperidin-1-yl]-2-oxoethyl}-4-(3-methyl-1,2,4-oxadiazol-5-yl)cyclohexanamine
   *trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(3-methyl-1,2,4-oxadiazol-5-yl)cyclohexanamine
80 : *cis*-4-({(1*R*)1-(4-chlorobenzyl)-2-[4-cyclohexyl-4-(1*H*-1,2,4-triazol-1-ylmethyl)piperidin-1-yl]-2-oxoethyl}amino)cyclohexanecarboxamide
   *trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarboxamide
81: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-isonicotinoylpiperidin-4-amine
82: *cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-1-(4-fluorophenyl)cyclohexanol
   *trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-1-(4-fluorophenyl)cyclohexanol
83: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(1-methyl-1*H*-imidazol-2-yl)carbonyl]piperidin-4-amine
84: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(5-methylisoxazol-3-yl)carbonyl]piperidin-4-amine
85: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(3,4-difluorobenzoyl)piperidin-4-amine
86: 1-[(1-*tert*-butyl-5-methyl-1*H*-pyrazol-3-yl)carbonyl]-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
87: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(3,5-dimethylisoxazol-4-yl)carbonyl]piperidin-4-amine
88: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(3-thienylcarbonyl)piperidin-4-amine
89: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(pyrrolidin-1-ylcarbonyl)piperidin-4-amine
90: 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-phenylpiperidine-1-carboxamide
91: 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*,*N*-dimethylpiperidine-1-carboxamide
92: 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*,*N*-diethylpiperidine-1-carboxamide
93: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(piperidin-1-ylcarbonyl)piperidin-4-amine
94: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(morpholin-4-ylcarbonyl)piperidin-4-amine
95: 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-methyl-*N*-phenylpiperidine-1-carboxamide
96: *N*-benzyl-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-methylpiperidine-1-carboxamide
97: *N*-{(1*R*)-1-(4-chlorobenzyl)-2-[4-cyclohexyl-4-(1*H*-1,2,4-triazol-1-ylmethyl)piperidin-1-yl]-2-oxoethyl}-4-methoxycyclohexanamine
98: 4-[4-(benzyloxy)phenyl]-*N*-{(1*R*)-1-(4-chlorobenzyl)-2-[4-cyclohexyl-4-(1*H-*1,2,4-triazol-1-ylmethyl)piperidin-1-yl]-2-oxoethyl}cyclohexanamine
100: *N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2-methoxyacetamide
101: 2-{[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-2-oxoethyl acetate
102: 2-(benzyloxy)-*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]acetamide
103: 3-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-1,3-oxazolidin-2-one
   3-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-1,3-oxazolidin-2-one
104: *cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(2-methoxyethyl)cyclohexane-1,4-diamine
   trans-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N'*-(2-methoxyethyl)cyclohexane-1,4-diamine
105: *cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-morpholin-4-ylcyclohexanamine
   *trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-morpholin-4-ylcyclohexanamine
106: 1-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]pyrrolidin-2-one
   1-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]pyrrolidin-2-one
107: *N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(2-methoxyethoxy)cyclohexanamine
108: ethyl 4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidine-1-carboxylate
109: methyl 4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidine-1-carboxylate
110: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(2*S*)-piperidin-2-ylcarbonyl]piperidin-4-amine
111: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(2*R*)-piperidin-2-ylcarbonyl]piperidin-4-amine
112: *cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarbonitrile
   *trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarbonitrile
113: 1-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]piperidin-2-one
   1-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]piperidin-2-one
114: *N*-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]propanamide
115: *tert*-butyl (2-{[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-2-oxoéthyl)carbamate
116: *N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]glycinamide
117: *N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2-hydroxyacetamide
118: *N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2,2-dimethylpropanamide
   *N*-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2,2-dimethylpropanamide
119: *cis-N-*{(1*R*)*-1-*(4-chlorobenzyl)-2-[4-cyclohexyl-4-(1*H*-1,2,4-triazol-1-ylmethyl)piperidin-1-yl]-2-oxoethyl}-*N*-(4-methoxyphenyl)cyclohexane-1,4-diamine
   *trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N'*-(4-methoxyphenyl)cyclohexane-1,4-diamine
120: *cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(2*H*-tetrazol-5-yl)cyclohexanamine
   *trans*-*N*-(1*R*)-1-(4-chlorobenzyl)-2-[4-cyclohexyl-4-(1*H*-1,2,4-triazol-1-ylmethyl)piperidin-1-yl]-2-oxoethyl}-4-(2*H*-tetrazol-5-yl)cyclohexanamine
121: *2-*{[*cis-*4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}phenol
   2-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}phenol
122: 4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl acetate
123: *N*²-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-*N*,*N*-dimethylglycinamide
   *N*²-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-*N*,*N*-dimethylglycinamide
124: *N*²-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]glycinamide
   *N*²-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]glycinamide
125: 4-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]piperazin-2-one
   4-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]piperazin-2-one
126: *cis*-4-(4-acetylpiperazin-1-yl)-*N*-{(1*R*)-1-(4-chlorobenzyl)-2-[4-cycloh exyl-4-(1*H*-1,2,4-triazol-1-ylmethyl)piperidin-1-yl]-2-oxoethyl}cyclohexanamine
   *trans*-4-(4-acetylpiperazin-1-yl)-*N*-{(1*R*)-1-(4-chlorobenzyl)-2-[4-cyclohexyl-4-(1*H*-1,2,4-triazol-1-ylmethyl)piperidin-1-yl]-2-oxoethyl}cyclohexanamine
127: *N-*{(1*R)-*1-(4-chlorobenzyl)-2-[4-cyclohexyl-4-(1*H*-1,2,4-triazol-1-ylmethyl)piperidin-1-yl]-2-oxoethyl}-1-pyridin-2-ylpiperidin-4-amine
128: methyl *N*-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]glycinate
129: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(2,2-difluoroethyl)piperidin-4-amine
130: *cis-N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*-(4-chlorophenyl)cyclohexane-1,4-diamine
   *trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-chlorophenyl)cyclohexane-1,4-diamine
131: 4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl pivalate
132: *cis-N-*{(1*R)*-1-(4-chlorobenzyl)-2-[4-cyclohexyl-4-(1*H*-1,2,4-triazol-1-ylmethyl)piperidin-1-yl]-2-oxoethyl}-*N*-(4-fluoro-2-methylphenyl)cyclohexane-1,4-diamine
   *trans-N-*{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-fluoro-2-methylphenyl)cyclohexane-1,4-diamine
133: 4-{[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}benzonitrile
134: *N*-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]cyclopropanecarboxamide
135: *N-*{(1*R*)-1-(4-chlorobenzyl)-2-[4-cyclohexyl-4-(1*H*-1,2,4-triazol-1-ylmethyl)piperidin-1-yl]-2-oxoethyl}-1-(6-methylpyridazin-3-yl)piperidin-4-amine
136: methyl [4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidin-1-yl]acetate
137: *cis* ou *trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-morpholin-4-ylphenyl)cyclohexane-1,4-diamine
138: *cis*-*N*-(1*R*)-1-(4-chlorobenzyl)-2-[4-cyclohexyl-4-(1*H*-1,2,4-triazol-1-ylmethyl)piperidin-1-yl]-2-oxoethyl}-*N*'-(2,4-difluorophenyl)cyclohexane-1,4-diamine
   *trans-N-*{(1*R*)-1-(4-chlorobenzyl)-2-[4-cyclohexyl-4-(1*H-*1,2,4-triazol-1-ylmethyl)piperidin-1-yl]-2-oxoethyl}-*N*'-(2,4-difluorophenyl)cyclohexane-1,4-diamine
139: *cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*-(5-fluoropyridin-2-yl)cyclohexane-1,4-diamine
   *trans-N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*-(5-fluoropyridin-2-yl)cyclohexane-1,4-diamine
140: *N*-1*H*-1,2,3-benzotriazol-5-yl-*N*-{(1*R*)-1-(4-chlorobenzyl)-2-[4-cyclohexyl-4-(1*H*-1,2,4-triazol-1-ylmethyl)piperidin-1-yl]-2-oxoethyl}cyclohexane-1,4-diamirie
141: *N-*{(1*R*)-1-(4-chlorobenzyl)-2-[4-cyclohexyl-4-(1*H*-1,2,4-triazol-1-ylmethyl)piperidin-1-yl]-2-oxoethyl}-1-pyrimidin-2-ylpiperidin-4-amine
142: 1-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]imidazolidin-2-one
   1-[*trans*-4-({(1*R*)-1-(4-chlorobenzyl)-2-[4-cyclohexyl-4-(1*H*-1,2,4-triazo)-1-ylmethyl)piperidin-1-yl}-2-oxoethyl}amino)cyclohexyl]imidazolidin-2-one
143: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-cyclopropylpiperidin-4-amine
144: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-{[(2*S*)-4,4-difluoropiperidin-2-yl]carbonyl}piperidin-4-amine
145: *N-*{(1*R*)-1-(4-chlorobenzyl)-2-[4-cyclohexyl-4-(1*H*-1,2,4-triazol-1-ylmethyl)piperidin-1-yl]-2-oxoethyl}-4,4-difluorocyclohexanamine
146: *cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(3,4-difluorophenyl)cyclohexane-1,4-diamine
   *trans-N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(3,4-difluorophenyl)cyclohexane-1,4-diamine
147: *cis-N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-fluoro-3-methoxyphenyl)cyclohexane-1,4-diamine
   *trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-fluoro-3-methoxyphenyl)cyclohexane-1,4-diamine
148: *cis-N'-*{(1*R*)-1-(4-chlorobenzyl)-2-[4-cyclohexyl-4-(1*H*-1,2,4-triazol-1 - ylmethyl)piperidin-1-yl]-2-oxoethyl}-*N*-ethyl-*N*-(4-fluoro-3-methoxyphenyl)cyclohexane-1,4-diamine
   *trans-N'-*{(*1R*)-1-(4-chlorobenzyl)-2-[4-cyclohexyl-4-(1*H*-1,2,4-triazol-1-ylmethyl)piperidin-1-yl]-2-oxoethyl}-*N*-ethyl-*N*-(4-fluoro-3-methoxyphenyl)cyclohexane-1,4-diamine
149: *cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*-[4-(trifluoromethyl)phenyl]cyclohexane-1,4-diamine
   *trans-N-*{(1*R*)-1-(4-chlorobenzyl)-2-[4-cyclohexyl-4-(1*H*-1,2,4-triazol-1-ylmethyl)piperidin-1-yl]-2-oxoethyl}-*N*'-[4-(trifluoromethyl)phenyl]cyclohexane-1,4-diamine
150: *cis-N-*{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-fluoro-3-methylphenyl)cyclohexane-1,4-diamine
   *trans-N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-fluoro-3-methylphenyl)cyclohexane-1,4-diamine
151: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(4,4-difluoro-L-prolyl)piperidin-4-amine
152: 1-(1*H*-benzimidazol-2-yl)-*N*-{(1*R*)-1-(4-chlorobenzyl)-2-[4-cyclohexyl-4-(1*H* 1,2,4-triazol-1-ylmethyl)piperidin-1-yl]-2-oxoethyl}piperidin-4-amine
153: 1-(2,1-benzisoxazol-3-ylcarbonyl)-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
154: 1-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidin-1-yl]butan-2-one
155: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(2,2,2-trifluoroethyl)piperidin-4-amine
156: 4-({(1*R*)-1-(4-chlorobenzyl)-2-[4-cyclohexyl-4-(1*H*-1,2,4-triazo)-1 - ylmethyl)piperidin-1-yl]-2-oxoethyl}amino)-*N*-(4-methoxyphenyl)piperidine-1-carboxamide
157: 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(4-fluorophenyl)piperidine-1-carboxamide
158: 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(2,4-difluorophenyl)piperidine-1-carboxamide
159: 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(3,4-difluorophenyl)piperidine-1-carboxamide
160: 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(2-fluorophenyl)piperidine-1-carboxamide
161: 4-({(1*R*)-1-(4-chlorobenzyl)-2-[4-cyclohexyl-4-(1*H*-1,2,4-triazol-1-yimethyl)piperidin-1-yl]-2-oxoethyl}amino)-*N*-(2-methoxyphenyl)piperidine-1-carboxamide
162: 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-[4-(dimethylamino)phenyl]piperidine-1-carboxamide
163: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(5-fluoro-1*H*-indol-2-yl)carbonyl]piperidin-4-amine
164: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(pyrazin-2-ylcarbonyl)piperidin-4-amine
165: *N*-{(1*R*)-1-(4-chlorobenzyl)-2-[4-cyclohexyl-4-(1*H*-1,2,4-triazol-1-yimethyl)piperidin-1-yl]-2-oxoethyl}-1-[(5-phenyl-1,3-oxazol-4-yl)carbonyl]piperidin-4-amine
166: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(isoxazol-5-ylcarbonyl)piperidin-4-amine
167: *3-*[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-6-fluoro-1,3-benzoxazol-2(3*H*)-one
   3-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-6-fluoro-1, 3-benzoxazol-2(3*H*)-one
168: *N*-[*cis*-4-({(1*R*)-1-(4-chlorobenzyl)-2-[4-cyclohexyl-4-(1*H*-1, 2,4-triazol-1-ylmethyl)piperidin-1-yl]-2-oxoethyl}amino)cyclohexyl]pyridine-2-carboxamide
169: 2-{[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-5-fluorophenol ou
   2-{[*trans*-4-({(1*R*)-1-(4-chlorobenzyl)-2-[4-cyclohexyl-4-(1*H*-1,2,4-triazol-1-yimethyl)piperidin-1-yl]-2-oxoethyl}amino)cyclohexyl]amino}-5-fluorophenol
170: *N-*{(1*R*)-1-(4-chlorobenzyl)-2-[4-cyclohexyl-4-(1*H*-1,2,4-triazol-1-ylmethyl)piperidin-1-yl]-2-oxoethyl}-1-(quinolin-2-ylcarbonyl)piperidin-4-amine
171: *N-*{(1*R*)-1-(4-chlorobenzyl)-2-[4-cyclohexyl-4-(1*H*-1,2,4-triazol-1-ylmethyl)piperidin-1-yl]-2-oxoethyl}-1-[(3-methylpyridin-2-yl)carbonyl]piperidin-4-amine
172: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(1*H*-1,2,4-triazol-3-ylcarbonyl)piperidin-4-amine
173: *N-*[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2,1-benzisoxazole-3-carboxamide
174: 1-(1,3-benzothiazol-2-ylcarbonyl)-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
175: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(6-methylpyridin-2-yl)carbonyl]piperidin-4-amine
176: 1-(1-benzofuran-2-ylcarbonyl)-*N*-{(1*R*)-1-(4-chlorobenzyl)-2-[4-cyclohexyl-4-(1H-1,2,4-triazol-1-ylmethyl)piperidin-1-yl]-2-oxoethyl}piperidin-4-amine
177: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(6-fluoropyridin-2-yl)carbonyl]piperidin-4-amine
178: *N*-{(1*R*)-1-(4-chlorobenzyl)-2-[4-cyclohexyl-4-(1*H*-1,2,4-triazol-1-ylmethyl)piperidin-1-yl]-2-oxoethyl-[(1-phenyl-1*H*-pyrazol-5-yl)carbonyl]piperidin-4-amine
179: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(2,4-difluorobenzoyl)piperidin-4-amine
180: *cis*-*N*-(1,3-benzothiazol-2-ylmethyl)-*N*'-{(1*R*)-1-(4-chlorobenzyl)-2-[4-cyclohexyl-4-(1*H*-1,2,4-triazol-1-ylmethyl)piperidin-1-yl]-2-oxoethyl}cyclohexane-1,4-diamine
181: *cis-N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(1,3-thiazol-2-ylmethyl)cyclohexane-1,4-diamine
182: *2-*{[*cis-4-*({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-5-fluoro-*N*,*N-*dimethylbenzamide
   2-{[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-5-fluoro-*N*,*N-*dimethylbenzamide
183: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(6-phenylpyridin-2-yl)carbonyl]piperidin-4-amine
184: *trans*-*N*-(*tert*-butyl)-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarboxamide
185: *cis*-4-({(1*R*)-1-(4-chlorobenzyl)-2-[4-cyclohexy)-4-(1*H*-1,2,4-triazol-1-ylmethyl)piperidin-1-yl]-2-oxoethyl}amino)-*N*-(3,4-difluorophenyl)cyclohexanecarboxamide
186: *cis*-*N*-{(1S)-1-(4-chlorobenzyl)-2-[4-cyclohexyl-4-(1*H*-1,2,4-triazo)-1-ylmethyl)piperidin-1-yl]-2-oxoethyl}cyclohexane-1,4-diamine
187: *trans-N*-{(1S)-1-(4-chlorobenzyl)-2-[4-cyclohexyl-4-(1*H*-1,2,4-triazol-1-ylmethyl)piperidin-1-yl]-2-oxoethyl}cyclohexane-1,4-diamine

Parmi les composés préférés de formule I dans laquelle X est CR₆R₇, on peut citer ceux dont les noms suivent
9: 4-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]phenol
12: *cis-N-*{(1*R*)-1-(4-chlorobenzyl)-2-[4-cyclohexyl-4*-*(1*H*-1,2,4-triazol-1-ylmethyl)pipéridin-1-yl]-2-oxoethyl}cyclohexane-1,4-diamine
13: *trans-N-*{(1*R*)-1-(4-chlorobenzyl)-2-[4-cyclohexyl-4-(1*H*-1,2,4-triazol-1-ylmethyl)piperidin-1-yl]-2-oxoethyl}cyclohexane-1,4-diamine
22: *cis-N-*{(1*R*)-1-(4-chlorobenzyl)-2-[3-cyclohexyl-3-(1*H*-1,2,4-triazol-1-ylmethyl)-8-azabicyclo[3.2.1]oct-8-yl]-2-oxoethyl}cyclohexane-1,4-diamine
23: *trans*-*N*-{(1*R*)-1-(4-chlorobenzyl)-2-[3-cyclohexyl-3-(1*H*-1,2,4-triazol-1-ylmethyl)-8-azabicyclo[3.2.1]oct-8-yl]-2-oxoethyl}cyclohexane-1,4-diamine
32: *N-*{(1*R*)-1-(4-chlorobenzyl)-2-[4-cyclohexyl-4-(1*H*-1,2,4-triazol-1-ylmethyl)piperidin-1-yl]-2-oxoethyl}-1,4-dioxaspiro[4.5]decan-8-amine
33: *N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*,*N*-dimethylcyclohexane-1,4-diamine
34: 4-(aminomethyl)-*N*-{(1*R*)-1-(4-chlorobenzyl)-2-[4-cyclohexyl-4-(1*H*-1,2,4-triazol-1-ylmethyl)piperidin-1-yl]-2-oxoethyl}cyclohexanamine
36: *cis-* 4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanol
   *trans-* 4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanol
38: *N*-{(1*R*)-1-(4-chlorobenzyl)-2-[4-cyclohexyl-4-(1*H*-1,2,4-triazol-1-ylmethyl)piperidin-1-yl]-2-oxoethyl}-4-phenylcyclohexanamine
40: *cis-* 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-1-phenylcyclohexanol
   *trans-* 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-1-phenylcyclohexanol
41: *cis*- 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-1-phenylcyclohexanecarbonitrile
   *trans-* 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-1-phenylcyclohexanecarbonitrile
44: *cis* -*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*-(4-fluorophenyl)cyclohexane-1,4-diamine
   *trans-N-*{(*1R*)-1-(4-chlorobenzyl)-2-[4-cyclohexyl-4-(1*H*-1,2,4-triazol-1-ylmethyl)piperidin-1-yl]-2-oxoethyl}-*N*'-(4-fluorophenyl)cyclohexane-1,4-diamine
45: *N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2,2,2-trifluoroacetamide
   *N*-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2,2,2-trifluoroacetamide
46: *N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]acetamide
   *N-*[*trans-*4-({(1*R*)1-(4-chlorobenzyl)-2-[4-cyclohexyl-4-(1*H*-1,2,4 triazol-1-ylmethyl)piperidin-1-yl]-2-oxoethyl}amino)cyclohexyl]acetamide
50: *cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*-methylcyclohexane-1,4-diamine
55: *N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-*N*-methylacetamide
56: *N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(1,3-dihydro-2*H*-isoindol-2-yl)cyclohexanamine
57: *N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-*N*-methylbenzamide
58: ethyl *cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarboxylate
   ethyl *trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarboxylate
59: *cis-N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(trifluoromethyl)cyclohexanamine
   *trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(trifluoromethyl)cyclohexanamine
62: *trans-N-*{(1*R*)-1-(4-chlorobenzyl)-2-[4-cyclohexyl-4-(1*H*-1,2,4-triazol-1-ylmethyl)piperidin-1-yl]-2-oxoethyl}-*N*-phenylcyclohexane-1,4-diamine
63: *cis-N-*{(1*R*)-1-(4-chlorobenzyl)-2-[4-cyclohexyl-4-(1*H*-1,2,4-triazol-1-ylmethyl)piperidin-1-yl]-2-oxoethyl}-*N*-phenylcyclohexane-1,4-diamine
64: *N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(2,5-dimethyl-2,5-dihydro-1*H*-pyrrol-1-yl)cyclohexanamine
65: *N*-benzyl-*N*'-{(1*R*)-1-(4-chlorobenzyl)-2-[4-cyclohexyl-4-(1*H*-1,2,4-triazol-1-ylmethyl)piperidin-1-yl]-2-oxoethyl}-*N*-methylcyclohexane-1,4-diamine
66: *N-*{(1*R*)-1-(4-chlorobenzyl)-2-[4-cyclohexyl-4-(1*H*-1,2,4-triazol-1-ylmethyl)piperidin-1-yl]-2-oxoethyl}-4-pyrrolidin-1-ylcyclohexanamine
67: 2-{[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}ethanol
68: 2-{benzyl[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}ethanol
69: *N*-{(1*R*)-1-(4-chlorobenzyl)-2-[4-cyclohexyl-4-(1*H*-1,2,4-triazol-1 - ylmethyl)piperidin-1-yl]-2-oxoethyl}-*N*-methyl-*N*-phenylcyclohexane-1,4-diamine
70: *N*'-{(1*R*)-1-(4-chlorobenzyl)-2-[4-cyclohexyl-4-(1*H*-1,2,4-triazol-1-ylmethyl)piperidin-1-yl]-2-oxoethyl}-*N*-(4-tluorophenyl)-*N*-methylcyclohexane-1,4-diamine
*71: cis ou trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarboxylic acid
72 : *cis ou trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarboxylic acid
73 : *cis ou trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*,*N*-diethylcyclohexanecarboxamide
74: *cis* ou *trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*,*N*-diethylcyclohexanecarboxamide
75: *cis ou trans*-4-({(1*R*)-(4-chlorobenzyl)-2-[4-cyclohexyl-4-(1*H*-1,2,4-triazol-1-ylmethyl)piperidin-1-yl]-2-oxoethyl}amino)-*N*,*N*-dimethylcyclohexanecarboxamide
76: *cis* ou *trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*,*N*-dimethylcyclohexanecarboxamide

Parmi les composés préférés de formule I dans laquelle X est CR₆R₇, on peut citer ceux dont les noms suivent
78: *cis*-*N*-benzyl-4-({(1*R*)-1-(4-chlorobenzyl)-2-[4-cyclohexyl-4-(1*H*-1,2,4-triazol-1 - ylmethyl)piperidin-1-yl]-2-oxoethyl}amino)-*N*-methylcyclohexanecarboxamide
   *trans-N-*benzyl-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-methylcyclohexanecarboxamide
79: *cis-N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(3-methyl-1,2,4-oxadiazol-5-yl)cyclohexanamine
   *trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(3-methyl-1,2,4-oxadiazol-5-yl)cyclohexanamine
80 : *cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarboxamide
   *trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarboxamide
82: *cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-1-(4-fluorophenyl)cyclohexanol
   *trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-1-(4-fluorophenyl)cyclohexanol
97: *N*-{(1*R*)-1-(4-chlorobenzyl)-2-[4-cyclohexyl-4-(1*H*-1,2,4-triazol-1-ylmethyl)piperidin-1-yl]-2-oxoethyl}-4-methoxycyclohexanamine
98: 4-[4-(benzyloxy)phenyl]-*N*-{(1*R*)-1-(4-chlorobenzyl)-2-[4-cyclohexyl-4-(1*H* 1,2,4-triazol-1-ylmethyl)piperidin-1-yl]-2-oxoethyl}cyclohexanamine
99: 4-(benzyloxy)-*N*-{(1*R*)-1-(4-chlorobenzyl)-2-[4-cyclohexyl-4-(1*H*-1,2,4-triazol-1-ylmethyl)piperidin-1-yl]-2-oxoethyl}cyclohexanamine
100: *N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2-methoxyacetamide
102: 2-(benzyloxy)-*N-*[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]acetamide
103: 3-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-1,3-oxazolidin-2-one
   3-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-1,3-oxazolidin-2-one
104: *cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(2-methoxyethyl)cyclohexane-1,4-diamine
   *trans*-*N-*{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(2-methoxyethyl)cyclohexane-1,4-diamine
105: *cis*-*N*-{(1*R*)-1-(4-chlorobenzyl)-2-[4-cyclohexyl-4-(1*H*-1,2,4-triazol-1 - ylmethyl)piperidin-1-yl]-2-oxoethyl}-4-morpholin-4-ylcyclohexanamine
   *trans-N-*{(1R)-1-(4-chlorobenzyl)-2-[4-cyclohexyl-4-(1*H*-1,2,4-triazol-1-ylmethyl)piperidin-1-yl]-2-oxoethyl}-4-morpholin-4-ylcyclohexanamine
106: 1-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]pyrrolidin-2-one
   1-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]pyrrolidin-2-one
107: *N*-{(1*R*)-1-(4-chlorobenzyl)-2-[4-cyclohexyl-4-(1*H*-1,2,4-triazol-1-ylmethyl)pipéridin-1-yl]-2-oxoethyl}-4-(2-methoxyethoxy)cyclohexanamine
112: *cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarbonitrile
   *trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarbonitrile
113: 1-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]piperidin-2-one
   1-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]piperidin-2-one
114: *N*-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]propanamide
116: *N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]glycinamide
117: *N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2-hydroxyacetamide
118: *N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2,2-dimethylpropanamide
   *N*-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2,2-dimethylpropanamide
119: *cis-N-*{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-methoxyphenyl)cyclohexane-1,4-diamine
   *trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-methoxyphenyl)cyclohexane-1,4-diamine
120: *cis-N-*{(1*R*)-1-(4-chlorobenzyl)-2-[4-cyclohexyl-4-(1*H*-1,2,4-triazol-1-ylmethyl)piperidin-1-yl]-2-oxoethyl}-4-(2*H-*tetrazol-5-yl)cyclohexanamine
   *trans-N-*{(1*R*)-1-(4-chlorobenzyl)-2-[4-cyclohexyl-4-(1*H*-1,2,4-triazol-1-ylmethyl)piperidin-1-yl]-2-oxoethyl}-4-(2*H*-tetrazol-5-yl)cyclohexanamine
121: 2-{[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}phenol
   2-{[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}phenol
122: 4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl acetate
123: *N*²-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-*N*,*N*-dimethylglycinamide
   *N²-*[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-*N,N*-dimethylglycinamide -
124: *N*²-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]glycinamide
   *N²-*[*trans*-4-({(1*R*)-1-(4-chlorobenzyl)-2-[4-cyclohexyl-4-(1*H*-1,2,4-triazol-1 - ylmethyl)piperidin-1-yl]-2-oxoethyl}amino)cyclohexyl]glycinamide
125: *4-*[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]piperazin-2-one
   4-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]piperazin-2-one
126: *cis*-4-(4-acetylpiperazin-1-yl)-*N*-{(1*R*)-1-(4-chlorobenzyl)-2-[4-cyclohexyl-4-(1*H*-1,2,4-triazol-1-ylmethyl)piperidin-1-yl]-2-oxoethyl}cyclohexanamine
   *trans*-4-(4-acetylpiperazin-1-yl)-*N*-{(1*R*)-1-(4-chlorobenzyl)-2-[4-cyclohexyl-4-(1*H-*1,2,4-triazol-1-ylmethyl)piperidin-1-yl]-2-oxoethyl}cyclohexanamine
130: *cis-N-*{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-chlorophenyl)cyclohexane-1,4-diamine
   *trans-N*{(1*R*)-1-(4-chlorobenzyl)-2-[4-cyclohexyl-4-(1*H*-1,2,4-triazol-1-ylmethyl)piperidin-1-yl]-2-oxoethyl}-*N*-(4-chlorophenyl)cyclohexane-1,4-diamine
131: 4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl pivalate
132: *cis-N-*{(1*R*)-1-(4-chlorobenzyl)-2-[4-cyclohexyl-4-(1*H*-1,2,4-triazol-1-ylmethyl)piperidin-1-yl]-2-oxoethyl}-*N*-(4-fluoro-2-methylphenyl)cyclohexane-1,4-diamine
   *trans-N-*{(1*R*)-1-(4-chlorobenzyl)-2-[4-cyclohexyl-4-(1*H*-1,2,4-triazol-1-ylmethyl)piperidin-1-yl]-2-oxoethyl}-*N*-(4-fluoro-2-methylphenyl)cyclohexane-1,4-diamine
133: 4-{[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}benzonitrile
134: *N-*[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]cyclopropanecarboxamide
138: *cis-N-*{(1*R*)-1-(4-chlorobenzyl)-2-[4-cyclohexyl-4-(1*H*-1,2,4-triazol-1-ylmethyl)piperidin-1-yl]-2-oxoethyl}-*N*-(2,4-difluorophenyl)cyclohexane-1,4-diamine
   *trans-N*{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(2,4-difluorophenyl)cyclohexane-1,4-diamine
139: *cis*-*N*-(1*R*)-1-(4-chlorobenzyl)-2-[4-cyclohexyl-4-(1*H*-1,2,4-triazol-1-ylmethyl)piperidin-1-yl]-2-oxoethyl}-*N*'-(5-fluoropyridin-2-yl)cyclohexane-1,4-diamine
   *trans-N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(5-fluoropyridin-2-yl)cyclohexane-1,4-diamine
140: *N*-1*H*-1,2,3-benzotriazol-5-yl-*N*'-{(1*R*)-1-(4-chlorobenzyl)-2-[4-cyclohexyl-4-(1*H*-1,2,4-triazol-1-ylmethyl)piperidin-1-yl]-2-oxoethyl}cyclohexane-1,4-diamine
142: 1-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]imidazolidin-2-one
   1-[*trans*-4-({(1*R*)-1-(4-chlorobenzyl)-2-[4-cyclohexyl-4-(1*H*-1,2,4-triazol-1 - ylmethyl)piperidin-1-yl]-2-oxoethyl}amino)cyclohexyl]imidazolidin-2-one
146: *cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*-(3,4-difluorophenyl)cyclohexane-1,4-diamine
   *trans-N-*{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(3,4-difluorophenyl)cyclohexane-1,4-diamine
147: *cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-fluoro-3-methoxyphenyl)cyclohexane-1,4-diamine
   *trans-N-*{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-fluoro-3-methoxyphenyl)cyclohexane-1,4-diamine
148: *cis*-*N*'-{(1*R*)-1-(4-chlorobenzyl)-2-[4-cyclohexyl-4-(1*H*-1,2,4-triazol-1-ylmethyl)piperidin-1-yl]-2-oxoethyl}-*N*-ethyl-*N*-(4-fluoro-3-methoxyphenyl)cyclohexane-1,4-diamine
   *trans*-*N'*-{(1*R*)-1-(4-chlorobenzyl)-2-[4-cyclohexyl-4-(1*H*-1,2,4-triazol-1-ylmethyl)piperidin-1-yl]-2-oxoethyl}-*N*-ethyl-*N*-(4-fluoro-3-methoxyphenyl)cyclohexane-1,4-diamine
149: *cis-N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-[4-(trifluoromethyl)phenyl]cyclohexane-1,4-diamine
   *trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-[4-(trifluoromethyl)phenyl]cyclohexane-1,4-diamine
150: *cis-N-*{(1*R*)-1-(4-chlorobenzyl)-2-[4-cyclohexyl-4-(1*H*-1,2,4-tiazol-1-ylmethyl)piperidin-1-yl]-2-oxoethyl}-*N*'-(4-fluoro-3-methylphenyl)cyclohexane-1,4-diamine
   *trans-N-*{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-fluôro-3-methylphenyl)cyclohexane-1,4-diamine
167: *3-*[*trans-*4-({(1*R*)*-*1*-*(4-chlorobenzyl)-2-[4-cyclohexyl-4-(1*H*-1,2,4-triazol-1-ylmethyl)piperidin-1-yl]-2-oxoethyl}amino)cyclohexyl]-6-fluoro-1,3-benzoxazol-2(3H)-one
   3-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-6-fluoro-1,3-benzoxazol-2(3*H*)-one
168: *N-*[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]pyridine-2-carboxamide
169: 2-{[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-5-fluorophenol ou
   2-{[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-5-fluorophenol
173: *N-*[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2,1-benzisoxazole-3-carboxamide
180: *cis-N-*(1,3-benzothiazol-2-ylmethyl)-*N*'-{(1*R*)-1-(4-chlorobenzyl)-2-[4-cyclohexyl-4-(1*H*-1,2,4-triazol-1-ylmethyl)piperidin-1-yl]-2-oxoethyl}cyclohexane-1,4-diamine
181: *cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(1,3-thiazol-2-ylmethyl)cyclohexane-1,4-diamine
182: 2-{[*cis*-4-({(1*R*)-1-(4-chlorobenzyl)-2-[4-cyclohexyl-4-(1*H*-1,2,4-triazol-1 - ylmethyl)piperidin-1-yl]-2-oxoethyl}amino)cyclohexyl]amino}-5-fluoro-*N*,*N-*dimethylbenzamide
   2-{[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-5-fluoro-*N*,*N-*dimethylbenzamide
184: *trans-N-*(*tert*-butyl)-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarboxamide
185: *cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(3,4-difluorophenyl)cyclohexanecarboxamide
186: *cis-N-*{(1S)-1-(4-chlorobenzyl)-2-[4-cyclohexyl-4-(1*H*-1,2,4-triazol-1-ylmethyl)piperidin-1-yl]-2-oxoethyl}cyclohexane-1,4-diamine
187: *trans*-*N*-{(1S)-1-(4-chlorobenzyl)-2-[4-cyclohexyl-4-(1*H*-1,2,4-triazol-1-ylmethyl)piperidin-1-yl]-2-oxoethyl}cyclohexane-1,4-diamine

Parmi les composés préférés de formule I dans laquelle X est CR₆R₇, on peut citer ceux dont les noms suivent
101: 2-{[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-2-oxoethyl acetate
115: *tert*-butyl (2-{[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-2-oxoethyl)carbamate
137: *cis* ou *trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*-(4-morpholin-4-ylphenyl)cyclohexane-1,4-diamine
145: *N*-{(1*R*)-1-4-chlorobenzyl)-2-[4-cyclohexyl-4-(1*H*-1,2,4-triazol-1-ylmethyl)piperidin-1-yl]-2-oxoethyl}-4,4-difluorocyclohexanamine

Parmi les composés préférés de formule I tans laquelle X est NR₁₀, on peut citer ceux dont les noms suivent
4: 1-benzyl-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
5: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(2-phenylethyl)piperidin-4-amine
6: 2-[4-({(1*R*)1-(4-chlorobenzyl)-2-[4-cyclohexyl-4-(1*H*-1,2,4-triazol-1-ylmethyl)piperidin-1-yl]-2-oxoethyl}amino)piperidin-1-yl]ethanol
7: 3-[4-({(1*R*)-1-(4-chlorobenzyl)-2-[4-cyclohexyl-4-(1*H*-1,2,4-triazol-1 - ylmethyl)piperidin-1-yl]-2-oxoethyl}amino)piperidin-1-yl]propan-1-ol
8: 4-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidin-1-yl]butan-1-ol
10: *tert*-butyl 4-({(1*R*)-1-(4-chlorobenzyl)-2-[4-cyclohexyl-4-(1*H*-1,2,4-triazol-1-ylmethyl)pipéridin-1-yl]-2-oxoethyl}amino)piperidine-1-carboxylate
14: *N*-{(1*R*)-1-(4-chlorobenzyl)-2-[4-cyclohexyl-4-(1*H*-1,2,4-triazol-1-ylmethyl)piperidin-1-yl]-2-oxoethyl}-8-azabicyclo[3.2.1]octan-3-amine
15 : *N*-{(1*R*)-1-(4-chlorobenzyl)-2-[4-cyclohexyl-4-(1*H*-1,2,4-triazol-1-ylmethyl)piperidin-1-yl]-2-oxoethyl}-8-methyl-8-azabicyclo[3.2.1]octan-3-amine
16: *N*-{(1*R*)-1-(4-chlorobenzyl)-2-[4-cyclohexyl-4-(1*H*-1,2,4-triazol-1-ylmethyl)piperidin-1-yl]-2-oxoethyl}-9-methyl-9-azabicyclo[3.3.1]nonan-3-amine
17: *N*-{(1*R*)-1-(4-chlorobenzyl)-2-[4-cyclohexyl-4-(1*H*-1,2,4-triazol-1-ylmethyl)piperidin-1-yl]-2-oxoethyl}quinuolidin-3-amine
18: *N*-{(1*R*)-1-(4-chlorobenzyl)-2-[4-cyclohexyl-4-(1*H*-1,2,4-triazol-1-ylmethyl)piperidin-1-yl]-2-oxoethyl}azepan-4-amine
19: . *N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-3-amine
20: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-phenylpiperidin-4-amine
21: *N*-{(1*R*)-1-(4-chlorobenzyl)-2-[3-cyclohexyl-3-(1*H*-1,2,4-triazol-1-ylmethyl)-8-azabicyclo[3.2.1]oct-8-yl]-2-oxoethyl}piperidin-4-amine
24: 1-benzyl-*N*-{(1*R*)-1-(4-chlorobenzyl)-2-[4-cyclohexyl-4-(1*H*-1,2,4-triazol-1-ylmethyl)piperidin-1-yl]-2-oxoethyl}pyrrolidin-3-amine
28: 1-benzoyl-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
29: 1-acetyl-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
35: 3-({(1*R*)-1-(4-chlorobenzyl)-2-[4-cyclohexyl-4-(1*H*-1,2,4-triazol-1 - ylmethyl)piperidin-1-yl]-2-oxoethyl}amino)-8-methyl-8-azabicyclo[3.2.1]octan-6-ol
37: *N*-{(1*R*)-1-(4-chlorobenzyl)-2-[4-cyclohexyl-4-(1*H*-1,2,4-triazol-1 - ylmethyl)piperidin-1-yl]-2-oxoethyl}-1-(trifluoroacetyl)piperidin-4-amine
39: 4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidine-1-carboxamide
47: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(4-fluorobenzoyl)piperidin-4-amine
48: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(cyclopentylcarbonyl)piperidin-4-amine
49: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(cyclobutylcarbonyl)piperidin-4-amine
51: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(4-methylphenyl)sulfonyl]piperidin-4-amine
52: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(pyridin-2-ylcarbonyl)piperidin-4-amine
53: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(phenylacetyl)piperidin-4-amine
54: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(methylsulfonyl)piperidin-4-amine
60: *N*-{(1*R*)-1-(4-chlorobenzyl)-2-[4-cyclohexyl-4-(1*H*-1,2,4-triazol-1-ylmethyl)piperidin-1-yl]-2-oxoethyl}-2-phenylpiperidin-4-amine
61: (1*S*,3*R*,5*S*,7*S*)-4-({(1*R*)-1-(4-chlorobenzyl)-2-[4-cyclohexyl-4-(1*H*-1,2,4-triazol-1-ylmethyl)piperidin-1-yl]-2-oxoethyl}amino)adamantan-1-ol

Parmi les composés préférés de formule I dans laquelle X est NR₁₀, on peut citer ceux dont les noms suivent
81: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-isonicotinoylpiperidin-4-amine .
83: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(1-methyl-1*H*-imidazol-2-yl)carbonyl]piperidin-4-amine
84: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(5-methylisoxazol-3-yl)carbonyl]piperidin-4-amine
85: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(3,4-difluorobenzoyl)piperidin-4-amine
86: 1-[(1-*tert*-butyl-5-methyl-1*H*-pyrazol-3-yl)carbonyl]-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
87: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(3,5-dimethylisoxazol-4-yl)carbonyl]piperidin-4-amine
88: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(3-thienylcarbonyl)piperidin-4-amine
89: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(pyrrolidin-1-ylcarbonyl)piperidin-4-amine
90: 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-phenylpiperidine-1-carboxamide
91: 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*,*N*-dimethylpiperidine-1-carboxamide
92: 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*,*N*-diethylpiperidine-1-carboxamide
93: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(piperidin-1-ylcarbonyl)piperidin-4-amine
94: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(morpholin-4-ylcarbonyl)piperidin-4-amine
95: 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-methyl-*N*-phenylpiperidine-1-carboxamide
96: *N*-benzyl-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-methylpiperidine-1-carboxamide
108: ethyl 4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidine-1-carboxylate
109: methyl 4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidine-1-carboxylate
110: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(2*S*)-piperidin-2-ylcarbonyl]piperidin-4-amine
111: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(2*R*)-piperidin-2-ylcarbonyl]piperidin-4-amine
127: *N*-{(1*R*)-1-(4-chlorobenzyl)-2-[4-cyclohexyl-4-(1*H*-1,2,4-triazol-1 - ylmethyl)piperidin-1-yl]-2-oxoethyl}-1-pyridin-2-ylpiperidin-4-amine
128: methyl *N*-4-({(1*R*)-(4-chlorobenzyl)-2-[4-cyclohexyl-4-(1*H*-1,2,4-triazol-1-ylmethyl)piperidin-1-yl]-2-oxoethyl}amino)cyclohexyl]glycinate
129: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(2,2-difluoroethyl)piperidin-4-amine
135: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(6-methylpyridazin-3-yl)piperidin-4-amine
136: methyl [4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidin-1-yl]acetate
141: *N-*{(1*R*)-1-(4-chlorobenzyl)-2-[4-cyclohexyl-4-(1*H*-1,2,4-triazol-1-ylmethyl)piperidin-1-yl]-2-oxoethyl}-1-pyrimidin-2-ylpiperidin-4-amine
143: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-cyclopropylpiperidin-4-amine
144: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-{[(2*S*)-4,4-difluoropiperidin-2-yl]carbonyl}piperidin-4-amine
151: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(4,4-difluoro-L-prolyl)piperidin-4-amine
152: 1-(1*H*-benzimidazol-2-yl)-*N*-{(1*R*)-1-(4-chlorobenzyl)-2-[4-cyclohexyl-4-(1*H-*1,2,4-triazol-1-ylmethyl)piperidin-1-yl]-2-oxoethyl}piperidin-4-amine
153: 1-(2,1-benzisoxazol-3-ylcarbonyl)-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
155: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(2,2,2-trifluoroethyl)piperidin-4-amine
156: 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(4-methoxyphenyl)piperidine-1-carboxamide
157: 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(4-fluorophenyl)piperidine-1-carboxamide
158: 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(2,4-difluorophenyl)piperidine-1-carboxamide
159: 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(3,4-difluorophenyl)piperidine-1-carboxamide
160: 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(2-fluorophenyl)piperidine-1-carboxamide
161: 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(2-methoxyphenyl)piperidine-1-carboxamide
162: 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-[4-(dimethylamino)phenyl]piperidine-1-carboxamide
163: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(5-fluoro-1*H*-indol-2-yl)carbonyl]piperidin-4-amine
164: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(pyrazin-2-ylcarbonyl)piperidin-4-amine
166: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(isoxazol-5-ylcarbonyl)piperidin-4-amine
170: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(quinolin-2-ylcarbonyl)piperidin-4-amine
171: *N*-{(1*R*)-(4-chlorobenzyl)-2-[4-cyclohexyl-4-(1*H*-1,2,4-triazol-1-ylmethyl)piperidin-1-yl]-2-oxoethyl}-1-[(3-methylpyridin-2-yl)carbonyl]piperidin-4-amine
172: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(1*H*-1,2,4-triazol-3-ylcarbonyl)piperidin-4-amine
174: 1-(1,3-benzothiazol-2-ylcarbonyl)-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
175: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(6-methylpyridin-2-yl)carbonyl]piperidin-4-amine
176: 1-(1-benzofuran-2-ylcarbonyl)-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
177: *N*-{(1*R*)-1-(4-chlorobenzyl)-2-[4-cyclohexyl-4-(1*H*-1,2,4-triazol-1 - ylmethyl)piperidin-1-yl]-2-oxoethyl}-1-[(6-fluoropyridin-2-yl)carbonyl]piperidin-4-amine
179: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(2,4-difluorobenzoyl)piperidin-4-amine

Parmi les composés préférés de formule I dans laquelle X est NR₁₀, on peut citer ceux dont les noms suivent
154: 1-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidin-1-yl]butan-2-one
165: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(5-phenyl-1,3-oxazol-4-yl)carbonyl]piperidin-4-amine
178: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(1-phenyl-1*H*-pyrazol-5-yl)carbonyl]piperidin-4-amine
183: *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(6-phenylpyridin-2-yl)carbonyl]piperidin-4-amine

Selon un autre objet l'invention se rapporte à un médicament, caractérisé en ce qu'il comprend un composé de formule (I) tel que décrit ci-dessus ou un sel d'addition de ce composé à un acide pharmaceutiquement acceptable, ou encore un hydrate ou un solvat du composé de formule (I).

Selon un autre objet l'invention se rapporte à une composition pharmaceutique, caractérisée en ce qu'elle comprend un composé de formule (I) tel que décrit ci-dessus ou un sel pharmaceutiquement acceptable, un hydrate ou un solvat de ce composé, ainsi qu'au moins un excipient pharmaceutiquement acceptable.

Selon un autre objet l'invention se rapporte à l'utilisation d'un composé de formule (I) tel que décrit ci dessus pour la préparation d'un médicament destiné au traitement et à la prévention de l'obésité, du diabète et des dysfonctions sexuelles pouvant affecter les deux sexes, en particulier des dysfonctionnements érectiles, au traitement des maladies cardiovasculaires ainsi que dans des applications anti-inflammatoires ou dans le traitement de la dépendance alcoolique.

Selon un autre objet l'invention se rapporte à un procédé de préparation d'un composé de formule (I) caractérisé en ce que l'on réalise une amination réductrice d'un composé de formule (V) : 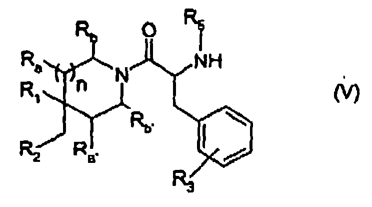 en présence d'un dérivé du groupe R₄ de type cétone, R₁, R₂, R₃, R₄, R₅, Rₐ, R_{a'}, R_{b}, R_{b'} et n étant tels que définis plus haut dans le texte.

Dans ce qui suit, on entend par groupe protecteur (Pg) un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans « Protective Groups in Organic Synthesis », Green W. et al., 1999, 3rd Edition (John Wiley & Sons, Inc., New York).

Selon un autre objet l'invention se rapporte aux composés de formules (IV) et (V) ; 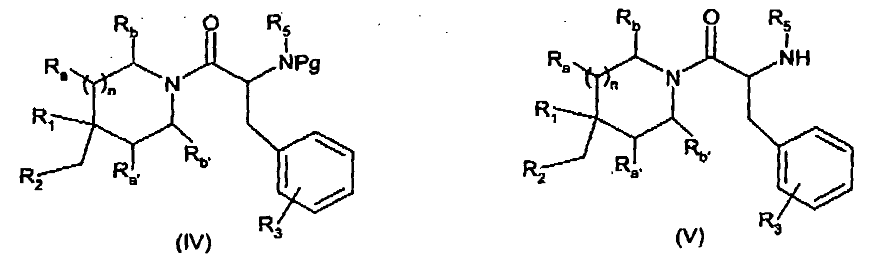 dans lesquelles R₁, Rₐ, R_{a'}, R_{b} et R_{b}, sont tels que définis plus haut dans le texte, Pg représente un groupe protecteur et :
n = 1, Rₐ et R_{a'}, identiques ou différents l'un de l'autre, représentent un atome d'hydrogène ou un groupe alkyle ou cycloalkyle, et R_{b} et R_{b'} forment ensemble, avec les atomes de carbone du cycle auquel ils sont rattachés, un pont carboné comprenant de 4 à 5 chaînons.

Selon un autre objet l'invention se rapporte aux composés de formules (VI), (XXVIII) et (XXIX), dans lesquels R₁, R₂, R₃, R₅, R_{a,} R_{a'}, R_{b}, R_{b'} et n sont tels que définis plus haut dans le texte et dans lesquels R₄ représente un groupe de formule (a) ou (b) telle que définie plus haut dans le texte et Pg représente un groupe protecteur d'amine ou d'hydroxyle:

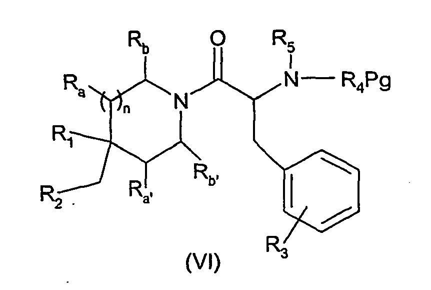

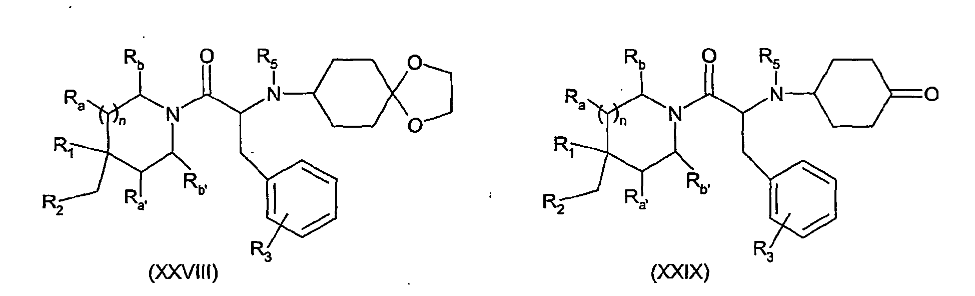

Selon un autre objet l'invention se rapporte aux composés de formule (II) : 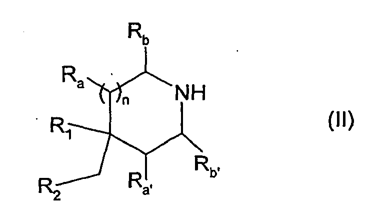 dans laquelle R₁, Rₐ, R_{a'}, R_{b} et R_{b'} sont tels que définis plus haut dans le texte, Pg représente un groupe protecteur et :
n = 1, Rₐ et R_{a'}, identiques ou différents l'un de l'autre, représentent un atome d'hydrogène ou un groupe alkyle ou cycloalkyle, et R_{b} et R_{b'} forment ensemble, avec les atomes de carbone du cycle auquel ils sont rattachés, un pont carboné comprenant de 4 à 5 chaînons.

On entend par groupe partant (Lg), dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un mésyle, tosyle, triflate, acétyle, etc. Des exemples de groupes partants ainsi que des références pour leur préparation sont donnés dans « March's Advanced Organic Chemistry », J. March et al., 5th Edition, 2001, EMlnter Ed.

On entend par groupement Boc un groupement t-butoxycarbonyl, Bn un groupement benzyle, CBz un groupement benzyloxycarbonyl, Fmoc un groupement 9-fluorenylmethyl-carbamate, et par h les heures.

Conformément à l'invention, on peut préparer les composés de formule générale (I) selon le procédé présenté dans le schéma 1.

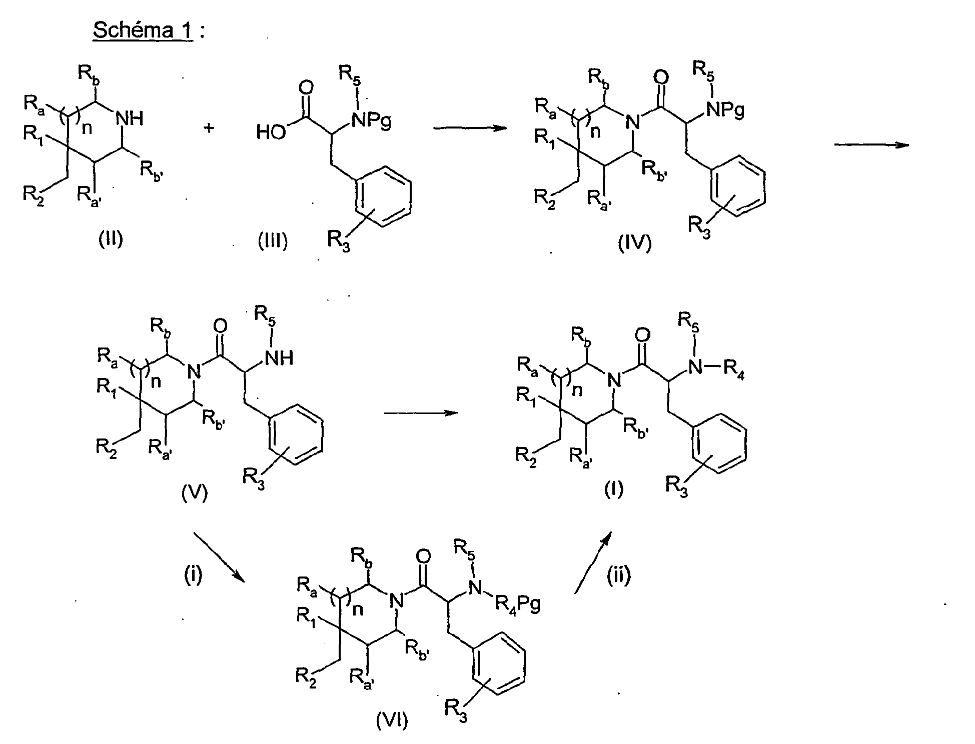

Selon le schéma 1, les composés de formule (IV) peuvent être préparés par couplage entre les intermédiaires de formule (II) et un aminoacide de formule (III) dont la fonction amine est protégée par un groupement protecteur Pg (par exemple un groupement Boc, CBz, Bn ou Fmoc), dans des conditions de couplage peptidique classiques, en utilisant par exemple comme agent couplant le dicyclocarbodiimide, le chlorhydrate du 1-(3-diméthylaminopropyl)-3-éthylcarbodiimide ou le bromo-tris-pyrrolidino-phosphonium hexafluorophosphate, en présence ou non d'hydroxybenzotriazole, et en utilisant comme base organique la triéthylamine ou la diisopropyléthylamine dans un solvant tel que le dioxane, le dichlorométhane ou l'acétonitrile.

Les aminoacides de formule générale (III) sont disponibles commercialement ou peuvent être préparés par des méthodes décrites dans la littérature (Williams, R.M., Synthesis of Optically Active α-Aminoacids, Pergamon Press, Oxford, 1989).

Les composés de formule (V) sont obtenus par déprotection de la fonction amine des composés de formule (IV), par des méthodes choisies parmi celles connues de l'Homme de l'art. Elles comprennent entre autres l'utilisation d'acide trifluoroacétique ou d'acide chlorhydrique dans le dichlorométhane, le dioxane, le tétrahydrofurane ou le diéthyléther dans le cas d'une protection par un groupement tert-butoxycarbonyle, l'hydrogénation avec le métal approprié dans le méthanol ou l'éthanol dans le cas d'un CBz ou d'un benzyle (Bn), et de pipéridine pour un groupement Fmoc, à des températures variant de -10°C à 100°C.

Dans une dernière étape, les composés de formule (I) sont obtenus par amination réductrice, réalisée en mettant les composés de formule (V) en présence d'un dérivé du groupe R₄ de type cétone, en utilisant un réducteur tel que le borohydrure de sodium, le triacétoxyborohydrure de sodium ou le cyanoborohydrure de sodium, en présence ou non d'un acide de Bronsted (tel que l'acide chlorhydrique) ou de Lewis (tel que le tétraisopropoxyde de titane) dans un solvant tel que le dichloroéthane, le dichlorométhane, l'acide acétique ou le méthanol, à des températures comprises entre -10°C et 30°C.

Les dérivés du groupe R₄ de type cétone peuvent être commerciaux ou obtenus par des méthodes connues de l'Homme de l'art, par exemple par acylation de la fonction amine ou hydroxyle libre du dérivé de type cétone, ou par amination réductrice de la fonction amine libre du dérivé de type cétone, cette fonction cétone étant libre ou protégée par des groupements tels que des acétals ou sous forme d'hydroxyle.

Selon une variante de la dernière étape du schéma 1, les composés de formule générale (I) dans lesquels R₄ répond à la formule (a) ou (b) peuvent également être préparés en réalisant :
- étape (i): une amination réductrice, réalisée en mettant les composés de formule (V) en présence d'un dérivé du groupe R₄ de type cétone, comme décrit ci-dessus, ledit groupe R₄ portant un groupe Pg protecteur d'amine ou d'hydroxyle, puis
- étape (ii) : déprotection de la fonction amine du composé de formule (VI) par des méthodes choisies parmi celles connues de l'Homme de l'art, comme décrit précédemment.

Alternativement, les composés de formule (VI) conduisant aux composés de formule (I) peuvent être préparés selon le procédé présenté dans le schéma 2.

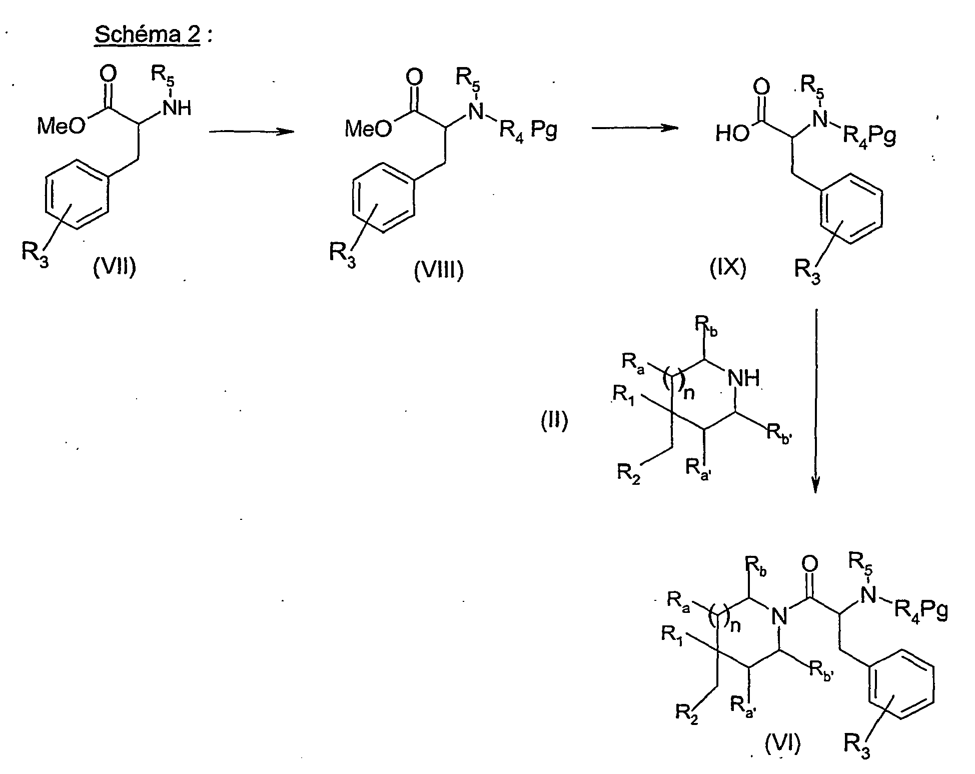

Selon le schéma 2, les composés de formule (VIII) peuvent être obtenus par amination réductrice, comme décrit précédemment, réalisée à partir des aminoacides de formule (VII). L'aminoacide de formule (VII) est disponible commercialement quand R₅ = H, ou peut être préparé par des méthodes décrites dans la littérature (Williams, R.M., Synthesis of Optically Active α-Aminoacids, Pergamon Press, Oxford, 1989). Dans les cas où R₅ représente un groupe alkyle, les aminoacides de formule (VII) peuvent être préparés par alkylation de l'aminoacide commercial protégé sur la fonction amine, selon les méthodes d'alkylation connues de l'Homme de l'art.

Les composés de formule (IX) peuvent être synthétisés par saponification des esters de formule (VIII), par exemple en présence d'hydroxyde de sodium ou d'hydroxyde de lithium dans un solvant tel que le méthanol, le tétrahydrofurane ou l'eau, ou un mélange de ces solvants.

Les composés de formule (VI) peuvent ensuite être préparés par couplage peptidique entre les intermédiaires de formule (II) et l'aminoacide de formule (IX), dans des conditions de couplage peptidique classiques, telles que celles décrites dans le schéma 1.

Les composés de formules (II) peuvent être obtenus selon le procédé présenté dans le schéma 3.

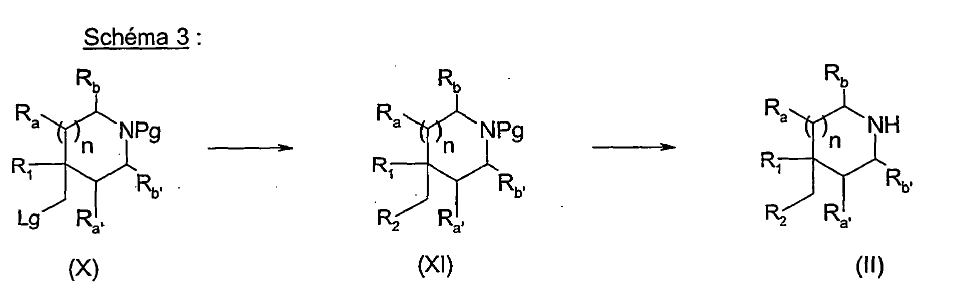

Selon le schéma 3, les composés de formule (XI) sont synthétisés par substitution du groupement partant Lg des intermédiaires de formule (X) par l'anion d'un hétéroaryle, dans un solvant tel que le diméthylformamide, à des températures comprises entre 20 et 200°C. Les composés de formule (II) sont ensuite obtenus par déprotection du groupement amine des composés de formule (XI), où Pg est un groupement protecteur d'amine tel que défini dans le schéma 1, selon des méthodes choisies parmi celles connues de l'Homme de l'art, comme décrit précédemment en rapport avec le schéma 1.

Dans le cas où R₁ représente un groupe cyclohexyle, les composés de formule (X) peuvent être préparés selon le schéma 4, adapté à partir de Sehbat et coll. (J. Med. Chem. (2002), 45, 4589).

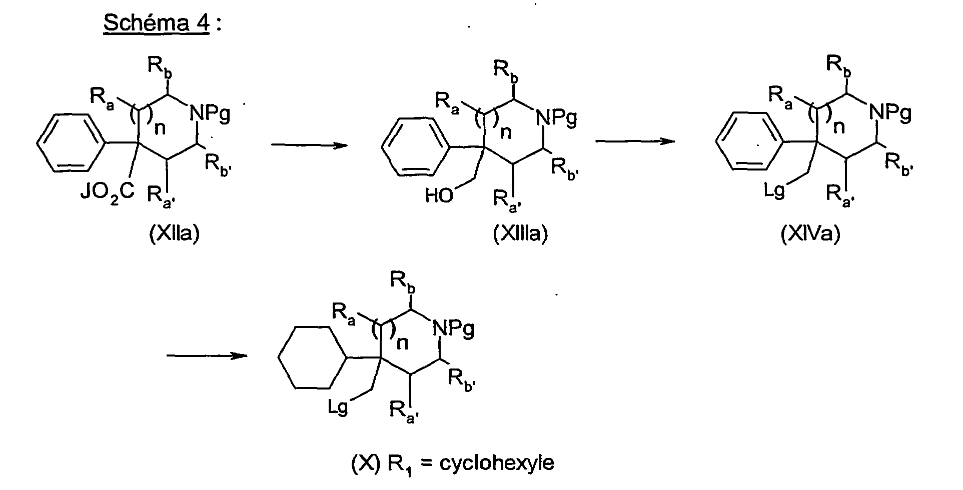

Dans les composés de formule (XIIa), J représente un atome d'hydrogène ou un groupe alkyle. Ces composés sont disponibles commercialement pour Rₐ = R_{a'} = R_{b} = R_{b'} = Het n = 1.

Les composés de formule (XIIIa) sont obtenus par réduction des acides ou esters de formule (XIIa) en utilisant des réducteurs tels que l'hydrure de lithium aluminium ou, après formation d'un anhydride mixte en présence de chloroformiate d'isobutyle et de triéthylamine dans le tétrahydrofurane ou le dioxane, le borohydrure de sodium dans le méthanol ou l'éthanol à des températures variant de -40°C à 10°C.

Les composés de formule (XIVa) sont préparés par transformation du groupement hydroxyle des composés de formule (XIIIa) en groupe partant, tel que le mésylate ou le tosylate, dans des conditions connues de l'Homme de l'art, par exemple par action du chlorure de méthanesulfonyle ou du chlorure de p-toluène sulfonyle en présence d'une base organique telle que la triéthylamine, à des températures variant de -20°C à la température ambiante.

Les composés de formule (X) sont ensuite formés par hydrogénation du groupement phényle des composés de formule (XIVa) en présence d'un catalyseur tel que Pd/C ou Rh/alumine à des pressions variant de 5 bars à 100 bars et à des températures variant de 20°C à 80°C, dans un solvant tel que le méthanol, l'éthanol ou l'acide acétique.

Les composés de formule (XIIa), et plus généralement les composés de formule (XII) ci-après, dans laquelle J représente un atome d'hydrogène ou un groupe alkyle, peuvent être préparés selon les schémas qui suivent. Les composés de formule (XII) peuvent se subdiviser en différentes formules (XIIb) et (XIId) :
- les composés de formule (XIIb) correspondent à la formule (XII) dans laquelle n = 1 et Rₐ, R_{a'}, R_{b} et R_{b'}, identiques ou différents les uns des autres, représentent un atome d'hydrogène ou un groupe alkyle ou cycloalkyle ;
- les composés de formule (XIId) correspondent à la formule (XII) dans laquelle n = 1, Rₐ et R_{a'}, identiques ou différents l'un de l'autre, représentent un atome d'hydrogène ou un groupe alkyle ou cycloalkyle, et R_{b} et R_{b'} forment ensemble, avec les atomes de carbone du cycle auquel ils sont rattachés, un pont carboné comprenant de 4 à 5 chaînons (t = 1 ou 2).

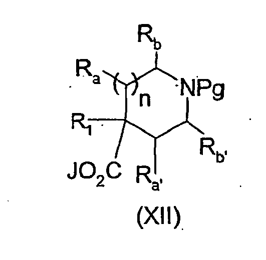

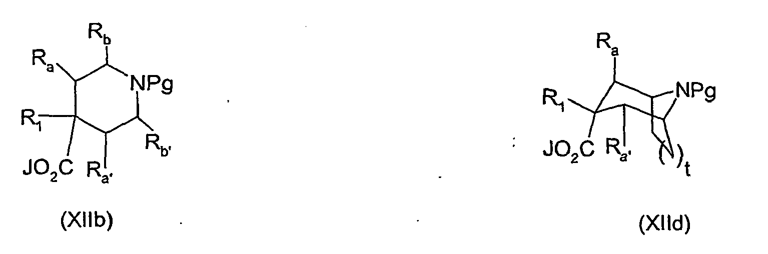

Les procédés de préparation des intermédiaires de synthèse de types pipéridine (XIIb) et tropane (XIId) sont décrits dans les schémas ci-dessous.

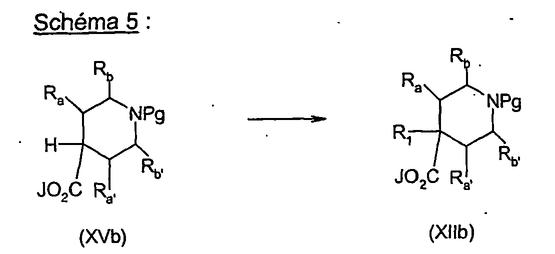

Selon le schéma 5, les pipéridines de formule (Xllb) peuvent être préparées par alkylation des composés de formule (XVb), disponibles commercialement, par un dérivé halogéné des groupes R₁ (où R₁ est un groupe alkyle ou cycloalkyle), lui aussi disponible commercialement.

Les tropanes de formule (XIId) peuvent être préparées selon le procédé décrit dans le schéma 7, d'après les travaux de Daum et coll. décrits dans J. Med. Chem. (1975), 18, 496.

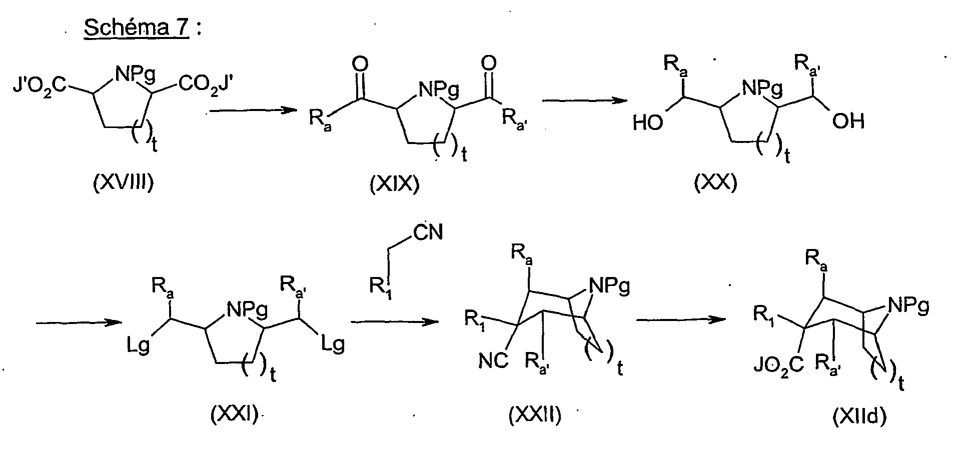

Les diesters de départ (XVIII), dans lesquels J' représente un groupement alkyle, et qui sont disponibles commercialement, peuvent être réduits en composés (XIX) par une réaction de Grignard, puis réduits en composés (XX), par exemple par l'action de l'hydrure de lithium aluminium ou d'un borane dans un solvant tel que le tétrahydrofurane ou le diéthyléther à des températures variant de -78°C à la température ambiante.

Alternativement, quand Rₐ = R_{a'} = H, on peut passer en une seule étape des composés de formule (XVIII) aux composés de formule (XX) dans lesquels Rₐ = R_{a'} = H, en utilisant un réducteur tel que l'hydrure de lithium aluminium dans un solvant tel que le tétrahydrofurane ou le diéthyléther à des températures variant de -78°C à la température ambiante.

Puis les groupements hydroxyles des composés (XX) sont transformés en groupements partants Lg, par exemple par mésylation ou tosylation, notamment par action de chlorure de méthanesulfonyle en présence de triéthylamine à des températures variant de -20°C à la température ambiante, ou en utilisant du chlorure de thionyle à des températures variant de 20°C à 120°C.

Les composés de formule (XXII) peuvent être préparés par réaction des nitriles de formule R₁-CH₂-CN sur les composés de formules (XXI), en présence d'une base telle que l'hydrure de sodium dans un solvant tel que le diméthylformamide, ou en présence de diisopropylamidure de lithium dans un solvant tel que le tétrahydrofurane ou le diéthylether, à des températures variant de -78°C à 100°C.

Alternativement, on peut utiliser un réactif de formule (R₁)'-CH₂-CN, dans lequel (R₁)' représente un précurseur du groupe R₁ ; par exemple si R₁ représente un groupe cycloalkyle sur le composé de formule (I), alors un intermédiaire de préparation de formule (XXII) peut comporter un groupe (R₁)' = phényle, qui pourra être hydrogéné dans une étape ultérieure pour produire le groupe souhaité R₁ = cycloalkyle.

Les composés de formule (XIId) dans lesquels J représente un atome d'hydrogène peuvent ensuite être obtenus par hydrolyse acide du groupement nitrile des composés de formule (XXII) à des températures variant de 100°C à 200°C, dans des solvants tels que le méthanol, l'éthanol ou l'eau. Les acides utilisés sont par exemple des acides minéraux, tels que l'acide chlorhydrique ou sulfurique.

Les composés de formule (XII), dont les procédés de préparation ont été décrits dans les schémas 5 et 7 ci-dessus, sont convertis en composés de formule (X), utilisables comme produits de départ dans le schéma 3, par réduction en alcool de la fonction -CO₂J puis introduction d'un groupe partant, comme décrit précédemment dans le schéma 4.

Selon une variante du schéma 1, dans le cas où les composés de formule (I) comprennent, en tant que groupe R₄, un groupe de formule (a) dans laquelle X = -N(R₁₀)-, où R₁₀ est un groupe alkyle substitué par un hydroxyle (c'est-à-dire R₁₀ est un groupe de formule -(CH₂)ₓ-OH, où x est un nombre entier compris entre 1 et 4), alors un intermédiaire de préparation desdits composés de formule (I) peut être un composé de formule (XXVI), obtenu selon le schéma 8.

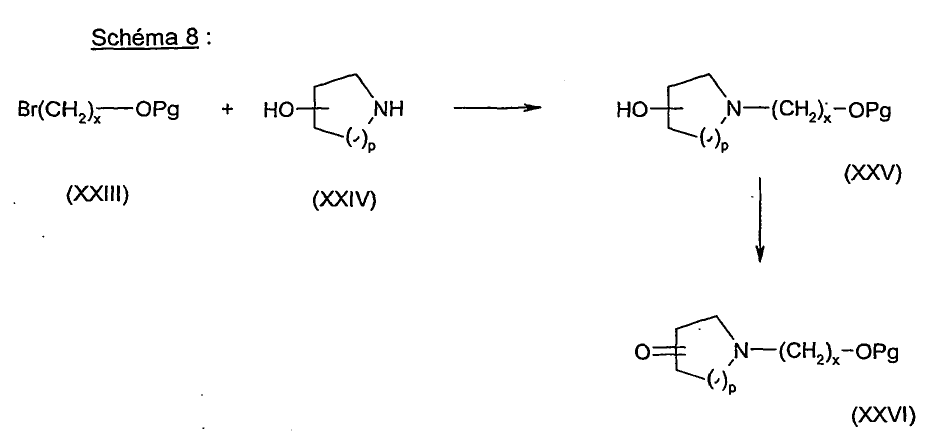

Selon le schéma 8, les composés de formule (XXIII) peuvent être préparés à partir des bromoalcools dont la fonction hydroxyle est protégée (Pg) selon des méthodes choisies parmi celles connues de l'Homme de l'art. Elles comprennent entre autres l'utilisation de dihydropyrane dans des conditions de catalyse acide, dans des solvants tels que le dichlorométhane.

Les composés de formule (XXV) peuvent être formés par substitution du brome des composés (XXIII) par la fonction amine des composés de formule (XXIV), en présence d'une base minérale telle que le carbonate de sodium dans des solvants tels que le diméthylformamide ou le toluène, à des températures variant de 0°C à 100°C.

Les composés de formule (XXVI) peuvent être obtenus par oxydation de la fonction hydroxyle présente sur la partie cyclique des composés de formule (XXV), par exemple en présence de chlorure d'oxalyle, de diméthylsulfoxyde et d'une base organique telle que la triéthylamine ou la diisopropylamine ou d'un complexe de chrome, à des températures variant de -78°C à 60°C.

Les composés de formule (XXVI) ainsi obtenus peuvent ensuite réagir avec les composés de formule (V), comme décrit dans le schéma 1 (étape d'amination réductrice).

Selon une autre variante du schéma 1, dans le cas où les composés de formule (I) comprennent, en tant que groupe R₄, un groupe de formule (a) de type cyclohexyle, c'est-à-dire un groupe de formule (a) où p = 2 et X = -C(R₆)(R₇)-, où R₆ représente un groupe -OR₈, R₇ et R₈ étant tels que définis ci-avant, alors la préparation des composés de formule (I) peut être réalisée comme décrit dans le schéma 9.

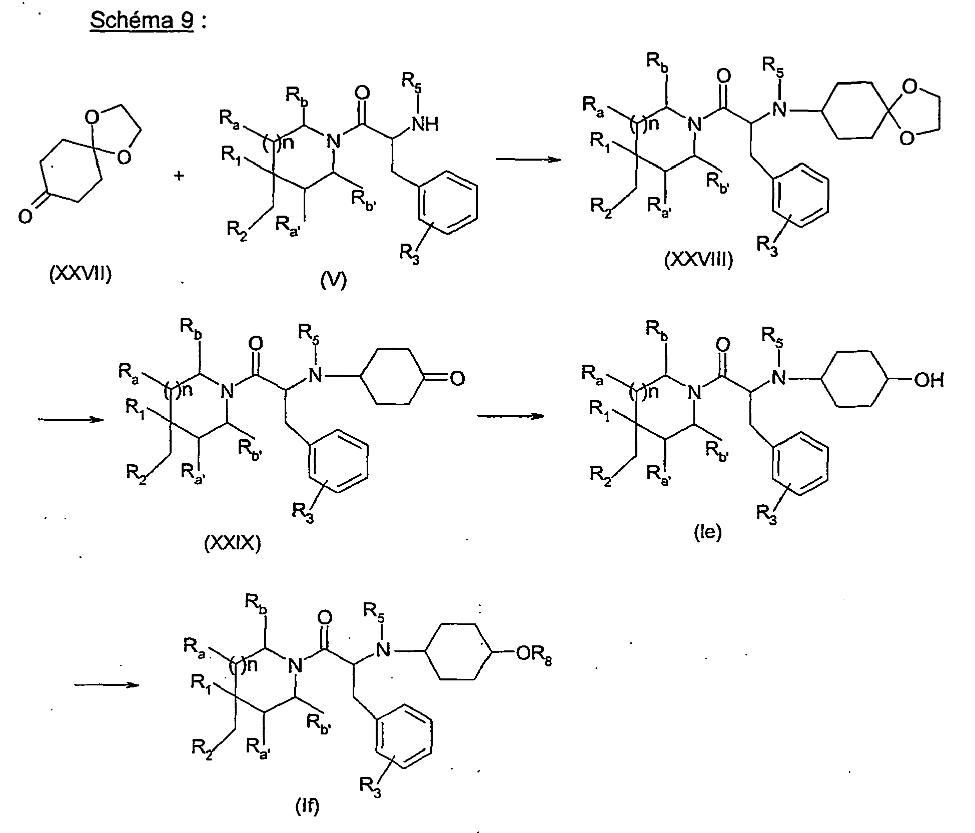

Selon le schéma 9, les composés de formule (XXVIII) peuvent être obtenus par une amination réductrice entre les composés de formule (XXVII) commerciaux et les composés de formule (V) dans des conditions telles que décrites dans le schéma 1.

La déprotection de la fonction oxo du composé de formule (XXVIII) en présence d'un acide tel que l'acide chlorhydrique ou le pyridinium tosylate dans le tétrahydrofurane ou l'acétone conduit au composé de formule (XXIX).

Les composés de formule (le) sont préparés par réduction des composés de formule (XXIX) dans des conditions telles que décrites dans le schéma 7.

Quand R₈ est différent d'un atome d'hydrogène, on réalise une fonctionnalisation des composés de formule (le), par exemple une alkylation en présence d'une base telle que l'hydrure de sodium et d'un dérivé du groupe R₈ comportant un groupement partant Lg, ce qui aboutit aux composés de formule (If).

Selon une autre variante du schéma 1, dans le cas où les composés de formule (I) comprennent, en tant que groupe R₄, un groupe de formule (a) de type cyclohexyle, c'est-à-dire un groupe de formule (a) où p = 2 et X = -C(R₆)(R₇)-, dans lequel R₆ représente un groupe -NR₈R₉, R₇, R₈ et R₉ étant tels que définis ci-avant, alors la préparation des composés de formule (I) peut être réalisée comme décrit dans le schéma 10.

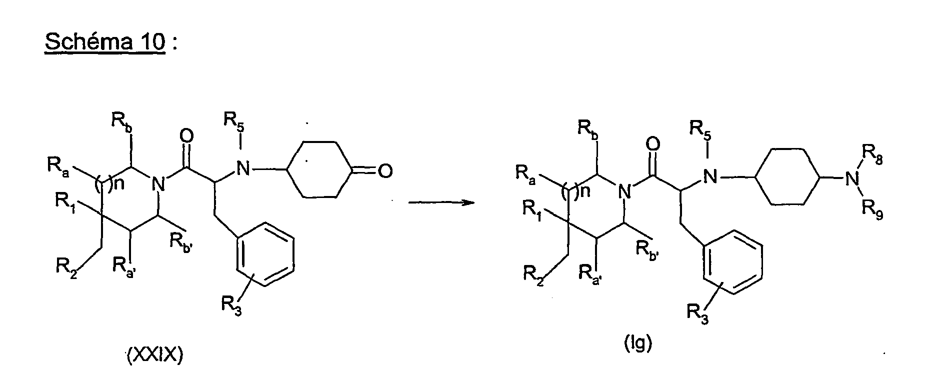

Selon le schéma 10, les composés de formule (Ig) peuvent être obtenus par une amination réductrice entre les composés de formule (XXIX) décrits dans le schéma 9 et des amines de formule R₈R₉NH, dans des conditions telles que décrites dans le schéma 1.

Dans les schémas 1 à 10, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme de l'art.

La présente invention a également pour objet les composés de formule (VI), (VIII), (IX), (XIId), (XXVIII) et (XXIX), utiles en tant qu'intermédiaires de synthèse des composés de formule (I).

D'autres composés utiles en tant qu'intermédiaires de synthèse des composés de formule (I), et qui font partie de l'invention, sont les composés de formule (II), (IV), (V), (X), (XI), (XIIa), (XIIb), (XIIIa) et (XIVa), dans desquelles
n = 1, Rₐ et Rₐ, identiques ou différents l'un de l'autre, représentent un atome d'hydrogène ou un groupe alkyle ou cycloalkyle, et R_{b} et R_{b'} forment ensemble, avec les atomes de carbone du cycle auquel ils sont rattachés, un pont carboné comprenant de 4 à 5 chaînons (t = 1 ou 2).

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

### Exemple 1: N-{(1R)-1-(4-chlorobenzyl)-2-[4-cyclohexyl-4-(1H-1,2,4-triazol-1-ylmethyl)piperidin-1-yl]-2-oxoethyl}piperidin-4-amine (composé n° 1)

### 1.1 : tert-butyl 4-(hydroxymethyl)-4-phenylpiperidine-1-carboxylate

On dissout sous azote 48 g d'acide 1-(*tert*-butoxycarbonyl)-4-phenylpiperidine-4-carboxylique commercial dans 437 mL de tétrahydrofurane anhydre. Le milieu est refroidi à -20°C et on additionne alors 24 mL de triéthylamine suivi de 21 g de chloroformiate d'isobutyle. Après 1 h d'agitation, le précipité formé est filtré. Le filtrat est refroidi à -20°C et 17,8g de borohydrure de sodium sont ajoutés par portions. L'agitation est maintenue pendant 1h. On ajoute alors 125 mL de méthanol, puis une solution d'acide sulfurique 2N à 0°C. On extrait au dichlorométhane jusqu'à épuisement de la phase aqueuse. La phase organique est alors lavée avec une solution aqueuse de soude 2N. Après séchage sur Na₂SO₄ et concentrée à sec. On obtient alors 30,7 g de *tert*-butyl 4-(hydroxymethyl)-4-phenylpiperidine-1-carboxylate.

### 1.2 : tert-butyl 4-{[(methylsulfonyl)oxy]methyl}-4-phenylpiperidine-1-carboxylate

On place 2,3 g de *tert*-butyl 4-(hydroxymethyl)-4-phenylpiperidine-1-carboxylate dans 70 mL de dichlorométhane à 0°C. Après addition de 1,68 mL de triéthylamine, on ajoute lentement le chlorure de mésylate à 0°C. Après 1h d'agitation à température ambiante, 30 mL d'une solution saturée aqueuse de chlorure d'ammonium sont ajoutés. On extrait au dichlorométhane jusqu'à épuisement de la phase aqueuse. La phase organique est lavée avec H₂O, avec une solution aqueuse de carbonate de sodium à 1% puis de nouveau avec H₂O. Après séchage sur MgSO₄ et concentration à sec, le brut est repris à l'acétate d'éthyle et au n-heptane. Après précipitation, on obtient 2,72 g *tert*-butyl 4-{[(methylsulfonyl)oxy]methyl}-4-phenylpiperidine-1-carboxylate.

### 1.3 : tert-butyl 4-cyclohexyl-4-{[(methylsulfonyl)oxy]methyl}piperidine-1-carboxylate

Dans un réacteur, on place 15 g de *tert*-butyl 4-{[(methylsulfonyl)oxy]methyl}-4-phenylpiperidine-1-carboxylate dans 406 mL d'éthanol, puis on ajoute 4,18 g de rhodium sur charbon à 5%. Le réacteur est ensuite agité à 30°C sous une pression d'hydrogène de 100 bars pendant 4h. Après filtration du catalyseur sur filtre Whatman et lavage au dichlorométhane, on concentre le filtrat et on obtient 14,76 g de *tert*-butyl 4-cyclohexyl-4-{[(methylsulfonyl)oxy]methyl}piperidine-1-carboxylate.

### 1.4 : tert-butyl 4-cyclohexyl-4-(1H-1,2,4-triazol-1-ylmethyl)piperidine-1-carboxylate

On introduit 7,83 g de *tert*-butyl 4-cyclohexyl-4-{[(methylsulfonyl)oxy]methyl}-piperidine-1-carboxylate et 5,68 g de 1,2,4-triazole sodique dans un ballon de 100 mL sous N₂. On ajoute 42 mL de diméthylformamide. Après réaction au micro-onde à 150°C pendant 1h30 et avec une puissance de 150W, on hydrolyse et on extrait à l'acétate d'éthyle jusqu'à épuisement de la phase aqueuse. La phase organique est lavée avec H₂O et concentrée à sec. Après cristallisation dans l'hexane, on obtient 4,42 g de *tert-*butyl 4-cyclohexyl-4-(1*H*-1,2,4-triazol-1-ylmethyl)piperidine-1-carboxylate.

### 1.5: 4-cyclohexyl-4-(1H-1,2,4,-triazol-1-ylmethyl)piperidine

On place 11,2 g de *tert*-butyl 4-cyclohexyl-4-(1*H*-1,2,4-triazol-1-ylmethyl)-piperidine-1-carboxylate dans 80 mL d'acide chlorhydrique 4N dans le dioxane. Le milieu réactionnel est agité pendant 16h à température ambiante. Après évaporation à sec, on essore le précipité obtenu et on rince au diéthyléther. Le chlorhydrate ainsi obtenu est séché sur P₂O₅ sous pression réduite. On obtient 9,65 g de 4-cyclohexyl-4-(1*H*-1,2,4-triazol-1-ylmethyl)piperidine.

### 1.6 : 9H-fluoren-9-ylmethyl {(1R)-1-(4-chlorobenzyl)-2-[4-cyclohexyl-4-(1H-1,2,4-triazol-1-ylmethyl)piperidin-1-yl]-2-oxoethyl}carbamate

On solubilise 2,98 g de 4-cyclohexyl-4-(1*H*-1,2,4-triazol-1-ylmethyl)piperidine dans 100 mL de dichlorométhane en présence de 5,06 g de 4-chloro-D-Fmoc-phénylalanine, de 1,62 g d'hydroxybenzotriazole, de 2,3 g de chlorhydrate de 1-(3-diméthylaminopropyl) -3-éthyl-carbodiimide et de 2,3 mL de diisopropyléthylamine. Le mélange est agité 16h à température ambiante. Après évaporation à sec, le résidu est lavé avec une solution saturée aqueuse de chlorure d'ammonium puis avec H₂O. Après séchage sur Na₂SO₄ et concentration à sec, le brut obtenu est chromatographié en éluant avec un gradient de méthanol dans le dichlorométhane variant de 0% à 3%. On obtient 7,8 g de 9H-fluoren-9-ylmethyl {(1*R*)-1-(4-chlorobenzyl)-2-[4-cyclohexyl-4-(1*H*-1,2,4-triazol-1-ylmethyl)piperidin-1-yl]-2-oxoethyl}carbamate.

### 1.7 : (2R)-3-(4-chlorophenyl)-1-[4-cyclohexyl-4-(1H-1,2,4-triazol-1-ylmethyl) piperidin-1-yl]-1-oxopropan-2-amine

On solubilise 6,98g de 9H-fluoren-9-ylmethyl {(1*R*)-1-(4-chlorobenzyl)-2-[4-cyclohexyl-4-(1*H*-1,2,4-triazol-1-ylmethyl)piperidin-1-yl]-2-oxoethyl}carbamate dans 82 mL de dichlorométhane, puis on ajoute 10,6 mL de pipéridine. L'agitation est maintenue pendant 16h à température ambiante sous N₂. Après concentration du milieu réactionnel, le brut obtenu est chromatographié en éluant avec un gradient de méthanol/ammoniaque dans le dichlorométhane variant de 99/1/0,1 à 9010/1 pour conduire à 4,19 g de (2*R*)-3-(4-chlorophenyl)-1-[4-cyclohexyl-4-(1*H*-1,2,4-triazol-1-ylmethyl)piperidin-1-yl]-1-oxopropan-2-amine.

### 1.8 : tert-butyl 4-({(1R)-1-(4-chlorobenzyl)-2-[4-cyclohexyl-4-(1H-1,2,4-triazol-1-ylmethyl)piperidin-1-yl]-2-oxoethyl}amino)piperidine-1-carboxylate

On solubilise 0,20g de (2*R*)-3-(4-chlorophenyl)-1-[4-cyclohexyl-4-(1*H*-1,2,4-triazol-1-ylmethyl)piperidin-1-yl]-1-oxopropan-2-amine dans 2,3 mL de dichlorométhane en présence de 0,93 g de *N*-Boc-pipéridone. L'agitation est maintenue 10 min à 0°C puis on additionne 0,13 g de triacétoxyborohydrure de sodium sous N₂. L'agitation est maintenue pendant 18h à température ambiante. Après hydrolyse, on extrait au dichlorométhane jusqu'à épuisement de la phase aqueuse. La phase organique est lavée avec une solution aqueuse saturée de carbonate de sodium. Après séchage avec MgSO₄ et concentration à sec, on obtient 0,29 g de *tert*-butyl 4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidine-1-carboxylate qui est utilisé tel quel par la suite.

### 1.9: Chlorhydrate de N-{(1R)-1-(4-chlorobenzyl)-2-[4-cyclohexyl-4-(1H-1,2,4-triazol-1-ylmethyl)piperidin-1-yl]-2-oxoethyl}piperidin-4-amine

On place 0,29 g de *tert*-butyl 4-({(1*R*)-1-(4-chlorobenzyl)-2-[4-cyclohexyl-4-(1*H-*1,2,4-triazol-1-ylmethyl)piperidin-1-yl]-2-oxoethyl}amino)piperidine-1-carboxylate dans 1,17 mL d'acide chlorhydrique 4N dans le dioxane. Le milieu réactionnel est trituré pendant 20 min à température ambiante puis on essore le précipité obtenu et on rince au diéthyléther. Le chlorhydrate ainsi obtenu est séché sur P₂O₅ sous pression réduite. On obtient 0,26 g du chlorhydrate de *N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine sous forme d'un solide blanc. Point de fusion = 255°C; M+H⁺ = 513, [α]_{D}²⁰ = -2,0° (c=1,002g/100mL, MeOH).

### Exemple 2: N-{(1R)-1-(4-chlorobenzyl)-2-[4-cyclohexyl-4-(1H-1,2,4-triazol-1-ylmethyl)piperidin-1-yl]-2-oxoethyl}cyclohexanamine (composé n° 3)

On solubilise 0,25g de (2*R*)-3-(4-chlorophenyl)-1-[4-cyclohexyl-4-(1*H*-1,2,4-triazol-1-ylmethyl)piperidin-1-yl]-1-oxopropan-2-amine, obtenu à l'étape 1.7, dans 2,9 mL de dichlorométhane en présence de 0,06 g de cyclohexanone. Le milieu réactionnel est refroidi à 0°C puis on additionne 0,16g de triacétoxyborohydrure de sodium sous N₂. L'agitation est maintenue pendant 18h à température ambiante. Après hydrolyse, on extrait au dichlorométhane jusqu'à épuisement de la phase aqueuse. La phase organique est lavée avec une solution aqueuse saturée de carbonate de sodium. Après séchage avec MgSO₄ et concentration à sec, le brut obtenu est chromatographié sur gel de silice en éluant avec un mélange 9/1 de dichlorométhane et de méthanol. On obtient 0,25 g de *N*-{(1*R*)-1-(4-chlorobenzyl)-2-[4-cyclohexyl-4-(1*H*-1,2,4-triazol-1-ylmethyl) piperidin-1-yl]-2-oxoethyl}cyclohexanamine sous forme d'un solide blanc.

Point de fusion = 60°C; M+H⁺ = 512.

### Exemple 3: 2-[4-({(1R)-1-(4-chlorobenzyl)-2-[4-cyclohexyl-4-(1H-1,2,4-triazol-1-ylmethyl)piperidin-1-yl]-2-oxoethyl}amino)piperidin-1-yl]ethanol (composé n° 6)

### 3.1 : 2-(2-bromoethoxy)tetrahydro-2H-pyrane

On place 3,97 mL de bromoéthanol dans 44 mL de tétrahydrofurane. La solution est refroidie à -10°C sous N₂. On ajoute alors 5,51 mL de 3,4-dihydro-2*H*-pyrane et 0,20 g-d'acide p-toluène sulfonique. Le milieu réactionnel est agité pendant 16h à -10°C. Après dilution dans du diéthyléther, la phase organique est lavée avec une solution aqueuse saturée d'hydrogénocarbonate de sodium puis avec H₂O séchée sur Na₂SO₄ et concentrée à sec pour conduire à 11 g de 2-(2-bromoethoxy)tetrahydro-2*H*-pyrane.

### 3.2 : 1-[2-(tetrahydro-2H-pyran-2-yloxy)ethyl]piperidin-4-ol

On dissout 2,72 g de 2-(2-bromoethoxy)tetrahydro-2*H*-pyrane, 1,31 g de 4-hydroxy-piperidine et 2,33 g de carbonate de potassium dans 130 mL de diméthylformamide sous N₂. Après agitation pendant 16h, on rajoute 1,31 g de 4-hydroxypiperidine et 2,33 g de carbonate de potassium. L'agitation est poursuivie pendant .72h. Après hydrolyse aqueuse, on extrait au dichlorométhane jusqu'à épuisement de la phase aqueuse. Les phases organiques sont lavées à l'eau, séchées sur Na₂SO₄ et concentrée à sec. Le brut obtenu est chromatographié sur gel de silice en éluant avec un gradient de méthanol/ammoniaque dans le dichlorométhane variant de 0% à 90/10/1 pour conduire à 1,25 g de 1-[2-(tetrahydro-2*H*-pyran-2-yloxy)ethyl]piperidin-4-ol.

### 3.3 : 1-[2-(tetrahydro-2H-pyran-2-yloxy)ethyl]piperidin-4-one

On place 0,68 mL de chlorure d'oxalyle dans 15 mL de dichlorométhane et le tout est refroidi à -78°C. On ajoute alors lentement 0,99 mL de diméthylsulfoxide dilué dans 2 mL de dichlorométhane puis 0,97 g de 1-[2-(tetrahydro-2*H*-pyran-2-yloxy)ethyl] piperidin-4-ol dilué dans 5 mL de dichlorométhane. Le milieu réactionnel est agité 30 min. On additionne alors lentement 2,49 mL de triéthylamine toujours à -78°C. L'agitation est poursuivie pendant 4h à température ambiante. Après hydrolyse, on extrait au dichlorométhane jusqu'à épuisement de la phase aqueuse. Les phases organiques sont lavées avec une solution aqueuse à 20% d'hydrogénocarbonate de sodium, séchées sur Na₂SO₄ et concentrée à sec. On obtient 0,89 g de 1-[2-(tetrahydro-2*H*-pyran-2-yloxy)ethyl]piperidin-4-one.

### 3.4 : N-{(1R)-1-(4-chlorobenzyl)-2-[4-cyclohexyl-4-(1H-1,2,4-triazol-1-ylmethyl)piperidin-1-yl]-2-oxoethyl}-1-[2-(tetrahydro-2H-pyran-2-yloxy)ethyl]piperidin-4-amine

On solubilise 0,30 g de (2*R*)-3-(4-chlorophenyl)-1-[4-cyclohexyl-4-(1*H*-1,2,4-triazol-1-ylmethyl)piperidin-1-yl]-1-oxopropan-2-amine, obtenu à l'étape 1.7, dans 3,5 mL de dichlorométhane en présence de 0,16 g de 1-[2-(tetrahydro-2*H*-pyran-2-yloxy)ethyl]piperidin-4-one et on laisse agiter 30 min à 0°C, puis on additionne 0,19 g de triacétoxyborohydrure de sodium sous N₂ pendant 10 min à 0°C puis l'agitation est maintenue pendant 18h à température ambiante. Après hydrolyse avec une solution aqueuse saturée de carbonate de potassium, on extrait au dichlorométhane jusqu'à épuisement de la phase aqueuse. Après séchage avec MgSO₄ et concentration à sec, le brut obtenu est chromatographié sur gel de silice en éluant avec un gradient de méthanol dans le dichlorométhane variant de 25% à 75%. On obtient 0,44 g de *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[2-(tetrahydro-2*H*-pyran-2-yloxy)ethyl]piperidin-4-amine.

### 3.5 : 2-[4-({(1R)-1-(4-chlorobenzyl)-2-[4-cyclohexyl-4-(1H-1,2,4-triazol-1-ylmethyl)piperidin-1-yl]-2-oxoethyl}amino)piperidin-1-yl]ethanol

On place 0,32 g de *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[2-(tetrahydro-2*H*-pyran-2-yloxy)ethyl]piperidin-4-amine dans 1,05 mL d'acide chlorhydrique 4N dans le dioxane. Le milieu réactionnel est agité pendant 18h à température ambiante. On essore le précipité obtenu et on rince au diéthyléther. Le chlorhydrate ainsi obtenu est séché sur P₂O₅ sous pression réduite. On obtient 0,21 g du chlorhydrate de 2-[4-({(1*R*)-1-(4-chlorobenzyl)-2-[4-cyclohexyl-4-(1*H-*1,2,4-triazol-1-ylmethyl)piperidin-1-yl]-2-oxoethyl}amino)piperidin-1-yl]ethanol sous forme d'un solide blanc.

Point de fusion = 257°C; M+H⁺ =557, [α]_{D}²⁰ = -2,4° (c=0,9905g/100 mL, MeOH).

### Exemple 4: N-{(1R)-1-(4-chlorobenzyl)-2-[4-cyclohexyl-4-(1H-1,2,4-triazol-1-ylmethyl)piperidin-1-yl]-2-oxoethyl}tetrahydro-2H-pyran-4-amine (composé n° 2) -

On solubilise 0,25 g de (2*R*)-3-(4-chlorophenyl)-1-[4-cyclohexyl-4-(1*H*-1,2,4-triazol-1-ylmethyl)pipéridin-1-yl]-1*-oxopropan-2-amine obtenu à l'étape 1.7 dans 2,9 mL de dichlorométhane en présence de 0,06 g de tetrahydro-4*H*-pyran-4-one. Le milieu réactionnel est refroidi à 0°C puis on additionne 0,16 g de triacétoxyborohydrure de sodium sous N₂. L'agitation est maintenue pendant 18h à température ambiante. Après hydrolyse avec une solution aqueuse de bicarbonate de soude, on extrait au dichlorométhane jusqu'à épuisement de la phase aqueuse. La phase organique est séchée avec MgSO₄ et concentrée à sec. Le brut obtenu est chromatographié sur gel de silice en éluant avec un mélange de dichlorométhane et de méthanol (9/1). On obtient 0,256 g de *N*-{(1*R*)-1-(4-chlorobenzyl)-2-[4-cyclohexyl-4-(1*H*-1,2,4-triazol-1-ylmethyl)-piperidin-1-yl]-2-oxoethyl}tetrahydro-2*H*-pyran-4-amine sous forme d'un solide blanc.

Point de fusion = 75°C; M+H⁺ = 514, [α]_{D}²⁰ = -1,7° (c=0,994g/100mL, MeOH).

RMN ¹H (200MHz, CDCl₃) : 7,96 (s, 1H), 7,90 (s, 1H), 7,25 (d, J=8 Hz, 2H), 7,14 (d, J=8 Hz, 2H), 4,18-2,10 (m, 14H), 2,05-0,82 (m, 19H). Analyse élémentaire: exp %C= 64,14, %H : 7,69, %N : 13,22 ; th : %64,43, %H : 7,89 ,%N : 13,42

### Exemple 5: Chlorhydrate de N-{(1R)-1-(4-chlorobenzyl)-2-[3-cyclohexyl-3-(1H-1,2,4-triazol-1-ylmethyl)-8-azabicyclo[3.2.1]oct-8-yl]-2-oxoethyl}piperidin-4-amine (composé n° 21)

### 5.1 : (2R, 5S)-1-benzylpyrrolidine-2,5-dimethanol

On place 64,84 mL d'hydrure de lithium aluminium (1 N dans le diéthyléther) à 0°C sous N₂. On ajoute alors 6,6 g de diethyl (2R, 5S)-1-benzylpyrrolidine-2,5-dicarboxylate dans 21 mL de diéthyléther. A la fin de l'addition, le milieu réactionnel est agité à reflux pendant 1 h. Après refroidissement à 0°C, On ajoute 6,5 mL d'eau et l'agitation est maintenue 1h à température ambiante. La solution est filtrée et le précipité formé est rincé plusieurs fois au diéthyléther. Le filtrat est ensuite séché sur Na₂SO₄ et concentré à sec pour conduire à 4,5 g de (2R, 5S)-1-benzylpyrrolidine-2,5-dimethanol.

### 5.2 : (2R, 5S)-bis(chloromethyl)-1-benzylpyrrolidine

On solubilise 4,5 g de (2R, 5S)-1-benzylpyrrolidine-2,5-dimethanol dans 68 mL de toluène puis on ajoute à 0°C 3,7 mL de chlorure de thionyle. Après chauffage à 70°C pendant 2h, on évapore à sec. Le solide obtenu est trituré dans du tolène, essoré et séché sur P₂O₅. On obtient 5,3 g de (2R, 5S)-bis(chloromethyl)-1-benzylpyrrolidine.

### 5.3 : 8-benzyl-3-phenyl-8-azabicyclo[3.2.1]octane-3-carbonitrile

On solubilise 10 g de (2R, 5S)-bis(chloromethyl)-1-benzylpyrrolidine dans 171 mL de diméthylformamide et 5,2 mL de phénylacétonitrile sont ajoutés. On ajoute alors par portions 4,07g d'hydrure de sodium et le milieu réactionnel est agité pendant 3h à température ambiante, puis pendant 1h à 100°C. Après refroidissement, le milieu réactionnel est versé sur de la glace. Après addition d'eau, on extrait alors à l'acétate d'éthyle jusqu'à épuisement de la phase aqueuse. Les phases organiques sont lavées à l'eau, avec une solution aqueuse saturée de chlorure de sodium puis séchées sur MgSO₄ et concentrée à sec. Le brut obtenu est chromatographié sur gel de silice en éluant avec un gradient de méthanol dans le dichlorométhane variant de 0% à 2,5% pour conduire à 7,75 g de 8-benzyl-3-phenyl-8-azabicyclo[3.2.1]octane-3-carbonitrile.

### 5.4 : 8-benzyl-3-phenyl-8-azabicyclo[3.2.1]octane-3-carboxylic acid

On ajoute 8,9 mL d'acide sulfurique et 4,15 mL d'eau à 7,75 g de 8-benzyl-3-phenyl-8-azabicyclo[3.2.1]octane-3-carbonitrile. On chauffe à 170°C pendant 1h puis à 130°C. On ajoute alors 6 mL d'éthanol et l'agitation est maintenue pendant 3h à la même température. Après refroidissement, on verse le milieu réactionnel sur de la glace, et on basifie avec de la soude aqueuse 2N. On extrait alors à l'acétate d'éthyle jusqu'à épuisement de la phase aqueuse. Les phases organiques sont lavées à l'eau, avec une solution aqueuse saturée de d'hydrogénocarbonate de sodium, à l'eau, avec une solution aqueuse saturée de chlorure de sodium puis séchées sur MgSO₄ et concentrée à sec. Les phases aqueuses sont alors traitées avec 100 g de résine DOWEX^{®} 50X2. La résine est ensuite essorée et lavée avec de l'eau, du tétrahydrofurane, puis du méthanol, Le composé est alors relargué avec une solution 2N d'ammoniaque dans le méthanol. Après concentration, on obtient 1,5 g du composé endo 8-benzyl-3-phenyl-8-azabicyclo[3.2.1]octane-3-carboxylic acid sous forme de sel d'ammoniaque. La suite de la synthèse concerne le composé endo.

### 5.5 : 3-phenyl-8-azabicyclo[3.2.1]octane-3-carboxylic acid

On solubilise 1 g de 8-benzyl-3-phenyl-8-azabicyclo[3.2.1]octane-3-carboxylic acid dans 19,5 mL de méthanol et on ajoute 1,86 g de formiate d'ammonium et 0,5 g de Pd/C 10% (50% dans H2O). On chauffe au reflux pendant 2h. Après filtration, on concentre à sec. On obtient 0,68 g de 3-phenyl-8-azabicyclo[3.2.1]octane-3-carboxylic acid, utilisé tel quel dans la suite de la synthèse.

### 5.6 : 8-(tert-butoxycarbonyl)-3-phenyl-8-azabicyclo[3.2.1]octane-3-carboxylic acid

On solubilise 0,68 g de 3-phenyl-8-azabicyclo[3.2.1]octane-3-carboxylic acid dans un mélange de 15 mL de tétrahydrofurane et 8,85 mL de soude aqueuse 1N. Après 15 min d'agitation, on ajoute 0,95 g de di-*tert*-butyl-dicarbonate. L'agitation est maintenue pendant 18h. Puis on refroidit le milieu réactionnel à 0°C et on ajoute du sulfate de potassium jusqu'à pH acide, puis H₂O. On extrait à l'acétate d'éthyle jusqu'à épuisement de la phase aqueuse. Les phases organiques sont lavées à l'eau, puis avec une solution aqueuse saturée de chlorure de sodium puis séchées sur MgSO₄ et concentrée à sec. Le brut obtenu est chromatographié sur gel de silice en éluant avec un gradient de méthanol dans le dichlorométhane variant de 0% à 10% pour conduire à 0,32g de 8-(*tert-*butoxycarbonyl)-3-phenyl-8-azabicyclo[3.2.1]octane-3-carboxylic acid.

### 5.7 : tert-butyl 3-(hydroxymethyl)-3-phenyl-8-azabicyclo[3.2.1]octane-8-carboxylate

On place 0,27 g de 8-(*tert*-butoxycarbonyl)-3-phenyl-8-azabicyclo[3.2.1]octane-3-carboxylic acid dans 4 mL de tétrahydrofurane à 0°C sous N₂ et on ajoute 1,63 mL de borane BH₃-THF 1 N. L'agitation est maintenue à température ambiante pendant 72h. On ajoute alors 0,8 mL de borane 1 N et on agite pendant 5h. Après ajout de méthanol, on concentre à sec. On ajoute alors un mélange glace, H₂O et d'une solution aqueuse d'acide chlorhydrique 1 N. On extrait à l'acétate d'éthyle jusqu'à épuisement de la phase aqueuse. Les phases organiques sont lavées à l'eau, puis avec une solution aqueuse saturée de chlorure de sodium puis séchées sur MgSO₄ et concentrées à sec. Le brut obtenu est chromatographié sur gel de silice en éluant avec un gradient de méthanol dans le dichlorométhane variant de 0% à 3% pour conduire à 0,11g de *tert*-butyl 3-(hydroxymethyl)-3-phenyl-8-azabicyclo[3.2.1]octane-8-carboxylate.

### 5.8 : tert-butyl 3-{[(methylsulfonyl)oxy]methyl}-3-phenyl-8-azabicyclo[3.2.1]octane-8-carboxylate

On place 0,44 g de *tert*-butyl 3-(hydroxymethyl)-3-phenyl-8-azabicyclo[3.2.1]octane-8-carboxylate dans 2 mL de dichlorométhane à 0°C sous N₂. Puis on ajoute 0,05 mL de chlorure de mésyle et 0,10 mL de triéthylamine. L'agitation à température ambiante est maintenue 3h. On ajoute alors 0,025 mL de chlorure de mésyle et 0,05 mL de triéthylamine. Après 2h d'agitation, on ajoute de la glace et une solution saturée aqueuse d'hydrogénocarbonate de sodium. On extrait à l'acétate d'éthyle jusqu'à épuisement de la phase aqueuse. Les phases organiques sont lavées à l'eau, puis avec une solution aqueuse saturée de chlorure de sodium puis séchées sur MgSO₄ et concentrée à sec. On obtient 0,17 g de *tert*-butyl 3-{[(methylsulfonyl)oxy]methyl}-3-phenyl-8-azabicyclo[3.2.1]octane-8-carboxylate qui est utilisé tel quel dans la suite de la synthèse.

### 5.9 : tert-butyl 3-cyclohexyl-3-{[(methylsulfonyl)oxy]methyl}-8-azabicyclo[3.2.1] octane-8-carboxylate

On place 0,17 g de *tert*-butyl 3-{[(methylsulfonyl)oxy]methyl}-3-phenyl-8-azabicyclo[3.2.1]octane-8-carboxylate dans un réacteur de haute pression, on solubilise dans l'éthanol et on ajoute 0,22 g de Rh/C à 5%. Le réacteur est ensuite agité sous une pression d'hydrogène de 110 bars pendant 6h. Le milieu réactionnel est filtré et concentré à sec. On obtient 0,14 g de *tert*-butyl 3-cyclohexyl-3-{[(methylsulfonyl) oxy]methyl}-8-azabicyclo[3.2.1]octane-8-carboxylate utilisé tel quel dans la suite de la synthèse.

### 5.10 : tert-butyl 3-cyclohexyl-3-(1H-1,2,4-triazol-1-ylmethyl)-8-azabicyclo[3.2.1] octane-8-carboxylate

On place 0,09 g de *tert*-butyl 3-cyclohexyl-3-{[(methylsulfonyl)oxy]methyl}-8-azabicyclo[3.2.1]octane-8-carboxylate dans 0.6 mL de HMPA en présence de 0,063 g de 1,2,4-triazole sodique. Après réaction au micro-onde à 140°C pendant 20min et avec une puissance de 30W, on hydrolyse et on extrait à l'acétate d'éthyle jusqu'à épuisement de la phase aqueuse. La phase organique est lavée avec H₂O puis avec une solution saturée de chlorure de sodium et concentrée à sec. Le brut obtenu est chromatographié sur gel de silice en éluant avec un gradient de méthanol dans le dichlorométhane variant de 0% à 3%. On obtient 0,017 g de *tert*-butyl 3-cyclohexyl-3-(1*H*-1,2,4-triazol-1-ylmethyl)-8-azabicyclo[3.2.1]octane-8-carboxylate.

### 5.11 : 3-cyclohexyl-3-(1H-1,2,4-triazol-1-ylmethyl)-8-azabicyclo[3.2.1]octane

On place 0,05g de *tert*-butyl 3-cyclohexyl-3-(1*H*-1,2,4-triazol-1-ylmethyl)-8-azabicyclo[3.2.1]octane-8-carboxylate dans 0,6 mL d'acide chlorhydrique 4N dans le dioxane. Le milieu réactionnel est agité pendant 5h à température ambiante. Après évaporation à sec, on obtient 0,05 g de 3-cyclohexyl-3-(1*H*-1,2,4-triazol-1-ylmethyl)-8-azabicyclo[3.2.1]octane utilisé tel quel par la suite.

### 5.12 : Methyl N-[1-(tert-butoxycarbonyl)piperidin-4-yl]-4-chloro-D-phenylalaninate

On solubilise 10 g d'ester méthylique de la p-D-chlorophenyl alanine dans 248 mL de dichlorométhane en présence de 8,8 g de N-Boc-pipéridone et de 14,4 g de triacétoxyborohydrure de sodium sous N₂. L'agitation est maintenue pendant 18h à température ambiante. Après addition de méthanol et évaporation à sec, le brut est repris avec solution aqueuse saturée d'hydrogénocarbonate de sodium, et on extrait à l'acétate d'éthyle jusqu'à épuisement de la phase aqueuse. Après séchage sur MgSO₄ et concentration à sec, on obtient 15,87 g de methyl *N*-[1-(*tert*-butoxycarbonyl)piperidin-4-yl]-4-chloro-D-phenylalaninate.

### 5.13 : N-[1-(tert-butoxycarbonyl)piperidin-4-yl]-4-chloro-D-phenylalanine

On solubilise 15,8 g de methyl *N*-[1-(*tert*-butoxycarbonyl)piperidin-4-yl]-4-chloro-D-phenylalaninate dans 200 mL d'un mélange tétrahydrofurane/eau (1/1) et on ajoute 3,35 g d'hydroxyde de lithium hydrate. L'agitation est maintenue 16h à température ambiante. On ajoute du sulfate de potassium jusqu'à pH 7. Le précipité obtenu est essoré et rincé au diéthyléther. Après séchage sur P₂O₅, on obtient 11,38 g de *N*-[1-(*tert-*butoxycarbonyl)piperidin-4-yl]-4-chloro-D-phenylalanine.

### 5.14 : tert-butyl 4-({(1R)-1-(4-chlorobenzyl)-2-[3-cyclohexyl-3-(1H-1,2,4-triazol-1-ylmethyl)-8-azabicyclo[3.2.1]oct-8-yl]-2-oxoethyl}amino)piperidine-1-carboxylate,

On solubilise 0,05 g de 3-cyclohexyl-3-(1*H* 1;2;4-triazol-1-ylmethyl)-8-azabicyclo [3.2.1]octane obtenu à l'étape 5.11 dans 2,4 mL de dichlorométhane en présence de 0,083 g de *N*-[1-(*tert*-butoxycarbonyl)piperidin-4-yl]-4-chloro-D-phenylalanine obtenu à l'étape 5.13; de 0,029 g d'hydroxybenzotriazole, de 0,041 g de chlorhydrate de 1-(3-diméthylaminopropyl)-3-éthyl-carbodiimide et de 0,09 mL de diisopropyléthylamine. Le mélange est agité 16h à température ambiante. Après. évaporation à sec, le résidu est repris avec une solution aqueuse de soude 1N et d'acétate d'éthyle. On extrait à l'acétate d'éthyle jusqu'à épuisement de la phase aqueuse. La phase organique est lavée avec H₂O, puis une solution saturée aqueuse de chlorure de sodium. Après séchage sur MgSO₄ et concentration à sec, le brut obtenu est chromatographié en éluant avec un gradient de méthanol dans le dichlorométhane variant de 0% à 10%. On obtient 0,066 g de *tert*-butyl 4-({(1*R*)-1-(4-chlorobenzyl)-2-[3-cyclohexyl-3-(1*H*-1,2,4-triazol-1-ylmethyl)-8-azabicyclo[3.2.1]oct-8-yl]-2-oxoethyl}amino)piperidine-1-carboxylate.

### 5.15: N-{(1R)-1-(4-chlorobenzyl)-2-[3-cyclohexyl-3-(1H-1,2,4-triazol-1-ylmethyl)-8-azabicyclo[3.2.1]oct-8-yl]-2-oxoethyl}piperidin-4-amine

On place 0,066 g de *tert*-butyl 4-({(1*R*)-1-(4-chlorobenzyl)-2-[3-cyclohexyl-3-(1*H-*1,2,4-triazol-1-ylmethyl)-8-azabicyclo[3.2.1]oct-8-yl]-2-oxoethyl}amino)piperidine-1-carboxylate dans 0,4 mL d'acide chlorhydrique 4N dans le dioxane. Le milieu réactionnel est agité pendant 4h à température ambiante. Après évaporation à sec, on reprend le résidu avec une solution aqueuse de soude 1N et à l'acétate d'éthyle. On extrait à l'acétate d'éthyle jusqu'à épuisement de la phase aqueuse. La phase organique est lavée avec H₂O puis une solution saturée aqueuse de chlorure de sodium. Après séchage sur MgSO₄ et concentration à sec, le brut obtenu est chromatographié en éluant avec un mélange de dichlorométhane et de méthanol 97,5/2,5, puis avec un mélange dichlorométhane/Méthanol/ammoniaque 9/1/0,1. On obtient 0,020 g de *N*-{(1*R*)-1-(4-chlorobenzyl)-2-[3-cyclohexyl-3-(1*H*-1,2,4-triazol-1-ylmethyl)-8-azabicyclo[3.2.1]oct-8-yl]-2-oxoethyl}piperidin-4-amine.

### 5.16: Chlorhydrate de N-{(1R)-1-(4-chlorobenzyl)-2-[3-cyclohexyl-3-(1H-1,2,4-triazol-1-ylmethyl)-8-azabicyclo[3.2.1]oct-8-yl]-2-oxoethyl}piperidin-4-amine

On place 0,02 g de *N*-{(1*R*)-1-(4-chlorobenzyl)-2-[3-cyclohexyl-3-(1*H*-1,2.4-triazol-1-ylmethyl)-8-azabicyclo[3.2.1]oct-8-yl]-2-oxoethyl}piperidin-4-amine dans 0.4 mL de dichlorométhane et on ajoute 0,74 mL d'acide chlorhydrique 0,1N dans l'isopropanol. Après concentration à sec, on reprend avec H₂O et on lyophilise la solution. On obtient 0,024 g de chlorhydrate de *N*-{(1*R*)-1-(4-chlorobenzyl)-2-[3-cyclohexyl-3-(1*H*-1,2,4-triazol-1-ylmethyl)-8-azabicyclo[3.2.1]oct-8-yl]-2-oxoethyl}piperidin-4-amine.

Point de fusion > 270°C; M+H⁺ = 540.

### Exemple 6: Chlorhydrate de 1-benzoyl-N-{(1R)-1-(4-chlorobenzyl)-2-[4-cyclohexyl-4-(1H-1,2,4-triazol-1-ylmethyl)piperidin-1-yl]-2-oxoethyl}piperidin-4-amine (composé n° 28)

### 6.1 : 1-benzoyl-N-{(1R)-1-(4-chlorobenzyl)-2-[4-cyclohexyl-4-(1H-1,2,4-triazol-1-ylmethyl)piperidin-1-yl]-2-oxoethyl}piperidin-4-amine

On solubilise 0,19g de (2*R*)-3-(4-chlorophenyl)-1-[4-cyclohexyl-4-(1*H*-1,2,4-triazol-1-ylmethyl)piperidin-1-yl]-1-oxopropan-2-amine obtenu à l'étape 1.7 dans 2,3 mL de dichlorométhane en présence de 0,12 g de 1-benzoylpiperidin-4-one. On additionne 0,22 g de triacétoxyborohydrure de sodium sous N₂. L'agitation est maintenue pendant 18h à température ambiante. Après hydrolyse avec une solution saturée aqueuse d'hydrogénocarbonate de sodium, on extrait au dichlorométhane jusqu'à épuisement de la phase aqueuse. La phase organique est lavée à l'eau puis avec une solution aqueuse saturée de chorure de sodium. Après séchage avec MgSO₄ et concentration à sec, le brut obtenu est chromatographié sur gel de silice en éluant avec un gradient de méthanol dans le dichlorométhane de 0% à 10%. On obtient 0,17g de 1-benzoyl-*N*-{(1*R*)-1-(4-chlorobenzyl)-2-[4-cydohexyl-4-(1*H*-1,2,4-triazol-1-ylmethyl) piperidin-1-yl]-2-oxoethyl}piperidin-4-amine.

### 6.2 : Chlorhydrate de 1-benzoyl-N-{(1R)-1-(4-chlorobenzyl)-2-[4-cyclohexyl-4-(1H-1,2,4-triazol-1-ylmethyl)piperidin-1-yl]-2-oxoethyl}piperidin-4-amine

On place 0,19 g de 1-benzoyl-*N*-{(1*R*)-1-(4-chlorobenzyl)-2-[4-cyclohexyl-4-(1*H* 1,2,4-triazol-1-ylmethyl)piperidin-1-yl]-2-oxoethyl}piperidin-4-amine dans 2 mL de méthanol et on ajoute 2,7 mL d'acide chlorhydrique 0,1N dans l'isopropanol. Après évaporation à sec, le milieu réactionnel est trituré puis on essore le précipité obtenu et on rince au diéthyléther. Le chlorhydrate ainsi obtenu est séché sur P₂O₅ sous pression réduite. On obtient 0,17 g du chlorhydrate de 1-benzoyl-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine sous forme d'un solide blanc.

Point de fusion > 200°C; M+H⁺ = 617, [α]_{D}²⁰ = +3,9 (0,331 g/100mL, MeOH).

### Exemple 7: Chlorhydrate de N-{(1R)-1-(4-chlorobenzyl)-2-[4-cyctohexyl-4-(1H-1,2,4-triazol-1-ylmethyl)piperidin-1-yl]-2-oxoethyl}-4-(1,3-dihydro-2H-isoindol-2-yl)cyclohexanamine (composé n° 56)

### 7.1: N-{(1R)-1-(4-chlorobenzyl)-2-[4-cyclohexyl-4-(1H-1,2,4-triazol-1-ylmethyl) piperidin-1-yl]-2-oxoethyl}-1,4-dioxaspiro[4.5]decan-8-amine

On solubilise 0,65g de (2*R*)-3-(4-chlorophenyl)-1-[4-cyclohexyl-4-(1*H*-1,2,4-triazol-1-ylmethyl)piperidin-1-yl]-1-oxopropan-2-amine obtenu à l'étape 1.7 dans 15 mL de dichlorométhane en présence de 0,3 g d'acétal de 1,4-cyclohexanedione monoéthylène. On additionne 0,63 g de triacétoxyborohydrure de sodium sous N₂. L'agitation est maintenue pendant 48h à température ambiante. Après hydrolyse avec une solution saturée aqueuse d'hydrogénocarbonate de sodium, on extrait au dichlorométhane jusqu'à épuisement de la phase aqueuse. La phase organique est lavée à l'eau puis avec une solution aqueuse saturée de chorure de sodium. Après séchage avec MgSO₄ et concentration à sec, le brut obtenu est chromatographié sur gel de silice en éluant avec un mélange de dichlorométhane et de méthanol 95/5. On obtient 0,85 g de *N*-{(1*R*)-1-(4-chlorobenzyl)-2-[4-cyclohexyl-4-(1*H*-1,2,4-triazol-1-ylmethyl)piperidin-1-yl]-2-oxoethyl}-1,4-dioxaspiro[4.5] decan-8-amine.

### 7.2 : 4-({(1R)-1-(4-chlorobenzyl)-2-[4-cyclohexyl-4-(1H-1,2,4-triazol-1-ylmethyl) piperidin-1-yl]-2-oxoethyl}amino)cyclohexanone

On solubilise 0,86 g de *N*-{(1*R*)-1-(4-chlorobenzyl)-2-[4-cyclohexyl-4-(1*H*-1,2,4-triazol-1-ylmethyl)piperidin-1-yl]-2-oxoethyl}-1,4-dioxaspiro[4.5]decan-8-amine dans 25 mL d'acide chlorhydrique aqueux 6N et on chauffe à 60°C pendant 18h. On ajoute alors 3 mL d'acide chlorhydrique aqueux 12N et on chauffe à 60°C pendant 24h. Après refroidissement du milieu réactionnel, on ajoute 150 mL de dichlorométhane et 50 mL d'H₂O Puis on additionne lentement du carbonate de potassium jusqu'à pH 10. On extrait au dichlorométhane jusqu'à épuisement de la phase aqueuse. Après séchage avec MgSO₄ et concentration à sec, on obtient 0,88 g de 4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanone qui est utilisé tel quel par la suite.

### 7.3 : N-{(1R)-1-(4-chlorobenzyl)-2-[4-cyclohexyl-4-(1H-1,2,4-triazol-1-ylmethyl) piperidin-1-yl]-2-oxoethyl}-4-(1,3-dihydro-2H-isoindol-2-yl)cyclohexanamine

On solubilise 0,35 g de 4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanone dans 6,7 mL de dichlorométhane en présence de 0,09 g d'isoindoline. On additionne 0,28 g de triacétoxyborohydrure de sodium sous N₂. L'agitation est maintenue pendant 18h à température ambiante. Après hydrolyse avec une solution aqueuse de soude 1N, on extrait au dichlorométhane jusqu'à épuisement de la phase aqueuse. Après séchage avec MgSO₄ et concentration à sec, le brut obtenu est chromatographié sur gel de silice en éluant avec un mélange de dichlorométhane et de méthanol 9/1. On obtient 0,2g de *N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(1,3-dihydro-2*H*-isoindol-2-yl)cyclohexanamine.

### 7.4: Chlorhydrate de N-{(1R)-1-(4-chlorobenzyl)-2-[4-cyclohexyl-4-(1H-1,2,4-triazol-1-ylmethyl)piperidin-1-yl]-2-oxoethyl}-4-(1,3-dihydro-2H-isoindol-2-yl) cyclohexanamine

On place 0,2 g de *N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(1,3-dihydro-2*H*-isoindol-2-yl)cyclohexanamine dans 5 mL de méthanol et on ajoute 3,18 mL d'acide chlorhydrique 0,1N dans l'isopropanol. Après évaporation à sec, le milieu réactionnel est trituré dans le diéthyléther puis on essore le précipité obtenu et on rince au diéthyléther. Le chlorhydrate ainsi obtenu est séché sur P₂O₅ sous pression réduite. On obtient 0,15 g du chlorhydrate de *N*-{(1*R*)-1-(4-chlorobenzyl)2-[4-cyclohexyl-4-(1*H*-1,2,4-triazol-1-ylmethyl)piperidin-1-yl]-2-oxoethyl}-4-(1,3-dihydro-2*H*-isoindol-2-yl)cyclohexanamine sous forme d'un solide blanc. Point de fusion = 215°C; M+H⁺ = 629.

### Exemple 8: Chlorhydrate de N-{(1R)-1-(4-chlorobenzyl)-2-[4-cyclohexyl-4-(1H-1,2,4-triazol-1-ylmethyl)piperidin-1-yl]-2-oxoethyl}-8-methyl-8-azabicyclo[3.2.1] octan-3-amine (composé n° 15)

### 8.1 :N-{(1R)-1-(4-chlorobenzyl)-2-[4-cyclohexyl-4-(1H-1,2,4-triazol-1-ylmethyl) piperidin-1-yl]-2-oxoethyl}-8-methyl-8-azabicyclo[3.2.1]octan-3-amine

On solubilise 0,30g de (2*R*)-3-(4-chlorophenyl)-1-[4-cyclohexyl-4-(1*H*-1,2,4-triazol-1-ylmethyl)piperidin-1-yl]-1-oxopropan-2-amine obtenue à l'étape 1.7 dans 3,2 mL de dichlorométhane en présence de 0,11 g de tropinone. On additionne ensuite 0,30 g de triacétoxyborohydrure de sodium sous N₂. L'agitation est maintenue pendant 18h à température ambiante. Après hydrolyse avec une solution aqueuse de soude 0,5N, on extrait à l'acétate d'éthyle jusqu'à épuisement de la phase aqueuse. La phase organique est lavée avec une solution aqueuse saturée de chlorure de sodium. Après séchage avec MgSO₄ et concentration à sec, le brut obtenu est chromatographié sur gel de silice en éluant avec un mélange de dichlorométhane/méthanol/ammoniaque variant de 95/5/0 à 90/10/0,1. On obtient 0,145 g de *N*-{(1*R*)-1-(4-chlorobenzyl)-2-[4-cyclohexyl-4-(1*H*-1,2,4-triazol-1-ylmethyl)piperidin-1-yl]-2-oxoethyl}-8-methyl-8-azabicyclo[3.2.1]octan-3-amine.

### 8.2: Chlorhydrate de N-{(1R)-1-(4-chlorobenzyl)-2-[4-cyclohexyl-4-(1H-1,2,4-triazol-1-ylmethyl)piperidin-1-yl]-2-oxoethyl}-8-methyl-8-azabicyclo[3.2.1]octan-3-amine

On place 0,145g de *N*-{(1*R*)-1-(4-chlorobenzyl)-2-[4-cyclohexyl-4-(1*H*-1,2,4-triazol-1-ylmethyl)piperidin-1-yl]-2-oxoethyl}-8-methyl-8-azabicyclo[3.2.1]octan-3-amine dans 2 mL de dichlorométhane et on ajoute 5,2 mL d'acide chlorhydrique 0,1 N dans l'isopropanol. Après concentration à sec, le milieu réactionnel est repris à l'acétate d'éthyle et trituré. Puis on essore le précipité obtenu et on rince à l'acétate d'éthyle. Le chlorhydrate ainsi obtenu est séché sur P₂O₅ sous pression réduite. On obtient 0,095 g du chlorhydrate de *N*-{(1*R*)-1-(4-chlorobenzyl)-2-[4-cyclohexyl-4-(1*H*-1,2,4-triazo1-1-ylmethyl)piperidin-1-yl]-2-oxoethyl}-8-methyl-8-azabicyclo[3.2.1]octan-3-amine.

Point de fusion = 262°C; M+H⁺ = 553.

### Exemple 9 : chlorhydrate de N-benzyl-4-({(1R)-1-(4-chlorobenzyl)-2-[4-cyclohexyl-4-(1H-1,2,4-triazol-1-ylmethyl)piperidin-1-yl]-2-oxoethyl}amino)-N-methylcyclohexanecarboxamide (composé n°78)

### 9.1 : N-benzyl-4-hydroxy-N-methylcyclohexanecarboxamide

On solubilise 2,0 g de l'acide carboxylique du 4-hydroxycyclohexane dans 69 mL de dichlorométhane en présence de 3,58 mL de N-methyl benzylamine, de 3,74 g d'hydroxybenzotriazole, 5,32 g de chlorhydrate de 1-(3-diméthylaminopropyl)-3-éthyl-carbodiimide et de 4,94 mL de diisopropyléthylamine. Le mélange est agité 16h à température ambiante. Après hydrolyse, on ajoute 14 mL d'une solution aqueuse d'acide chlorhydrique 1 N. On extrait au dichlorométhane jusqu'à épuisement de la phase aqueuse. La phase organique est lavée avec H₂O, puis une solution saturée aqueuse de chlorure de sodium. Après séchage sur MgSO₄ et concentration à sec, le brut obtenu est chromatographié en éluant avec un gradient de méthanol dans le dichlorométhane variant de 0% à 10%. On obtient 3,57 g de *N*-benzyl-4-hydroxy-*N-*methylcyclohexanecarboxamide (mélange de stéréoisomères cis et trans).

### 9.2: N-benzyl-N-methyl-4-oxocyclohexanecarboxamide

On solubilise 3,57 g de *N*-benzyl-4-hydroxy-*N*-methylcyclohexanecarboxamide dans 50 mL de diméthylsulfoxide en présence de 12,07 mL de triéthylamine. On ajoute alors goutte à goutte le complexe de trioxide de soufre-pyridine dissous dans 25 mL de diméthylsulfoxide de telle sorte que la température du milieu réactionnel ne dépasse pas 25°C. Après agitation 2h à température ambiante, le milieu est hydrolysé. Après extraction au dichlorométhane jusqu'à épuisement de la phase aqueuse, la phase organique est lavée deux fois avec une solution aqueuse d'acide chlorhydrique 1 N puis avec H₂O. Après séchage sur MgSO₄ et concentration à sec, on obtient 3,07 g de *N-*benzyl-*N*-methyl-4-oxocyclohexanecarboxamide qui est utilisé tel quel par la suite.

### 9.3 : N-benzyl-4-({(1R)-1-(4-chlorobenzyl)-2-[4-cyclohexyl-4-(1H-1,2,4-triazol-1-ylmethyl)piperidin-1-yl]-2-oxoethyl}amino)-N-methylcyclohexanecarboxamide

On solubilise 0,30g de (2*R*)-3-(4-chlorophenyl)-1-[4-cyclohexyl-4-(1*H*-1,2,4-triazol-1-ylmethyl)piperidin-1-yl]-1-oxopropan-2-amine obtenue à l'étape 1.7 dans 7 mL de dichlorométhane en présence de 0,26 g de *N*-benzyl-*N*-methyl-4-oxocyclohexanecarboxamide obtenu à l'étape 9.2. On additionne ensuite 0,30 g de triacétoxyborohydrure de sodium sous N₂. L'agitation est maintenue pendant 18h à température ambiante. Après hydrolyse avec une solution aqueuse saturée de carbonate de sodium, on extrait au dichlorométhane jusqu'à épuisement de la phase aqueuse. La phase organique est lavée avec H₂O puis avec une solution aqueuse saturée de chlorure de sodium. Après séchage sur MgSO₄ et concentration à sec, le brut obtenu est chromatographié sur gel de silice en éluant avec un mélange de dichlorométhane/acétone/méthanol variant de 100/0/0 à 75/25/5. On obtient 0,36 g et 0,090 g de *N*-benzyl-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-methylcyclohexanecarboxamide, stéréoisomères (R, cis) et (R, trans) de configuration non déterminée.

### 9.4: chlorhydrate de N-benzyl-4-({(1R)-1-(4-chlorobenzyl)-2-[4-cyclohexyl-4-(1H-1,2,4-triazol-1-ylmethyl)piperidin-1-yl]-2-oxoethyl}amino)-N methylcyclohexanecarboxamide

On place 0,36 g de *N*-benzyl-4-({(1*R*)-1-(4-chlorobenzyl)-2-[4-cyclohexyl-4-(1*H-*1,2,4-triazol-1-ylmethyl)piperidin-1-yl]-2-oxoethyl}amino)-*N* methylcyclohexanecarboxamide, stéréoisomère pur (R, cis) ou (R, trans), dans 2 mL de dichlorométhane et on ajoute 5,2 mL d'acide chlorhydrique 0,1N dans l'isopropanol. Après concentration à sec, le milieu réactionnel est repris à l'acétate d'éthyle et trituré. Puis on essore le précipité obtenu et on rince à l'acétate d'éthyle. Le chlorhydrate ainsi obtenu est séché sur P₂O₅ sous pression réduite. On obtient 0,095 g du chlorhydrate de *N*-benzyl-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-methylcyclohexanecarboxamide, stéréoisomère pur (R, cis) ou (R, trans).

Point de fusion = 160°C; M+H⁺= 663 ; [α]_{D}²⁰ = +7,1 (0,3525 g/100mL, DMSO).

### Exemple 10 : Chlorhydrate de N-{(1R)-1-(4-chlorobenzyl)-2-[4-cyclohexyl-4-(1H-1,2,4-triazol-1-ylmethyl)piperidin-1-yl]-2-oxoethyl}-4-(3-methyl-1,2,4-oxadiazol-5-yl)cyclohexanamine (composé n°79)

### 10.1 : 4-oxocyclohexanecarboxylic acid

On solubilise 8,5 g de carboxylate d'éthyle du 4-oxocyclohexane dans 68 mL de méthanol et 45 mL de H₂O. On ajoute à 0°C 3,56 g d'hydroxyde de lithium hydrate. Après agitation à température ambiante pendant 4g, le milieu réactionnel est acidifié à pH2 avec une solution aqueuse d'acide chlorhydrique 3N. Le méthanol est évaporé et on extrait à l'acétate d'éthyle jusqu'à épuisement de la phase aqueuse. Après séchage sur MgSO₄, et concentration à sec, on obtient 6,7 g de 4-oxocyclohexanecarboxylic acid.

### 10.2 : N-{[(4-oxocyclohexyl)carbonyl]oxy}ethanimidamide

On solubilise 2,0 g de 4-oxocyclohexanecarboxylic acid dans 70 mL de dichlorométhane en présence de 1,15 g de *N*-hydroxyethanimidamide, 1,90 g d'hydroxybenzotriazole, et de 1,95 g de diisopropyl carbodiimide. Le mélange est agité 16h à température ambiante. Après hydrolyse, on ajoute une solution aqueuse de soude 1 N jusqu'à pH 12. On extrait au dichlorométhane jusqu'à épuisement de la phase aqueuse. Après séchage sur MgSO₄ et concentration à sec, le brut obtenu est repris dans 10 mL d'acétate d'éthyle. La diisopropyle urée est filtrée, et le filtrat est concentré. On obtient 1,38 g de *N*-{[(4-oxocyclohexyl)carbonyl]oxy}ethanimidamide.

### 10.3: 4-(3-methyl-1,2,4-oxadiazol-5-yl)cyclohexanone

On solubilise 1,38 g de *N*-{[(4-oxocyclohexyl)carbonyl]oxy}ethanimidamide dans 58 mL d'éthanol et 22 mL d'H₂O. On ajoute 1,37 g d'acétate de sodium. Le milieu réactionnel est chauffé à 90°C pendant 18h. Après refroidissement jusqu'à température ambiante, l'éthanol est évaporé. On extrait au dichlorométhane jusqu'à épuisement de la phase aqueuse. Après séchage sur MgSO₄ et concentration à sec, le brut obtenu est chromatographié sur gel de silice en éluant avec un gradient de méthanol dans le dichlorométhane variant de 0% à 2%. On obtient 0,5 g de 4-(3-methyl-1,2,4-oxadiazol-5-yl)cyclohexanone.

### 10.4 : N-{(1R)-1-(4-chlorobenzyl)-2-[4-cyclohexyl-4-(1H-1,2,4-triazol-1-ylmethyl)piperidin-1-yl]-2-oxoethyl}-4-(3-methyl-1,2,4-oxadiazol-5-yl)cyclohexanamine

On solubilise 0,30 g de (2*R*)-3-(4-chlorophenyl)-1-[4-cyclohexyl-4-(1H-1,2,4-triazol-1-ylmethyl)piperidin-1-yl]-1-oxopropan-2-amine obtenue à l'étape 1.7 dans 7 mL de dichlorométhane en présence de 0,19g de 4-(3-methyl-1,2,4-oxadiazol-5-yl)cyclohexanone obtenu à l'étape 10.3. On additionne ensuite 0,30g de triacétoxyborohydrure de sodium sous N₂. L'agitation est maintenue pendant 18h à température ambiante. Après hydrolyse avec une solution aqueuse saturée d'hydrogénocarbonate de sodium, on extrait au dichlorométhane jusqu'à épuisement de la phase aqueuse. La phase organique est lavée avec H₂O puis avec une solution aqueuse saturée de chlorure de sodium. Après séchage sur MgSO₄ et concentration à sec, le brut obtenu est chromatographié sur gel de silice en éluant avec un mélange de dichlorométhane/acétone/méthanol variant de 100/0/0 à 70/25/5. On obtient 0,26 g et 0,11 g de *N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(3-methyl-1,2,4-oxadiazol-5-yl)cyclohexanamine, stéréoisomères (R, cis) et (R, trans) de configuration non déterminée.

### 10.5 : chlorhydrate de N-{(1R)-1-(4-chlorobenzyl)-2-[4-cyclohexyl-4-(1H-1,2,4-triazol-1-ylmethyl)piperidin-1-yl]-2-oxoethyl}-4-(3-methyl-1,2,4-oxadiazol-5-yl)cyclohexanamine

On place 0,26 g de *N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(3-methyl-1,2,4-oxadiazol-5-yl)cyclohexanamine, stéréoisomère pur (R,cis) ou (R,trans) dans 2 mL de méthanol et on ajoute 4,32 mL d'acide chlorhydrique 0,1 N dans l'isopropanol. Après concentration à sec, le milieu réactionnel est repris au diéthyléther et trituré. Puis on essore le précipité obtenu et on rince au diéthyléther. Le chlorhydrate ainsi obtenu est séché sur P₂O₅ sous pression réduite. On obtient 0,27 g du chlorhydrate de *N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(3-methyl-1,2,4-oxadiazol-5-yl)cyclohexanamine, stéréoisomère pur (R,cis) ou (R,trans).

Point de fusion >200°C; M+H⁺= 598 ; [α]_{D}²⁰ = +10,4 (0,5345 g/100mL, DMSO).

### Exemple 11 : Chlorhydrate de 4-({(1R)-1-(4-chlorobenzyl)-2-[4-cyclohexyl-4-(1H-1,2,4-triazol-1-ylmethyl)piperidin-1-yl]-2-oxoethyl}amino)-1-(4-fluorophenyl)cyclohexanol (composé n°82)

### 11.1 : 4-(4-fluorophenyl)-4-hyqroxycyclohexanone

On place 10,7 mL de n-butyllithium 2,5N dans 5 mL de diéthyléther anhydre à - 35°C. Puis on ajoute 2,94 mL de 4-bromofluorobenzène de telle sorte que la température ne dépasse pas -30°C. Après agitation 10 min à -10°C, on ajoute lentement cette suspension à 3,0 g de 1,4-cyclohexanedione dans 60 mL de tétrahydrofurane placé à - 78°C. L'agitation du milieu à -78°C est maintenue 1h. Après hydrolyse avec une solution aqueuse saturée de chlorure d'ammonium, on extrait la phase aqueuse à l'acétate d'éthyle jusqu'à épuisement de la phase aqueuse. La phase organique est lavée avec une solution aqueuse saturée de chlorure de sodium. Après séchage sur MgSO₄ et concentration à sec, le brut obtenu est chromatographié sur gel de silice en éluant avec un mélange de cyclohexane/acétate d'éthyle variant de 8/2 à 6/4. On obtient 0,77 g de 4-(4-fluorophenyl)-4-hydroxycyclohexanone.

### 11.2 : 4-({(1R)-1-(4-chlorobenzyl)-2-[4-cyclohexyl-4-(1H-1,2,4-triazol-1-ylmethyl)piperidin-1-yl]-2-oxoethyl}amino)-1-(4-fluorophenyl)cyclohexanol

On solubilise 0,20g de (2*R*)-3-(4-chlorophenyl)-1-[4-cyclohexyl-4-(1*H*-1,2,4-triazol-1-ylmethyl)piperidin-1-yl]-1-oxopropan-2-amine obtenue à l'étape 1.7 dans 4,7 mL de dichlorométhane en présence de 0,145 g de 4-(4-fluorophenyl)-4-hydroxycyclohexanone obtenue à l'étape 11.1. On additionne ensuite 0,25g de triacétoxyborohydrure de sodium sous N₂. L'agitation est maintenue pendant 18h à température ambiante. Après hydrolyse avec une solution aqueuse saturée d'hydrogénocarbonate de sodium, on extrait au dichlorométhane jusqu'à épuisement de la phase aqueuse. La phase organique est lavée avec H₂O. Après séchage sur MgSO₄ et concentration à sec, le brut obtenu est chromatographié sur gel de silice en éluant avec un mélange de dichlorométhane/acétone/méthanol variant de 100/0/0 à 70/25/5. On obtient 0,10 g et 0,15 g de 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-1-(4-fluorophenyl)cyclohexanol, stéréoisomères (R, cis) et (R, trans) de configuration non déterminée.

### 11.3: chlorhydrate de 4-({(1R)-1-(4-chlorobenzyl)-2-[4-cyclohexyl-4-(1H-1,2,4-triazol-1-ylmethyl)piperidin-1-yl]-2-oxoethyl}amino)-1-(4-fluorophenyl)cyclohexanol

On place 0,10 g de 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-1-(4-fluorophenyl)cyclohexanol, stéréoisomère pur (R,cis) ou (R, trans) dans 2 mL de méthanol et on ajoute 4,32 mL d'acide chlorhydrique 0,1 N dans l'isopropanol. Après concentration à sec, le milieu réactionnel est repris au diéthyléther et trituré. Puis on essore le précipité obtenu et on rince au diéthyléther. Le chlorhydrate ainsi obtenu est séché sur P₂O₅ sous pression réduite. On obtient 0,1 g du chlorhydrate de 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-1-(4-fluorophenyl)cyclohexanol, stéréoisomère pur (R,cis) ou (R, trans).

Point de fusion = 150°C; M+H⁺ = 625

### Exemple 12 : Chlorhydrate de 4-({(1R)-1-(4-chlorobenzyl)-2-[4-cyclohexyl-4-(1H-1,2,4-triazol-1-ylméthyl)piperidin-1-yl]-2-oxoethyl}amino)-N-phenylpiperidine-1-carboxamide (composé n°90)

### 12.1 : 4-oxo-N-phenylpiperidine-1-carboxamide

On place 0,91 mL de phénylisocyanate dans 42 mL de dichlorométhane. Puis on ajoute 1,36 g de pipéridin-4-one et 2,32 g de carbonate de potassium. Après agitation 18h à température ambiante, on hydrolyse le milieu réactionnel et on extrait au dichlorométhane jusqu'à épuisement de la phase aqueuse. La phase organique est lavée avec une solution aqueuse d'acide chlorhydrique 1N. Après séchage sur MgSO₄ et concentration à sec, le brut obtenu est chromatographié sur gel de silice en éluant avec un gradient de méthanol dans le dichlorométhane variant de 0% à 1%. On obtient 1,85 g de 4-oxo-*N*-phenylpiperidine-1-carboxamide sous la forme d'un solide blanc.

### 12.2 : 4-({(1R)-1-(4-chlorobenzyl)-2-[4-cyclohexyl-4-(1H-1,2,4-triazol-1-ylmethyl)piperidin-1-yl]-2-oxoethyl}amino)-N-phenylpiperidine-1-carboxamide

On solubilise 0,25g de (2*R*)-3-(4-chlorophenyl)-1-[4-cyclohexyl-4-(1*H*-1,2,4-triazol-1-ylmethyl)piperidin-1-yl]-1-oxopropan-2-amine obtenue à l'étape 1.7 dans 2,9 mL de dichlorométhane en présence de 0,13 g de 4-oxo-*N*-phenylpiperidine-1-carboxamide obtenu à l'étape 12.1. On additionne ensuite 0,16 g de triacétoxyborohydrure de sodium sous N₂. L'agitation est maintenue pendant 18h à température ambiante. On ajoute alors 0,013 g de et 0,016 g de triacétoxyborohydrure de sodium. L'agitation set maintenue 24h. Après hydrolyse avec une solution aqueuse d'hydroxyde de sodium 1N, on extrait au dichlorométhane jusqu'à épuisement de la phase aqueuse. Après séchage sur MgSO₄ et concentration à sec, le brut obtenu est chromatographié sur gel de silice en éluant avec un mélange de dichlorométhane/acétate d'éthyle/méthanol/ammoniaque variant de 95/5/01/0 à 85/15/3/0.3. On obtient 0,35 g de 4-({(1*R*)-1-(4-chlorobenzyl)-2-[4-cyclohexyl-4-(1*H*-1,2,4-triazo)-1-ylmethyl)piperidin-1-yl]-2-oxoethyl}amino)-*N-*phenylpiperidine-1-carboxamide.

### 12.3: chlorhydrate de 4-({(1R)-1-(4-chlorobenzyl)-2-[4-cyclohexyl-4-(1H-1,2,4-triazol-1-ylmethyl)piperidin-1-yl]-2-oxoethyl}amino)-N-phenylpiperidine-1-carboxamide

On place 0,35 g de 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-phenylpiperidine-1-carboxamide dans 2 mL d'acétate d'éthyle et on ajoute 0,32 mL d'acide chlorhydrique 2N dans le diéthyl éther. Après concentration à sec, le milieu réactionnel est repris à l'acétate d'éthyle et trituré. Puis on essore le précipité obtenu et on rince à l'acétate d'éthyle. Le chlorhydrate ainsi obtenu est séché sur P₂O₅ sous pression réduite. On obtient 0,31 g du chlorhydrate de 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-phenylpiperidine-1-carboxamide.

Point de fusion = 198°C; M+H⁺ = 635 ; [α]_{D}²⁰ = +11,4 (0,861g/100mL, DMSO).

### Exemple 13 : Chlorhydrate de 3-[4-({(1R)-1-(4-chlorobenzyl)-2-[4-cyclohexyl-4-(1H-1,2,4-triazol-1-ylmethyl)piperidin-1-yl]-2-oxoethyl}amino)cyclohexyl]-1,3-oxazolidin-2-one (composé n°103)

### 13.1 : 2-(1,4-dioxaspiro[4.5]dec-8-ylamino)ethanol

On solubilise 3,12 g de 1,4-dioxaspiro[4.5]decan-8-one dans 80 mL de dichlorométhane en présence de 1,16 g d'éthanol amine. On additionne ensuite 6,75 g de triacétoxyborohydrure de sodium sous N₂. L'agitation est maintenue pendant 18h à température ambiante. Après hydrolyse avec une solution aqueuse de soude 1N, on extrait au dichlorométhane jusqu'à épuisement de la phase aqueuse. Après séchage sur MgSO₄ et concentration à sec, on obtient 4,0 g de 2-(1,4-dioxaspiro[4.5]dec-8-ylamino)ethanol utilisé tel quel par la suite.

### 13.2: 3-(1,4-dioxaspiro[4.5]dec-8-yl)-1,3-oxazolidin-2-one

On place 1,47 g de disphosgène dans 50 mL de dichlorométhane sous N₂ et à 0°C. On ajoute goutte à goutte 1,0 g de 2-(1,4-dioxaspiro[4.5]dec-8-ylamino)ethanol obtenu à l'étape 13.1 en mélange avec 3,59 mL de triéthylamine. L'agitation est maintenue 5h à température ambiante. Après évaporation à sec, le brut obtenu est repris au dichlorométhane. La phase organique est lavée deux fois avec une solution aqueuse d'acide chlorhydrique 1N, puis avec H₂O et une solution aqueuse saturée de chlorure de sodium. Après séchage sur MgSO₄ et concentration à sec, le brut obtenu est chromatographié sur gel de silice en éluant avec un gradient de méthanol dans le dichlorométhane variant de 0% à 2%. On obtient 1,19 g de 3-(1,4-dioxaspiro[4.5]dec-8-yl)-1,3-oxazolidin-2-one.

### 13.3 : 3-(4-oxocyclohexyl)-1,3-oxazolidin-2-one

On solubilise 0,75 g de 3-(1,4-dioxaspiro[4.5]dec-8-yl)-1,3-oxazolidin-2-one dans 27,5 mL d'HCl 6N. Le milieu réactionnel est chauffé à 65°C pendant 5h. Après retour à température ambiante, on ajoute lentement du carbonate de sodium jusqu'à pH 9. On extrait au dichlorométhane jusqu'à épuisement de la phase aqueuse. La phase organique est lavée avec H₂O. Après séchage sur MgSO₄, le brut obtenu est chromatographié sur gel de silice en éluant avec un gradient de méthanol dans le dichlorométhane de 0% à 10% On obtient 0,11g de 3-(4-oxocyclohexyl)-1,3-oxazolidin-2-one.

### 13.4 : 3-[4-({(1R)-1-(4-chlorobenzyl)-2-[4-cyclohexyl-4-(1H-1,2,4-triazol-1-ylmethyl)piperidin-1-yl]-2-oxoethyl}amino)cyclohexyl]-1,3-oxazolidin-2-one

On solubilise 0,26 g de (2*R*)-3-(4-chlorophenyl)-1-[4-cyclohexyl-4-(1*H*-1,2,4-triazol-1-ylmethyl)piperidin-1-yl]-1-oxopropan-2-amine obtenue à l'étape 1.7 dans 6 mL de dichlorométhane en présence de 0,12 g de 3-(4-oxocyclohexyl)-1,3-oxazolidin-2-one obtenu à l'étape 13.3. On additionne ensuite 0,17g de triacétoxyborohydrure de sodium sous N₂. L'agitation est maintenue pendant 18h à température ambiante. Après hydrolyse avec une solution aqueuse saturée d'hydrogénocarbonate de sodium, on extrait au dichlorométhane jusqu'à épuisement de la phase aqueuse. La phase organique est lavée avec H₂O puis avec une solution aqueuse saturée de chlorure de sodium. Après séchage sur MgSO₄ et concentration à sec, le brut obtenu est chromatographié sur gel de silice en éluant avec un mélange de dichlorométhane/acétone/méthanol variant de 100/0/0 à 70/25/5. On obtient 0,18 g et 0,16 g de 3-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-1,3-oxazolidin-2-one, stéréoisomères (R, cis) et (R, trans) de configuration non déterminée.

### 13.5: chlorhydrate de 3-[4-({(1R)-1-(4-chlorobenzyl)-2-[4-cyclohexyl-4-(1H-1,2,4-triazol-1-ylmethyl)piperidin-1-yl]-2-oxoethyl}amino)cyclohexyl]-1,3-oxazolidin-2-one

On place 0,18 g de 3-(4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-1,3-oxazolidin-2-one, stéréoisomère pur (R,cis) ou (R,trans), dans 2 mL de méthanol et on ajoute 3,0 mL d'acide chlorhydrique 0,1 N dans l'isopropanol. Après concentration à sec, le milieu réactionnel est repris au diéthyléther et trituré. Puis on essore le précipité obtenu et on rince au diéthyléther. Le chlorhydrate ainsi obtenu est séché sur P₂O₅ sous pression réduite. On obtient 0,17 g du chlorhydrate de 3-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-1,3-oxazolidin-2-one, stéréoisomère pur (R,cis) ou (R,trans).

Point de fusion = 163°C; M+H⁺ = 598 ; [α]_{D}²⁰ = +12,4 (0,899g/100mL, DMSO).

### Exemple 14 : Chlorhydrate de 1-[4-({(1R)-1-(4-chlorobenzyl)-2-[4-cyclohexyl-4-(1H-1,2,4-triazol-1-ylmethyl)piperidin-1-yl]-2-oxoethyl}amino)cyclohexyl]pyrrolidin-2-one (composé n°106)

### 14.1: 1-(1,4-dioxaspiro[4.5]dec-8-yl)pyrrolidin-2-one

On solubilise 1,8 g de 1,4-dioxaspiro[4.5]decan-8-one dans 100 mL de dichlorométhane en présence de 3,02 g du carboxylate d'éthyle de l'acide 4-aminobutyrique. On additionne ensuite 3,54 g de triacétoxyborohydrure de sodium et 6,96 mL de triéthylamine sous N₂. L'agitation est maintenue pendant 18h à température ambiante. On obtient 1,5 g de 1-(1,4-dioxaspiro[4.5]dec-8-yl)pyrrolidin-2-one.

### 14.2: 1-(4-oxocyclohexyl)pyrrolidin-2-one

On place 1,5 g de 1-(1,4-dioxaspiro[4.5]dec-8-yl)pyrrolidin-2-one dans 22 mL d'acide chlorhydrique 6N. Le milieu réactionnel est agité 18h à température ambiante, puis il est hydrolysé avec une solution aqueuse de soude 1 N. On extrait au dichlorométhane jusqu'à épuisement de la phase aqueuse. Après séchage sur MgSO₄ et concentration à sec, on obtient 0,45 g de 1-(4-oxocyclohexyl)pyrrolidin-2-one qui est utilisé tel quel par la suite.

### 14.3 : 1-[4-({(1R)-1-(4-chlorobenzyl)-2-[4-cyclohexyl-4-(1H-1,2,4-triazol-1-ylmethyl)piperidin-1-yl]-2-oxoethyl}amino)cyclohexyl]pyrrolidin-2-one

On solubilise 0,34g de (2*R*)-3-(4-chlorophenyl)-1-[4-cyclohexyl-4-(1*H*-1,2,4-triazol-1-ylmethyl)piperidin-1-yl]-1-oxopropan-2-amine obtenue à l'étape 1.7 dans 8 mL de dichlorométhane en présence de 0,17 g de 1-(4-oxocyclohexyl)pyrrolidin-2-one obtenu à l'étape 14.2. On additionne ensuite 0,25 g de triacétoxyborohydrure de sodium sous N₂. L'agitation est maintenue pendant 18h à température ambiante. Après hydrolyse avec une solution aqueuse saturée d'hydrogénocarbonate de sodium, on extrait au dichlorométhane jusqu'à épuisement de la phase aqueuse. La phase organique est lavée avec H₂O puis avec une solution aqueuse saturée de chlorure de sodium. Après séchage sur MgSO₄ et concentration à sec, le brut obtenu est chromatographié sur gel de silice en éluant avec un mélange de dichlorométhane/acétone/méthanol variant de 100/0/0 à 70/25/5. On obtient 0,25 g et 0,21 g de 1-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyljpyrrolidin-2-one, stéréoisomères (R, cis) ou (R, trans) de configuration non déterminée.

### 14.4: chlorhydrate de 1-[4-({(1R)-1-(4-chlorobenzyl)-2-[4-cyclohexyl-4-(1H-1,2,4-triazol-1-ylmethyl)piperidin-1-yl]-2-oxoethyl}amino)cyclohexyl]pyrrolidin-2-one

On place 0,25 g de 1-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]pyrrolidin-2-one, stéréoisomère pur (R,cis) ou (R,trans) dans 2 mL d'acétate d'éthyle et on ajoute 0,21 mL d'acide chlorhydrique 2N dans le diéthyl éther. Après concentration à sec, le milieu réactionnel est repris à l'acétate d'éthyle et trituré. Puis on essore le précipité obtenu et on rince à l'acétate d'éthyle. Le chlorhydrate ainsi obtenu est séché sur P₂O₅ sous pression réduite. On obtient 0,24 g du chlorhydrate de 1-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]pyrrolidin-2-one, stéréoisomère pur (R,cis) ou (R,trans).

Point de fusion >200°C; M+H⁺ = 595 ; [α]_{D}²⁰ = +12,0 (0,901g/100mL, DMSO).

### Exemple 15 : Chlorhydrate de N-{(1R)-1-(4-chlorobenzyl)-2-[4-cyclohexyl-4-(1H-1,2,4-triazol-1-ylmethyl)piperidin-1-yl]-2-oxoethyl}-4-(2-methoxyethoxy)cyclohexanamine (exemple 107)

### 15.1 : 8-(2-methoxyethoxy)-1,4-dioxaspiro[4.5]decane

On place 1,32 g de 4-hydroxycyclohexanone dans 17 mL de diméthylformamide anhydre sous N₂. On ajoute 0,40 g d'hydrure de sodium. Le milieu réactionnel est agité à température ambiante pendant 1h. On ajoute alors 1,57 mL de 2-bromo méthyl éther. Le milieu réactionnel est agité à température ambiante pendant 18h. On rajoute alors 0,2 g d'hydrure de sodium et 0,78 mL de 2-bromo méthyl éther. Le milieu réactionnel est agité à température ambiante pendant 24h. le milieu réactionnel est alors versé sur de la glace. On extrait à l'acétate d'éthyle jusqu'à épuisement de la phase aqueuse. La phase organique est lavée avec H₂O. Après séchage sur MgSO₄ et concentration à sec, le brut obtenu est chromatographié sur gel de silice en éluant avec un gradient de méthanol dans le dichlorométhane variant de 0% à 2%. On obtient 0,77 g de 8-(2-methoxyethoxy)-1,4-dioxaspiro[4.5]decane.

### 15.2: 4-(2-methoxyethoxy)cyclohexanone

On.place 0,77 g de 8-(2-methoxyethoxy)-1,4-dioxaspiro[4.5]decane dans 11,9 mL d'acide chlorhydrique 6N et on chauffe à 60°C pendant 24h. On ajoute alors 4 mL d'acide chlorhydrique 12N et on poursuit le chauffage pendant 24h. On refroidit le milieu réactionnel à 0°C et on ajoute du carbonate de sodium. On extrait la phase aqueuse au dichlorométhane jusqu'à épuisement de la phase aqueuse. La phase organique est lavée avec H₂O. Après séchage sur Na₂SO₄ et concentration à sec, le brut obtenu est chromatographié sur gel de silice en éluant avec un gradient de méthanol dans le dichlorométhane variant de 0% à 2%. On obtient 0,38 g de 4-(2-methoxyethoxy)cyclohexanone.

### 15.3 : N-{(1R)-1-(4-chlorobenzyl)-2-[4-cyclohexyl-4-(1H-1,2,4-triazol-1-ylmethyl)piperidin-1-yl]-2-oxoethyl}-4-(2-methoxyethoxy)cyclohexanamine

On solubilise 0,5 g de (2*R*)-3-(4-chlorophenyl)-1-[4-cyclohexyl-4-(1*H*-1,2,4-triazol-1-ylmethyl)piperidin-1-yl]-1-oxopropan-2-amine obtenue à l'étape 1.7 dans 11,6 mL de dichlorométhane en présence de 0,24 g de 4-(2-methoxyethoxy)cyclohexanone obtenu à l'étape 15.2. On additionne ensuite 0,37 g de triacétoxyborohydrure de sodium sous N₂. L'agitation est maintenue pendant 18h à température ambiante. Après hydrolyse, on extrait au dichlorométhane jusqu'à épuisement de la phase aqueuse. La phase organique est lavée avec H₂O puis avec une solution aqueuse saturée de chlorure de sodium. Après séchage sur MgSO₄ et concentration à sec, le brut obtenu est chromatographié sur gel de silice en éluant avec un mélange de dichlorométhane/acétone/méthanol variant de 100/0/0 à 70/25/5. On obtient 0,53 g de *N-*{(1*R*)-1-(4-chlorobenzyl)-2-[4-cyclohexyl-4-(1*H*-1,2,4-triazol-1-ylmethyl)piperidin-1-yl]-2-oxoethyl}-4-(2-methoxyethoxy)cyclohexanamine, mélange de stéréoisomères (R, cis) et (R, trans).

### 15.4: chlorhydrate de N-{(1R)-1-(4-chlorobenzyl)-2-[4-cyclohexyl-4-(1H-1,2,4-triazol-1-ylmethyl)piperidin-1-yl]-2-oxoethyl}-4-(2-methoxyethoxy)cyclohexanamine

On place 0,53 g de *N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(2-methoxyethoxy)cyclohexanamine dans 2 mL d'acétate d'éthyle et on ajoute 0,21 mL d'acide chlorhydrique 0,5N dans le diéthyl éther. Après concentration à sec, le milieu réactionnel est repris à l'acétate d'éthyle et trituré. Puis on essore le précipité obtenu et on rince à l'acétate d'éthyle. Le chlorhydrate ainsi obtenu est séché sur P₂O₅ sous pression réduite. On obtient 0,54 g du chlorhydrate de *N*-{(1*R*)-{4-chlorobenzyl)-2-[4-cyclohexyl-4-(1*H*-1,2,4-triazol-1-ylmethyl)piperidin-1-yl]-2-oxoethyl}-4-(2-methoxyethoxy)cyclohexanamine, mélange de stéréoisomères (R, cis) et (R, trans).

Point de fusion = 257°C; M+H⁺ = 586 ;

### Exemple 16: Chlorhydrate de N-{(1R)-1-(4-chlorobenzyl)-2-[4-cyclohexyl-4-(1H-1,2,4-triazol-1-ylmethyl)piperidin-1-yl]-2-oxoethyl}-1-[(2S)-piperidin-2-ylcarbonyl]piperidin-4-amine (composé n°110)

### 16.1: tert-butyl (2S)-2-[(4-oxopiperidin-1-yl)carbonyl]piperidine-1-carbaxylate

On place 0,68 g de pipéridin-4-one dans 51 mL de dichlorométhane en présence de 1,15 g de (2*S*)-1-(*tert*-butoxycarbonyl)piperidine-2-carboxylic acid, de 0,68g d'hydroxybenzotriazole, de 0,97 g de chlorhydrate de 1-(3-diméthylaminopropyl)-3-éthyl-carbodiimide et de 1,79 mL de diisopropyléthylamine. Le mélange est agité 18h à température ambiante. Après évaporation à sec et hydrolyse, on extrait au dichlorométhane jusqu'à épuisement de la phase aqueuse. La phase organique est lavée avec H₂O, puis avec une solution aqueuse saturée de chlorure de sodium. Après séchage sur MgSO₄ et concentration à sec, le brut obtenu est chromatographié en éluant avec un gradient de méthanol dans le dichlorométhane variant de 0% à 10%. On obtient 1,56 g de *tert*-butyl (2*S*)-2-[(4-oxopiperidin-1-yl)carbonyl]piperidine-1-carboxylate.

### 16.2: tert-butyl (2S)-2-{[4-({(1R)-1-(4-chlorobenzyl)-2-[4-cyclohexyl-4-(1H-1,2,4-triazol-1-ylmethyl)piperidin-1-yl]-2-oxoethyl}amino)piperidin-1-yl]carbonyl}piperidine-1-carboxylate

On solubilise 0,3 g de (2*R*)-3-(4-chlorophenyl)-1-[4-cyclohexyl-4-(1*H*-1,2,4-triazol-1-ylmethyl)piperidin-1-yl]-1-oxopropan-2-amine obtenue à l'étape 1.7 dans 7 mL de dichlorométhane en présence de 0,48 g de *tert*-butyl (2*S*)-2-[(4-oxopiperidin-1-yl)carbonyl]piperidine-1-carboxylate obtenu à l'étape 16.1. On additionne ensuite 0,22 g de triacétoxyborohydrure de sodium sous N₂. L'agitation est maintenue pendant 18h à température ambiante. Après un ajout de 0,3 g de (2*R*)-3-(4-chlorophenyl)-1-[4-cyclohexyl-4-(1*H*-1,2,4-triazol-1-ylmethyl)piperidin-1-yl]-l-oxopropan-2-amine et 0,47 g de triacétoxyborohydrure de sodium, le milieu réactionnel est agité 24h. Après hydrolyse avec une solution aqueuse saturée d'hydrogénocarbonate de sodium, on extrait au dichlorométhane jusqu'à épuisement de la phase aqueuse. La phase organique est lavée avec H₂O puis avec une solution aqueuse saturée de chlorure de sodium. Après séchage sur MgSO₄ et concentration à sec, le brut obtenu est chromatographié sur gel de silice en éluant avec un gradient de méthanol dans le dichlorométhane variant de 0% à 10%. On obtient 0,39g de *tert*-butyl (2*S*)-2-{[4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidin-1-yl]carbonyl}piperidine-1-carboxylate.

### 16.3: N-{(1R)-1-(4-chlorobenzyl)-2-[4-cyclohexyl-4-(1H-1,2,4-triazol-1-ylmethyl)piperidin-1-yl]-2-oxoethyl}-1-[(2S)-piperidin-2-ylcarbonyl]piperidin-4-amine

On solubilise 0,39 g de *tert*-butyl (2*S*)-2-{[4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidin-1-yl]carbonyl}piperidine-1-carboxylate dans 1 mL de dioxane. On ajoute 1,35 mL d'acide chlorhydrique 4N dans le dioxane. Le milieu réactionnel est agité 18h à température ambiante. Après évaporation à sec, le résidu est repris au dichlorométhane. On ajoute une solution aqueuse saturée d'hydrogénocarbonate de sodium. On extrait au dichlorométhane jusqu'à épuisement de la phase aqueuse. La phase organique est lavée avec H₂O, puis avec une solution saturée aqueuse de chlorure de sodium. Après séchage sur MgSO₄ et concentration à sec, le brut obtenu est chromatographié en éluant avec un mélange dichlorométhane/méthanol/ammoniaque variant de 100/0/0 à 90/10/1. On obtient 0,30 g de *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(2*S*)-piperidin-2-ylcarbonyl]piperidin-4-amine.

### 16.4: N-{(1R)-1-(4-chlorobenzyl)-2-[4-cyclohexyl-4-(1H-1,2,4-triazol-1-ylmethyl)piperidin-1-yl]-2-oxoethyl}-1-[(2S)-piperidin-2-ylcarbonyl]piperidin-4-amine

On place 0,3 g de *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(2*S*)-piperidin-2-ylcarbonyl]piperidin-4-amine dans 2 mL de dichlorométhane et on ajoute 2,4 mL d'acide chlorhydrique 0,2N dans le diéthyléther. Après concentration à sec, le milieu réactionnel est repris au diéthyléther et trituré. Puis on essore le précipité obtenu et on rince au diéthyléther. Le chlorhydrate ainsi obtenu est séché sur P₂O₅ sous pression réduite. On obtient 0,23 g du chlorhydrate de *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(2*S*)-piperidin-2-ylcarbonyl]piperidin-4-amine.

Point de fusion = 207°C; M+H⁺ = 627 ; [α]_{D}²⁰ = +4,9 (0,921g/100mL, DMSO).

### Exemple 17 : Chlorhydrate de 4-({(1R)-1-(4-chlorobenzyl)-2-[4-cyclohexyl-4-(1H-1,2,4-triazol-1-ylmethyl)piperidin-1-yl]-2-oxoethyl}amino)cyclohexanecarbonitrile (composé n°112)

### 17.1 : 4-[(trimethylsilyl)oxy]cyclohex-3-ene-1-carbonitrile

On solubilise 14,2 g de 2-(triméthylsiloxy)-1,3-butadiène et 5,3 g d'acrylonitrile dans 35 mL de toluène anhydre. On ajoute 0,11 g d'hydroquinone et le milieu réactionnel est chauffé à 140°C pendant 24h. Après évaporation à sec, on obtient 4,0 g de 4-[(trimethylsilyl)oxy]cyclohex-3-ene-1-carbonitrile, utilisé tel quel par la suite.

### 17.2:4-oxocyclohexanecarbonitrile

On place 4,0 g de 4-[(trimethylsilyl)oxy]cyclohex-3-ene-1-carbonitrile dans 7 mL d'une solution aqueuse d'acide sulfurique à 2%. Après une agitation pendant 30 min, le milieu réactionnel est hydrolysé avec une solution aqueuse de chlorure d'ammonium saturée. On extrait au dichlorométhane jusqu'à épuisement de la phase aqueuse. La phase organique est lavée avec une solution aqueuse saturée de chlorure de sodium. Après séchage sur MgSO₄ et concentration à sec, on obtient 2,6 g de 4-oxocyclohexanecarbonitrile, utilisé tel quel par la suite.

### 17.3 : 4-({(1R)-1-(4-chlorobenzyl)-2-[4-cyclohexyl-4-(1H-1,2,4-triazol-1-ylmethyl)piperidin-1-yl]-2-oxoethyl}amino)cyclohexanecarbonitrile

On solubilise 1,08g de (2*R*)-3-(4-chlorophenyl)-1-[4-cyclohexyl-4-(1*H*-1,2,4-triazol-1-ylmethyl)piperidin-1-yl]-1-oxopropan-2-amine obtenue à l'étape 1.7 dans 25 mL de dichlorométhane en présence de 0,615 g de 4-oxocyclohexanecarbonitrile obtenu à l'étape 17.2. On additionne ensuite 1,32 g de triacétoxyborohydrure de sodium sous N₂. L'agitation est maintenue pendant 18h à température ambiante. Après hydrolyse avec une solution aqueuse saturée d'hydrogénocarbonate de sodium, on extrait au dichlorométhane jusqu'à épuisement de la phase aqueuse. La phase organique est lavée avec H₂O puis avec une solution aqueuse saturée de chlorure de sodium. Après séchage sur MgSO₄ et concentration à sec, le brut obtenu est chromatographié sur gel de silice en éluant avec un mélange de dichlorométhane/acétone/méthanol variant de 100/0/0 à 70/25/5. On obtient 0,55 g et 0,25 g de 4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarbonitrile, stéréoisomères (R, cis) et (R, trans), de configuration non déterminée.

### 17.4: chlorhydrate de 4-({(1R)-1-(4-chlorobenzyl)-2-[4-cyclohexyl-4-(1H-1,2,4-triazol-1-ylmethyl)piperidin-1-yl]-2-oxoethyl}amino)cyclohexanecarbonitrile

On place 0,2 g de 4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarbonitrile, stéréoisomère pur (R,cis) ou (R,trans) dans 2 mL de dichlorométhane et on ajoute 1,86 mL d'acide chlorhydrique 0,2N dans le diéthyl éther. Après concentration à sec, le milieu réactionnel est repris au diéthyl éther et trituré. Puis on essore le précipité obtenu et on rince au diéthyl éther. Le chlorhydrate ainsi obtenu est séché sur P₂O₅ sous pression réduite. On obtient 0,21 g du chlorhydrate de 4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarbonitrile, stéréoisomère pur (R,cis) ou (R,trans).

Point de fusion = 272°C; M+H⁺ = 540 ; [α]_{D}²⁰ = +6,4 (c=0,8g/100 mL, DMSO)

### Exemple 18: Chlorhydrate de N-[cis-4-({(1R)-1-(4-chlorobenzyl)-2-[4-cyclohexyl-4-(1H-1,2,4-triazol-1-ylmethyl)piperidin-1-yl]-2-oxoethyl}amino)cyclohexyl]glycinamide (composé n°116)

### 18.1 : methyl N-{cis-4-[(tert-butoxycarbonyl)amino]cyclohexyl}-4-chloro-D-phenylalaninate

On place 10 g de chlorhydrate de methyl 4-chloro-L-phenylalaninate en présence de 8,5 g de *tert*-butyl (4-oxocyclohexyl)carbamate dans 200 mL de dichlorométhane. On ajoute 11,0 g de triacétoxyborohydrure de sodium. L'agitation à température ambiante est maintenue pendant 18h. La solution est hydrolysée avec une solution aqueuse saturée d'hydrogénocarbonate de sodium et extraite au dichlorométhane jusqu'à épuisement de la phase aqueuse. Après séchage sur MgSO₄ et concentration à sec, le brut obtenu est chromatographié sur gel de silice en éluant avec un mélange dichlorométhane/acétate d'éthyle/méthanol/ammoniaque variant de 95/5/1/0,1 à 85/15/3/0,3. On obtient 6,1 g de methyl *N*-{*cis*-4-[(*tert*-butoxycarbonyl)amino]cyclohexyl}-4-chloro-D-phenylafaninate et 7,4 g de methyl *N*-{trans-4-[(*tert*-butoxycarbonyl)amino]cyclohexyl}-4-chloro-D-phenylalaninate.

### 18.2 : N-{cis-4-[(tert-butoxycarbonyl)amino]cyclohexyl}-4-chloro-D-phenylalanine

On place 4,8 g de methyl *N*-{*cis*-4-[(*tert*-butoxycarbonyl)amino]cyclohexyl}-4-chloro-D-phenylalaninate dans 180 mL d'un mélange H₂O/THF/MeOH puis on ajoute 0,83 g d'hydroxyde de lithium hydrate à 0°C. L'agitation est maintenue à température ambiante pendant 18h. Après évaporation du méthanol et tétrahydrofurane, on lyophilise le milieu réactionnel. On obtient 4,5 g de carboxylate de lithium de *N-*{*cis-4-*[(*tert-*butoxycarbonyl)amino]cyclohexyl}-4-chloro-D-phenylalanine.

### 18.3 : tert-butyl [cis-4-({(1R)-1-(4-chlorobenzyl)-2-[4-cyclohexyl-4-(1H-1,2,4-triazol-1-ylmethyl)piperidin-1-yl]-2-oxoethyl}amino)cyclohexyl]carbamate

On place 2,0 g de 4-cyclohexyl-4-(1*H*-1,2,4-triazol-1-ylmethyl)piperidine obtenu à l'étape 1.5 dans 40 mL de dichlorométhane en présence de 3,2 g de carboxylate de lithium de *N*-{*cis*-4-[(*tert* butoxycarbonyl)amino]cyclohexyl}-4-chloro-D-phenylalanine obtenu à l'étape 18.2, de 1,1 g d'hydroxybenzotriazole, de 1,5 g de chlorhydrate de 1-(3-diméthylaminopropyl)-3-éthyl-carbodiimide et de 1,68 mL de diisopropyléthylamine. Le mélange est agité 16h à température ambiante. Après évaporation à sec et hydrolyse, on extrait à l'acétate d'éthyle jusqu'à épuisement de la phase aqueuse. La phase organique est lavée avec une solution aqueuse d'acide chlorhydrique 1N, puis avec une solution aqueuse de soude 1N, et avec H₂O. Après séchage sur MgSO₄ et concentration à sec, le brut obtenu est chromatographié en éluant avec un gradient de méthanol dans le dichlorométhane variant de 0% à 5%. On obtient 2,4 g de *tert*-butyl [*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]carbamate.

### 18.4: cis-N-{(1R)-1-(4-chlorobenzyl)-2-[4-cyclohexyl-4-(1H-1,2,4-triazol-1-ylmethyl)piperidin-1-yl]-2-oxoethyl}cyclohexane-1,4-diamine

On place 2,4 g *tert*-butyl [*cis*-4-({(1*R*)-1-(4-chlorobenzyl)-2-[4-cyclohexyl-4-(1*H* 1,2,4-triazol-1-ylmethyl)piperidin-1-yl]-2-oxoethyl}amino)cyclohexyl]carbamate dans 5 mL de dioxane. Puis on ajoute 9,6 mL d'acide chlorhydrique 4N dans le dioxane. Le milieu réactionnel est agité pendant 18h à température ambiante. Après évaporation à sec, on reprend le résidu avec une solution aqueuse saturée d'hydrogénocarbonate de sodium et à l'acétate d'éthyle. On extrait au dichlorométhane jusqu'à épuisement de la phase aqueuse. La phase organique est lavée avec H₂O, puis avec une solution saturée aqueuse de chlorure de sodium. Après séchage sur MgSO₄ et concentration à sec, on obtient 1,98 g de *cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}cyclohexane-1,4-diamine.

### 18.5 : tert-butyl (2-{[cis-4-({(1R)-1-(4-chlorobenzyl)-2-[4-cyclohexyl-4-(1H-1,2,4-triazol-1-ylmethyl)piperidin-1-yl]-2-oxoethyl}amino)cyclohexyl]amino}-2-oxoethyl)carbamate

On place 0,7 g de *cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}cyclohexane-1,4-diamine obtenu à l'étape 18.4 dans 40 mL de dichlorométhane en présence de 0,23 g de Boc-Gly-OH, de 0,18 g d'hydroxybenzotriazole, de 0,25 g de chlorhydrate de 1-(3-diméthylaminopropyl)-3-éthyl-carbodiimide et de 0,24 mL de diisopropyléthylamine. Le mélange est agité 18h à température ambiante. Après évaporation à sec et hydrolyse, on extrait au dichlorométhane jusqu'à épuisement de la phase aqueuse. La phase organique est lavée avec H₂O, puis avec une solution saturée aqueuse de chlorure de sodium. Après séchage sur MgSO₄ et concentration à sec, le brut obtenu est chromatographié en éluant avec un gradient de méthanol dans le dichlorométhane variant de 0% à 10%. On obtient 0,75 g de *tert*-butyl (2-{[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-2-oxoethyl)carbamate.

### 18.6 : N-[cis-4-({(1R)-1-(4-chlorobenzyl)-2-[4-cyclohexyl-4-(1H-1,2,4-triazol-1-ylmethyl)piperidin-1-yl]-2-oxoethyl}amino)cyclohexyl]glycinamide

On solubilise 0,45 g de *tert*-butyl (2-{[*cis*-4-({(l*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-2-oxoethyl)carbamate dans 1 mL de dioxane. On ajoute 1,63 mL d'acide chlorhydrique 4N dans le dioxane, puis environ 1 mL de méthanol. Le milieu réactionnel est agité 18h à température ambiante. Après évaporation à sec, le résidu est repris au dichlorométhane. On ajoute une solution aqueuse saturée d'hydrogénocarbonate de sodium. On extrait au dichlorométhane jusqu'à épuisement de la phase aqueuse. La phase organique est lavée avec H₂O, puis avec une solution saturée aqueuse de chlorure de sodium. Après séchage sur MgSO₄ et concentration à sec, le brut obtenu est chromatographié en éluant avec un mélange dichlorométhane/méthanol/ammoniaque variant de 100/0/0 à 90/10/1. On obtient 0,35 g de *N-*[*cis-4-*({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]glycinamide.

### 18.7: chlorhydrate de N-[cis-4-({(1R)-1-(4-chlorobenzyl)-2-[4-cyclohexyl-4-(1H-1,2,4-triazol-1-ylmethyl)piperidin-1-yl]-2-oxoethyl}amino)cyclohexyl]glycinamide

On place 0,35 g de *N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]glycinamide dans 2 mL de dichlorométhane et on ajoute 3,0 mL d'acide chlorhydrique 0,2N dans le diéthyléther. Après concentration à sec, le milieu réactionnel est repris au diéthyléther et trituré. Puis on essore le précipité obtenu et on rince au diéthyléther. Le chlorhydrate ainsi obtenu est séché sur P₂O₅ sous pression réduite. On obtient 0,3 g du chlorhydrate de *N-*[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]glycinamide.

Point de fusion = 128°C; M+H⁺ = 584 ; [α]_{D}²⁰ = +0,7 (0,938g/100mL, DMSO).

### Exemple 19 : Chlorhydrate de N-{(1R)-1-(4-chlorobenzyl)-2-[4-cyclohexyl-4-(1H-1,2,4-triazol-1-ylmethyl)piperidin-1-yl]-2-oxoethyl}-4-(2,3-dihydro-1H-tetrazol-5-yl)cyclohexanamine (composé n°120)

### 19.1 : N-{(1R)-1-(4-chlorobenzyl)-2-[4-cyclohexyl-4-(1H-1,2,4-triazol-1-ylmethyl)piperidin-1-yl]-2-oxoethyl}-4-(2,3-dihydro-1H-tetrazol-5-yl)cyclohexanamine

On place 0,3 g de 4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarbonitrile, stéréoisomère pur (R,cis) ou (R,trans) obtenu à l'étape 17.3 dans 3 mL de diméthylformamide en présence de 0,43 g d'azoture de sodium et de 0,36 g de chlorure d'ammonium dans un tube scellé. Après réaction au micro-onde à 140°C pendant 3h, on évapore le diméthylformamide, et on reprend le brut obtenu dans le méthanol. Après filtration, le filtrat est concentré à sec et chromatographié sur C18 en éluant avec un mélange eau/acétonitrile variant de 80/20 à 100/0. On obtient 0,13 g de *N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(2,3-dihydro-1*H*-tetrazol-5-yl)cyclohexanamine, stéréoisomère pur (R,cis) ou (R,trans).

### 19.2 chlorhydrate de N-{(1R)-1-(4-chlorobenzyl)-2-[4-cyclohexyl-4-(1H-1,2,4-triazol-1-ylmethyl)piperidin-1-yl]-2-oxoethyl}-4-(2,3-dihydro-1H-tetrazol-5-yl)cyclohexanamine

On place 0,2 g de *N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(2,3-dihydro-1*H*-tetrazol-5-yl)cyclohexanamine, stéréoisomère pur (R, cis) ou (R, trans) dans 2 mL de dichlorométhane et on ajoute 1,86 mL d'acide chlorhydrique 0,2N dans le diéthyl éther. Après concentration à sec, le milieu réactionnel est repris au diéthyl éther et trituré. Puis on essore le précipité obtenu et on rince au diéthyl éther. Le chlorhydrate ainsi obtenu est séché sur P₂O₅ sous pression réduite. On obtient 0,095g du chlorhydrate de *N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(2,3-dihydro-1*H-*tetrazol-5-yl)cyclohexanamine, stéréoisomère pur (R,cis) ou (R,trans).

Point de fusion = 249°C; M+H⁺= 586

### Exemple 20 : Chlorhydrate de N-{(1R)-1-(4-chlorobenzyl)-2-[4-cyclohexyl-4-(1H-1,2,4-triazol-1-ylmethyl)piperidin-1-yl]-2,oxoethyl}-1-pyridin-2-ylpiperidin-4-amine (composé n°127)

### 20.1 : 8-pyridin-2-yl-1,4-dioxa-8-azaspiro[4.5]decane

On place 1,35 mL de 1,4-dioxa-8-azaspiro[4.5]decane en présence de 4,54 mL de 2-fluoropyridine dans un tube scellé. Après réaction au micro-onde à 100W et 150°C pendant 15 min, on hydrolyse et on ajoute dichlorométhane. Le milieu est alors basifié avec une solution aqueuse de soude 1 N. On extrait au dichlorométhane jusqu'à épuisement de la phase aqueuse. Après séchage sur MgSO₄ et concentration à sec, le brut obtenu est chromatographié en éluant avec un gradient de méthanol dans le dichlorométhane variant de 0% à 2%. On obtient 1,6 g de 8-pyridin-2-yl-1,4-dioxa-8-azaspiro[4.5]decane.

### 20.2 : 1-pyridin-2-ylpiperidin-4-one

On place 1,6 g de 8-pyridin-2-yl-1,4-dioxa-8-azaspiro[4.5]decane dans une solution aqueuse d'acide chlorhydrique 6N. Le milieu réactionnel est chauffé à 60°C pendant 24h. Après refroidissement, on hydrolyse avec du carbonate de sodium jusqu'à pH 8. On extrait au dichlorométhane jusqu'à épuisement de la phase aqueuse. Après séchage sur MgSO₄ et concentration à sec, on obtient 2,23 g de 1-pyridin-2-ylpiperidin-4-one, utilisé tel quel par la suite.

### 20.3 : N-{(1R)-1-(4-chlorobenzyl)-2-[4-cyclohexyl-4-(1H-1,2,4-triazol-1-ylmethyl)piperidin-1-yl]-2-oxoethyl}-1-pyridin-2-ylpiperidin-4-amine

On solubilise 0,3 g de (2*R*)-3-(4-chlorophenyl)-1-[4-cyclohexyl-4-(1*H*-1,2,4-triazol-1-ylmethyl)piperidin-1-yl]-1-oxopropan-2-amine obtenue à l'étape 1.7 dans 7 mL de dichlorométhane en présence de 0,25 g de 1-pyridin-2-ylpiperidin-4-one obtenu à l'étape 20.2. On additionne ensuite 0,22 g de triacétoxyborohydrure de sodium sous N₂. L'agitation est maintenue pendant 18h à température ambiante. Après hydrolyse avec une solution aqueuse saturée d'hydrogénocarbonate de sodium, on extrait au dichlorométhane jusqu'à épuisement de la phase aqueuse. La phase organique est lavée avec H₂O puis avec une solution aqueuse saturée de chlorure de sodium. Après séchage sur MgSO₄ et concentration à sec, le brut obtenu est chromatographié sur gel de silice en éluant avec un mélange de dichlorométhane/acétone/méthanol variant de 100/0/0 à 90/10/1. On obtient 0,4 g de *N*-{(1*R*)-1-(4-chlorobenzyl)-2-[4-cyclohexyl-4-(1*H-*1,2,4-triazo(-1-ylmethyl)piperidin-1-yl]-2-oxoethyl}-1-pyridin-2-ylpiperidin-4-amine.

### 20.4: chlorhydrate de N-{(1R)-1-(4-chlorobenzyl)-2-[4-cyclohexyl-4-(1H-1,2,4-triazol-1-ylmethyl)piperidin-1-yl]-2-oxoethyl}-1-pyridin-2-yfpiperidin-4-amine

On place 0,58 g de *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-pyridin-2-ylpiperidin-4-amine dans 2 mL de dichlorométhane et on ajoute 3,47 mL d'acide chlorhydrique 0,2N dans le diéthyléther. Après concentration à sec, le milieu réactionnel est repris au diéthyléther et trituré. Puis on essore le précipité obtenu et on rince au diéthyléther. Le chlorhydrate ainsi obtenu est séché sur P₂O₅ sous pression réduite. On obtient 0,37 g du chlorhydrate de *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-pyridin-2-ylpiperidin-4-amine.

Point de fusion >296°C; M+H⁺ = 593 ; [α]_{D}²⁰ = +14,3 (0,938g/100mL, DMSO).

### Exemple 21: Chlorhydrate de 3-[4-({(1R)-1-(4-chlorobenzyl)-2-[4-cyclohexyl-4-(1H-1,2,4-triazol-1-ylmethyl)piperidin-1-yl]-2-oxoethyl}amino)cyclohexyl]-6-fluoro-1,3-benzoxazol-2(3H)-one (composé n°167)

### 21:1 : 2-(1,4-dioxaspiro[4.5]dec-8-ylamino)-5-fluorophenol

On place 2,5 g de 1,4-dioxaspiro[4.5]decan-8-one dans 78 mL d'acide acétique. On ajoute 2,0 g de 2-amino-5-fluorophénol, 10,0 g de triacétoxyborohydrure de sodium, et 11,2 g de sulfate de sodium. Le milieu réactionnel est agité 24h à température ambiante. Après évaporation de l'acide acétique, le résidu est traité avec une solution aqueuse de soude 1N. On extrait à l'acétate d'éthyle jusqu'à épuisement de la phase aqueuse. Après séchage sur MgSO₄ et concentration à sec, le brut obtenu est cristallisé dans de l'heptane. Les cristaux obtenus sont essorés, rincés à l'heptane. On obtient 1,8 g de 2-(1,4-dioxaspiro[4.5]dec-8-ylamino)-5-fluorophenol.

### 21.2: 4-[(4-fluoro-2-hydroxyphenyl)amino]cyclohexanone

On place 1,8 g de 2-(1,4-dioxaspiro[4.5]dec-8-ylamino)-5-fluorophenol dans une solution aqueuse d'acide chlorhydrique 6N. Le milieu réactionnel est chauffé à 60°C pendant 18h. Après refroidissement, on hydrolyse avec une solution aqueuse de soude jusqu'à pH 8. On extrait à l'acétate d'éthyle jusqu'à épuisement de la phase aqueuse. La phase organique est lavée avec une solution aqueuse saturée de chlorure de sodium. Après séchage sur MgSO₄ et concentration à sec, le brut obtenu est chromatographié sur gel de silice en éluant avec un gradient de méthanol dans le dichlorométhane variant de 0% à 10%. On obtient 1 g de composé qui est chromatographié dans un mélange heptane/acétate d'éthyle variant de 8/2 à 4/6. On obtient 0,64 g de 4-[(4-fluoro-2-hydroxyphenyl)amino]cyclohexanone.

### 21.3 : 6-fluoro-3-(4-oxocyclohexyl)-1,3-benzoxazol-2(3H)-one

On solubilise 0,24 g 4-[(4-fluoro-2-hydroxyphenyl)amino]cyclohexanone dans 11 mL de dichlorométhane anhydre et on ajoute 0,27 g de carbonyldiimidazole. Le milieu réactionnel est agité 18h à température ambiante. Après addition 0,05 g de carbonyldiimidazole supplémentaire, l'agitation est maintenue 5h. Après concentration à sec et hydrolyse, on extrait à l'acétate d'éthyle jusqu'à épuisement de la phase aqueuse. La phase organique est lavée avec une solution aqueuse saturée de chlorure de sodium. Après séchage sur MgSO₄ et concentration à sec, le brut obtenu est chromatographié sur gel de silice en éluant avec un gradient de méthanol dans le dichlorométhane variant de 0% à 5%. On obtient 0,3 g de 6-fluoro-3-(4-oxocyclohexyl)-1,3-benzoxazol-2(3*H*)-one.

### 21.4 : 3-[4-({(1R)-1-(4-chlorobenzyl)-2-[4-cyclohexyl-4-(1H-1,2,4-triazol-1-ylmethyl)piperidin-1-yl]-2-oxoethyl}amino)cyclohexyl]-6-fluoro-1,3-benzoxazol-2(3H)-one

On solubilise 0,5 g de (2*R*)-3-(4-chlorophenyl)-1-[4-cyclohexyl-4-(1*H*-1,2,4-triazol-1-ylmethyl)piperidin-1-yl]-1-oxopropan-2-amine obtenue à l'étape 1.7 dans 7 mL de dichlorométhane en présence de 0,29 g de 6-fluoro-3-(4-oxocyclohexyl)-1,3-benzoxazol-2(3*H*)-one obtenu à l'étape 21.3. On additionne ensuite 0,37 g de triacétoxyborohydrure de sodium sous N₂. L'agitation est maintenue pendant 18h à température ambiante. Après concentration à sec et hydrolyse, on extrait à l'acétate d'éthyle jusqu'à épuisement de la phase aqueuse. La phase organique est lavée avec H₂O puis avec une solution aqueuse saturée de chlorure de sodium. Après séchage sur MgSO₄ et concentration à sec, le brut obtenu est chromatographié sur gel de silice en éluant avec un mélange dichlorométhane/acétate d'éthyle/méthanol variant de 100/0/0 à 7/2/1. On obtient 0,24 g et 0,2 g de 3-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-6-fluoro-1,3-benzoxazol-2(3*H*)-one, stéréoisomères (R, cis) et (R, trans) de configuration non déterminée.

### 21.5 : 3-[4-({(1R)-1-(4-chlorobenzyl)-2-[4-cyclohexyl-4-(1H-1,2,4-triazol-1-ylmethyl)piperidin-1-yl]-2-oxoethyl}amino)cyclohexyl]-6-fluoro-1,3-benzoxazol-2(3H)-one

On place 0,15 g de 3-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-6-fluoro-1,3-benzoxazol-2(3*H*)-one, stéréoisomère pur (R,cis) ou (R,trans) dans 2 mL de dichlorométhane et on ajoute 2,23 mL d'acide chlorhydrique 0,2N dans le diéthyléther. Après concentration à sec, le milieu réactionnel est repris dans l'acétate d'éthyle et trituré. Puis on essore le précipité obtenu et on rince à l'acétate d'éthyle. Le chlorhydrate ainsi obtenu est séché sur P₂O₅ sous pression réduite. On obtient 0,10 g du chlorhydrate de 3-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-6-fluoro-1,3-benzoxazol-2(3*H*)-one, stéréoisomère pur (R,cis) ou (R,trans).

Point de fusion = 286°C; M+H⁺= 664

### Exemple 22: Chlorhydrate de 2-{[4-({(1R)-1-(4-chlorobenzyl)-2-[4-cyclohexyl-4-(1H-1,2,4-triazol-1-ylmethyl)piperidin-1-yl]-2-oxoethyl}amino)cyclohexyl]amino}-5-fluoro-N,N-dimethylbenzamide (composé n°182)

### 22.1 : methyl 2-(1,4-dioxaspiro[4.5]dec-8-ylamino)-5-fluorobenzoate

On place 4,1 g de 1,4-dioxaspiro[4.5]decan-8-one dans 88 mL d'acide acétique. On ajoute 3,0 g du 2-amino-5-fluorobenzoic acid methyl ester, 11,3 g de triacétoxyborohydrure de sodium, et 12,6 g de sulfate de sodium. Le milieu réactionnel est agité 18h à température ambiante. Après addition de 1,5 g de 1,4-dioxaspiro[4.5]decan-8-one et agitation 24h suplémentaire, l'acide acétique est évaporé: Le résidu est traité avec une solution aqueuse de soude 3N. On extrait à l'acétate d'éthyle jusqu'à épuisement de la phase aqueuse. Après séchage sur MgSO₄ et concentration à sec, le brut obtenu est chromatographié sur gel de silice en éluant avec un gradient de méthanol dans le dichlorométhane variant de 0% à 5% . On obtient 0,67 g de methyl 2-(1,4-dioxaspiro[4.5]dec-8-ylamino)-5-fluorobenzoate.

### 22.2 : 2-(1,4-dioxaspiro[4.5]dec-8-ylamino)-5-fluorobenzoic acid

On place 0,65 g de methyl 2-(1,4-dioxaspiro[4.5]dec-8-ylamino)-5-fluorobenzoate dans 21. mL de méthanol et on ajoute 8,3 mL d'une solution de soude aqueuse 1 N. Après addition de 5 mL de tétrahydrofurane et chauffage à 70°C pendant 2h30, on acidifie à pH 2-3 avec une solution aqueuse d'acide chlorhydrique 1N. On extrait au dichlorométhane jusqu'à épuisement de la phase aqueuse. La phase organique est lavée avec une solution aqueuse saturée de chlorure de sodium, on sèche sur MgSO₄ et on concentre à sec. On obtient 0,58 g de 2-(1,4-dioxaspiro[4.5]dec-8-ylamino)-5-fluorobenzoic acid utilisé tel quel par la suite.

### 22.3 : 2-(1,4-dioxaspiro[4.5]dec-8-ylamino)-5-fluoro-N,N-dimethylbenzamide

On place 0,58 g de 2-(1,4-dioxaspiro[4.5]dec-8-ylamino)-5-fluorobenzoic acid obtenu à l'étape 22.2 dans 20 mL de dichlorométhane en présence de 0,8 g du chlorhydrate de diméthylamine, de 0,27 g d'hydroxybenzotriazole, de 0,38 g de chlorhydrate de 1-(3-diméthylaminopropyl)-3-éthyl-carbodiimide et de 2,4 mL de diisopropyléthylamine. Le mélange est agité 48h à température ambiante. Après évaporation à sec et hydrolyse avec une solution aqueuse de soude 1N, on extrait au dichlorométhane jusqu'à épuisement de la phase aqueuse. La phase organique est lavée avec une solution saturée aqueuse de chlorure de sodium. Après séchage sur MgSO₄ et concentration à sec, le brut obtenu est chromatographié en éluant avec un gradient de méthanol dans le dichlorométhane variant de 0% à 10%. On obtient 0,4 g de 2-(1,4-dioxaspiro[4.5]dec-8-ylamino)-5-fluoro-*N*,*N*-dimethylbenzamide.

### 22.4 : 5-fluoro-N,N-dimethyl-2-[(4-oxocyclohexyl)amino]benzamide

On place 0,38 g de 2-(1,4-dioxaspiro[4.5]dec-8-ylamino)-5-fluoro-*N*,*N-*dimethylbenzamide dans 1,7 mL d'une solution aqueuse d'acide chlorhydrique 2N et on agite à 40 °C pendant 2h. Après concentration à sec, le résidu est repris avec une solution aqueuse de soude 1 N et extrait à l'acétate d'éthyle jusqu'à épuisement de la phase aqueuse. La phase organique est lavée avec H₂O puis avec une solution saturée aqueuse de chlorure de sodium. Après séchage sur MgSO₄ et concentration à sec, le brut obtenu est chromatographié en éluant avec un gradient de méthanol dans le dichlorométhane variant de 0% à 10%. On obtient 0,25 g de 5-fluoro-*N,N-*dimethyl-2-[(4-oxocyclohexyl)amino]benzamide.

### 22.5 : 2-{[4-({(1R)-1-(4-chlorobenzyl)-2-[4-cyclohexyl-4-(1H-1,2,4-triazol-1-ylmethyl)piperidin-1-yl]-2-oxoethyl}amino)cyclohexyl]amino}-5-fluoro-N,N-dimethylbenzamide

On solubilise 0,35g de (2*R*)-3-(4-chlorophenyl)-1-[4-cyclohexyl-4-(1*H*-1,2,4-triazol-1-ylmethyl)piperidin-1-yl]-1-oxopropan-2-amine obtenue à l'étape 1.7 dans 4 mL de dichlorométhane en présence de 0,25 g de 5-fluoro-*N,N-*dimethyl-2-[(4-oxocyclohexyl)amino]benzamide obtenu à l'étape 22.4. On additionne ensuite 0,22 g de triacétoxyborohydrure de sodium sous N₂. L'agitation est maintenue pendant 18h à température ambiante. Après concentration à sec et hydrolyse avec une solution aqueuse de soude 1N, on extrait à l'acétate d'éthyle jusqu'à épuisement de la phase aqueuse. La phase organique est lavée avec H₂O pu avec une solution aqueuse saturée de chlorure de sodium. Après séchage sur MgSO₄ et concentration à sec, le brut obtenu est chromatographié sur gel de silice en éluant avec un mélange dichlorométhane/acétate d'éthyle/méthanol variant de 100/0/0 à 7/2,5/0,5. On obtient 0,22 g et 0,1 g de 2-{[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-5-fluoro-*N,N-*dimethylbenzamide, stéréoisomères (R, cis) et (R, trans) de configuration non déterminée.

### 22.6 chlorhydrate de 2-{[4-({(1R)-1-(4-chlorobenzyl)-2-[4-cyclohexyl-4-(1H-1,2,4-triazol-1-ylmethyl)piperidin-1-yl]-2-oxoethyl}amino)cyclohexyl]amino}-5-fluoro-N,N-dimethylbenzamide

On place 0,22 g de 2-{[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-5-fluoro-*N,N-*dimethylbenzamide, stéréoisomère pur (R,cis) ou (R,trans) dans 2 mL de dichlorométhane et on ajoute 3,15 mL d'acide chlorhydrique 0,2N dans le diéthyléther. Après concentration à -sec, le milieu réactionnel est repris dans l'acétate d'éthyle et trituré. Puis on essore le précipité obtenu et on rince à l'acétate d'éthyle. Le chlorhydrate ainsi obtenu est séché sur P₂O₅ sous pression réduite. On obtient 0,19 g du chlorhydrate de 2-{[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-5-fluoro-*N*,*N*-dimethyl.benzamide, stéréoisomère pur (R,cis) ou (R,trans).

Point de fusion = 145°C; M+H⁺ =692

Le tableau qui suit illustre les structures chimiques et les propriétés physiques de quelques exemples de composés selon l'invention, à savoir des composés de formule (I bis), correspondant à des composés de formule (I) dans lesquels R₁ représente un groupe cyclohexyle, R₂ représente un groupe 1,2,4-triazolyle, Rₐ = R_{a'} = R₅ = H, n = 1 et R₃ représente un atome de chlore situé en position para sur le noyau phényle auquel il est rattaché. Dans ce tableau :
- l'atome de carbone portant le groupé 4-Cl-benzyle présente la configuration (R),
- dans la colonne « sel », « - » représente un composé sous forme de base libre, alors que « HCl » représente un composé sous forme de chlorhydrate et « CF₃COOH » un composé sous forme de trifluoroacétate,
- « PF » représente le point de fusion du composé, et
- Me, Et, tBu et Bn représentent respectivement des groupes méthyle, éthyle, tertiobutyle et benzyle.

**Tableaux**

| | | | | | |
|---|---|---|---|---|---|
| 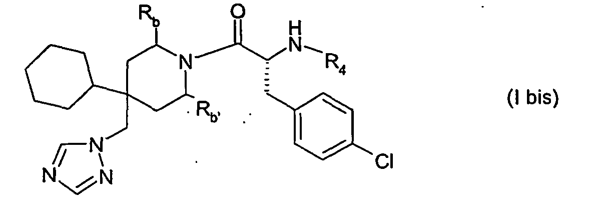 | | | | | |

| **N°** | **R_{b}** | **R_{b'}** | **R₄** | **Sel** | **PF (°C)** |
|---|---|---|---|---|---|
| **1** | H | H | 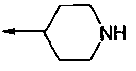 | HCl | 255 |
| **2** | H | H | 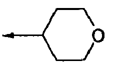 | - | 75 |
| **3** | H | H | 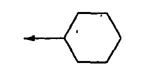 | - | 60 |
| **4** | H | H | 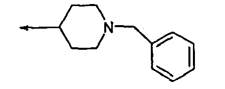 | - | 60 |
| **5** | H | H | 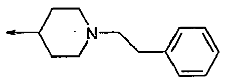 | - | 78 |
| **6** | H | H | 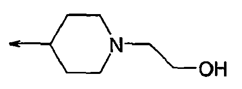 | HCl | 257 |
| **7** | H | H | 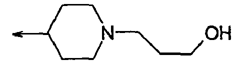 | - | - |
| **8** | H | H | 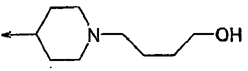 | HCl | 266 |
| **9** | H | H | 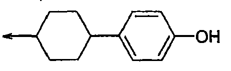 | - | 100 |
| **10** | H | H | 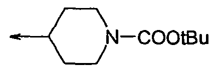 | - | 175 |
| **11** | H | H | 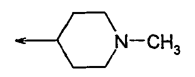 | HCl | 264 |
| **12** | H | H | 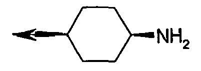 | HCl | 159 |
| **13** | H | H | 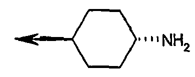 | HCl | 245 |
| **14** | H | H | 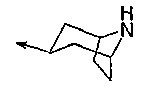 | HCl | 236 |
| **15** | H | H | 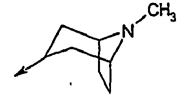 | HCl | 262 |
| **16** | H | H | 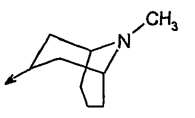 | HCl | > 280 |
| **17** | H | H | 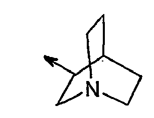 | HCl | 260 |
| **18** | H | H | 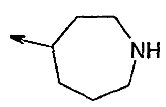 | HCl | 244 |
| **19** | H | H | 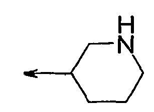 | HCl | 120 |
| **20** | H | H | 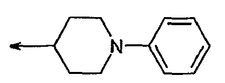 | HCl | 200 |
| **21** | CH₂CH₂ | | 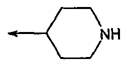 | HCl | > 270 |
| **22** | CH₂CH₂ | | 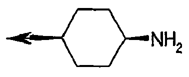 | HCl | 215 |
| **23** | CH₂CH₂ | | 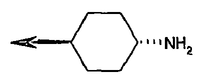 | HCl | 205 |
| **24** | H | H | 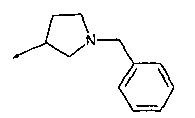 | HCl | 137 |
| **25** | H | H | 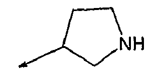 | HCl | 160 |
| **26** | H | H | 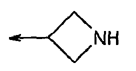 | HCl | 182 |
| **27** | H | H | 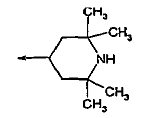 | HCl | 198 |
| **28** | H | H | 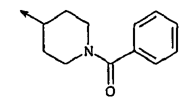 | HCl | > 200 |
| **29** | H | H | 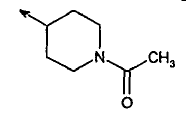 | - | > 200 |
| **30** | H | H | 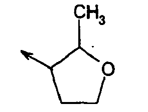 | HCl | 252 |
| **31** | H | H | 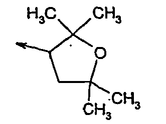 | HCl | 260 |
| **32** | H | H | 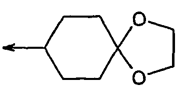 | HCl | 275 |
| **33** | H | H | 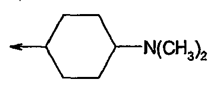 | HCl | 190 |
| **34** | H | H | 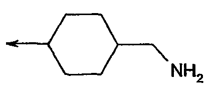 | HCl | 295 |
| **35** | H | H | 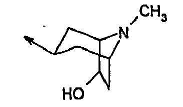 | HCl | 153 |
| **36** | H | H | 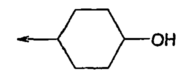 | HCl | 128 ou 132* |
| **37** | H | H | 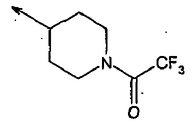 | CF₃COOH | > 200 |
| **38** | H | H | 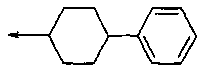 | CF₃COOH | > 200 |
| **39** | H | H | 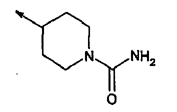 | HCl | 186 |
| **40** | H | H | 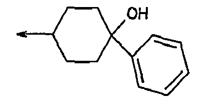 | HCl | 250 ou 260* |
| **41** | H | H | 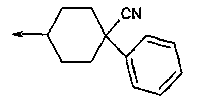 | HCl | 270 |
| **42** | H | H | 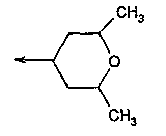 | HCl | 280 ou 260* |
| **43** | H | H | 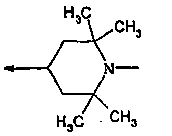 | HCl | 140 |
| **44** | H | H | 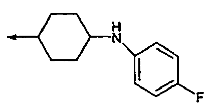 | HCl ou - | > 205 ou 100* |
| **45** | H | H | 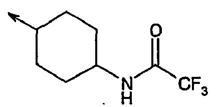 | HCl | 192 ou 140* |
| **46** | H | H | 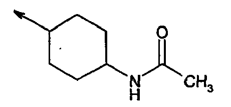 | HCl | 157 ou 118* |
| **47** | H | H | 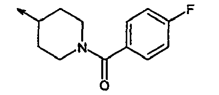 | - | 82 |
| **48** | H | H | 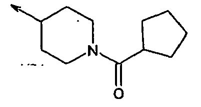 | - | 65 |
| **49** | H | H | 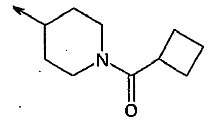 | - | 88 |
| **50** | H | H | 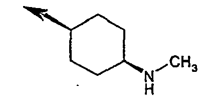 | HCl | 210 |
| **51** | H | H | 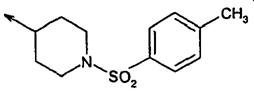 | - | 96 |
| **52** | H | H | 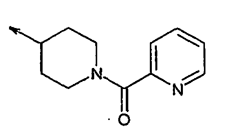 | - | 90 |
| **53** | H | H | 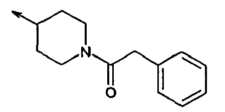 | HCl | 155 |
| **54** | H | H | 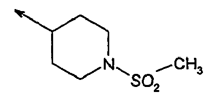 | HCl | > 200 |
| **55** | H | H | 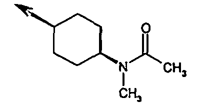 | HCl | 190 |
| **56** | H | H | 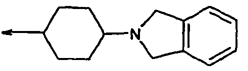 | HCl | 215 |
| **57** | H | H | 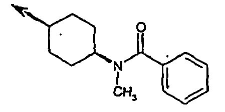 | HCl | 270 |
| **58** | H | H | 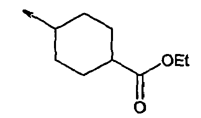 | - | 67* |
| **59** | H | H | 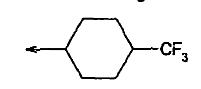 | HCl | 267 ou 268* |
| **60** | H | H | 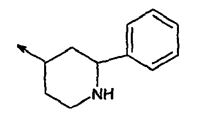 | HCl | 270 |
| **61** | H | H | 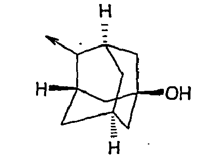 | HCl | > 270 |
| **62**** | H | H | 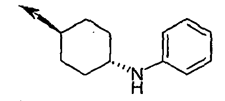 | HCl | > 00 |
| **63**** | H | H | 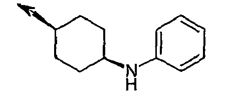 | HCl | 110 |
| **64** | H | H | 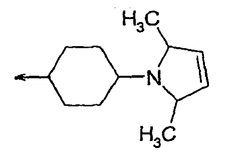 | HCl | 103 |
| **65**** | H | H | 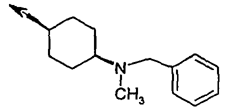 | HCl | 88 |
| **66** | H | H | 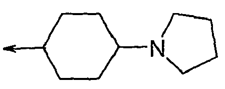 | HCl | 105 |
| **67** | H | H | 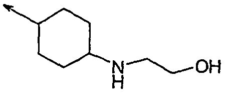 | HCl | 103 |
| **68** | H | H | 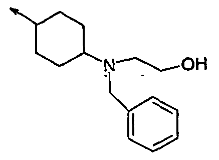 | HCl | 114 |
| **69** | H | H | 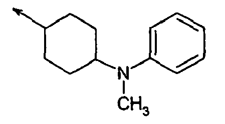 | HCl | 130 |
| **70** | H | H | 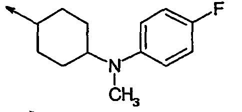 | HCl | 111 |
| **71**** | H | H | 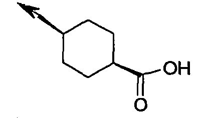 | HCl | 178 |
| **72**** | H | H | 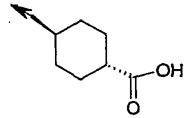 | HCl | > 200 |
| **73**** | H | H | 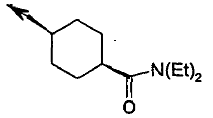 | HCl | > 200 |
| **74**** | H | H | 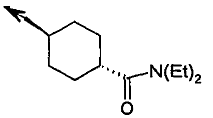 | HCl | > 200 |
| **75**** | H | H | 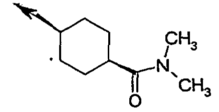 | HCl | > 200 |
| **76**** | H | H | 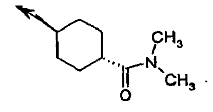 | HCl | 63 |
| **77** | H | H | 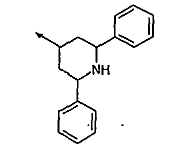 | HCl | 212 |
| **78** | H | H | 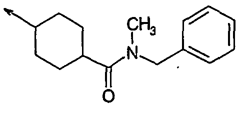 | HCl | 160 ou >200* |
| **79** | H | H | 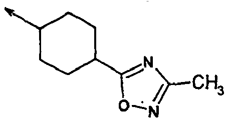 | HCl | >200 ou 239* |
| **80** | H | H | 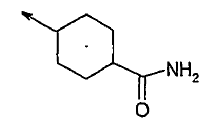 | HCl | 145 ou 118* |
| **81** | H | H | 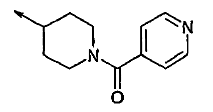 | HCl | 165 |
| **82** | H | H | 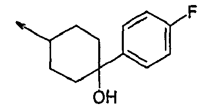 | HCl | 150 ou 155* |
| **83** | H | H | 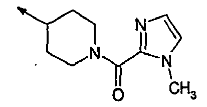 | HCl | 160 |
| **84** | H | H | 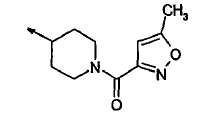 | HCl | 85 |
| **85** | H | H | 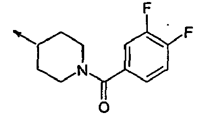 | HCl | 140 |
| **86** | H | H | 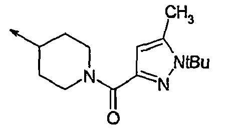 | HCl | >220 |
| **87** | H | H | 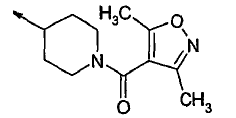 | HCl | 210 |
| **88** | H | H | 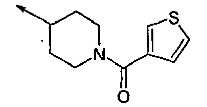 | HCl | 110 |
| **89** | H | H | 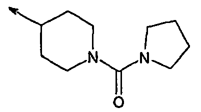 | HCl | 275 |
| **90** | H | H | 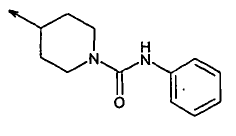 | HCl | 198 |
| **91** | H | H | 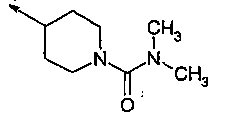 | HCl | 255 |
| **92** | H | H | 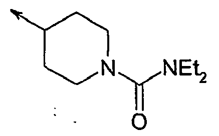 | HCl | 288 |
| **93** | H | H | 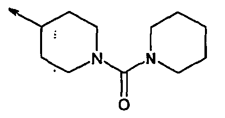 | HCl | 275 |
| **94** | H | H | 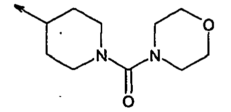 | HCl | 259 |
| **95** | H | H | 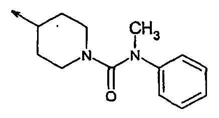 | HCl | 265 |
| **96** | H | H | 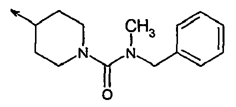 | HCl | 245 |
| **97** | H | H | 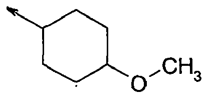 | HCl | >240 |
| **98** | H | H | 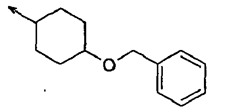 | HCl | >240 |
| **99** | H | H | 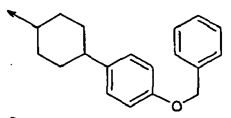 | HCl | 198 ou 252* |
| **100** | H | H | 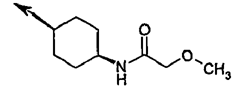 | HCl | 110 |
| **101** | H | H | 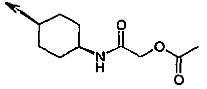 | HCl | 144 |
| **102** | H | H | 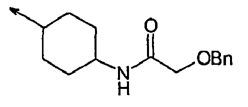 | HCl | 102 |
| **103** | H | H | 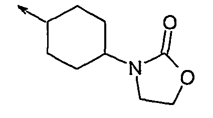 | HCl | 163 ou >200* |
| **104** | H | H | 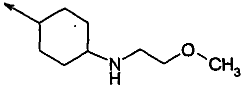 | HCl | 79 ou 93* |
| **105** | H | H | 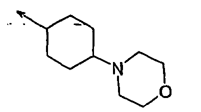 | HCl | 119 ou >200* |
| **106** | H | H | 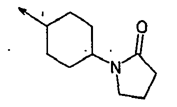 | HCl | 174 ou >200* |
| **107** | H | H | 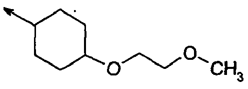 | HCl | 257 |
| **108** | H | H | 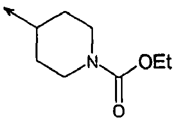 | HCl | 273 |
| **109** | H | H | 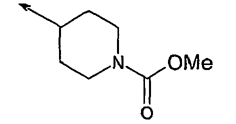 | HCl | 273 |
| **110** | H | H | 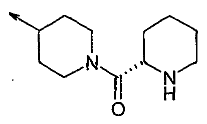 | HCl | 207 |
| **111** | H | H | 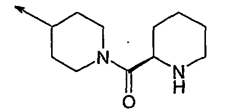 | HCl | 179 |
| **112** | H | H | 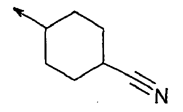 | HCl | 272 ou 279* |
| **113** | H | H | 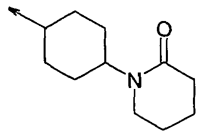 | HCl | 177 ou 183* |
| **114** | H | H | 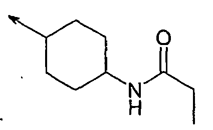 | HCl | 158 |
| **115** | H | H | 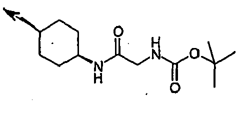 | - | 102 |
| **116** | H | H | 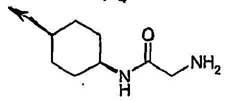 | HCl | 128 |
| **117** | H | H | 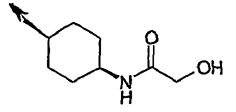 | HCl | 179 |
| **118** | H | H | 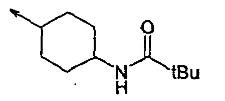 | HCl | 158 ou 254* |
| **119** | H | H | 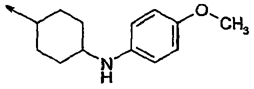 | HCl | 225 ou 200* |
| **120** | H | H | 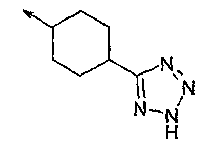 | HCl | 249 ou 228* |
| **121** | H | H | 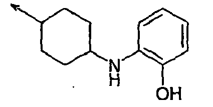 | HCl | 130 ou 136* |
| **122** | H | H | 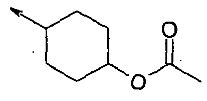 | - | 61 |
| **123** | H | H | 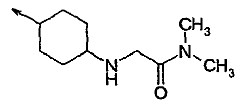 | HCl | 165 ou 256* |
| **124** | H | H | 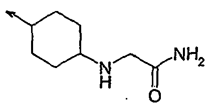 | HCl | 172 ou 173* |
| **125** | H | H | 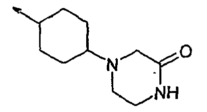 | HCl | 204 ou 254* |
| **126** | H | H | 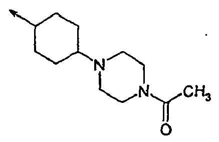 | HCl | 194 ou 267* |
| **127** | H | H | 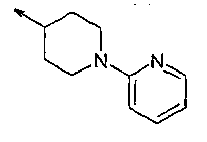 | HCl | >255 |
| **128** | H | H | 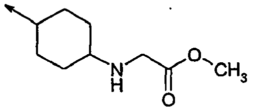 | HCl | 131 |
| **129** | H | H | 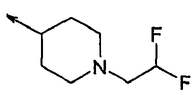 | HCl | 221 |
| **130** | H | H | 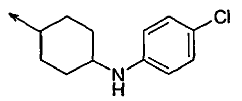 | HCl | 280 ou 201* |
| **131** | H | H | 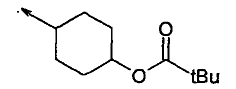 | Fumaric acid | 120 |
| **132** | H | H | 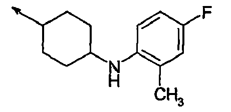 | HCl | 221 ou 267* |
| **133** | H | H | 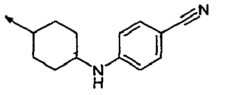 | HCl | 132 |
| **134** | H | H | 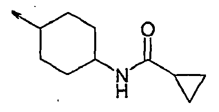 | HCl | 178 |
| **135** | H | H | 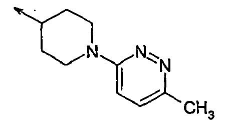 | HCl | 273 |
| **136** | H | H | 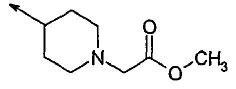 | HCl | 193 |
| **137**** | H | H | 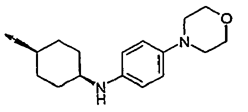 | HCl | 233 |
| **138** | H | H | 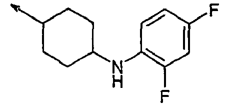 | HCl | 177 ou 152* |
| **139** | H | H | 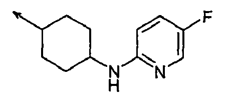 | HCl | 132 ou 165* |
| **140** | H | H | 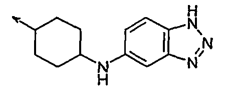 | HCl | 205 |
| **141** | H | H | 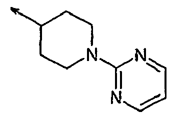 | HCl | 294 |
| **142** | H | H | 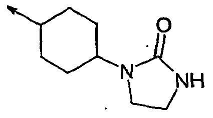 | HCl | 173 ou 177* |
| **143** | H | H | 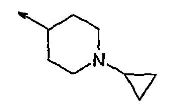 | HCl | 213 |
| **144** | H | H | 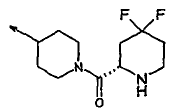 | HCl | 245 |
| **145** | H | H | 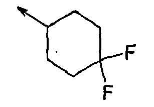 | HCl | 292 |
| **146** | H | H | 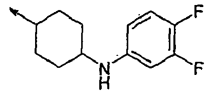 | HCl | 255 ou 235* |
| **147** | H | H | 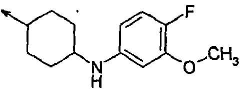 | HCl | 175 ou 184* |
| **148** | H | H | 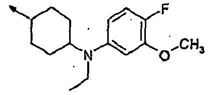 | HCl | 160 ou 222* |
| **149** | H | H | 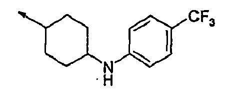 | HCl | 183 ou 185* |
| **150** | H | H | 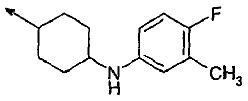 | HCl | 248 ou 220* |
| **151** | H | H | 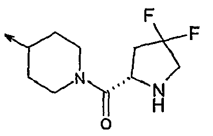 | HCl | 237 |
| **152** | H | H | 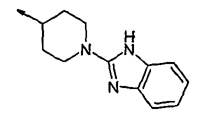 | - | 228 |
| **153** | H | H | 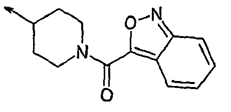 | HCl | 225 |
| **154** | H | H | 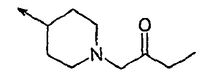 | CF3COOH | 80 |
| **155** | H | H | 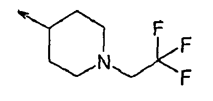 | HCl | 272 |
| **156** | H | H | 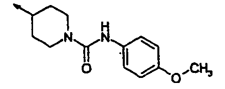 | HCl | 150. |
| **157** | H | H | 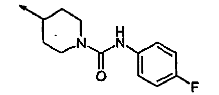 | HCl | 177 |
| **158** | H | H | 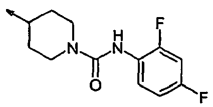 | HCl | 168 |
| **159** | H | H | 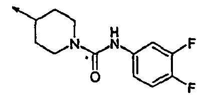 | HCl | 190 |
| **160** | H | H | 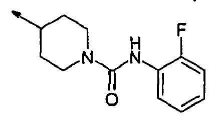 | HCl | 167 |
| **161** | H | H | 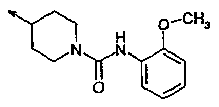 | HCl | 182 |
| **162** | H | H | 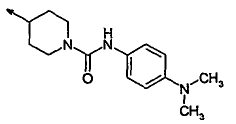 | HCl | 205 |
| **163** | H | H | 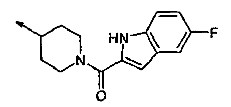 | HCl | 211 |
| **164** | H | H | 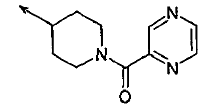 | HCl | 285 |
| **165** | H | H | 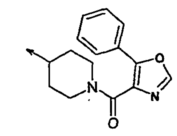 | HCl | 278 |
| **166** | H | H | 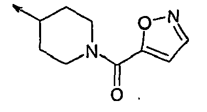 | HCl | 236 |
| **167** | H | H | 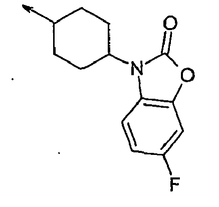 | HCl | 286 ou 236* |
| **168** | H | H | 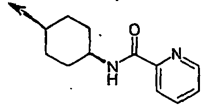 | HCl | 208 |
| **169**** | H | H | 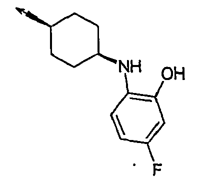 | HCl | 242 |
| **170** | H | H | 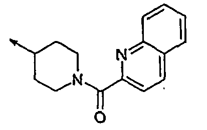 | HCl | 221 |
| **171** | H | H | 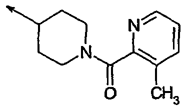 | HCl | 269 |
| **172** | H | H | 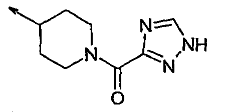 | HCl | 212 |
| **173** | H | H | 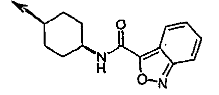 | HCl | 192 |
| **174** | H | H | 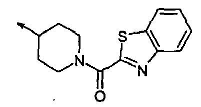 | HCl | 268 |
| **175** | H | H | 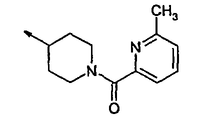 | HCl | 229 |
| **176** | H | H | 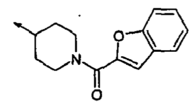 | HCl | 203 |
| **177** | H | H | 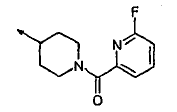 | HCl | 273 |
| **178** | H | H | 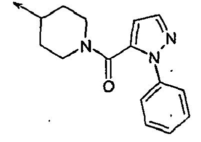 | HCl | 203 |
| **179** | H | H | 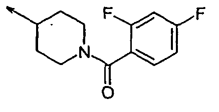 | HCl | 266 |
| **180** | H | H | 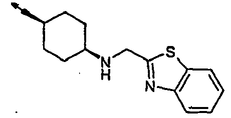 | HCl | 115 |
| **181** | H | H | 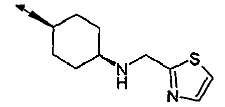 | HCl | 120 |
| **182** | H | H | 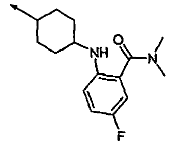 | HCl | 145 ou 155* |
| **183** | H | H | 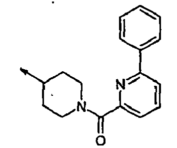 | HCl | 261 |
| **184** | H | H | 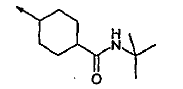 | HCl | 178 ou 248* |
| **185** | H | H | 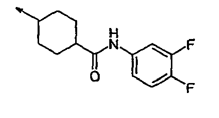 | HCl | 187 ou 224* |

| | | | | | |
|---|---|---|---|---|---|
| * selon l'isomère (cis ou trans) ** configuration cis ou trans non déterminée, représentation arbitraire | | | | | |

Le tableau qui suit illustre les structures chimiques et les propriétés physiques de quelques exemples de composés selon l'invention, à savoir des composés de formule (I ter), correspondant à des composés de formule (I) dans lesquels R₁ représente un groupe cyclohexyle, R₂ représente un groupe 1,2,4-triazolyle, Rₐ = R_{a'} = R₅ = H, n = 1 et R₃ représente un atome de chlore situé en position para sur le noyau phényle auquel il est rattaché. Dans ce tableau :
- l'atome de carbone portant le groupe 4-Cl-benzyle présente la configuration (S),
- dans la colonne « sel », « HCl » représente un composé sous forme de chlorhydrate,
- *« PF » représente le point de fusion du composé*.

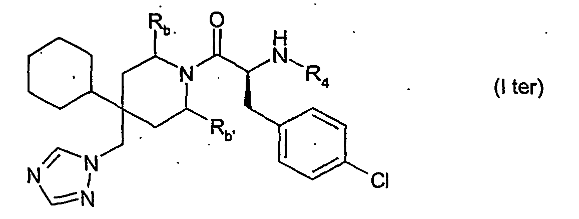

| **N°** | **R_{b}** | **R_{b'}** | R₄ | **Sel** | **PF (°C)** |
|---|---|---|---|---|---|
| **186** | H | H | 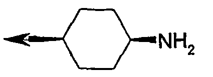 | HCl | 153 |
| **187** | H | H | 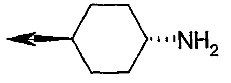 | HCl | 246 |

Les composés selon l'invention ont fait l'objet d'essais pharmacologiques permettant de déterminer leur effet agoniste des récepteurs aux mélanocortines, en particulier leur effet agoniste du récepteur MC3 et/ou MC4.

### Evaluation de l'affinité des composés de formule (I) selon l'invention envers les récepteurs MC3 et MC4

Ce test d'affinité est réalisé par la mesure de la liaison du [¹²⁵I]-[Nle⁴-D-Phe⁷]-α-MSH aux membranes cellulaires : le déplacement de ce radio-ligand est utilisé pour identifier des inhibiteurs de la liaison spécifique aux récepteurs recombinants mélanocortine.

Pour ce test, on utilise des membranes préparées à partir des cellules CHO-K1 exprimant le récepteur humain MC4 à forte densité (Euroscreen) ou des membranes achetées (Perkin Elmer Life Sciences, Receptor Biology) des cellules HEK-293 exprimant des récepteurs hMC3. Les cellules CHO-K1 transfectées avec le gène du récepteur hMC4 (Euroscreen) sont ensemencées dans le milieu de culture DMEM/ Nutrient Mix F12 contenant 10% sérum de veau (Biowhittaker), 1% pyruvate de sodium, 1% L-glutamine, 1% acides aminés non-essentiels, 0.4 mg/ml généticine (G418) et 0.5% PenStrep, ces produits étant fournis par Gibco/BRI, sauf le sérum de veau. Les cellules sont grattées à 80% de confluence et les culots de cellules sont congelés à -80°C.

Un tube de cellules (environ 70 x 10⁶ cellules) est décongelé sur glace et resuspendu dans 10 ml de tampon de binding [25 mM HEPES, pH 7.0, 1 mM MgCl₂, 1.5 mM CaCl₂, 100 mM NaCl, 1 mM 1,10-phenanthroline et 1 tablette de Complète (inhibiteur de protéases de Roche) dans 50 ml de tampon] par polytronnage 20 s. La suspension est centrifugée 20 min à 19500 t/min à 4°C. Le surnageant est jeté et le culot est resuspendu dans 5 ml tampon de binding. Par un test de Bradford, on dose la quantité de protéines présentes dans l'échantillon et on ajuste la concentration à 3 µg/25 µl par dilution dans du tampon de binding.

[¹²⁵I]-[Nle⁴, D-Phe⁷]-α-MSH est dilué dans du tampon de binding + 0.2% BSA. Des billes SPA (wheatgerm agglutinine polyvinyltoluene, Amersham Pharmacia Biotech), sont hydratées dans le tampon de binding + 0.2% BSA et ensuite mélangées avec l'homogénat de cellules pour obtenir 3 µg de protéines cellulaires et 250 µg de billes dans 50 µl. Les produits à tester (dilués dans 10% DMSO), en quantité de 10 µl à une concentration de 10 fois la concentration finale, sont distribués dans une plaque blanche 96 puits à fond clair (CORNING 3604 Polystyrene Non-Binding Surface). Le binding non-spécifique est défini par NDP-αMSH à 10⁻⁷ M. Le binding total est mesuré par le nombre de coups par minute en présence du radio-ligand seul. La distribution de la suspension membranes-billes (50 µl/puits) est suivie par la distribution de la solution de [¹²⁵I]-[Nle⁴, D-Phe⁷]-α-MSH, 40 µl/puits (100 pM concentration finale), pour un volume final de 100 µl/puits. Après 6 h d'incubation à température ambiante, le comptage est fait dans un compteur de scintillation Microbeta TriLux. La valeur IC₅₀ des composés correspond à la concentration qui déplace la liaison spécifique du radio-ligand de 50%.

On détermine ainsi que les composés selon l'invention présentent une affinité pour les récepteurs MC3 et/ou MC4. Leurs IC₅₀ envers les récepteurs MC3 et MC4 sont inférieures à 10 µM, pour la plupart comprises entre 1 nM et 1 µM. A titre d'exemples, les composés n° 1, 2 et 12 du tableau présentent respectivement des IC₅₀ de 0,25 µM, 0,57 µM et 0,20 µM envers le récepteur MC4.

### Evaluation de l'activité agoniste des composés de formule (I) selon l'invention envers les récepteurs MC3 et MC4

Un test fonctionnel est utilisé pour discriminer l'activité agoniste de l'activité antagoniste. Pour cela, on dose la formation d'adénosine - mono-phosphate cyclique (AMPc) générée par l'activation du récepteur MC3 ou du récepteur MC4.

Les cellules CHO-K1, exprimant le récepteur humain MC4 à une densité modérée (Euroscreen), sont ensemencées dans le milieu de culture DMEM / Nutrient Mix F12 (Gibco/BRI) contenant 10% de sérum de veau, 0.5% pyruvate de sodium, 1% L-glutamine, 1% acides aminés non-essentiels, 200 mg/l hygromycine B et 0.5% PenStrep, ces produits étant fournis par Gibco/BRI, sauf le sérum de veau (Biowhittaker) et l'hygromycine B (Sigma).

Les cellules CHO(dhfr-) exprimant le récepteur humain MC3 sont ensemencées dans le milieu de culture MEM Eagle (Sigma) contenant 10% de sérum de veau dialysé, 1 % L-glutamine, 1 % pyruvate de sodium, 20mg/500 ml L-proline, 0.3 mg/ml Geneticin et 0.5% PenStrep, ces produits étant fournis par Gibco/BRI, sauf le sérum de veau dialysé (Cambrex) et la L-proline (Sigma).

Les composés à tester (dilués dans 10% DMSO), en quantité de 10 µl à une concentration de 10 fois la concentration finale, sont ajoutés aux plaques de cellules (volume final = 100 µl/puits). Après 1 heure d'incubation (37°C, 5% CO₂) la quantité du AMPc est dosée utilisant des kits du TROPIX (Appelera) selon la documentation du fournisseur. L'activité intrinsèque des composés est calculée en comparant la stimulation d'AMPc par ces composés à la stimulation induite par 30 nM de NDPαMSH (100% maximum). La valeur EC₅₀ des composés correspond à la concentration qui produit 50% de la stimulation maximale obtenue avec ce composé.

On détermine ainsi que les composés selon l'invention sont des agonistes des récepteurs MC3 et/ou MC4. Ils présentent des EC₅₀ envers les récepteurs MC3 et MC4 inférieures à 10 µM, pour la plupart comprises entre 1 nM et 1 µM. A titre d'exemples, les composés n° 1, 2 et 12 du tableau présentent respectivement des EC₅₀ de 0,20 µM, 0,11 µM et 0,10 µM envers le récepteur MC3, et de 0,05 µM, 0,06 µM et 0,02 µM envers le récepteur MC4.

Les composés selon l'invention présentant une activité agoniste des récepteurs aux mélanocortines, ils peuvent donc être utilisés pour la préparation de médicaments: Ainsi, selon un autre de ses aspects, l'invention a pour objet des médicaments qui comprennent un composé de formule (I), ou un sel d'addition de ce dernier à un acide pharmaceutiquement acceptable, ou encore un hydrate ou un solvat du composé de formule (I).

Ces médicaments trouvent leur emploi en thérapeutique, dans les pathologies dans lesquelles les récepteurs aux mélanocortines, en particulier les récepteurs MC3 et/ou MC4, sont impliqués: il s'agit notamment du traitement et de la prévention de l'obésité, du diabète et des dysfonctions sexuelles pouvant affecter les deux sexes, telles que les dysfonctionnements érectiles, les maladies cardiovasculaires telles que les infarcus du myocarde ou l'hypertension ainsi que dans des applications antiinflammatoires ou dans le traitement de la dépendance alcoolique.

Selon un autre de ses aspects, la présente invention concerne des compositions pharmaceutiques comprenant, en tant que principe actif, un composé selon l'invention: Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable. Lesdits excipients sont choisis selon la forme pharmaceutique et le mode d'administration souhaité, parmi les excipients habituels qui sont connus de l'Homme de l'art.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

Une forme d'administration préférée est la voie orale.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs 15,0 | mg |
| Hydroxypropyl-méthylcellulose | 2,25 mg |
| Stéarate de magnésium | 3,0 mg |

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvats.

## Revendications

1. Composé répondant à la formule (I) : 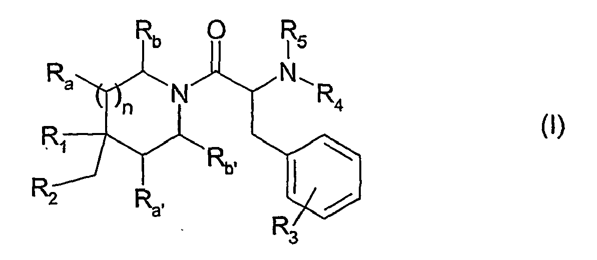 dans laquelle :
n est égal à 1,
Rₐ, R_{a'}, R_{b} et R_{b'} sont identiques ou différents les uns des autres et représentent un atome d'hydrogène ou un groupe alkyle ou cycloalkyle, R_{b} et R_{b'} pouvant former ensemble, avec les atomes de carbone du cycle auquel ils sont rattachés, un pont carboné comprenant de 4 à 5 chaînons,
R₁ représente un groupe alkyle ou cycloalkyle,
R₂ représente un groupe hétéroaryle,
R₃ représente 1 à 3 groupes, identiques ou différents les uns des autres, situés en des positions quelconques du noyau auquel ils sont rattachés et choisis parmi les atomes d'halogène et les groupes alkyles, cycloalkyles, -OR, -NRR', -CO-NRR', -NR-CO-R', -NR-CO-NRR', -NR-COOR', -NO₂, -CN et -COOR,
R₅ représente un atome d'hydrogène ou un groupe alkyle ou cycloalkyle,
R₄ est choisi parmi les groupes de formules (a), (b) et (c), ci-dessous, éventuellement substitués par un groupe oxo, ou mono ou polysubstitué par un groupe aryl ou hétéroaryle: 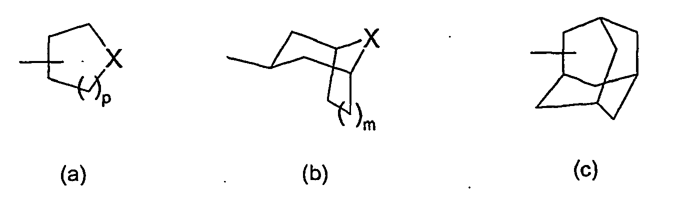
dans lesquelles:
p = 0, 1, 2 ou 3,
m = 0, 1 ou 2,
et soit
a) **X** représente un chaînon -N(R₁₀)-, où
**R₁₀** est choisi parmi :
un groupe -(CH₂)ₓ-OR₈, -(CH₂)ₓ-COOR₈, -(CH₂)ₓ-NR₈R₉, -(CH₂)ₓ-CO-NR₈R₉ ou -(CH₂)ₓ-NR₈-COR₉, -(CH₂)ₓ-COR₈ dans lesquels x = 1, 2, 3 ou 4,
un groupe cycloalkyle ou hétérocycloalkyle fusionné avec un groupe aryle ou hétéroaryle,
un groupe cycloalkyle, hétérocycloalkyle, aryle, hétéroaryle, alkylaryle, alkylhétéroaryle, -CO-alkyle, -CO-cycloalkyle, -CO-hétérocycloalkyle, -CO-aryle, -CO-hétéroaryle, -CO-alkylaryle, -CO-alkylhétéroaryle, -CS-alkyle, -CS-cycloalkyle, -CS-hétérocycloalkyle, -CS-aryle, -CS-hétéroaryle, -CS-alkylaryle, -CS-alkylhétéroaryle, -CS-NR₈R₉, -C(=NH)-NR₈R₉, -SO₂-alkyle -SO₂-cycloalkyle, -SO₂-hétérocycloalkyle, -SO₂-aryle, -SO₂-hétéroaryle, -SO₂-alkylaryle, -SO₂-alkylhétéroaryle ou -SO₂-NR₈R₉,
les groupes alkyles, cycloalkyles, hétérocycloalkyles, aryles ou hétéroaryles étant éventuellement substitués par 1 ou plusieurs groupes choisis parmi les atomes d'halogène et les groupes R , R', OR, NRR', -CO-NRR', -NRCOR', NRCONRR', -NO₂, CN, -COOR, OCOR,COR,, OCONRR', NRCOOR' ;
les groupes cycloalkyles ou hétérocycloalkyles étant éventuellement fusionnés avec un groupe aryle ou hétéroaryle ;
ou bien R₁₀ forme, avec l'atome d'azote auquel il est rattaché et un atome de carbone situé en une position quelconque de la structure cyclique de la formule (a), mais non adjacent audit atome d'azote, un pont comprenant de 3 à 5 chaînons,
**R₈** et **R₉** sont choisis indépendamment l'un de l'autre parmi un atome d'hydrogène et les groupes alkyles, cycloalkyles, hétérocycloalkyles, aryles, hétéroaryles, alkylaryles, alkylhétéroaryles, -CO-alkyles, -CO-cycloalkyles, -CO-hétérocycloalkyles, -CO-aryles, -CO-hétéroaryles, -CO-alkylaryles, -CO-alkylhétéroaryles, -SO₂-alkyles, -SO₂-cycloalkyles, -SO₂-hétérocycloalkyles, -SO₂-aryles, -SO₂-hétéroaryles, -SO₂-alkylaryles, -SO₂-alkylhétéroaryles, -C(=NH)-NRR', -COOR, -CO-NRR', -CS-NRR' et -(CH₂)ₓ-OR, où x = 0, 1, 2, 3 ou 4, les groupes alkyles, cycloalkyles, hétérocycloalkyles, aryles et hétéroaryles étant éventuellement substitués par un ou plusieurs groupes choisis parmi les atomes d'halogène et les groupes R , R', OR, NRR', - CO-NRR', -NRCOR', NRCONRR', -NO₂, CN, -COOR, OCOR,COR,, OCONRR', NRCOOR' ;
ou bien **R₈** et **R₉** forment ensemble un cycloalkyle ou un hétérocycloalkyle ;
**R** et **R**' représentent indépendamment l'un de l'autre un atome d'hydrogène ou un groupe alkyle, cycloalkyle, hétérocycloalkyle, aryle, hétéroaryle, alkylaryle ou alkylhétéroaryle, ou peuvent former ensemble un cycloalkyle ou un hétérocycloalkyle.
Soit,
b) **X** représente un chaînon -C(R₆)(R₇)- où
**R₆** est choisi parmi :
. un atome d'hydrogène, un atome d'halogène,
. un groupe -(CH₂)ₓ-OR₈, -(CH₂)ₓ-COOR₈, -(CH₂)ₓ-NR₈R₉, -(CH₂)ₓ-CO-NR₈R₉ ou -(CH₂)ₓ-NR₈-COR₉, dans lesquels x = 0, 1, 2, 3 ou 4,
. un groupe alkyle, cycloalkyle, hétérocycloalkyle, aryle, hétéroaryle, alkylaryle, alkylhétéroaryle, -CO-alkyle, -CO-cycloalkyle, -CO-hétérocycloalkyle, -CO-aryle, -CO-hétéroaryle, -CO-alkylaryle ou -CO-alkylhétéroaryle, -CS-alkyle, -CS-cycloalkyle, -CS-hétérocycloalkyle, -CS-aryle, -CS-hétéroaryle, -CS-alkylaryle, -CS-alkylhétéroaryle, -CS-NR₈R₉, -C(=NH)-NR₈R₉,
. un groupe cycloalkyle ou hétérocycloalkyle fusionné ou non situé en position spiro sur le cycle de formule (a) auquel il est rattaché,
. un groupe cycloalkyle ou hétérocycloalkyle fusionné avec un groupe aryle ou hétéroaryle,
les groupe alkyles, cycloalkyles, hétérocycloalkyles, aryles ou hétéroaryles étant éventuellement substitués par 1 ou plusieurs groupes choisis parmi les atomes d'halogène et les groupes R , R', OR, NRR', -CO-NRR', -NRCOR', NRCONRR', -NO₂, CN, -COOR, OCOR, COR, OCONRR', NRCOOR' ;
les groupes cycloalkyles ou hétérocycloalkyles étant éventuellement fusionnés avec un groupe aryle ou hétéroaryle,
**R**₇ est choisi parmi les atomes d'hydrogène et d'halogène et les groupes alkyles, cycloalkyles, aryles, hétéroaryles, alkylaryles, alkylhétéroaryles, -OR, -O-aryles, -O-hétéroaryles, -O-alkylaryles, -O-alkylhétéroaryles, -NRR', -CO-NRR', -NR-CO-R', -NR-CO-NRR', -NR-COOR', -NO₂, -CN et-COOR,
**R₈** et **R₉** sont choisis indépendamment l'un de l'autre parmi un atome d'hydrogène et les groupes alkyles, cycloalkyles, hétérocycloalkyles, aryles, hétéroaryles, alkylaryles, alkylhétéroaryles, -CO-alkyles, -CO-cycloalkyles, -CO-hétérocycloalkyles, -CO-aryles, -CO-hétéroaryles, -CO-alkylaryles, -CO-alkylhétéroaryles, -SO₂-alkyles, -SO₂-cycloalkyles, -SO₂-hétérocycloalkyles, -SO₂-aryles, -SO₂-hétéroaryles, -SO₂-alkylaryles, -SO₂-alkylhétéroaryles, -C(=NH)-NRR', -COOR, -CO-NRR', -CS-NRR' et -(CH₂)ₓ-OR, où x = 0, 1, 2, 3 ou 4, les groupes alkyles, cycloalkyles, hétérocycloalkyles, aryles et hétéroaryles étant éventuellement substitués par un ou plusieurs groupes choisis parmi les atomes d'halogène et les groupes R , R', OR, NRR', - CO-NRR', -NRCOR', NRCONRR', -NO₂, CN, -COOR, OCOR, COR, OCONRR', NRCOOR';
ou bien **R₈** et **R₉** forment ensemble un cycloalkyle ou un hétérocycloalkyle ;
**R** et **R**' représentent indépendamment l'un de l'autre un atome d'hydrogène ou un groupe alkyle, cycloalkyle, hétérocycloalkyle, aryle, hétéroaryles, alkylaryle ou alkylhétéroaryle, ou peuvent former ensemble un cycloalkyle ou un hétérocycloalkyle.
à l'état de base ou de sel d'addition à un acide, ainsi qu'à l'état d'hydrate ou de solvat.

2. Composé de formule (I) selon la revendication 1, **caractérisé en ce que R₄** est choisi parmi les groupes de formules (a), (b) et (c) éventuellement mono ou polysubstitué par un groupe aryl ou hétéroaryle où X représente un chaînon -C(R₆)(R₇)-, dans lequel
**R₆** est choisi parmi ;
. un atome d'hydrogène,
. un groupe -(CH₂)ₓOR₈, -(CH₂)ₓ-COOR₈, -(CH₂)ₓ-NR₈R₉, -(CH₂)ₓ-CO-NR₈R₉ ou -(CH₂)ₓ-NR₈-COR₉, dans lesquels x = 0, 1, 2, 3 ou 4,
. un groupe alkyle, cycloalkyle, hétérocycloalkyle, aryle, hétéroaryle, alkylaryle, alkylhétéroaryle, -CO-alkyle, -Co-cycloalkyle, -CO-hétérocycloalkyle, -co-aryle, -CO-hétéroaryle, -CO-alkylaryle ou -CO-alkylhétéroaryle,
. un groupe cycloalkyle ou hétérocycloalkyle situé en position spiro sur le cycle de formule (a) auquel il est rattaché,
. un groupe cycloalkyle ou hétérocycloalkyle fusionné avec un groupe aryle ou hétéroaryle,
**R₇** est choisi parmi les atomes d'hydrogène et d'halogène et les groupes alkyles, cycloalkyles, aryles, hétéroaryles, alkylaryles, alkylhétéroaryles, -OR, -O-aryles, -O-hétéroaryles, -O-alkylaryles, -O-alkylhétéroaryles, -NRR', -CO-NRR', -NR-CO-R', -NR-CO-NRR', -NR-COOR', -NO₂, -CN et -COOR,
**R₈** et **R₉** sont choisis indépendamment l'un de l'autre parmi un atome d'hydrogène et les groupes alkyles, cycloalkyles, hétérocycloalkyles, aryles, hétéroaryles, alkylaryles, alkylhétéroaryles, -CO-alkyles, -CO-cycloalkyles, -CO-hétérocycloalkyles, -CO-aryles, -CO-hétéroaryles, -CO-alkylaryles, -CO-alkylhétéroaryles, -SO₂-alkyles, -SO₂-cycloalkyles, -SO₂-hétérocycloalkyles, -SO₂-aryles, -SO₂-hétéroaryles, -SO₂-alkylaryles, -SO₂-alkylhétéroaryles, -C(=NH)-NRR', -COOR, -CO-NRR', -CS-NRR' et -(CH₂)ₓ-OR, où x = 0, 1, 2, 3 ou 4 ;
**R** et **R**' représentent indépendamment l'un de l'autre un atome d'hydrogène ou un groupe alkyle, cycloalkyle, hétérocycloalkyle, aryle, hétéroaryle, alkylaryle ou alkylhétéroaryle.

3. Composé de formule (I) selon la revendication 1, **caractérisé en ce que R₄** est choisi parmi les groupes de formules (a), (b) et (c) où X représente un chaînon - C(R₆)(R₇)-, dans lequel R₆ est choisi parmi un atome d'halogène ou un groupe cycloalkyle ou hétérocycloalkyle fusionné ou non situé en position spiro sur le cycle de formule (a) auquel il est rattaché.

4. Composé de formule (I) selon la revendication 1, **caractérisé en ce que R**₄ est choisi parmi les groupes de formules (a), (b) et (c) où X représente un chaînon - C(R₆)(R₇)-, dans lequel R6 est choisi parmi -CS-alkyle, -CS-cycloalkyle, -CS-hétérocycloalkyle, -CS-aryle, -CS-hétéroaryle, -CS-alkylaryle, -CS-alkylhétéroaryle, -CS-NR₈R₉, -C(=NH)-NR₈R₉.

5. Composé de formule (I) selon la revendication 1, **caractérisé en ce que R₄** est choisi parmi les groupes de formules (a), (b) et (c) où X représente un chaînon - C(R₆)(R₇)-, dans lequel les groupes alkyles, cycloalkyles, hétérocycloalkyles, aryles ou hétéroaryles sont éventuellement substitués par 1 ou plusieurs groupes choisis parmi R ou R', OCOR, COR; OCONRR', NRCOOR'.

6. Composé de formule (I) selon la revendication 1, **caractérisé en ce que R₄** est choisi parmi les groupes de formules (a), (b) et (c) où X représente un chaînon - C(R₆)(R₇)-, dans lequel les groupes cycloalkyles ou hétérocycloalkyles sont éventuellement fusionnés avec un groupe aryle ou hétéroaryle.

7. Composé de formule (I) selon la revendication 1, **caractérisé en ce R₄** est choisi parmi les groupes de formules (a), (b) et (c) où X représente un chaînon -C(R₆)(R₇)-, dans lequel **R₈** et **R₉** choisis indépendamment l'un de l'autre représentent des groupes alkyles, cycloalkyles, hétérocycloalkyles, aryles et hétéroaryles étant éventuellement substitués par un ou plusieurs groupes choisis parmi les groupes R , R', COR, OCOR, OCONRR', NRCOOR' ;
ou bien **R₈** et **R₉** forment ensemble un cycloalkyle ou un hétérocycloalkyle.

8. Composé de formule (I) la revendication 1, **caractérisé en ce que R₄** est choisi parmi les groupes de formules (a), (b) et (c) où X représente un chaînon -C(R₆)(R₇)-, dans lequel **R** et **R**' peuvent former ensemble un cycloalkyle ou un hétérocycloalkyle.

9. Composé de formule (I) selon l'une quelconque des revendications 1 à 8, **caractérisé en ce que R₇** est l'hydrogène.

10. Composé de formule (I) selon l'une des revendications 1 à 9, **caractérisé en ce que R₄** représente le groupe de formule a) où p=2 tel que défini ci-dessous :

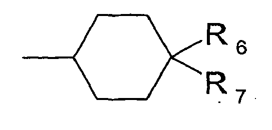

11. Composé de formule (I) selon la revendication 1, **caractérisé en ce que**
**R₄** est choisi parmi les groupes de formules (a), (b) et (c) éventuellement mono ou polysubstitué par un groupe aryl ou hétéroaryle où X représente un chaînon -N(R₁₀)-dans lequel
**R₁₀** est choisi parmi :
un groupe -CO-NR₈R₉, -COOR₈
un groupe -(CH₂)ₓ-OR₈, -(CH₂)ₓ-COOR₈, -(CH₂)ₓ-NR₈R₉, -(CH₂)ₓ-CO-NR₈R₉ ou -(CH₂)ₓ-NR₈-COR₉, dans lesquels x = 1, 2, 3 ou 4,
. un groupe cycloalkyle ou hétérocycloalkyle fusionné avec un groupe aryle ou hétéroaryle,
un groupe cycloalkyle, hétérocycloalkyle, aryle, hétéroaryle, alkylaryle, alkylhétéroaryle, , -CO-cycloalkyle, -CO-hétérocycloalkyle, , -CO-hétéroaryle, -CO-alkylaryle, -CO-alkylhétéroaryle, -CS-alkyle, -CS-cycloalkyle, -CS-hétérocycloalkyle, -CS-aryle, -CS-hétéroaryle, -CS-alkylaryle, -CS-alkylhétéroaryle, -CS-NR₈R₉, -C(=NH)-NR₈R₉, , -SO₂-cycloalkyle, -SO₂-hétérocycloalkyle, , -SO₂-hétéroaryle, -SO₂-alkylaryle, -SO₂-alkylhétéroaryle ou -SO₂-NR₈R₉ ;
ou bien **R₁₀** forme, avec l'atome d'azote auquel il est rattaché et un atome de carbone situé en une position quelconque de la structure cyclique de la formule (a), mais non adjacent audit atome d'azote, un pont comprenant de 3 à 5 chaînons.
**R₈** et **R₉** sont choisis indépendamment l'un de l'autre parmi un atome d'hydrogène et les groupes alkyles, cycloalkyles, hétérocycloalkyles, aryles, hétéroaryles, alkylaryles, alkylhétéroaryles, -CO-alkyles, -CO-cycloalkyles, -CO-hétérocycloalkyles, -CO-aryles, -CO-hétéroaryles, -CO-alkylaryles, -CO-alkylhétéroaryles, -SO₂-alkyles, -SO₂-cycloalkyles, -SO₂-hétérocycloalkyles, -SO₂-aryles, -SO₂-hétéroaryles, -SO₂-alkylaryles, -SO₂-alkylhétéroaryles, -C(=NH)-NRR', -COOR, -CO-NRR', -CS-NRR' et -(CH₂)ₓ-OR, où x = 0, 1, 2, 3 ou 4 ;
**R** et **R**' représentent indépendamment l'un de l'autre un atome d'hydrogène ou un groupe alkyle, cycloalkyle, hétérocycloalkyle, aryle, hétéroaryle, alkylaryle ou alkylhétéroaryle.

12. Composé de formule (I) selon la revendication 1, **caractérisé en ce que R₄** est choisi parmi les groupes de formules (a), (b) et (c) éventuellement substitué par un groupe oxo où X représente un chaînon -N(R₁₀).

13. Composé de formule (I) selon la revendication 1, **caractérisé en ce que R₄** est choisi parmi les groupes de formules (a), (b) et (c) où X représente un chaînon -N(R₁₀)-dans lequel **R₈** et **R₉** choisis indépendamment l'un de l'autre représentent des groupes alkyles, cycloalkyles, hétérocycloalkyles, aryles et hétéroaryles étant éventuellement substitués par un ou plusieurs groupes choisis parmi les atomes d'halogène et les groupes R , R', OR, NRR', -CO-NRR', -OCOR, NRCOR', NRCONRR', COR, -NO₂, CN, - COOR, OCONRR', NRCOOR' ;
ou bien **R₈** et **R₉** forment ensemble un cycloalkyle ou un hétérocycloalkyle.

14. Composé de formule (I) selon la revendication 1, **caractérisé en ce que R₄** est choisi parmi les groupes de formules (a), (b) et (c) où X représente un chaînon -N(R₁₀)-dans lequel **R₁₀** est, -(CH₂)ₓ-COR₈ dans lequel x = 1, 2, 3 ou 4.

15. Composé de formule (I) selon la revendication 1, **caractérisé en ce que R₄** est choisi parmi les groupes de formules (a), (b) et (c) où X représente un chaînon -N(R₁₀)-dans lequel les groupes alkyles, cycloalkyles, hétérocycloalkyles, aryles ou hétéroaryles sont éventuellement substitués par 1 ou plusieurs groupes choisis parmi R, R' OCOR, COR, OCONRR' ou NRCOOR'.

16. Composé de formule (I) selon la revendication 1, **caractérisé en ce que R₄** est choisi parmi les groupes de formules (a), (b) et (c) où X représente un chaînon -N(R₁₀)-dans lequel les groupes cycloalkyles ou hétérocycloalkyles sont éventuellement fusionnés avec un groupe aryle ou hétéroaryle.

17. Composé de formule (I) selon l'une des revendications 1, 11 à 16 **caractérisé en ce que R₄** représente le groupe de formule a) où p=2 tel que défini ci-dessous :

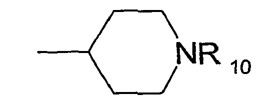

18. Composé de formule (I) selon l'une quelconque des revendications 1 à 17, **caractérisé en ce que R₁** représente un groupe cycloalkyle,
à l'état de base ou de sel d'addition à un acide, ainsi qu'à l'état d'hydrate ou de solvat.

19. Composé de formule (I) selon l'une quelconque des revendications 1 à 18, **caractérisé en ce que R₂** représente un groupe triazolyle,
à l'état de base ou de sel d'addition à un acide, ainsi qu'à l'état d'hydrate ou de solvat.

20. Composé de formule (I) selon l'une quelconque des revendications 1 à 19, **caractérisé en ce que R₃** représente 1 à 3 groupes, identiques ou différents les uns des autres, choisis parmi les atomes d'halogène,
à l'état de base ou de sel d'addition à un acide, ainsi qu'à l'état d'hydrate ou de solvat.

21. Composé de formule (I) selon l'une quelconque des revendications 1 à 20, **caractérisé en ce que R₅** représente un atome d'hydrogène,
à l'état de base ou de sel d'addition à un acide, ainsi qu'à l'état d'hydrate ou de solvat.

22. Composé de formule (I) selon l'une quelconque des revendications 1 à 21, **caractérisé en ce que** :
- n = 1 et Rₐ = R_{a'} = R_{b} = R_{b'} = H, ou
- n = 1, Rₐ = R_{a'} = H et R_{b} et R_{b'} forment ensemble, avec les atomes de carbone du cycle auquel ils sont rattachés, un pont carboné comprenant 4 chaînons,
à l'état de base ou de sel d'addition à un acide, ainsi qu'à l'état d'hydrate ou de solvat.

23. Composés dont les noms suivent
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(2-phenylethyl)piperidin-4-amine
4-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]phenol
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}cyclohexane-1,4-diamine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-8-methyl-8-azabicyclo[3.2.1]octan-3-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-9-methyl-9-azabicyclo[3.3.1]nonan-3-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-phenylpiperidin-4-amine
1-benzoyl-*N*-{(1*R*)-1-(4-chlorobenzyl)-2-[4-cyclohexyl-4-(1*H-*1,2,4-triazol-1-ylmethyl)piperidin-1-yl]-2-oxoethyl}piperidin-4-amine
1-acetyl-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1,4-dioxaspiro[4.5]decan-8-amine
*N*'-{(1*R*)-1-(4-chlorobenzyl)-2-[4-cyclohexyl-4-(1*H*-1,2,4-triazol-1-ylmethyl)piperidin-1-yl]-2-oxoethyl}-*N*,*N*-dimethylcyclohexane-1,4-diamine
4-(aminomethyl)-*N*-{(1*R*)-1-(4-chlorobenzyl)-2-[4-cyclohexyl-4-(1*H*-1,2,4-triazol-1-ylmethyl)piperidin-1-yl]-2-oxoethyl}cyclohexanamine
3-({(1*R*)-1-(4-chlorobenzyl)-2-[4-cyclohexyl-4-(1*H*-1,2,4-triazol-1-ylmethyl)piperidin-1-yl]-2-oxoethyl}amino)-8-methyl-8-azabicyclo[3.2.1]octan-6-ol
*cis*- 4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanol
*trans-* 4-({(1*R*)-1-(4-chlorobenzyl)-2-[4-cyclohexyl-4-(1*H*-1,2,4-tiazol-1-ylmethyl)piperidin-1-yl]-2-oxoethyl}amino)cyclohexanol
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(trifluoroacetyl)piperidin-4-amine
4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidine-1-carboxamide
*cis*- 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-1-phenylcyclohexanol
*trans-* 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-1-phenylcyclohexanol
*cis* -*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-fluorophenyl)cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-fluorophenyl)cyclohexane-1,4-diamine
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2,2,2-trifluoroacetamide
*N*-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2,2,2-trifluoroacetamide
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]acetamide
*N*-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]acetamide
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(4-fluorobenzoyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(cyclopentylcarbonyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(cyclobutylcarbonyl)piperidin-4-amine
*cis*-*N*-{(1*R*)-1-(4-chlorobenzyl)-2-[4-cyclohexyl-4-(1*H*-1,2,4-triazot-1-ylmethyl)piperidin-1-yl]-2-oxoethyl}-*N*'-methylcyclohexane-1,4-diamine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(pyridin-2-ylcarbonyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(phenylacetyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(methylsulfonyl)piperidin-4-amine
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-*N*-methylacetamide
*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(1,3-dihydro-2*H*-isoindol-2-yl)cyclohexanamine
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-*N*-methylbenzamide
ethyl *cis*-4-({(1*R*)-1-(4-chlorobenzyl)-2-[4-cyclohexyl-4-(1*H*-1,2,4-triazot-1-ylmethyl)piperidin-1-yl]-2-oxoethyl}amino)cyclohexanecarboxylate
ethyl *trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarboxylate
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N'*-phenylcyclohexane-1,4-diamine
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N'*-phenylcyclohexane-1,4-diamine
*N'*-{(1*R*)-1-(4-chlorobenzyl)-2-[4-cyclohexyl-4-(1*H*-1,2,4-triazol-1-ylmethyl)piperidin-1-yl]-2-oxoethyl}-*N*-methyl-*N*-phenylcyclohexane-1,4-diamine
*N'*-{(1*R*)-1-(4-chlorobenzyl)-2-[4-cyclohexyl-4-(1*H*-1,2,4-triazol-1-ylmethyl)piperidin-1-yl]-2-oxoethyl}-*N*-(4-fluorophenyl)-*N*-methylcyclohexane-1,4-diamine
*cis ou trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*,*N*-diethylcyclohexanecarboxamide
*cis ou trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*,*N*-diethylcyclohexanecarboxamide
*cis ou trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*,*N*-dimethylcyclohexanecarboxamide
*cis ou trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N,N*-dimethylcyclohexanecarboxamide
*cis*-*N*-benzyl-4-({(1*R*)-1-(4-chlorobenzyl)-2-[4-cydohexyl-4-(1*H*-1,2,4-triazol-1-ylmethyl)piperidin-1-yl]-2-oxoethyl}amino)-*N*-methylcyclohexanecarboxamide
*trans*-*N*-benzyl-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-methylcyclohexanecarboxamide
*cis*-*N*-{(1*R*)-1-(4-chorobenzyl)-2-[4-cyclohexyl-4-(1*H*-1,2,4-triazol-1-ylmethyl)piperidin-1-yl]-2-oxoethyl}-4-(3-methyl-1,2,4-oxadiazol-5-yl)cyclohexanamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(3-methyl-1,2,4-oxadiazol-5-yl)cyclohexanamine
*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarboxamide
*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarboxamide
*N*-{(1*R*)-(4-chlorobenzyl)-2-[4-cyclohexyl-4-(1*H*-1,2,4-triazol-1-ylmethyl)piperidin-1-yl]-2-oxoethyl}-1-isonicotinoylpiperidin-4-amine
*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-1-(4-fluorophenyl)cyclohexanol
*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-1-(4-fluorophenyl)cyclohexanol
*N*-{(1*R*)-1-(4-chlorobenzyl)-2-[4-cyclohexyl-4-(1*H*-1,2,4-triazo)-1 - ylmethyl)piperidin-1-yl]-2-oxoethyl}-1-[(1-methyl-1*H*-imidazol-2-yl)carbonyl]piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(5-methylisoxazol-3-yl)carbonyl]piperidin-4-amine
*N*-{(1*R*)1-(4-chlorobenzyl)-2-[4-cyclohexyl-4-(1*H*-1,2,4-triazol-1-ylmethyl)piperidin-1-yl]-2-oxoethyl}-1-(3,4-difluorobenzoyl)piperidin-4-amine
1-[(1-*tert*-butyl-5-methyl-1*H*-pyrazol-3-yl)carbonyl]-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
*N*-{(1*R*)-1(4-chlorobenzyl)-2-[4-cyclohexyl-4-(1*H*-1,2,4-triazol-1-ylmethyl)piperidin-1-yl]-2-oxoethyl}-1-[(3,5-dimethylisoxazol-4-yl)carbonyl]piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(3-thienylcarbonyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethy}1-(pyrrolidin-1-ylcarbonyl)piperidin-4-amine
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-phenylpiperidine-1-carboxamide
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N,N*-dimethylpiperidine-1-carboxamide
4-({(1*R*)-1-(4-chlorobenzyl)-2-[4-cyclohexyl-4-(1*H*-1,2,4-triazol-1-ylmethyl)piperidin-1-yl]-2-oxoethyl}amino)*-N,N*-diethylpiperidine-1-carboxamide
*N*-{(1*R*)1-(4-chlorobenzyl)-2-[4-cyclohexyl-4-(1*H*-1,2,4-triazol-1-ylmethyl)piperidin-1-yl]-2-oxoethyl}-1-(piperidin-1-ylcarbonyl)piperidin-4-amine
*N*-{(1*R*)1-(4-chlorobenzyl)-2-[4-cyclohexyl-4-(1*H*-1,2,4-triazol-1-ylmethyl)piperidin-1-yl]-2-oxoethyl}-1-(morpholin-4-ylcarbonyl)piperidin-4-amine
4-({(1*R*)1-(4-chlorobenzyl)-2-[4-cyclohexyl-4-(1*H*-1,2,4-triazol-1-ylmethyl)piperidin-1-yl]-2-oxoethyl}amino)-*N*-methyl-*N*-phenylpiperidine-1-carboxamide
*N*-benzyl-4-({(1*R*)-1-(4-chlorobenzyl)2-[4-cyclohexyl-4-(1*H*-1,2,4-triazol-1-ylmethyl)piperidin-1-yl]-2-oxoethyl}amino)-*N*-methylpiperidine-1-carboxamide
*N*-{(1*R*)1-(4-chlorobenzyl)-2-[4-cyclohexyl-4-(1*H*-1,2,4-triazol-1-ylmethyl)piperidin-1-yl]-2-oxoethyl}-4-methoxycyclohexanamine
4-[4-(benzyloxy)phenyl]-*N*-{(1*R*)-1-(4-chlorobenzyl)-2-[4-cyclohexyl-4-(1*H*-1,2,4-triazol-1-ylmethyl)piperidin-1-yl]-2-oxoethyl}cyclohexanamine
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2-methoxyacetamide
2-{[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-2-oxoethyl acetate
2-(benzyloxy)-*N*-[*cis*-4-({(1*R*)-1-(4-chlorobenzyl)-2-[4-cyclohexyl-4-(1*H-*1,2,4-triazol-1-ylmethyl)piperidin-1-yl]-2-oxoethyl}amino)cyclohexyl]acetamide
3-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-1,3-oxazolidin-2-one
3-[*trans*-4-({(1*R*)-(4-chlorobenzyl)-2-[4-cyclohexyl-4-(1*H*-1,2,4-triazol-1-ylmethyl)piperidin-1-yl]-2-oxoethyl}amino)cyclohexyl]-1,3-oxazolidin-2-one
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(2-methoxyethyl)cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(2-methoxyethyl)cyclohexane-1,4-diamine
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-morpholin-4-ylcyclohexanamine
*trans*-*N-*{(1*R*)-1-(4-chlorobenzyl)-2-[4-cyclohexyl-4-(1*H*-1,2,4-triazol-1-ylmethyl)piperidin-1-yl]-2-oxoethy}-4-morpholin-4-ylcyclohexanamine
1-[*cis*-4-({(1*R*)-1-(4-chlorobenzyl)-2-[4-cyclohexyl-4-(1*H-*1,2,4-triazol-1-ylmethl)piperidin-1-yl]-2-oxoethyl}amino)cyclohexyl]pyrrolidin-2-one
1-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]pyrrolidin-2-one
*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(2-methoxyethoxy)cyclohexanamine
ethyl 4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidine-1-carboxylate
methyl 4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidine-1-carboxylate
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(2S)-piperidin-2-ylcarbonyl]piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(2*R*)-piperidin-2-ylcarbonyl]piperidin-4-amine
*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarbonitrile
*trans*-4-({(1*R*)-1-(4-chlorobenzyl)-2-[4-cyclohexyl-4-(1*H-*1,2,4-triazol-1-ylmethyl)piperidin-1-yl]-2-oxoethyl}amino)cyclohexanecarbonitrile
1-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexy]piperidin-2-one
1-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]piperidin-2-one
*N*-[4-({(*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]propanamide
*tert*-butyl(2-{[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyljamino}-2-oxoethyl)carbamate
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]glycinamide
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2-hydroxyacetamide
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2,2-dimethylpropanamide
*N*-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2,2-dimethylpropanamide
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-methoxyphenyl)cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-methoxyphenyl)cyclohexane-1,4-diamine
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(2*H*-tetrazol-5-yl)cyclohexanamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(2*H-*tetrazol-5-yl)cyclohexanamine
2-{[*cis*-4-({(1*R*)1-(4-chlorobenzyl)-2-[4-cyclohexyl-4-(1*H*-1,2,4-triazol-1-ylmethyl)piperidin-1-yl]-2-oxoethyl}amino)cyclohexyl]amino}phenol
2-{[*trans*-4-({(1*R*)-(4-chlorobenzyl)-2-[4-cydohexyl-4-(1*H*-1,2,4-triazol-1-ylmethyl)piperidin-1-yl]-2-oxoethyl}amino)cyclohexyl]amino}phenol
4-({(1*R*)-(4-chlorobenzyl)-2-[4-cyclohexyl-4-(1*H*-1,2,4-triazol-1-ylmethyl)piperidin-1-yl]-2-oxoethyl}amino)cyclohexyl acetate
*N*²-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-*N*,*N*-dimethylglycinamide
*N*²-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-*N*,*N*-dimethylglycinamide
*N*²-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]glycinamide
*N*²-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]glycinamide
4-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]piperazin-2-one
4-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]piperazin-2-one
*cis*-4-(4-acetylpiperazin-1-yl)-*N*-{(1*R*)-1-(4-chlorobenzyl)-2-[4-cyclohexyl-4-(1*H-*1,2,4-triazol-1-ylmethyl)piperidin-1-yl]-2-oxoethyl}cyclohexanamine
*trans*-4-(4-acetylpiperazin-1-yl)-*N*-{(1*R*)-1-(4-chlorobenzyl)-2-[4-cyclohexyl-4-(1*H*-1,2,4-triazol-1-ylmethyl)piperidin-1-yl]-2-oxoethyl}cyclohexanamine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-pyridin-2-ylpiperidin-4-amine
methyl *N*-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]glycinate
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(2,2-difluoroethyl)piperidin-4-amine
*cis*-*N*{(1*R*)-1-(4-chlorobenzyl)-2-[4-cyclohexyl-4-(1*H* 1,2,4-triazol-1-ylmethyl)piperidin-1-yl]-2-oxoethyl}-*N*'-(4-chlorophenyl)cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-chlorophenyl)cyclohexane-1,4-diamine
4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl pivalate
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-fluoro-2-methylphenyl)cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-fluoro-2-methylphenyl)cyclohexane-1,4-diamine
4-{[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}benzonitrile
*N*-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]cyclopropanecarboxamide
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(6-methylpyridazin-3-yl)piperidin-4-amine
methyl [4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidin-1-yl]acetate
*cis* ou *trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-morpholin-4-ylphenyl)cyclohexane-1,4-diamine
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-(2,4-difluorophenyl)cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(2,4-difluorophenyl)cyclohexane-1,4-diamine
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(5-fluoropyridin-2-yl)cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(5-fluoropyridin-2-yl)cyclohexane-1,4-diamine
*N*-1*H*-1,2,3-benzotriazol-5-yl-*N*'-{(1*R*)-1-(4-chlorobenzyl)-2-[4-cyclohexyl-4-(1*H-*1,2,4-triazol-1-ylmethyl)piperidin-1-yl]-2-oxoethyl}cyclohexane-1,4-diamine
*N*-{(1*R*)-1-(4-chlorobenzyl)-2-[4-cyclohexyl-4-(1*H*-1,2,4-triazol-1-ylmethyl)piperidin-1-yl]-2-oxoethy}-1-pyrimidin-2-ylpiperidin-4-amine
1-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]imidazolidin-2-one
1-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}lamino)cyclohexyl]imidazolidin-2-one
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-cyclopropylpiperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-{[(2*S*)-4,4-difluoropiperidin-2-yl]carbonyl}piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-4,4-difluorocyclohexanamine
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(3,4-difluorophenyl)cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*-(3,4-difluorophenyl)cyclohexane-1,4-diamine
*cis*-*N*-{(1*R*)-1-(4-chlorobenzyl)-2-[4-cyclohexyl-4-(1*H-*1,2,4-triazol-1-ylmethyl)piperidin-1-yl]-2-oxoethyl}-*N*'-(4-fluoro-3-methoxyphenyl)cyclohexane-1,4-diamine
*trans*-*N*-{(1*R*)-(4-chlorobenzyl)-2-[4-cyclohexyl-4-(1*H*-1,2,4-triazol-1-ylmethyl)piperidin-1-yl]-2-oxoethyl}-*N*'-(4-fluoro-3-methoxyphenyl)cyclohexane-1,4-diamine
*cis*-*N*'-{(1*R*)-1-(4-chlorobenzyl)-2-[4-cyclohexyl-4-(1*H*-1,2,4-triazol-1-ylmethyl)piperidin-1-yl]-2-oxoethyl}-*N*-ethyl-*N*-(4-fluoro-3-methoxyphenyl)cyclohexane-1,4-diamine
*trans*-*N*'-{(1*R*)-1-(4-chlorobenzyl)-2-[4-cyclohexyl-4-(1*H*-1,2,4-triazol-1-ylmethyl)piperidin-1-yl]-2-oxoethyl}-*N*-ethyl-*N*-(4-fluoro-3-methoxyphenyl)cyclohexane-1,4-diamine
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-[4-(trifluoromethyl)phenyl]cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-[4-(trifluoromethyl)phenyl]cyclohexane-1,4-diamine
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-fluoro-3-methylphenyl)cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-fluoro-3-methylphenyl)cyclohexane-1,4-diamine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(4,4-difluoro-L-prolyl)piperidin-4-amine
1-(1*H*-benzimidazol-2-yl)-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
1-(2,1-benzisoxazol-3-ylcarbonyl)-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
1-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidin-1-yl]butan-2-one
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(2,2,2-trifluoroethyl)piperidin-4-amine
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(4-methoxyphenyl)piperidine-1-carboxamide
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(4-fluorophenyl)piperidine-1-carboxamide
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(2,4-difluorophenyl)piperidine-1-carboxamide
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(3,4-difluorophenyl)piperidine-1-carboxamide
4-({(1*R*)-1-(4-chlorobenzyl)-2-[4-cyclohexyl-4-(1H-1,2,4-triazo(-1-ylmethyl)piperidin-1-yl]-2-oxoethyl}amino)-*N*-(2-fluorophenyl)piperidine-1-carboxamide
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(2-methoxyphenyl)piperidine-1-carboxamide
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-[4-(dimethylamino)phenyl]piperidine-1-carboxamide
*N*-{(1*R*)1-(4-chlorobenzyl)-2-[4-cyclohexyl-4-(1*H*-1,2,4-triazol-1-ylmethyl)piperidin-1-yl]-2-oxoethyl}-1-[(5-fluoro-1*H*-indol-2-yl)carbonyl]piperidin-4-amine
*N*-{(1*R*)1-(4-chlorobenzyl)-2-[4-cyclohexyl-4-(1*H*-1,2,4-triazol-1-ylmethyl)piperidin-1-yl]-2-oxoethyl}-1-(pyrazin-2-ylcarbonyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(5-phenyl-1,3-oxazol-4-yl)carbonyl]piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(isoxazol-5-ylcarbonyl)piperidin-4-amine
3-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-6-fluoro-1,3-benzoxazol-2(3*H*)-one
3-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-6-fluoro-1,3-benzoxazol-2(3*H*)-one
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]pyridine-2-carboxamide
2-{[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-5-fluorophenol ou
2-{[*trans*-4-({(1*R*)1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-5-fluorophenol
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(quinolin-2-ylcarbonyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(3-methylpyridin-2-yl)carbonyl]piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(1*H*-1,2,4-triazol-3-ylcarbonyl)piperidin-4-amine
*N*-[*cis*-4-({(1*R*)1-(4-chlorobenzyl)-2-[4-cyclohexyl-4-(1*H*-1,2,4-triazol-1-ylmethyl)piperidin-1-yl]-2-oxoethyl}amino)cyclohexyl]-2,1-benzisoxazole-3-carboxamide
1-(1,3-benzothiazol-2-ylcarbonyl)-*N*-{(*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
*N*-{(1*R*)-(4-chlorobenzyl)-2-[4-cyclohexyl-4-(1*H*-1,2,4-triazol-1-ylmethyl)piperidin-1-yl]-2-oxoethyl}-1-[(6-methylpyridin-2-yl)carbonyl]piperidin-4-amine
1-(1-benzofuran-2-ylcarbonyl)-*N*-{(1*R*)-1-(4-chlorobenzyl)-2-[4-cydohexyl-4-(1*H-*1,2,4-triazol-1-ylmethyl)piperidin-1-yl]-2-oxoethyl}piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(6-fluoropyridin-2-yl)carbonyl]piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(1-phenyl-1*H*-pyrazol-5-yl)carbonyl]piperidin-4-amine
*N*{(1*R*)1-(4-chlorobenzyl)-2-[4-cyclohexyl-4-(1*H*-1,2,4-triazol-1-ylmethyl)piperidin-1-yl]-2-oxoethyl}-1-(2,4-difluorobenzoyl)piperidin-4-amine
*cis*-*N*-(1,3-benzothiazol-2-ylmethyl)-*N*'-{(1*R*)-1-(4-chlorobenzyl)-2-[4-cyclohexyl-4-(1*H*-1,2,4-triazol-1-ylmethyl)piperidin-1-yl]-2-oxoethyl}cyclohexane-1,4-diamine
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(1,3-thiazol-2-ylmethyl)cyclohexane-1,4-diamine
2-{[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-5-fluoro-*N*,*N* dimethylbenzamide
2-{[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-5-fluoro-*N*,*N* dimethylbenzamide
*N*-{(1*R*)-1-(4-chlorobenzyl)-2-[4-cyclohexyl-4-(1*H-*1,2,4-triazol-1-ylmethyl)piperidin-1-yl]-2-oxoethyl}-1-[(6-phenylpyridin-2-yl)carbonyl]piperidin-4-amine
*trans-N*-(*tert*-butyl-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarboxamide
*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(3,4-difluorophenyl)cyclohexanecarboxamide
*cis*-*N*-{(1S)-1-(4-chlorobenzyl)-2-[4-cyclohexyl-4-(1*H*-1,2,4-triazol-1-ylmethyl)piperidin-1-yl]-2-oxoethyl}cyclohexane-1,4-diamine
*trans*-*N*-{(1S)-1-(4-chlorobenzyl)-2-[4-cyclohexyl-4-(1*H*-1,2,4-triazol-1-ylmethyl)piperidin-1-yl]-2-oxoethyl}cyclohexane-1,4-diamine

24. Composés dont les noms suivent
4-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]phenol
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}cyclohexane-1,4-diamine
*cis*-*N*-{(1*R*)-1-(4-chlorobenzyl)-2-[3-cyclohexyl-3-(1*H-*1,2,4-triazol-1-ylmethyl)-8-azabicyclo[3.2.1]oct-8-yl]-2-oxoethyl}cyclohexane-1,4-diamine
*trans*-*N*-{(1*R*)-1-(4-chlorobenzyl)-2-[3-cyclohexyl-3-(1*H*-1,2,4-triazol-1-ylmethyl)-8-azabicyclo[3.2.1]oct-8-yl]-2-oxoethyl}cyclohexane-1,4-diamine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1,4-dioxaspiro[4.5]decan-8-amine
*N*-{(1*R*)-1-(4-chlorobenzyl)-2-[4-cyclohexyl-4-(1*H-*1,2,4-triazol-1-ylmethyl)piperidin-1-yl]-2-oxoethyl}-*N*,*N*-dimethylcyclohexane-1,4-diamine
4-(aminomethyl)-*N*-{(1*R*)-1-(4-chlorobenzyl)-2-[4-cyclohexyl-4-(1*H*-1,2,4-triazol-1-ylmethyl)piperidin-1-yl]-2-oxoethyl}cyclohexanamine
*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanol
*trans*-4-({(1*R*)-1-(4-chlorobenzyl)-2-[4-cyclohexyl-4-(1*H*-1,2,4-trazol-1-ylmethyl)piperidin-1-yl-2-oxoethyl}amino)cyclohexanol
*N*-{(1*R*)-1-(4-chlorobenzyl)-2-[4-cyclohexyl-4-(1*H*-1,2,4-triazol-1-ylmethyl)piperidin-1-yl]-2-oxoethy}-4-phenylcyclohexanamine
*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-1-phenylcyclohexanol
*trans*- 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-1-phenylcyclohexanol
*cis*- 4-({(1*R*)-1-(4-chlorobenzyl)-2-[4-cyclohexyl-4-(1*H*-1,2,4-tdazol-1 - ylmethyl)pipéridin-1-yl]-2-oxoethyl}amino)-1-phenylcyclohexanecarbonitrile
*trans*- 4-({(1*R*)-1-(4-chlorobenzyl)-2-[4-cyclohexyl-4-(1*H-*1,2,4-triazol-1-ylmethyl)piperidin-1-yl]-2-oxoethyl}amino)-1-phenylcyclohexanecarbonitrile
*cis* -*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-fluorophenyl)cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-fluorophenyl)cyclohexane-1,4-diamine
*N*-[*cis*-4-({(1*R*)-1-(4-chlorobenzy)-2-[4-cyclohexy)-4-(1*H*-1,2,4-triazol-1-ylmethyl)piperidin-1-yl]-2-oxoethyl}amino)cyclohexyl]-2,2,2-trifluoroacetamide
*N*-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2,2,2-trifluoroacetamide
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]acetamide
*N*-[tans-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]acetamide
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-methylcyclohexane-1,4-diamine
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-*N*-methylacetamide
*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(1,3-dihydro-2*H*-isoindol-2-yl)cyclohexanamine
*N*-[*cis*-4-({(1*R*)-1-(4-chlorobenzyl)-2-[4-cyclohexyl-4-(1*H-*1,2,4-triazol-1-ylmethyl)piperidin-1-yl]-2-oxoethyl}amino)cyclohexyl]-*N*-methylbenzamide
ethyl *cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarboxylate
ethyl *trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarboxylate
*cis*-*N*-{(1*R*)-1-(4-chlorobenzyl)-2-[4-cyclohexyl-4-(1*H-*1,2,4-triazol-1-ylmethyl)piperidin-1-yl]-2-oxoethyl}-4-(trifluoromethyl)cyclohexanamine
trans-*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(trifluoromethyl)cyclohexanamine
*trans*-*N*-{(1*R*)-1-(4-chlorobenzyl)2-[4-cyclohexyl-4-(1*H*-1,2,4-triazol-1-ylmethyl)piperidin-1-yl]-2-oxoethyl}-*N*-phenylcyclohexane-1,4-diamine
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-phenylcyclohexane-1,4-diamine
*N*-{(1*R*)1-(4-chlorobenzyl)-2-[4-cyclohexyl-4-(1*H*-1,2,4-triazol-1-ylmethyl)piperidin-1-yl]-2-oxoethyl}-4-(2,5-dimethyl-2,5-dihydro-1*H*-pyrrol-1-yl)cyclohexanamine
*N*-benzyl-*N*'-{(1*R*)-1-(4-chlorobenzyl)-2-[4-cyclohexyl-4-(1*H*-1,2,4-triazol-1-ylmethyl)piperidin-1-yl]-2-oxoethyl}-*N*-methylcyclohexane-1,4-diamine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-pyrrolidin-1-ylcyclohexanamine
2-{[4-({(1*R*)1-(4-chlorobenzyl)-2-[4-cyclohexyl-4-(1*H*-1,2,4-triazol-1-ylmethyl)piperidin-1-yl]-2-oxoethyl}amino)cyclohexyl]amino}ethanol
2-{benzyl[4-({(1*R*)-1-(4-chlorobenzl)-2-[4-cyclohexyl-4-(1*H*-1,2,4-triazol-1-ylmethyl)piperidin-1-yl]-2-oxoethyl]amino)cyclohexyl]amino}ethanol
*N*'-{(1*R*)-1-(4-chlorobenzyl)-2-[4-cyclohexyl-4-(1*H*-1,2,4-triazol-1-ylmethyl)piperidin-1-yl]-2-oxoethyl}-*N*-methyl-*N*-phenylcyclohexane-1,4-diamine
*N*'-{(1*R*}-1-(4-chlorobenzyl)-2-[4-cyclohexyl-4-(1*H*-1,2A-triazol-1-ylmethyl)piperidin-1-yl]-2-oxoethyl)-*N*-(4-fluorophenyl)-*N*-methylcyclohexane-1,4-diamine
*cis ou trans*-4-({(1*R*)-(4-chlorobenzyl)-2-[4-cyclohexyl-4-(1*H*-1,2,4-triazol-1-ylmethyl)piperidin-1-yl]-2-oxoethyl}amino)cyclohexanecarboxylic acid
*cis ou trans*-4-({(1*R*)-1-(4-chlorobenzyl)-2-[4-cydohexyl-4-(1*H*-1,2,4-triazol1-ylmethyl)piperidin-1-yl]-2-oxoethyl}amino)cyclohexanecarboxylic acid
*cis ou trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*,*N*-diethylcyclohexanecarboxamide
*cis ou trans*-4-({(1*R*)-1-(4-chlorobenzyl)-2-[4-cydohexyl-4-(1*H*-1,2,4-triazol-1-ylmethyl)piperidin-1-yl]-2-oxoethyl}amino)-*N*,*N*-diethylcyclohexanecarboxamide
*cis ou trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*,*N*-dimethylcyclohexanecarboxamide
*cis ou trans*-4-({(1*R*)1-(4-chlorobenzyl)-2-[4-cyclohexyl-4-(1*H*-1,2,4-triazol-1-ylmethyl)piperidin-1-yl]-2-oxoethyl}amino)-*N*,*N*-dimethylcyclohexanecarboxamide

25. Composés dont les noms suivent
*cis*-*N*-benzyl-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-methylcyclohexanecarboxamide
*trans*-*N*-benzyl-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N-*methylcyclohexanecarboxamide
*cis*-*N*-{(1*R*)-1-(4-chlorobenzyl)-2-[4-cydohexyl-4-(1*H*-1,2,4-triazol-1-ylmethyl)piperidin-1-yl]-2-oxoethyl}-4-(3-methyl-1,2,4-oxadiazol-5-yl)cyclohexanamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(3-methyl-1,2,4-oxadiazol-5-yl)cyclohexanamine
*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarboxamide
*trans*-4-({(1*R*)-1-(4-chlorobenzyl)-2-[4-cyclohexyl-4-(*1*H-1,2,4-triazol-1-ylmethyl)piperidin-1-yl]-2-oxoethyl}amino)cyclohexanecarboxamide
*cis*-4-({(1*R*)-(4-chlorobenzyl)-2-[4-cyclohexyl-4-(1*H*-1,2,4-triazol-1-ylmethyl)piperidin-1-yl]-2-oxoethyl}amino)-1-(4-fluorophenyl)cyclohexanol
*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-1-(4-fluorophenyl)cyclohexanol
*N*-{(1*R*)-1-(4-chlorobenzyl)-2-[4-cyclohexyl-4-(1*H*-1,2,4-triazol-ylmethyl)piperidin-1-yl]-2-oxoethyl}-4-methoxycyclohexanamine
4-[4-(benzyloxy)phenyl]-*N*-{(1*R*)-1(4-chlorobenzyl)-2-[4-cyclohexyl-4-(1*H*-1,2,4-triazol-1-ylmethyl)piperidin-1-yl]-2-oxoethyl}cyclohexanamine
4-(benzyloxy)-*N*-{(1*R*)-1-(4-chlorobenzyl)-2-[4-cyclohexyl-4-(1*H*-1,2,4-triazol-1-ylmethyl)piperidin-1-yl]-2-oxoethyl}cyclohexanamine
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2-methoxyacetamide
2-(benzyloxy)-*N*-[*cis*-4-({(1*R*)-1-(4-chlorobenzyl)-2-[4-cyclohexyl-4-(1*H-*1,2,4-triazol-1-ylmethyl)piperidin-1-yl]-2-oxoethyl}amino)cyclohexyl]acetamide
3-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-1,3-oxazolidin-2-one
3-[*trans*-4-({(1*R*)-1-(4-chlorobenzyl)-2-[4-cyclohexyl-4-(1*H*-1,2,4-triazol-1 - ylmethyl)piperidin-1-yl]-2-oxoethyl}amino)cyclohexyl]-1,3-oxazolidin-2-one
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(2-methoxyethyl)cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(2-methoxyethyl)cyclohexane-1,4-diamine
*cis*-*N*-{(1*R*)-1-(4-chlorobenzyl)-2-[4-cyclohexyl-4-(1*H-*1,2,4-triazol-1-ylmethyl)piperidin-1-yl]-2-oxoethyl}-4-morpholin-4-ylcyclohexanamine
*trans*-*N*-{(1R)-1-(4-chlorobenzyl)-2-[4-cyclohexyl-4-(1*H*-1,2,4-triazol-1-ylmethyl)piperidin-1-yl]-2-oxoethyl}-4-morpholin-4-ylcyclohexanamine
1-[*cis*-4-({(1*R*)-1-(4-chlorobenzyl)-2-[4-cydohexyl-4-(1*H*-1,2,4-triazol-1 - ylmethyl)piperidin-1-yl]-2-oxoethyl}amino)cyclohexyl]pyrrolidin-2-one
1-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]pyrrolidin-2-one
*N*-{(1*R*)-1-(4-chlorobenzyl)-2-[4-cyclohexyl-4-(1*H*-1,2,4-triazol-1-ylmethyl)piperidin-1-yl-2-oxoethyl}-4-(2-methoxyethoxy)cyclohexanamine
*cis*-4-({(1*R*)-1-(4-chlorobenzyl)-2-[4-cyclohexyl-4-(1*H* 1,2,4-triazol-1-ylmethyl)piperidin-1-yl]-2-oxoethyl}amino)cyclohexanecarbonitrile
trans-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarbonitrile
1-[*cis*-4-({(1*R*)-(4-chlorobenzyl)-2-[4-cyclohexyl-4-(1*H*-1,2,4-triazol-1-ylmethyl)piperidin-1-yl]-2-oxoethyl}amino)cyclohexyl]piperidin-2-one
1-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]piperidin-2-one
*N*-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]propanamide
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]glycinamide
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2-hydroxyacetamide
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2,2-dimethylpropanamide
*N*-[*trans*-4-({(1*R*)-1-(4-chlorobenzyl)-2-[4-cyclohexyl-4-(1*H*-1,2,4-triazol-ylmethyl)piperidin-1-yl]-2-oxoethyl}amino)cyclohexyl]-2,2-dimethylpropanamide
*cis*-N-{(1R)-(4-chlorobenzyl)-2-[4-cyclohexyl-4-(1*H*-1,2,4-triazol-1-ylmethyl)piperidin-1-yl]-2-oxoethyl}-*N*'-(4-methoxyphenyl)cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-methoxyphenyl)cyclohexane-1,4-diamine
*cis*-*N*-{(1R)-1-(4-chlorobenzyl)-2-[4-cyclohexyl-4-(1*H*-1,2,4-triazol-1-ylmethyl)piperidin-1-yl]-2-oxoethyl}-4-(2*H*-tetrazol-5-yl)cyclohexanamine
trans-*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(2*H* tetrazol-5-yl)cyclohexanamine
2-{[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}phenol
2-{[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}phenol
4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyciohexyl acetate
*N*²-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-*N*,*N*-dimethylglycinamide
*N*²-[*trans*-4-({(*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-*N*,*N*-dimethylglycinamide
*N*²-[*cis*-4-({(1*R*)-1-(4-chlorobenzyl)-2-[4-cyclohexyl-4-(1*H*-1,2,4-triazo)-1-ylmethyl)piperidin-1-yl]-2-oxoethyl}amino)cyclohexyl]glycinamide
*N*²-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]glycinamide
[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]piperazin-2-one
4-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]piperazin-2-one
*cis*-4-(4-acetylpiperazin-1-yl)-*N*-{(1*R*)-1-(4-chlorobenzyl)-2-[4-cyclohexyl-4-(1*H-*1,2,4-triazol-1-ylmethyl)piperidin-1-yl]-2-oxoethyl}cyclohexanamine
*trans*-4-(4-acetylpiperazin-1-yl)-*N*-{(1*R*)-1-(4-chlorobenzyl)-2-[4-cyclohexyl-4-(1*H*-1,2,4-triazol-1-ylmethyl)piperidin-1-yl]-2-oxoethyl}cyclohexanamine
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-chlorophenyl)cyclohexane-1,4-diamine
*trans*-*N*-{(1*R*)-(4-chlorobenzyl)-2-[4-cyclohexyl-4-(1*H*-1,2,4-triazol-1-ylmethyl)piperidin-1-yl]-2-oxoethyl}-*N*'-(4-chlorophenyl)cyclohexane-1,4-diamine
4-({(1*R*)-1-(4-chlorobenzyl)-2-[4-cyclohexyl-4-(1*H-*1,2,4-triazol-1-ylmethyl)piperidin-1-yl]-2-oxoethyl}amino)cyclohexyl pivalate
*cis*-*N*-{(1*R*)-1-(4-chlorobenzyl)-2-[4-cyclohexyl-4-(1*H-*1,2,4-triazol-1-ylmethyl)piperid in-1-yl]-2-oxoethyl}-*N*'-(4-fluoro-2-methylphenyl)cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-fluoro-2-methylpheny)cyclohexane-1,4-diamine
4-{[4-({(1*R*)-1-(4-chlorobenzyl)-2-[4-cyclohexyl-4-(1*H-*1,2,4-triazol-1-ylmethyl)piperidin-1-yl]-2-oxoethyl}amino)cyclohexyl]amino}benzonitrile
*N*-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]cyclopropanecarboxamide
*cis*-*N*-{(1*R*)-1-(4-chlorobenzyl)-2-[4-cyclohexyl-4-(1*H-*1,2,4-triazol-1-ylmethyl)piperidin-1-yl]-2-oxoethyl}-*N*'-(2,4-difluorophenyl)cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl-2-oxoethyl}-*N*'-(2,4-difluorophenyl)cyclohexane-1,4-diamine
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(5-fluoropyridin-2-yl)cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*-(5-fluoropyridin-2-yl)cyclohexane-1,4-diamine
*N*-1*H*-1,2,3-benzotriazol-5-yl-*N*'-{(1*R*)-1-(4-chlorobenzyl)-2-[4-cyclohexyl-4-(1*H-*1,2,4-triazol-1-ylmethyl)piperidin-1-yl]-2-oxoethyl}cyclohexane-1,4-diamine
1-[*cis*-4-({(1*R*)-1-(4-chlorobenzyl)-2-[4-cyclohexyl-4-(1*H-*1,2,4-triazol-1-ylmethyl)piperidin-1-yl]-2-oxoethyl}amino)cyclohexyl]imidazolidin-2-one
1-[*trans*-4-({(1*R*)-1-(4-chlorobenzyl)-2-[4-cyclohexyl-4-(1*H-*1,2,4-triazol-1-ylmethyl)piperidin-1-yl]-2-oxoethyl}amino)cyclohexyl]imidazolidin-2-one
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(3,4-difluorophenyl)cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(3,4-difluorophenyl)cyclohexane-1,4-diamine
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-fluoro-3-methoxyphenyl)cyclohexane-1,4-diamine
*trans*-*N*-{(1*R*)-1-(4-chlorobenzyl)-2-[4-cyclohexyl-4-(1*H-*1,2,4-triazol-1-ylmethyl)piperidin-1-yl]-2-oxoethyl}-*N*'-(4-fluoro-3-methoxyphenyl)cyclohexane-1,4-diamine
*cis*-*N*-{(1*R*)-1-(4-chlorobenzyl}-2-[4-cyclohexyl-4-(1*H*-1,2,4-triazol-1-ylmethyl)piperidin-1-yl]-2-oxoethyl}-*N*-ethyl-*N*-(4-fluoro-3-methoxyphenyl)cyclohexane-1,4-diamine
*trans*-*N*'-{(1*R*)-1-(4-chlorobenzyl)-2-[4-cyclohexyl-4-(1*H-*1,2,4-triazol-1-ylmethl)piperidin-1-yl]-2-oxoethyl}-*N*-ethyl-*N*-(4-fluoro-3-methoxyphenyl)cyclohexane-1,4-diamine
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-[4-(trifluoromethyl)phenyl]cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-[4-(trifluoromethyl)phenyl]cyclohexane-1,4-diamine
*cis*-*N*-{(1*R*)-1-(4-chlorobenzyl)-2-[4-cyclohexyl-4-1*H*-1,2,4-triazol-1-ylmethyl)piperidin-1-yl]-2-oxoethyl}-*N*'-(4-fluoro-3-methylphenyl)cyclohexane-1,4-diamine
*trans*-*N*-{(1*R*)-1-(4-chlorobenzyl-2-[4-cyclohexyl-4-(1*H-*1,2,4-triazol-1-ylmethyl)piperidin-1-yl]-2-oxoethyl}-*N*'-(4-fluoro-3-methylphenyl)cyclohexane-1,4-diamine
3-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-6-fluoro-1,3-benzoxazol-2(3*H*)-one
3-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-6-fluoro-1,3-benzoxazol-2(3*H*)-one
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]pyridine-2-carboxamide
2-{[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-5-fluorophenol ou
2-{[*trans*-4-({(1*R*)-1-(4-chlorobenzyl)-2-[4-cyclohexyl-4-(1*H-*1,2,4-triazol-1-ylmethyl)piperidin-1-yl]-2-oxoethyl}amino)cyclohexy]amino}-5-fluorophenol
*N*-[*cis*-4-({(1R)1-(4-chlorobenzyl)-2-[4-cyclohexyl-4-(1*H*-1,2,4-triazol-1-ylmethyl)piperidin-1-yl]-2-oxoethyl}amino)cyclohexyl]-2,1-benzisoxazole-3-carboxamide
*cis*-*N*-(1,3-benzothiazol-2-ylmethyl)-*N*'-{(1*R*)-1-(4-chlorobenzyl)-2-[4-cyclohexyl-4-(1*H*-1,2,4-triazol-1-ylmethyl)piperidin-1-yl]-2-oxoethyl}cyclohexane-1,4-diamine
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(1,3-thiazol-2-ylmethyl)cyclohexane-1,4-diamine
2-{[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-5-fluoro-*N,N* dimethylbenzamide
2-{[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-5-fluoro-*N*,*N* dimethylbenzamide
*trans*-*N*-(*tert*-butyl)-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarboxamide
*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(3,4-difluorophenyl)cyclohexanecarboxamide
*cis*-*N*-{(1S)-1-(4-chlorobenzyl)-2-[4-cyclohexyl-4-(1*H-*1,2,4-triazol-1-ylmethyl)piperidin-1-yl]-2-oxoethyl}cyclohexane-1,4-diamine
*trans*-*N*-{(1S)-1-(4-chlorobenzyl)-2-[4-cyclohexyl-4-(1*H*-1,2,4-triazol-1-ylmethyl)piperidin-1-yl]-2-oxoethyl}cyclohexane-1,4-diamine

26. Composés dont les noms suivent
2-{[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-2-oxoethyl acetate
: *tert*-butyl (2-{[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-2-oxoethyl)carbamate
*cis* ou *trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*-(4-morpholin-4-ylphenyl)cyclohexane-1,4-diamine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-4,4-difluorocyclohexanamine

27. Composés dont les noms suivent
1-benzyl-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
*N*-{(1*R*-1-(4-chlorobenzyl)-2-[4-cyclohexyl-4-(1*H*-1,2,4-triazol-1-ylmethyl)piperidin-1-yl]-2-oxoethy}-1-(2-phenylethyl)piperidin-4-amine
2-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidin-1-yl]ethanol
3-[4-({(1*R*)1-(4-chlorobenzyl)-2-[4-cyclohexyl-4-(1*H*-1,2,4-triazol-1-ylmethyl)piperidin-1-yl]-2-oxoethyl}amino)piperidin-1-yl]propan-1-ol
4-[4-({(1*R*)-1-(4-chlorobenzyl)-2-[4-cyclohexyl-4-(1*H-*1,2,4-triazol-1-ylmethyl)piperidin-1-yl]-2-oxoethyl}amino)piperidin-1-yl]butan-1-ol
*tert*-butyl 4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidine-1-carboxylate
*N*-{(1*R*)-1-(4-chlorobenzyl)-2-[4-cyclohexyl-4-(1*H*-1,2,4-triazol-1-ylmethyl)piperidin-1-yl]-2-oxoethyl}-8-azabicyclo[3.2.1]octan-3-amine
*N*-{(1*R*)-1-(4-chiorobenzyl)-2-[4-cyclohexyl-4-(1*H*-1,2,4-triazol-1-ylmethyl)piperidin-1-yl]-2-oxoethyl}-8-methyl-8-azabicyclo[3.2.1]octan-3-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-9-methyl-9-azabicyclo[3.3.1]nonan-3-amine
*N*-{(1*R*)-1(4-chlorobenzyl)-2-[4-cyclohexyl-4-(1*H*-1,2,4-triazol-1-ylmethyl)piperidin-1-yl]-2-oxoethyl}quinuclidin-3-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}azepan-4-amine
*N*{(1*R*)-1-(4-chlorobenzyl)-2-[4-cyclohexyl-4-(1*H*-1,2,4-triazol-1-ylmethyl)piperidin-1-yl]-2-oxoethyl}piperidin-3-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-phenylpiperidin-4-amine
*N*-{(1*R*)-1-(4-chlorobenzyl)-2-[3-cyclohexyl-3-(1*H-*1,2,4-triazol-1-ylmethyl)-8-azabicyclo[3.2.1]oct-8-yl]-2-oxoethyl}piperidin-4-amine
1-benzyl-*N*-{(1*R*)-1-(4-chlorobenzyl)-2-[4-cyclohexyt-4-(1*H*-1,2,4-triazol-1-ylmethyl)piperidin-1-yl]-2-oxoethyl}pyrrolid in-3-amine
1-benzoyl-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
1-acetyl-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
3-({(1*R*)-1-(4-chlorobenzyl)-2-[4-cyclohexyl-4-(1*H*-1,2,4-triazol-1-ylmethyl)piperidin-1-yl]-2-oxoethyl}amino)-8-methyl-8-azabicyclo[3.2.1]octan-6-ol
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(trifluoroacetyl)piperidin-4-amine
4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidine-1-carboxamide
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(4-fluorobenzoyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(cyclopentylcarbonyl)piperidin-4-amine
*N*-{(1*R*)-1-(4-chlorobenzyl)-2-[4-cyclohexyl-4-(1*H-*1,2,4-triazol-1-ylmethyl)pipéridin-1-yl]-2-oxoethyl}-1-(cyclobutylcarbonyl)piperidin-4-amine
*N*-{(1*R*)-1-(4-chlorobenzyl)-2-[4-cyclohexyl-4-(1*H-*1,2,4-triazol-1-ylmethyl)piperidin-1-yl]-2-oxoethyl}-1-[(4-methylphenyl)sulfonyl]piperidin-4-amine
*N*-{(1*R*)-1-(4-chlorobenzyl)-2-[4-cyclohexyl-4-(1*H-*1,2,4-triazol-1-ylmethyl)piperidin-1-yl]-2-oxoethyl}-1-(pyridin-2-ylcarbonyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(phenylacetyl)piperidin-4-amine
*N-*{(1*R*)-1-(4-chlorobenzyl)-2-[4-cyclohexyl-4-(1*H-*1,2,4-triazol-1-ylmethyl)piperidin-1-yl-2-oxoethyl}-1-(methylsuifonyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-2-phenylpiperidin-4-amine
(1*S*,3*R*,5*S,*7S)-4-({(1*R*)-1-(4-chlorobenzyl)-2-[4-cyclohexyl-4-(1*H*-1,2,4-triazol-1-ylmethyl)piperidin-1-yl]-2-oxoethyl}amino)adamantan-1-ol

28. Composés dont les noms suivent
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-isonicotinoylpiperidin-4-amine
*N*-{(1*R*)-1-(4-chlorobenzyl)-2-[4-cyclohexyl-4-(1*H-*1,2,4-triazol-1-ylmethyl)piperidin-1-yl]-2-oxoethyl}-[(1-methyl-1*H*-imidazol-2-yl)carbonyl]piperidin-4-amine
*N*-{(1*R*)-1-(4-chlorobenzyl)-2-[4-cyclohexyl-4-(1*H-*1,2,4-triazol-1-ylmethyl)piperidin-1-yl]-2-oxoethyl}-1-[(5-methylisoxazol-3-yl)carbonyl]piperidin-4-amine
*N*-{(1*R*)-1-(4-chlorobenzyl)-2-[4-cyclohexyl-4-(1*H-*1,2,4-triazol-1-ylmethyl)piperidin-1-yl]-2-oxoethyl}-1-(3,4-difluorobenzoyl)piperidin-4-amine
1-[(1-*tert*-butyl-5-methyl-1*H*-pyrazol-3-yl)carbonyl]-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(3,5-dimethylisoxazol-4-yl)carbonyl]piperidin-4-amine
*N-*{(1*R*)-1-(4-chlorobenzyl)-2-[4-cyclohexyl-4-(1*H-*1,2,4-triazol-1-ylmethyl)piperidin 1-yl]-2-oxbethyl}-1-(3-thienylcarbonyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(pyrrolidin-1-ylcarbonyl)piperidin-4-amine
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-phenylpiperidine-1-carboxamide 4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N,N*-dimethylpiperidine-1-carboxamide
4-({(1*R*)-1-(4-chlorobenzyl)-2-[4-cyclohexyl-4-(1*H*-1,2,4-triazol-1-ylmethyl)-piperidin-1-yl]-2-oxoethyl}amino)-*N,N*-diethylpiperidine-1-carboxamide
N-{(1*R*)-1-(4-chlorobenzyl)-2-[4-cyclohexyl-4-(1*H*-1,2,4-triazol-1-ylmethyl)piperidin-1-yl]-2-oxoethyl}-1-(piperidin-1-ylcarbonyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-(morpholin-4-ylcarbonyl)piperidin-4-amine
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-methyl-*N*-phenylpiperidine-1-carboxamide
*N*-benzyl-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-methylpiperidine-1,carboxamide
ethyl 4-({(1*R*)-1-(4-chlorobenzyl)-2-[4-cyclohexyl-4-(1*H*-1,2,4-triazo(-1-ylmethyl)piperidin-1-yl]-2-oxoethyl}amino)piperidine-1-carboxylate
: methyl 4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidine-1-carboxylate
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(2*S*)-piperidin-2-ylcarbonyl]piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(2*R*)-piperidin-2-ylcarbonyl]piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-pyridin-2-ylpiperidin-4-amine
methyl *N*-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]glycinate
*N*-{(1*R*)-1-(4-chlorobenzyl)-2-[4-cyclohexyl-4-(1*H*-1,2,-4-triazol-1-ylmethyl)piperidin-1-yl]-2-oxoethyl}-1-(2,2-difluoroethyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(6-methylpyridazin-3-yl)piperidin-4-amine
methyl [4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidin-1-yl]acetate
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-pyrimidin-2-ylpiperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-cyclopropylpiperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-{[(2*S*)-4,4-difluoropiperidin-2-yl]carbonyl}piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(4,4-difluoro-L-prolyl)piperidin-4-amine
1-(1*H*-benzimidazol-2-yl)-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
1-(2,1-benzisoxazol-3-ylcarbonyl)-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(2,2,2-trifluoroethyl)piperidin-4-amine
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(4-methoxyphenyl)piperidine-1-carboxamide
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(4-fluorophenyl)piperidine-1-carboxamide
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(2,4-difluorophenyl)piperidine-1-carboxamide
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(3,4-difluorophenyl)piperidine-1-carboxamide
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(2-fluorophenyl)piperidine-1-carboxamide
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(2-methoxyphenyl)piperidine-1-carboxamide
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-[4-(dimethylamino)phenyl]piperidine-1-carboxamide
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(5-fluoro-1*H*-indol-2-yl)carbonyl]piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(pyrazin-2-ylcarbonyl)piperidin-4-amine
*N*-{(1*R*)-1-(4-chlorobenzyl)-2-[4-cyclohexyl-4-(1*H* 1,2,4-triazol-1-ylmethyl)piperidin-1-yl]-2-oxoethyl}-1-(isoxazol-5-ylcarbonyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(quinolin-2-ylcarbonyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(3-methylpyridin-2-yl)carbonyl]piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(1*H*-1,2,4-triazol-3-ylcarbonyl)piperidin-4-amine
1-(1,3-benzothiazol-2-ylcarbonyl)-*N*-{(1*R*)1-(4-chlorobenzyl)-2-[4-cyclohexyl-4-(1*H*-1,2,4-triazol-1-ylmethyl)piperidin-1-yl]-2-oxoethyl}piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(6-methylpyridin-2-yl)carbonyl]piperidin-4-amine
1-(1-benzofuran-2-ylcarbonyl)-*N*-{(1*R*)-1-(4-chlorobenzyl)-2-[4-cyclohexyl-4-(1*H-*1,2,4-triazol-1-ylmethyl)piperidin-1-yl]-2-oxoethyl}piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(6-fluoropyridin-2-yl)carbonyl]piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(2,4-difluorobenzoyl)piperidin-4-amine

29. Composés dont les noms suivent
1-[4-({(1*R*)-1-(4-chlorobenzyl)-2-[4-cyclohexyl-4-(1*H*-1,2,4-triazol-1 - ylmethyl)piperidin-1-yl]-2-oxoethyl}amino)piperidin-1-yl]butan-2-one
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(5-phenyl-1,3-oxazol-4-yl)carbonyl]piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(1-phenyl-1*H*-pyrazol-5-yl)carbonyl]piperidin-4-amine
*N*{(1*R*)-1-(4-chlorobenzyl)-2-[4-cyclohexyl-4-(1*H*-1,2,4-triazol-1-ylmethyl)piperidin-1-yl]-2-oxoethyl}-1-[(6-phenylpyridin-2-yl)carbonyl]piperidin-4-amine

30. Médicament, **caractérisé en ce qu'**il comprend un composé de formule (I) selon l'une quelconque des revendications 1 à 29, ou un sel d'addition de ce composé à un acide pharmaceutiquement acceptable, ou encore un hydrate ou un solvat du composé de formule (I).

31. Composition pharmaceutique, **caractérisée en ce qu'**elle comprend un composé de formule (I) selon l'une quelconque des revendications 1 à 29, ou un sel pharmaceutiquement acceptable, un hydrate ou un solvat de ce composé, ainsi qu'au moins un excipient pharmaceutiquement acceptable.

32. Utilisation d'un composé de formule (I) selon l'une quelconque des revendications 1 à 29 pour la préparation d'un médicament destiné au traitement et à la prévention de l'obésité, du diabète et des dysfonctions sexuelles pouvant affecter les deux sexes, au traitement des maladies cardiovasculaires ainsi que dans des applications anti-inflammatoires ou dans le traitement de la dépendance alcoolique.

33. Utilisation selon la revendication 32, **caractérisée en ce que** lesdites dysfonctions sexuelles consistent en des dysfonctionnements érectile.

34. Procédé de préparation d'un composé de formule (I) selon l'une quelconque des revendications 1 à 22, **caractérisé en ce que** l'on réalise une amination réductrice d'un composé de formule (V) : 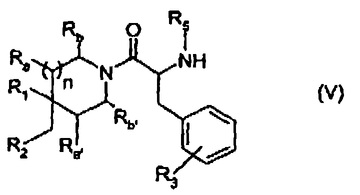 en présence d'un dérivé du groupe R₄ de type cétone, R₁, R₂, R₃, R₄, R₅, Rₐ, R_{a'}, R_{b}, R_{b'} et n étant tels que définis dans l'une quelconque des revendications 1 à 22.

35. Composés de formules (IV) et (V) ; 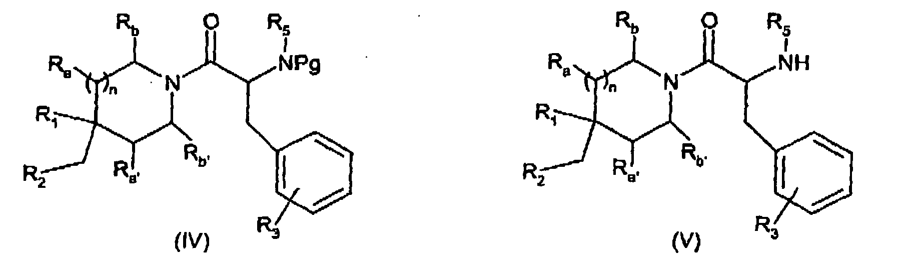 dans lesquelles R₁, R_{a,} R_{a'}, R_{b} et R_{b'} sont tels que définis dans l'une quelconque des revendications 1 à 22, Pg représente un groupe protecteur et :
n = 1, Rₐ et R_{a',} identiques ou différents l'un de l'autre, représentent un atome d'hydrogène ou un groupe allyle ou cycloalkyle, et R_{b} et R_{b'} forment ensemble, avec les atomes de carbone du cycle auquel ils sont rattachés, un pont carboné comprenant de 4 à 5 chaînons.

36. Composés de formules (VI), (XXVIII) et (XXIX), dans lesquels R₁, R₂, R₃, R₆, Rₐ, R_{a'}, R_{b}, R_{b'} et n sont tels que définis dans l'une quelconque des revendications 1 à 22 et dans lesquels R₄ représente un groupe de formule (a) ou (b) telle que définie dans l'une quelconque des revendications 1 à 17 et Pg représente un groupe protecteur d'amine ou d'hydroxyle:

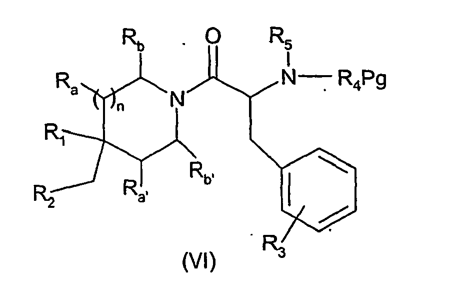

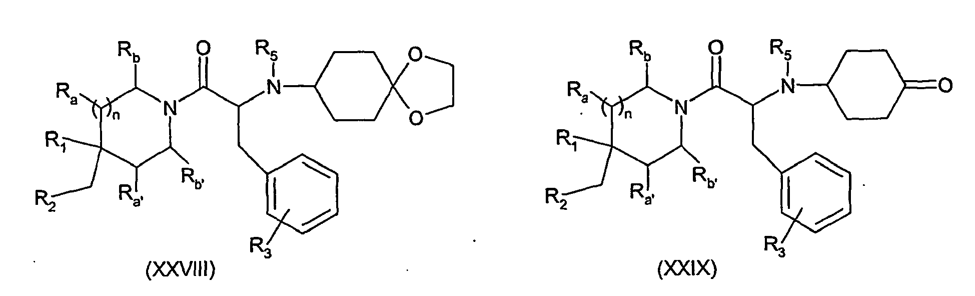

37. Composés de formule (II): 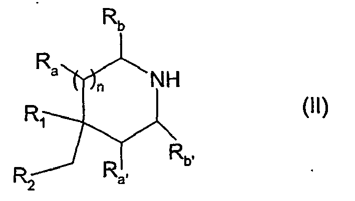 dans laquelle R₁, Rₐ, R_{a'}, R_{b} et R_{b'} sont tels que définis dans l'une quelconque des revendications 1 à 22, Pg représente un groupe protecteur et :
n = 1, Rₐ et R_{a'}, identiques ou différents l'un de l'autre, représentent un atome d'hydrogène ou un groupe alkyle ou cycloalkyle, et R_{b} et R_{b'} forment ensemble, avec les atomes de carbone du cycle auquel ils sont rattachés, un pont carboné comprenant de 4 à 5 chaînons.

## Claims

1. Compound corresponding to formula (I): 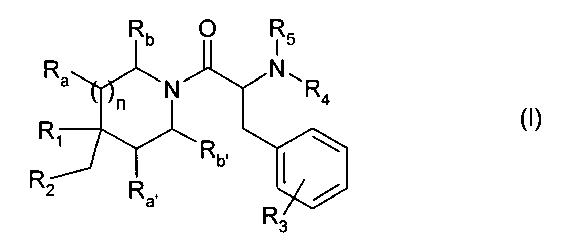 in which:
n is equal to 1,
**Rₐ**, **R**_{**a**'}, **R_{b}** and **R**_{**b**'} are identical to or different from one another and represent a hydrogen atom or an alkyl or cycloalkyl group, it being possible for R_{b} and R_{b'} to form, together with the carbon atoms of the ring to which they are attached, a carbon bridge comprising 4 or 5 members,
**R₁** represents an alkyl or cycloalkyl group,
**R₂** represents a heteroaryl group,
**R₃** represents 1 to 3 groups, which may be identical to or different from one another, located in any positions of the ring to which they are attached and chosen from halogen atoms, and alkyl, cycloalkyl, -OR, -NRR', -CO-NRR', -NR-CO-R', -NR-CO-NRR', -NR-COOR', -NO₂, -CN and -COOR groups,
**R₅** represents a hydrogen atom or an alkyl or cycloalkyl group,
**R₄** is chosen from the groups of formulae (a), (b) and (c) below, optionally substituted with an oxo group, or mono- or polysubstituted with an aryl or heteroaryl group: 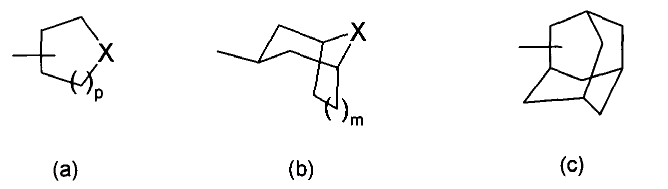
in which:
**p** = 0, 1, 2 or 3,
**m** = 0, 1 or 2,
and either
a) X represents a ring member -N(R₁₀)-, where
**R₁₀** is chosen from:
a group - (CH₂) ₓ-OR₈, - (CH₂)ₓ-COOR₈, - (CH₂)ₓNR₈R₉, - (CH₂)ₓ-CO-NR₈R₉, - (CH₂)ₓ-NR₈-COR₉, or -(CH₂)ₓ-COR₈ in which x = 1, 2, 3 or 4,
. a cycloalkyl or heterocycloalkyl group fused with an aryl or heteroaryl group,
a cycloalkyl, heterocycloalkyl, aryl, heteroaryl, alkylaryl, alkylheteroaryl, -CO-alkyl, -CO-cycloalkyl, -CO-heterocycloalkyl, -CO-aryl, -CO-heteroaryl, -CO-alkylaryl, -CO-alkylheteroaryl, -CS-alkyl, -CS-cycloalkyl, -CS-heterocycloalkyl, -CS-aryl, -CS-heteroaryl, -CS-alkylaryl, -CS-alkylheteroaryl, -CS-NR₈R₉, -C(=NH)-NR₈R₉, -SO₂-alkyl, -SO₂-cycloalkyl, -SO₂-heterocycloalkyl, -SO₂-aryl, -SO₂-heteroaryl, -SO₂-alkylaryl, -SO₂-alkylheteroaryl or -SO₂-NR₈R₉ group,
the alkyl, cycloalkyl, heterocycloalkyl, aryl or heteroaryl groups being optionally substituted with one or more groups chosen from halogen atoms and R, R', OR, NRR', -CO-NRR', -NRCOR', NRCONRR', -NO₂, CN, -COOR, OCOR, COR, OCONRR' and NRCOOR' groups;
the cycloalkyl or heterocycloalkyl groups being optionally fused with an aryl or heteroaryl group;
or else R₁₀ forms, with the nitrogen atom to which it is attached and a carbon atom located in any position of the cyclic structure of formula (a), but not adjacent to said nitrogen atom, a bridge comprising from 3 to 5 members,
**R₈** and **R₉** are chosen, independently of one another, from a hydrogen atom, and alkyl, cycloalkyl, heterocycloalkyl, aryl, heteroaryl, alkylaryl, alkylheteroaryl, -CO-alkyl, -CO-cycloalkyl, -CO-heterocycloalkyl, -CO-aryl, -CO-heteroaryl, -CO-alkylaryl, -CO-alkylheteroaryl, -SO₂-alkyl, -SO₂-cycloalkyl, -SO₂-heterocycloalkyl, -SO₂-aryl, -SO₂-heteroaryl, -SO₂-alkylaryl, -SO₂-alkylheteroaryl, -C(=NH)-NRR', -COOR, -CO-NRR', -CS-NRR' and -(CH₂)ₓ-OR groups, where x = 0, 1, 2, 3 or 4, the alkyl, cycloalkyl, heterocycloalkyl, aryl and heteroaryl groups being optionally substituted with one or more groups chosen from halogen atoms and R, R', OR, NRR', -CO-NRR', -NRCOR', NRCONRR', -NO₂, CN, -COOR, OCOR, COR, OCONRR' and NRCOOR' groups;
or else **R₈** and **R₉** together form a cycloalkyl or a heterocycloalkyl;
**R** and **R'** represent, independently of one another, a hydrogen atom, or an alkyl, cycloalkyl, heterocycloalkyl, aryl, heteroaryl, alkylaryl or alkylheteroaryl group, or can together form a cycloalkyl or a heterocycloalkyl;
or,
b) **X** represents a ring member -C(R₆) (R₇)-where
**R₆** is chosen from:
. a hydrogen atom, a halogen atom,
. a group - (CH₂)ₓ-OR₈, -(CH₂)ₓ-COOR₈, - (CH₂)ₓ-NR₈R₉, (CH₂)ₓ-CO-NR₈R₉ or - (CH₂)ₓ-NR₈-COR₉, in which x = 0, 1, 2, 3 or 4,
. an alkyl, cycloalkyl, heterocycloalkyl, aryl, heteroaryl, alkylaryl, alkylheteroaryl, -CO-alkyl, -CO-cycloalkyl, -CO-heterocycloalkyl, -CO-aryl, -CO-heteroaryl, -CO-alkylaryl, -CO-alkylheteroaryl, -CS-alkyl, -CS-cycloalkyl, -CS-heterocycloalkyl, -CS-aryl, -CS-heteroaryl, -CS-alkylaryl, -CS-alkylheteroaryl, -CS-NR₈R₉ or -C(=NH)-NR₈R₉ group,
. a cycloalkyl or heterocycloalkyl group, optionally fused, located in the spiro position on the ring of formula (a) to which it is attached,
. a cycloalkyl or heterocycloalkyl group fused with an aryl or heteroaryl group,
the alkyl, cycloalkyl, heterocycloalkyl, aryl or heteroaryl groups being optionally substituted with one or more groups chosen from halogen atoms and R, R', OR, NRR', -CO-NRR', -NRCOR', NRCONRR', -NO₂, CN, -COOR, OCOR, COR, OCONRR' and NRCOOR' groups;
the cycloalkyl or heterocycloalkyl groups being optionally fused with an aryl or heteroaryl group,
**R₇** is chosen from hydrogen and halogen atoms, and alkyl, cycloalkyl, aryl, heteroaryl, alkylaryl, alkylheteroaryl, -OR, -O-aryl, -O-heteroaryl, -O-alkylaryl, -O-alkylheteroaryl, -NRR', -CO-NRR', -NR-CO-R', -NR-CO-NRR', -NR-COOR', -NO₂, -CN and -COOR groups,
**R₈** and **R₉** are chosen, independently of one another, from a hydrogen atom, and alkyl, cycloalkyl, heterocycloalkyl, aryl, heteroaryl, alkylaryl, alkylheteroaryl, -CO-alkyl, -CO-cycloalkyl, -CO-heterocycloalkyl, -CO-aryl, -CO-heteroaryl, -CO-alkylaryl, -CO-alkylheteroaryl, -SO₂-alkyl, -SO₂-cycloalkyl, -SO₂-heterocycloalkyl, -SO₂-aryl, -SO₂-heteroaryl, -SO₂-alkylaryl, -SO₂-alkylheteroaryl, -C(=NH)-NRR', -COOR, -CO-NRR', -CS-NRR' and -(CH₂)ₓ-OR groups, where x = 0, 1, 2, 3 or 4, the alkyl, cycloalkyl, heterocycloalkyl, aryl and heteroaryl groups being optionally substituted with one or more groups chosen from halogen atoms and R, R', OR, NRR', -CO-NRR', -NRCOR', NRCONRR', -NO₂, CN, -COOR, OCOR, COR, OCONRR' and NRCOOR' groups;
or else **R₈** and **R₉** together form a cycloalkyl or a heterocycloalkyl;
**R** and **R**' represent, independently of one another, a hydrogen atom, or an alkyl, cycloalkyl, heterocycloalkyl, aryl, heteroaryl, alkylaryl or alkylheteroaryl group, or can together form a cycloalkyl or a heterocycloalkyl,
in the form of a base or of an addition salt with an acid, and also in the form of a hydrate or of a solvate.

2. Compound of formula (I) according to Claim 1, **characterized in that R₄** is chosen from the groups of formulae (a), (b) and (c) optionally mono- or polysubstituted with an aryl or heteroaryl group, where X represents a ring member -C(R₆) (R₇)-, in which
**R₆** is chosen from:
. a hydrogen atom,
. a group - (CH₂) x-OR₈, -(CH₂)ₓ-COOR₈, - (CH₂)ₓ-NR₈R₉, -(CH₂)ₓ-CO-NR₈R₉ or - (CH₂)ₓ-NR₈-COR₉, in which x = 0, 1, 2, 3 or 4,
. an alkyl, cycloalkyl, heterocycloalkyl, aryl, heteroaryl, alkylaryl, alkylheteroaryl, -CO-alkyl, -CO-cycloalkyl, -CO-heterocycloalkyl, -CO-aryl, -CO-heteroaryl, -CO-alkylaryl or -CO-alkylheteroaryl group,
. a cycloalkyl or heterocycloalkyl group located in the spiro position on the ring of formula (a) to which it is attached,
. a cycloalkyl or heterocycloalkyl group fused with an aryl or heteroaryl group,
**R₇** is chosen from hydrogen and halogen atoms, and alkyl, cycloalkyl, aryl, heteroaryl, alkylaryl, alkylheteroaryl, -OR, -0-aryl, -O-heteroaryl, -O-alkylaryl, -O-alkylheteroaryl, -NRR', -CO-NRR', -NR-CO-R', -NR-CO-NRR', -NR-COOR', -NO₂, -CN and -COOR groups,
**R₈** and **R₉** are chosen, independently of one another, from a hydrogen atom, and alkyl, cycloalkyl, heterocycloalkyl, aryl, heteroaryl, alkylaryl, alkylheteroaryl, -CO-alkyl, -CO-cycloalkyl, -CO-heterocycloalkyl, -CO-aryl, -CO-heteroaryl, -CO-alkylaryl, -CO-alkylheteroaryl, -SO₂-alkyl, -SO₂-cycloalkyl, -SO₂-heterocycloalkyl, -SO₂-aryl, -SO₂-heteroaryl, -SO₂-alkylaryl, -SO₂-alkylheteroaryl, -C(=NH)-NRR', -COOR, -CO-NRR', -CS-NRR' and -(CH2)ₓ-OR groups, where x = 0, 1, 2, 3 or 4,
**R** and **R**' represent, independently of one another, a hydrogen atom, or an alkyl, cycloalkyl, heterocycloalkyl, aryl, heteroaryl, alkylaryl or alkylheteroaryl group.

3. Compound of formula (I) according to Claim 1, **characterized in that R**₄ is chosen from the groups of formulae (a), (b) and (c) where X represents a ring member -C(R₆)(R₇)-, in which R₆ is chosen from a halogen atom, or a cycloalkyl or heterocycloalkyl group, optionally fused, located in the spiro position on the ring of formula (a) to which it is attached.

4. Compound of formula (I) according to Claim 1, **characterized in that R₄** is chosen from the groups of formulae (a), (b) and (c) where X represents a ring member -C(R₆) (R₇)-, in which R₆ is chosen from -CS-alkyl, -CS-cycloalkyl, -CS-heterocycloalkyl, -CS-aryl, -CS-heteroaryl, -CS-alkylaryl, -CS-alkylheteroaryl, -CS-NR₈R₉ and -C(=NH)-NR₈R₉.

5. Compound of formula (I) according to Claim 1, **characterized in that R₄** is chosen from the groups of formulae (a), (b) and (c) where X represents a ring member -C(R₆) (R₇)-, in which the alkyl, cycloalkyl, heterocycloalkyl, aryl or heteroaryl groups are optionally substituted with one or more groups chosen from R or R', OCOR, COR, OCONRR' and NRCOOR'.

6. Compound of formula (I) according to Claim 1, **characterized in that R₄** is chosen from the groups of formulae (a), (b) and (c) where X represents a ring member -C(R₆) (R₇)-, in which the cycloalkyl or heterocycloalkyl groups are optionally fused with an aryl or heteroaryl group.

7. Compound of formula (I) according to Claim 1, **characterized in that R₄** is chosen from the groups of formulae (a), (b) and (c) where X represents a ring member -C(R₆) (R₇)-, in which **R₈** and **R₉**, chosen independently of one another, represent alkyl, cycloalkyl, heterocycloalkyl, aryl and heteroaryl groups which are optionally substituted with one or more groups chosen from R, R', COR, OCOR, OCONRR' and NRCOOR' groups;
or else **R₈** and **R₉** together form a cycloalkyl or a heterocycloalkyl.

8. Compound of formula (I) according to Claim 1, **characterized in that R₄** is chosen from the groups of formulae (a), (b) and (c) where X represents a ring member -C(R₆) (R₇)-, in which **R** and **R**' can together form a cycloalkyl or a heterocycloalkyl.

9. Compound of formula (I) according to any one of Claims 1 to 8, **characterized in that R**₇ is hydrogen.

10. Compound of formula (I) according to one of Claims 1 to 9, **characterized in that R₄** represents the group of formula (a) where p=2 as defined below:

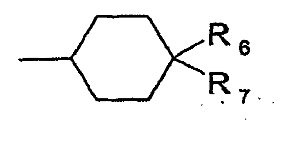

11. Compound of formula (I) according to Claim 1, **characterized in that R₄** is chosen from the groups of formulae (a), (b) and (c) optionally mono- or polysubstituted with an aryl or heteroaryl group, where X represents a ring member -N(R₁₀)- in which
**R₁₀** is chosen from:
a group -CO-NR₈R₉ or -COOR₈,
a group -(CH₂)ₓ-OR₈, -(CH₂)ₓ-COOR₈, -(CH₂)ₓ-NR₈R₉, -(CH₂) ₓ-CO-N₈R₉ or -(CH₂)ₓ-NR₈-COR₉, in which x = 1, 2, 3 or 4,
a cycloalkyl or heterocycloalkyl group fused with an aryl or heteroaryl group,
a cycloalkyl, heterocycloalkyl, aryl, heteroaryl, alkylaryl, alkylheteroaryl, -CO-cycloalkyl, -CO-heterocycloalkyl, -CO-heteroaryl, -CO-alkylaryl, -CO-alkylheteroaryl, -CS-alkyl, -CS-cycloalkyl, -CS-heterocycloalkyl, -CS-aryl, -CS-heteroaryl, -CS-alkylaryl, -CS-alkylheteroaryl, -CS-NR₈R₉, -C(=NH)-NR₈R₉, -SO₂-cycloalkyl, -SO₂-heterocycloalkyl, -SO₂-heteroaryl, -SO₂-alkylaryl, -SO₂-alkylheteroaryl or -SO₂-NR₈R₉ group,
or else **R₁₀** forms, with the nitrogen atom to which it is attached and a carbon atom located in any position of the cyclic structure of formula (a), but not adjacent to said nitrogen atom, a bridge comprising from 3 to 5 members,
**R₈** and **R₉** are chosen, independently of one another, from a hydrogen atom, and alkyl, cycloalkyl, heterocycloalkyl, aryl, heteroaryl, alkylaryl, alkylheteroaryl, -CO-alkyl, -CO-cycloalkyl, -CO-heterocycloalkyl, -CO-aryl, -CO-heteroaryl, -CO-alkylaryl, -CO-alkylheteroaryl, -SO₂-alkyl, -SO₂-cycloalkyl, -SO₂-heterocycloalkyl, -SO₂-aryl, -SO₂-heteroaryl, -SO₂-alkylaryl, -SO₂-alkylheteroaryl, -C (=NH) -NRR' , -COOR, -CO-NRR', -CS-NRR' and -(CH₂)ₓ-OR groups, where x = 0, 1, 2, 3 or 4,
**R** and **R**' represent, independently of one another, a hydrogen atom, or an alkyl, cycloalkyl, heterocycloalkyl, aryl, heteroaryl, alkylaryl or alkylheteroaryl group.

12. Compound of formula (I) according to Claim 1, **characterized in that R₄** is chosen from the groups of formulae (a), (b) and (c) optionally substituted with an oxo group, where X represents a ring member -N (R₁₀) .

13. Compound of formula (I) according to Claim 1, **characterized in that R₄** is chosen from the groups of formulae (a), (b) and (c) where X represents a ring member -N (R₁₀) -, in which **R₈** and **R₉**, chosen independently of one another, represent alkyl, cycloalkyl, heterocycloalkyl, aryl and heteroaryl groups which are optionally substituted with one or more groups chosen from halogen atoms and R, R', OR, NRR', -CO-NRR', -OCOR, NRCOR', NRCONRR', COR, -NO₂, CN, -COOR, OCONRR' and NRCOOR' groups;
or else **R₈** and **R₉** together form a cycloalkyl or a heterocycloalkyl.

14. Compound of formula (I) according to Claim 1, **characterized in that R₄** is chosen from the groups of formulae (a), (b) and (c) where X represents a ring member -N(R₁₀) -, in which **R₁₀** is -(CH₂)ₓ-COR₈ in which x = 1, 2, 3 or 4.

15. Compound of formula (I) according to Claim 1, **characterized in that R₄** is chosen from the groups of formulae (a), (b) and (c) where X represents a ring member -N(R₁₀)-, in which the alkyl, cycloalkyl, heterocycloalkyl, aryl or heteroaryl groups are optionally substituted with one or more groups chosen from R, R', OCOR, COR, OCONRR' or NRCOOR'.

16. Compound of formula (I) according to Claim 1, **characterized in that R₄** is chosen from the groups of formulae (a), (b) and (c) where X represents a ring member -N(R₁₀)-, in which the cycloalkyl or heterocycloalkyl groups are optionally fused with an aryl or heteroaryl group.

17. Compound of formula (I) according to one of Claims 1 and 11 to 16, **characterized in that R₄** represents the group of formula (a) where p=2 as defined below:

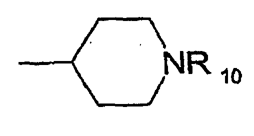

18. Compound of formula (I) according to any one of Claims 1 to 17, **characterized in that R₁** represents a cycloalkyl group,
in the form of a base or of an addition salt with an acid, and also in the form of a hydrate or of a solvate.

19. Compound of formula (I) according to any one of Claims 1 to 18, **characterized in that R₂** represents a triazolyl group,
in the form of a base or of an addition salt with an acid, and also in the form of a hydrate or of a solvate.

20. Compound of formula (I) according to any one of Claims 1 to 19, **characterized in that R₃** represents 1 to 3 groups, which may be identical to or different from one another, chosen from halogen atoms,
in the form of a base or of an addition salt with an acid, and also in the form of a hydrate or of a solvate.

21. Compound of formula (I) according to any one of Claims 1 to 20, **characterized in that R₅** represents a hydrogen atom,
in the form of a base or of an addition salt with an acid, and also in the form of a hydrate or of a solvate.

22. Compound of formula (I) according to any one of Claims 1 to 21, **characterized in that**:
- n = 1 and Rₐ = R_{a'} = R_{b} = R_{b'} = H, or
- n = 1, Rₐ = R_{a'} = H, and R_{b} and R_{b'} form, together with the carbon atoms of the ring to which they are attached, a carbon bridge comprising 4 members,
in the form of a base or of an addition salt with an acid, and also in the form of a hydrate or of a solvate.

23. Compounds having the following names:
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(2-phenylethyl)piperidin-4-amine
4-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]phenol
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}cyclohexane-1,4-diamine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-8-methyl-8-azabicyclo[3.2.1]octan-3-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-9-methyl-9-azabicyclo[3.3.1]nonan-3-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-phenylpiperidin-4-amine
1-benzoyl-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
1-acetyl-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1,4-dioxaspiro[4.5]decan-8-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N,N*-dimethylcyclohexane-1,4-diamine
4-(aminomethyl)-*N*-{(1*R*)-1-(4-chlorobenzyl)-2-[4-cyclohexyl-4-(1*H*-1,2,4-triazol-1-ymethyl)piperidin-1-yl]-2-oxoethyl}cyclohexanamine
3-({(1*R*)1-(4-chlorobenzyl)-2-[4-cyclohexyl-4-(1*H*-1,2,4-triazol-1-ylmethyl)piperidin-1-yl]-2-oxoethyl}amino)-8-methyl-8-azabicyclo[3.2.1]octan-6-ol
*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanol
*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanol
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(trifluoroacetyl)piperidin-4-amine
4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidine-1-carboxamide,
*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-1-phenylcyclohexanol
*trans*-4-({(1*R*)-1-(4-chlorobenzyl)-2-[4-cyclohexyl-4-(1*H*-1,2,4-triazol-1-ylmethyl)pipeidin-1-yl]-2-oxoethyl}amino)1-phenylcyclohexanol
*cis-N-*{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-fluorophenyl)cyclohexane-1,4-diamine
*trans*-*N*-{(1*R*)-1-(4-chlorobenzyl)-2-[4-cyclohexyl-4-(1*H*-1,2,4-triazol-1-ylmethyl)pipendin-1-yl]-2-oxoethyl}-*N*-(4-fluorophenyl)cyclohexane-1,4-diamine
*N*-[cis-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2,2,2-trifluoroacetamide
*N*-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2,2,2-trifluoroacetamide
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]acetamide
*N*-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]acetamide
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(4-fluorobenzoyl)piperidin-4-amine
-{(1*R*)-1-{4-chlorobenzyl)-2-[4-cyclohexyl-2-(1*H*-1,2,4-triazol-1-ylmethyl)piperidin-1-yl]-2-oxoethyl}-1-(cyclopentylcarbonyl)piperidin-4-amine
*N*-{(1*R*)-1-(4chlorobenzyl)-2-[4-cyclohexyl-4-(1*H*-1,2,4-triazol-1-ylmethyl)piperidin-1-yl]-2-oxoethy}-1-(cyclobutylcarbonyl)piperidin-4-amine
*cis*-*N*-{(1*R*)-1-(4-chlorobenzyl)2-[4-cyclohexyl-4-(1*H*-1,2,4-triazol-1-ylmethyl)piperidin-1-yl]-2-oxoethyl}-methylcyclohexane-1,4-diamine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(pyridin-2-ylcarbonyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(phenylacetyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(methylsulfonyl)piperidin-4-amine
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-*N*-methylacetamide
*N*-{(1*R*)-1-(4-chlorobenzyl)-2-[4-cyclohexyl-4-(1*H* 1,2,4-triazol-1-ylmethyl)piperidin-1-yl]-2-oxoethyl}-4-(1,3-dihydro-2*H*-isoindol-2-yl)cyclohexanamine
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-*N*-methylbenzamide
ethyl *cis*-4-({(1*R*)-1-chlorobenzyl)-2-[4-cyclohexyl-4-(1*H*-1,2,4-triazol-1-ylmethyl)piperidin-1-yl]-2-oxoethyl}amino)cyclohexanecarboxylate
ethyl *trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarboxylate
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-phenylcyclohexane-1,4-diamine
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethy}-*N*'-phenylcyclohexane-1,4-diamine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*-methyl-*N*-phenylcyclohexane-1,4-diamine
*N*-{(1*R*)-1-(4-chlorobenzyl)-2-[4-cyclohexyl-4-(1*H*-1,2,4-tiazol-1-ylmethyl)piperidin-1-yl]-2-oxoethyl}-*N*-(4-fluorophenyl)-*N*-methylcyclohexane-1,4-diamine
*cis or trans*-4-({(1*R*)-1-(4-chlorobenzyl)-2-[4-cyclohexyl-4-(1*H*-1,2,4-triazolylmethyl)piperidin-1-yl]-2-oxoethyl}amino)-*N,N*-diethylcyclohexanecarboxamide
*cis* or *trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N,N*-diethylcyclohexanecarboxamide
*cis* or *trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N,N*-dimethylcyclohexanecarboxamide
*cis* or *trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N,N*-dimethylcydohexanecarboxamide
*cis*-*N*-benzyl-4-({(1*R*)-1-(4-chlorobenzyl)-2-[4-cyclohexyl-4(1*H*-1,2,4-triazol-1-ylmethyl)piperidin-1-yl]-2-oxoethyl}amino)-*N*-methylcyclohexanecarboxamide
*trans*-*N*-benzyl-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-methylcyclohexanecarboxamide
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(3-methyl-1,2,4-oxadiazol-5-yl)cyclohexanamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(3-methyl-1,2,4-oxadiazol-5-yl)cyclohexanamine
*cis*-4-({(1*R*}-1-(4-chlorobenzyl)-2-[4-cyclohexyl-4-(1*H*-1,2,4-triazol-1-ylmethyl)piperidin-1-yl]-2-oxoethyl}amino)cyclohexanecarboxamide
*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarboxamide
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-isonicotinoylpiperidin-4-amine
*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-1-(4-fluorophenyl)cyclohexanol
*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-1-(4-fluorophenyl)cyclohexanol
*N*-{(1*R*)-1-(4-chlorobenzyl)-2-[4-cyclohexyl-4-(1*H*-1,2,4-triazol-1-ylmethyl)piperidin-1-yl]- 2-oxoethyl}-1-[(1-methyl-1*H*-imidazol-2-yl)carbonyl]piperidin-4-amine
*N*-{(1*R*)-1-(4-chlorobenzyl)-2-[4-cyclohexyl-4-(1*H*-1,2,4-tiazol-1-ylmethyl)piperidin-1-yl]-2-oxoethyl}-1-[(5-methylisoxazol-3-yl)carbonyl]piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(3,4-difluorobenzoyl)piperidin-4-amine
1-[(1-tert butyl-5-methyl-1*H*-pyrazol-3-yl)carbonyl]-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(3,5-dimethylisoxazol-4-yl)carbonyl]piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(3-thienylcarbonyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(pyrrolidin-1-ylcarbanyl)piperidin-4-amine
4-({(1*R*)-1-(4-chlorobenzyl)-2-[4-cyclohexyl-4-(1*H*-1,2,4-tiazol-1-ylmethyl)piperidin-1-yl]-2-oxoethyl}amino)-*N*-phenylpiperidine-1-carboxamide
4-({(1*R*)-1-(4-chlorobenzyl)-2-[4-cyclohexyl-4-(1*H*-1,2,4-triazol-1-ylmethyl)piperidin-1-yl]-2-oxoethyl}amino-*N,N*-dimethylpiperidine-1-carboxamide
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N,N*-diethylpiperidine-1-carboxamide
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(piperidin-1-ylcarbonyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(morpholin-4-ylcarbonyl)piperidin-4-amine
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-methyl-*N*-phenylpiperidine-1-carboxamide
*N*-benzyl-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-methylpiperidine-1-carboxamide
*N*-{(1*R*)-1-(4-chlorobenzyl)-2-[4-cyclohexyl4-(1*H*-1,2,4-triazol-1-ylmethyl)piperidin-1-yl]-2-oxoethyl}-4-methoxycyclohexanamine
4-[4-(benzyloxy)phenyl]-*N*-{(1*R*)-1-(4-chlorobenzyl)-2-[4-cyclohexyl-4-(1*H*-1,2,4-triazol-1-ylmethyl)piperidin-1-yl]-2-oxoethyl}cyclohexanamine
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2-methoxyacetamide
2-{[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-2-oxoethyl acetate
2-(benzyloxy)-*N*-[*cis*-4-({(*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]acetamide
3-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-1,3-oxazolidin-2-one
*3*-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-1,3-oxazolidin-2-one
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(2-methoxyethyl)cyclohexane-1,4-diamine
*trans*-*N*-{{1*R*)-1-(4-chlorobenzyl)-2-[4-cyclohexyl-4-(1*H*-1,2,4-triazol-1-ylmethyl)piperidin-1-yl]-2-oxoethyl}-*N*'-(2-methoxyethyl)cyclohexane-1,4-diamine
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-morpholin-4-ylcyclohexanamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-morpholin-4-ylcyclohexanamine
1-[*cis*-4-({(1*R*)-1-(4-chlorobenyl)-2-[4-cyclohexyl-4-(1*H*-1,2,4-triazol-1-ylmethyl)piperidin-1-yl]-2-oxoethyl}amino)cyclohexyl]pyrrolidin-2-one
1-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]pyrrolidin-2-one
*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(2-methoxyethoxy)cyclohexanamine
ethyl 4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidine-1-carboxylate
methyl 4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidine-1-carboxylate
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(2S)-piperidin-2-ylcarbonyl]piperidin-4-amine
*N*-{{1*R*)-1-(4-chlorobenzyl)-2-[4-cyclohexyl-4-(1*H*-1,2,4-triazol-1-ylmethyl)piperidin-1-yl]-2-oxoethyl}-1-[(2*R*)-piperidin-2-ylcarbonyl]piperidin-4-amine
*cis*-4-({(1*R*)-1-(4-chlorobenzyl-2-[4-cyclohexyl-4-(1*H*-1,2,4-triazol-1-ylmethyl)piperidin-1-yl]-2-oxoethyl}amino)cyclohexanecarbonitrile
*trans*-4-({(1*R*)-1-(4-chlorobenzyl)-2-[4-cyclohexyl-4-(1*H*-1,2,4-triazol-1-ylmethyl)piperidin-1-yl]- 2-oxoethyl}amino)cyclohexanecarbonitrile
1-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]piperidin-2-one
1-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]piperidin-2-one
*N*-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]propanamide
tert-butyl (2-{[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-2-oxoethyl)carbamate
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]glycinamide *N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2-hydroxyacetamide
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2,2-dimethylpropanamide
*N*-[*trans*-4-({(1*R*)-1-(4-chlorobenzyl)-2-[4-cyclohexyl-4-(1*H*,2,4-triazol-1-ylmethyl)piperidin-1-yl]-2-oxoethyl}amino)cyclohexyl]-2,2-dimethylpropanamide
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*-(4-methoxyphenyl)cyclohexane-1,4-diamine
*trans*-*N*{(1*R*)-1-(4-chlorobenzyl)-2-[4-cyclohexyl-4-(1*H*-1,2,4-triazol-1-ylmethyl)piperidin-1-yl]-2-oxoethyl}-*N*-(4-methoxyphenyl)cyclohexane-1,4-diamine
*cis-N-*{(*1R*)-1-(4-chlorobenzyl)-2-[4-cyclohexy[-4-(1*H*-1,2,4-triazol-1-ylmethyl)piperidin-1-yl]-2-oxoethyl}-4-(2*H*-tetrazol-5-yl)cyclohexanamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(2*H*-tetrazol-5-yl)cyclohexanamine
2-{[*cis*-4-({(1*R*)-1-(4-chlorobenzyl)2-[4-cyclohexyl-4-(1*H*-1-1,2,4-triazol-1-ylmethyl)piperidin-1-yl]-2-oxoethyl}amino)cyclohexyl]amino}phenol
2-{[*trans*4({(1*R*)-1-(4-chlorobenzyl)-2-[4-cyclohexyl-4-(1*H*-1,2,4-triazol-1-ylmethyl)piperidin-1-yl]-2-oxoethy}amino)cyclohexyl]amino}phenol
4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl acetate
*N*²-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-*N,N*-dimethylglycinamide
*N*²-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-*N,N*-dimethylglycinamide
*N*²-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]glycinamide
*N*²-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]glycinamide
4-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]piperazin-2-one
4-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]piperazin-2-one
*cis*-4-(4-acetylpiperazin-1-yl)-*N*-{(1*R*)-1-(4-chlorobenzyl)-2-[4-cyclohexyl-4-(1*H-*1,2,4-triazol-1-ylmethyl)piperidin-1-yl]-2-oxoethyl}cyclohexanamine
*trans*-4-(4-acetylpiperazin-1-yl)-*N*-{(1*R*)-1-(4-chlorobenzyl)-2-[4-cyclohexyl-4-(1*H*-1,2,4-triazol-1-ylmethyl)piperidin-1-yl]-2-oxoethyl}cyclohexanamine
*N*-{(1*R*)-1-(4-chlorobenyl)-2-[4-cyclohexyl-4-(1*H*-1,2,4-triazol-1-ylmethyl)piperidin-1-yl]-2-oxoethyl}-1-pyridin-2-ylpiperidin-4-amine
methyl *N*-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]glycinate
*N*-{(1*R*)-1-(4-chlorobenzyl)-2-[4-cyclohexyl-4-(1*H*-11,2,4-triazol-1-ylmethyl)piperidin-1-yl]-2-oxoethyl}-1-(2,2-difluoroethyl)piperidin-4-amine
*cis*-*N*-{(1R)-1-(4-chlorobenzyl)-2-[4-cyclohexyl-4-(1*H*-1,2,4-triazol-1-ylmethyl)piperidin-1-yl]-2-oxoethyl}-*N*-(4-chlorophenyl)cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl)-*N*-(4-chlorophenyl)cyclohexane-1,4-diamine
4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl pivalate
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-fluoro-2-methylphenyl)cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl]-2-cycloethyl}-*N*-(4-fluoro-2-methylphenyl)cyclohexane-1,4-diamine
4-{[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}benzonitrile
*N*-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]cyclopropanecarboxamide
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(6-methylpyridazin-3-yl)piperidin-4-amine
methyl [4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidin-1-yl]acetate
*cis* or *trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-morpholin-4-ylphenyl)cyclohexane-1,4-diamine
- *cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(2,4-difluorophenyl)cyclohexane-1,4-diamine
*trans-N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(2,4-difluorophenyl)cyclohexane-1,4-diamine
*cis*-*N*-{(1*R*)-1-(4-chlorobenzyl)-2-[4-cyclohexyl-4-(1*H*-1,2,4-triazol-ylmethyl)piperidin-1-yl]-2-oxoethyl}-*N*'-(5-fluoropyridin-2-yl)cyclohexane-1,4-diamine
*trans*-*N*-{(1*R*)-1-(4-chlorobenzyl)-2-[4-cyclohexyl-4-(1*H*-1,2,4-triazolylmethyl)piperidin-1-yl]-2-oxoethyl}-*N*'-(5-fluoropyridin-2-yl)cyclohexane-1,4-diamine
*N*-1*H*-1,2,3-benzotriazol-5-yl-*N*'-{(1*R*)-1-(4-chlorobenzyl)-2-[4-cyclohexyl-4-(1*H-*1,2,4-triazol-1-ylmethyl)piperidin-1-yl]-2-oxoethyl}cyclohexane-1,4-diamine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-pyrimidin-2-ylpiperidin-4-amine
1-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]imidazolidin-2-one
1-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]imidazolidin-2-one
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-cyclopropylpiperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-{[(2*S*)-4,4-difluoropiperidin-2-yl]carbonyl}piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-4,4-difluorocyclohexanamine
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(3,4-difluorophenyl)cyclohexane-1,4-diamine
trans-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(3,4-difluorophenyl)cyclohexane-1,4-diamine
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-fluoro-3-methoxyphenyl)cyclohexane-1,4-diamine
*trans-N-*{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-fluoro-3-methoxyphenyl)cyclohexane-1,4-diamine
*cis*-*N*'-{(1*R*)-1-(4-chlorobenzyl)-2-[4-cyclohexyl-4-(1*H*-1,2,4-triazol-1-ylmethyl)piperidin-1-yl]-2-oxoethyl}-*N*-ethyl-*N*-(4-fluoro-3-methoxyphenyl)cyclohexane-1,4-diamine
trans-*N*'-{(1*R*)-1-(4-chlorobenzyl)-2-[4-cyclohexyl-4-(1*H*-1,2,4-triazol-1-ylmethyl)piperidin-1-yl]-2-oxoethyl}-*N*-ethyl-*N*-(4-fluoro-3-methoxyphenyl)cyclohexane-1,4-diamine
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-[4-(trifluoromethyl)pheny]cyclohexane-1,4-diamine
trans-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-[4-(trifluoromethyl)phenyl]cyclohexane-1,4-diamine
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-fluoro-3-methylphenyl)cyclohexane-1,4-diamine
trans-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-fluoro-3-methylphenyl)cyclohexane-1,4-diamine
*N*-{(1*R*)-1-(4-chlorobenzyl)-2-[4-cyclohexyl-4-(1,2,4-triazol-1-ylmethyl)piperidin-1-yl]-2-oxoethyl}-1-(4,4-difluoro-L-prolyl)piperidin-4-amine
1-(1*H*-benzimidazol-2-yl)-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
1-(2,1-benzisoxazol-3-ylcarbonyl)-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
1-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidin-1-yl]butan-2-one
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(2,2,2-trifluoroethyl)piperidin-4-amine
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(4-methoxyphenyl)piperidine-1-carboxamide
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(4-fluorophenyl)piperidine-1-carboxamide
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(2,4-difluorophenyl)piperidine-1-carboxamide
4-({(1*R*)-1-(4-chlorobenzyl)-2-[4-cyclohexyl-4-(1*H-1*,2,4-triazol-1-ylmethyl)piperidin-1-yl]-2-oxoethyl}amino)-*N*-(3,4-difluorophenyl)piperidine-1-carboxamide
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(2-fluorophenyl)piperidine-1-carboxamide
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(2-methoxyphenyl)piperidine-1-carboxamide
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-[4-(dimethylamino)phenyl]piperidine-1-carboxamide
*N*-{(1*R*)-1-(4-chlorobenzyl)-2-[4-cyclophexyl-4-(1*H*-1,2,4-triazol-1-ylmethyl)piperidin-1-yl]-2-oxoethyl}-1-[(5-fluoro-1*H*-indol-2-yl)carbonyl]piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(pyrazin-2-ylcarbonyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(5-phenyl-1,3-oxazol-4-yl)carbonyl]piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(isoxazol-5-ylcarbonyl)piperidin-4-amine
3-[*trans*-4-({(1*R*)-1-(4-chlorobenzyl)-2-[4-cyclohexyl-4-(1*H*-1,2,4-triazol-1-ylmethyl)piperidin-1-y]-2-oxoethyl}amino)cyclohexyl]-6-fluoro-1,3-benzoxazol-2(3*H*)-one
3-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-6-fluoro-1,3-benzoxazol-2(3*H*)-one
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]pyridine-2-carboxamide
2-{[*cis*-4({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}5-fluorophenol or
2-{[*trans*-4-({(1*R*)-1-(4-chlorobenzyl)-2-[4-cyclohexy-4-(1*H*-1,2,4-triazol-1-ylmethyl)piperidin-1-yl]-2-oxoethyl}amino)cyclohexyl]amino}-5-fluorophenol
*N*-{(1*R*)1-(4-chlorobenzyl)-2-[4-cyclohexyl-4-(1*H*-1,2,4-triazol-1-ylmethyl)piperidin-1-yl]-2-oxoethyl}-1-(quinolin-2-ylcarbonyl)piperidin-4-amine
*N*-{(1*R*)-1-(4chlorobenzyl)-2-[4-cyclohexyl-4-(1*H*-1,2,4-triazol-1-ylmethyl)piperidin-1-yl]-2-oxoethyl}-1-[(3-methylpyridin-2-yl)carbonyl]piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(1*H*-1,2,4-triazol-3-ylcarbonyl)piperidin-4-amine
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2,1-benzisoxazole-3-carboxamide
1-(1,3-benzothiazol-2-ylcarbonyl)-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(6-methylpyridin-2-yl)carbonyl]piperidin-4-amine
1-(1-benzofuran-2-ylcarbonyl)-*N*-{(1*R*)-1-(4-chlorobenzyl)-2-[4-cyclohexyl-4-(1*H-*1,2,4-triazol-1-ylmethyl)piperidin-1-yl]-2-oxoethyl}piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(6-fluoropyridin-2-yl)carbonyl]piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(1-phenyl-1*H*-pyrazol-5-yl)carbonyl]piperidin-4-amine
*N*-{(1*R*)-1-(4-chlorobenzyl)-2-[4-cyclohexyl-4-(1*H*-1,2,4triazol-1-ylmethyl)piperidin-1-yl]-2-oxoethyl}-1-(2,4-difluorobenzoyl)piperidin-4-amine
*cis-N-*(1,3-benzothiazol-2-ylmethyl)-*N*'-{(1*R*)-1-(4-chlorobenzyl)-2-[4-cyclohexyl-4-(1*H*-1,2,4-triazol-1-ylmethyl)piperidin-1-yl]-2-oxoethyl}cyclohexane-1,4-diamine
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(1,3-thiazol-2-ylmethyl)cyclohexane-1,4diamine
2-{[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-5-fluoro-*N*,*N-*dimethylbenzamide
2-{[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-5-fluoro-*N*,*N-*dimethylbenzamide
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(6-phenylpyridin-2-yl)carbonyl]piperidin-amine
*trans*-*N*-(*tert*-butyl)-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarboxamide
*cis*-4-({(1*R*)-1-(4-chlorobenzyl)-2-[4-cyclohexyl-4(1*H*-1,2,4-triazol-1-ylmethyl)piperidin-1-yl]-2-oxoethyl}amino)-*N*-(3,4-difluorophenyl)cyclohexanecarboxamide
*cis*-*N*-{(1*S*)-1-(4-chlorobenzyl)-2-[4-cyclohexyl-4-(1*H*-1,2,4-triazol-1-ylmethyl)piperidin-1-yl]-2-oxoethyl}cyclohexane-1,4-diamine
*trans*-*N*-{(1*S*)1-(4-chlorobenzyl)-2-[4-cyclohexyl-4-(1*H*-1,2,4-triazol-1-ylmethyl)piperidin-1-yl]-2-oxoethyl}cyclohexane-1,4-diamine

24. Compounds having the following names:
4-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]phenol
*cis*-*N*-{(1*R*)1-(4-chlorobenzyl)-2-[4-cyclohexyl-4-(1*H*-1,2,4-triazol-1-ylmethyl)piperidin-1-yl]-2-oxoethyl}cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxethyl}cyclohexans-1,4-diamine
*cis*-*N*-{(1*R*)-1-(4-chlorobenzyl)-2-[3-cyclohexyl-3-(1H-1,2,4-triazol-1-ylmethyl)-8-azabicyclo[3.2.1]oct-8-yl]-2-oxoethyl}cyclohexane-1,4-diamine
*trans*-*N*-{(1*R*)-1-(4-chlorobenzyl)-2-[3-cyclohexyl-3-(1*H*-1,2,4-triazol-1-ylmethyl)-8-azabicyclo[3.2.1]oct-8-yl]-2-oxoethyl}cyclohexane-1,4-diamine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1,4-dioxaspiro[4.5]decan-8-amine
*N'*-{(1*R*)-1-(4-chlorobenzyl)-2-[4-cyclohexyl-4-(1*H*-1,2,4-triazol-1-ylmethyl)piperidin-1-yl]-2-oxoethyl}-*N*,*N*-dimethylcyclohexane-1,4-diamine
4-(aminomethyl)-*N*-{(1*R*)-1-(4-chlorobenzyl)-2-[4-cyclohexyl-4-(1*H*-1,2,4-triazol-1-ylmethyl)piperidin-1-yl]-2-oxoethyl}cyclohexanamine
*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanol
*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanol
*N*-{(1*R*)-(4-chlorobenzyl)-2-[4-cyclohexyl-4-(1*H*-1,2,4-triazol-1-ylmethyl)piperidin-1-yl]2-oxoethyl}-4-phenylcyclohexanamine
*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-1-phenylcyclohexanol
*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-1-phenylcyclohexanol
*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-1-phenylcyclohexanecarbonitrile
*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-1-phenylcyclohexanecarbonitrile
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-fluorophenyl)cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1₋yl]-2-oxoethyl}-*N*'-(4-fluorophenyl)cyclohexane-1,4-diamine
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2,2,2-trifluoroacetamide
*N*-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2,2,2-trifluoroacetamide
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]acetamide
*N*-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]acetamide
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-methylcyclohexane-1,4-diamine
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-*N*-methylacetamide
*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(1,3-dihydro-2*H*-isoindol-2-yl)cyclohexanamine
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-*N*-methylbenzamide
ethyl *cis*-4-({(1*R*)-1-(4-chlorobenzyl)-2-[4-cyclohexyl-4-(1*H*-1,2,4-triazol-1-ylmehyl)piperidin-1-yl]-2-oxoethyl}amino)cyclohexanecarboxylate
ethyl *trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarboxylate
*cis-N-*{(1*R*)-1-(4-chlorobenzyl)-2-[4-Cyclohexyl-4-(1*H*-1,2,4-tiazol-1-ylmethyl)piperidin-1-yl]-2-oxoethyl}-4-(trifluoromethyl)cyclohexanamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(trifluoromethyl)cyclohexanamine
*trans-N*-{(1*R*)-1-(4-chlorobenzyl)-2-[4-cyclohexyl-4(1*H*-1,2,4-triazol-1-ylmethyl)piperidin-1-yl]-2-oxoethyl}-*N*'-phenylcyclohexane-1,4-diamine
*cis*-*N*-{(1*R*)-1-(4-chlorobenzyl)-2-[4-cyclohexyl-4-(*H*-1,2,4-triazol-1-ylmethyl)piperidin-1-yl]-2-oxoethyl}-*N*'-phenylcyclohexane-1,4-diamine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(2,5-dimethyl-2,5-dihydro-1*H*-pyrrol-1-yl)cyclohexanamine
*N*-benzyl-*N*'-{(1*R*)-1-(4-chlorobenzyl)-2-[4-cyclohexyl-4-(1*H*-1,2,4-triazol-1-ylmethyl)piperidin-1-yl]-2-oxoethyl}-*N*-methylcyclohexane-1,4-diamine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-pyrrolidin-1-ylcyclohexanamine
2-{[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}ethanol
2-{benzyl[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}ethanol
*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*-methyl-*N*-phenylcyclohexane-1,4-diamine
*N*'-{(1*R*)-1-(4-chlorobenzyl)-2-[4-cyclohexyl-4-(1*H*-1,2,4-triazol-1-ylmethyl)piperidin-1-yl]-2-oxoethyl}-*N*-(4-fluorophenyl)-*N*-methylcyclohexane-1,4-diamine
*cis* or *trans*-4({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarboxylic acid
*cis* or *trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarboxylic acid
*cis or trans*-4-({(1*R*)-1-(4-chlorobenzyl)-2-[4-cyclohoxyl-4-(1*H*-1,2,4-triazol-1-ylmethyl)piperidin-1-yl]-2-oxoethyl}amino)-*N*,*N*-diethylcyclohexanecarboxamide
*cis* or *trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*,*N*-diethylcyclohexanecarboxamide
*cis* or *trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*,*N*-dimethylcyclohexanecarboxamide
*cis or trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*,*N*-dimemylcyclohexanecarboxamide

25. Compounds having the following names:
*cis*-*N*-benzyl-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-methylcyclohexanecarboxamide
*trans*-*N*-benzyl-4-({(1*R*)-1-(4-chlorobenzyl)-2-[4-cyclohexyl-4-(1*H*-1,2,4-triazol-1-ylmethyl)piperidin-1-yl]-2-oxoethyl}amino-*N*-methylcyclohexanecarboxamide
*cis*-*N*-{(1*R*)-1-(4-chlorobenzyl)-2-[4-cyclohexyl-4-(1*H*-1,2,4-tiazol-1-ylmethyl)piperidin-1-yl]-2-oxoethyl}-4-(3-methyl-1,2,4-oxadiazol-5-yl)cyclohexanamine
*trans*-*N*{(1*R*)-1-(4-chlorobenzyl)-2-[4-cyclohexyl-4-(1*H*-1,2,4-triazol-1-ylmethyl)piperidin-1-yl]-2-oxoethyl}-4-(3-methyl-1,2,4-oxadiazol-5-yl)cyclohexanamine
*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarboxamide
*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarbaxamide
*cis*-4-({(1*R*)-1-(4-chlorobenzyl)-2-[4-cyclohexyl-4-(1*H-*1,2,4-triazol-1-ylmethyl)piperidin-1-yl]-2-oxoethyl}amino)-1-(4-fluorophenyl)cyclohexanol
*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-1-(4-fluorophenyl)cyclohexanol
*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-methoxycyclohexanamine
4-[4-(benzyloxy)phenyl]-*N*{(1*R*)-1-(4-chlorobenzyl)-2-[4-cyclohexyl-4-(1*H*-1,2,4-triazol-1-ylmethyl)piperidin-1-yl]-2-oxoethyl}cyclohexanamine
4-(benzyloxy)-*N*-{(1*R*)-1-(4-chlorobenzyl)-2-[4-cyclohexyl-4-(1*H*-1,2,4-triazol-1-ylmethyl)piperidin-1-yl]-2-oxoethyl}cyclohexanamine
*N*-[*cis*-4-({(1*R*)-1-(4-chlorobenzylyl)-2-[4-cyclohexyl-4-(1*H*-1,2,4-triazol-1-ylmethyl)piperidin-1-yl]-2-oxoethyl}amino)cyclohexyl]-2-methoxyacetamide
2-(benzyloxy)-*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]acetamide
3-[*cis*-4-({(1*R*)-1-(4-chlorobenzyl)-2-[4-cyclohexyl-4-(1*H*-1,2,4-triazol-1-ylmethyl)piperidin1-yl]-2-oxoethyl}amino)cyclohexyl]-1,3-oxazolidin-2-one
3-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-1,3-oxazolidin-2-one
*cis-N-*{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(2-methoxyethyl)cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(2-methoxyethyl)cyclohexane-1,4-diamine
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl-4-morpholin-4-ylcyclohexanamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-morpholin-4-ylcyclohexanamine
1-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]pyrrolidin-2-one
1-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]pyrrolidin-2-one
*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(2-methoxyethoxy)cyclohexanamine
*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarbonitrile
*trans*-4-({(1*R*)-1-(4-chlorobenzyl)-2-[4-cyclohexyl-4-(1*H*-1,2,4-triazol-1-ylmethyl)piperidin-9-yl]-2-oxoethyl}amino)cyclohexanecarbonitrile
1-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]piperidin-2-one
1-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]piperidin-2-one
*N*-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]propanamide
*N*-[*cis*-4({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]glycinamide
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2-hydroxyacetamide
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2,2-dimethylpropanamide
*N*-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2,2-dimethylpropanamide
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-methoxyphenyl)cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-methoxyphenyl)cyclohexane-1,4-diamine
*cis*-*N*-{(1*R*)1-(4-chlorobenzyl)-2-[4-cyclohexyl-4-(1*H*-1,2,4-triazol-1-ylmethyl)piperidin-1-yl]-2-oxoethyl}-4-(2*H*-tetrazol-5-yl)cyclohexanamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-4-(2*H*-tetrazol-5-yl)cyclohexanamine
2-{[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}phenol
2-{[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}phenol
4-({(1*R*)-(4-chlorobenzyl)-2-[4-cyclohexyl-4-(1*H*-1,2,4-triazol-1-ylmethyl)piperidin-1-yl]-2-oxoethyl}amino)cyclohexyl acetate
*N²*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-*N*,*N*-dimethylglycinamide
*N²*-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-*N*,*N*-dimethylglycinamide
*N²*-[*cis*-4-({(1*R*)-1-(4-chlorobenzyl)-2-[4-cycloexyl-4-(1*H*-1,2,4-triazol-1-ylmethyl)piperidin-1--yl]-2-oxoethyl}amino)cyclohexyl]glycinamide
*N²*-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]glycinamide
[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]piperazin-2-one
4-[*trans-4-*({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexy]piperazin-2-one
*cis*-4-(4-acetylpiperazin-1-yl)-*N*-{(1*R*)-1-(4-chlorobenzyl)-2-[4-cyclohexyl-4-(1*H-*1,2,4-triazol-1-ylmethyl)piperidin-1-yl]-2-oxoethyl}cyclohexanamine
*trans*-4-(4-acetylpiperazin-1-yl)-*N*-{(1*R*)-1-(4-chlorobenzyl)-2-[4-cyclohexyl-4-(1*H*-1,2,4-triazol-1-ylmethyl)piperidin-1-yl]-2-oxoethyl}cyclohexanamine
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-chlorophenyl)cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-chlorophenyl)cyclohexane-1,4-diamine
4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl pivalate
*cis*-*N*-{(1R)-1-(4-chlorobenzyl)-2-[4-cyclohexyl-4-(1*H*-1,2,4-triazol-1-ylmethyl)piperidin-1-yl]-2-oxoethyl}-*N*'-(4-fluoro-2-methylphenyl)cyclohexane-1,4-diamine
*trans*-*N*-{(1*R*)-1-(4-chlorobenzyl)-2-[4-cyclohexyl-4-(1*H*-1,2,-triazol-1-ylmethyl)piperidin-1-yl]-2-oxoethyl}-*N*'-(4-fluoro-2-methylphenyl)cyclohexane-1,4-diamine
4-{[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}benzonitrile
*N*-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]cyclopropanecarboxamide
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(2,4-difluorophenyl)cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(2,4-difluorophenyl)cyclohexane-1,4-diamine
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(5-fluoropyridin-2-yl)cyclohexane-1,4-diamine
*trans*-*N*-{(1*R*)-1-(4-chlorobenzyl)-2-[4-cyclohexyl-4-(1H-1,2,4-triazol-1-ylmethyl)piperidin-1-yl]-2-oxoethyl}-*N*'-(5-fluoropyridin-2-yl)cyclohexane-1,4-diamine
*N*-1*H*-1,2,3-benzotriazol-5-yl-*N*'-{(1*R*)-1-(4-chlorobenzyl)-2-[4-cyclohexyl-4-(1*H-*1,2,4-triazol-1-ylmethyl)piperidin-1-yl]-2-oxoethyl}cyclohexane-1,4-diamine
1-[*cis*-4-({(1*R*)-1-(4-chlorobenzyl)-2-[4-cyclohexo-4-(1*H*-1,2,4-triazol-1-ylmethyl)piperidin-1-yl]-2-oxoethyl}amino)cyclohexyl]imidazolidin-2-one
1-[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]imidazolidin-2-one
*cis-N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(3,4-difluorophenyl)cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*-(3,4-difluorophenyl)cyclohexane-1,4-diamine
*cis*-*N*-{(1*R*)-1-(4-chlorohenzyl)-2-[4-cyclohexyl-4-(1*H*-1,2,4-triazol-1-ylmethyl)piperidin-1-yl]-2-oxoethyl}-*N*'-(4-fluoro-3-methoxyphenyl)cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-fluoro-3-methoxyphenyl)cyclohexane-1,4 diamine
*cis*-*N*'-{(1*R*)-1-(4-chlorobenzyl)-2-[4-cyclohexyl-4-(1*H*-1,2,4-triazol-1-ylmethyl)piperidin-1-yL]-2-oxoethyl}-*N*-ethyl-*N*-(4-fluoro-3-methoxyphenyl)cyclohexane-1,4-diamine
*trans-N*'-{(1*R*)-1-(4-chlorobenzyl)-2-[4-cyclohexyl-4-(1*H*-1,2,4-triazol-1-ylmethyl)piperidin-1-yl]-2-oxoethyl}-*N*-ethyl-*N*-(4-fluoro-3-methoxyphenyl)cyclohexane-1.4-diamine
*cis*-*N*-{(1*R*)-1-(4-chlorobenzyl)-2-[4-cyclohexyl-4-(1*H*-1,2,4-triazol-1-ylmethyl)piperidin-yl]-2-oxoethyl}-*N*'-[4-(trifluoromethyl)phenyl]cyclohexane-1,4-diamine
*trans*-*N*-{(1*R*)-1-(4-chlorobenzyl)-2-[4-cyclohexyl-4-(1H-1,2,4-triazol-1-ylmethyl)piperidin-1-yl]-2-oxoethyl}-*N*'-[4-(trifluoromethyl)phenyl]cyclohexane-1,4-diamine
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(4-fluoro-3-methylphenyl)cyclohexane-1,4-diamine
*trans*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*-(4-fluoro-3-methylphenyl)cyclohexane-1,4-diamine
3-[*trans*-4-({(*1*R)-1-(4-chlorobenzyl)-2-[4-cyclohexyl-4-(1*H*-1,2,4-triazol-1-ylmethyl)piperidin-1-yl]-2-oxoethyl}amino)cyclohexyl]-6-fluoro-1,3-benzoxazol-2(3*H*)-one
3-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-6-fluoro-1,3-benzoxazol-2(3*H*)-one
*N*-[*cis*-4-({(*1R*)*-1*-(4-chlorobenzyl)-2-[4-cyclohexy]-4-(1*H*-1,2,4-triazol-1-ylmethyl)piperidin-1-yl]-2-oxoethyl}amino)cyclohexyl]pyridine-2-carboxamide
2-{[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-5-fluorophenol or
2-{[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-5-fluorophenol
*N*-[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]-2,1-benzisoxazole-3-carboxamide
*cis*-*N*-(1,3-benzothiazol-2-ylmethyl)-*N*'-{(1*R*)-1-(4-chlorobenzyl)-2-[4-cyclohexyl-4-(1*H*-1,2,4-triazol-1-ylmethyl)piperidin-1-yl]-2-oxoethyl}cyclohexane-1,4-diamine
*cis*-*N*-{(1*R*)-1-(4-chlorobenzyl)-2-[4-cyclohexyl-4-(1*H*-1,2,4-triazol-1-ylmethyl)piperidin-1-yl]-2-oxoethyl}-*N*'-(1,3-thiazol-2-ylmethyl)cyclohexane-1,4-diamine
2-{[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-5-fluoro-*N*,*N-*dimethylbenzamide
2-{[*trans*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-5-fluoro-*N*,*N-*dimethylbenzamide
*trans*-*N*-(*tert*-butyl)-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexanecarboxamide
*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(3,4-difluorophenyl)cyclohexanecarboxamide
*cis*-*N*-{(1S)-1-(4-chlorobenzyl)-2-[4-cyclohexyl-4-(1*H*-1,2,4-triazol-1-ylmethyl)piperidin-1-yl]-2-oxoethyl}cyclohexane-1,4-diamine
*trans*-*N*{(1S)-1-(4-chlorobenzyl)-2-[4-cyclohexyl-4-(1*H*-1,2,4-triazol-1-ylmethyl)piperidin-1-yl]-2-oxoethyl}cyclohexane-1,4-diamine

26. Compounds having the following names:
2-{[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-2-oxoethyl acetate
*tert*-butyl (2-{[*cis*-4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]amino}-2-oxoethyl)carbamate
*cis* or *trans*-*N*-{(1*R*)-1-(4-chlorobenzyl)-2-[4-cylohexyl-4-(1*H*-1,2,4-triazol-1-ylmethyl)piperidin-1-yl]-2-oxoethyl}-*N*'-(4-morpholin-4-ylphenyl)cyclohexane-1,4-diamine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-4,4-difluorocyclohexanamine

27. Compounds having the following names:
1-benzyl-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(2-phenylethyl)piperidin-4-amine
2-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidin-1-yl]ethanol
3-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidin-1-yl]propan-1-ol
4-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidin-1-yl]butan-1-tol
*tert*-butyl 4-({(1*R*)-1-(4-chlorobenzyl)-2-[4cyclohexyl-4-(1*H*-1,2,4-triazol-1-ylmethyl)piperidin-1-yl]-2-oxoethyl}amino)piperidine-1-carboxylate
*N-*{(1*R*)-1-(4-chlorobenzyl)-2-[4-cyclohexyl-4-(1*H*-1,2,4-triazol-1-ylmethyl)piperidin-1-yl]-2-oxoethyl}-8-azabicyclo[3.2.1]octan-3-amine
*N*-{(1*R*)-1-(4-chlorobenzyl)-2-[4-cyclohexyl-4-*H*-(1,2,4-triazol-1-ylmethyl)piperidin-1-yl]-2-oxoethyl}-8-methyl-8-azabicyclo[3.2.1]octan-3-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-9-methyl-9-azabicyclo[3.3.1]nonan-3-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}quinuclidin-3-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}azepan-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-3-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-phenylpiperidin-4-amine
*N*-{(1*R*)-1-(4-chlorobenzyl)-2-[3-cyclohexyl-3-(1*H*-1,2,4-triazol-1-ylmethyl)-8-azabicyclo[3.2.1]oct-8-yl]-2-cxoethyl}piperidin-4-amine
1-benzyl-*N*-{(1*R*)-1-(4-chlorobenzyl)-2-[4-cyclohexyl-4-(1*H*-1,2,4-triazol-1-ylmethyl)piperidin-1-yl]-2-oxoethyl}pyrrolidin-3-amine
1-benzoyl-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
1-acetyl-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
3-({(1*R*)-1-(4-chlorobenzyl)-2-[4-cyclohexyl-4-(1*H*-1,2,4-triazol-1-ylmethyl)piperidin-1-yl]-2-oxoethyl}amino)-8-methyl-azabicyclo[3.2.1]octal-6-ol
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(trifluoroacetyl)piperidin-4-amine
4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidine-1-carboxamide
- *N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(4-fluorobenzoyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(cyclopentylcarbonyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(cyclobutylcarbonyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(4,methylphenyl)sulfonyl]piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(pyridin-2-ylcarbonyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(phenylacetyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(methylsulfonyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-2-phenylpiperidin-4-amine
(1*S*,3*R*,5*S*,7*S*)-4-({(1*R*)-1-(4-chlorobenzyl)-2-[4-cyclohexyl-4-(1*H*-1,2,4-triazol-1-ylmethyl)piperidin-1-yl]-2-oxoethyl}amino)adamantan-1-ol.

28. Compounds having the following names:
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-isonicotinoylpiperidin-4-amine.
*N*-{(1*R*)-1-(4-chlorobenzyl)-2-[4-cyclohexyl-4-(1*H*-1,2,4-triazol-ylmethyl)piperidin-1-yl]-2-oxoethyl}-1-[(1-methyl-1*H*-imidazol-2-yl)carbonyl]piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxosthyl}-1-[(5-methylisoxazol-3-yl)carbonyl]piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(3,4-difluorobenzoyl)piperidin-4-amine
1-[(1-*tert*-butyl-5-methyl-1*H*-pyrazol-3-yl)carbonyl]-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(3,5-dimethylisoxazol-4-yl)carbonyl]piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(3-thienylcarbonyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-cxoethyl}-1-(pyrrolidin-1-ylcarbonyl)piperidin-4-amine
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-phenylpiperidine-1-carboxamide
4({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*,*N*-dimethylpiperidine-1-carboxamide
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*,*N*-diethylpiperidine-1-carboxamide
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(piperidin-1-ylcarbonyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(morpholin-4-ylcarbonyl)piperidin-4-amine
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-methyl-*N* phenylpiperidine-1-carboxamide
*N*=benzyl-4-({(1*R*)-1-(4-chlorobenzyl)-2-[4-cyclohexyl-4-(1*H*-1,2,4-triazol-1-ylmethyl)piperidin-1-yl],2-oxoethyl}amino)-*N*-methylpiperidine-1-carboxamide
ethyl 4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidine-1-carboxylate
methyl 4-({(1*R*)-1-(4-chlorobenzyl)-2-[4-yclohexyl-4-(1*H*-1,2,4-triazol-1-ylmethyl)piperidin-1-yl]-2-oxoethyl}amino)piperidine-1-carboxylate
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(2*S*)-piperidin-2-ylcarbonyl]piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(2*R*)-piperidin-2-ylcarbonyl]piperidin-4-amine
*N*-{(1*R*)-1-(4-chlorobenryl)-2-[4-cyclohexyl-4-(1*H*-1,2,4-triazol-1-ylmethyl)piperidin-1-yl]-2-oxoethyl}-1-pyridin-2-ylpiperidin-4-amine
methyl *N-*[4-({(1*R*)-1-(4-chlorobenzyl)-2-[4-cyclohexyl-4-(1*H*-1,2,4-triazol-1-ylmethyl)piperidin-1-yl]-2-oxoethyl}amino)cyclohexyl]glycinate
*N*-{(1*R*)-1-(4-chlorobenzyl)-2-[4-cyclohexyl-4-(1*H*-1,2,4-triazol-1-ylmethyl)piperidin-1-yl]-2-cxoethyl}-1-(2,2-difluoroethyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(6-methylpyridazin-3-yl)piperidin-4-amine
methyl [4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidin-1-yl]acetate
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-pyrimidin-2-ylpiperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-cyclopropylpiperidin-4-amine
N-{(1*R*)-1-(4-chlorobenzyl)-2-[4-cyclohexyl-4-(1*H*-1,2,4-triazol-1-ylmethyl)piperidin-1-yl]-2-oxoethyl}-1-{[(2*S*)-4,4-difluoropiperidin-2-yl]carbonyl}piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(4,4-difluoro-L-prolyl)piperidin-4-amine
1-(1*H*-benzimidazol-2-yl)-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
1-(2,1-benzisoxazol-3-ylcarbonyl)-*N*-{(1*R*)1-(4-chlorobenzyl)-2-[4-cyclohexyl-4-(1*H*-1,2,4-triazol-1-ylmethyl)piperidin-1-yl]-2-oxoethyl]piperidin-4-amine
*N*-{(1*R)-1-(*4-chlorobenzyl)-2-[4-cyclohexyl-4-(1*H*-1,2,4-triazol-1-ylmethyl)piperidin-1-yl]-2-oxoethyl}-1-(2,2,2-trifluoroethyl)piperidin-4-amine
4-({(1*R*}-1-{4-chlorobenzyl}-2-[4-cyclohexyl-4-(1*H*-1,2,4-triazol-1-ylmethyl)piperidin-1-yl]-2-oxoethyl}amino)-*N*-(4-methoxyphenyl)piperidine-1-carboxamide
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(4-fluorophenyl)piperidine-1-carboxamide
4-({(1*R*)*-1-*(4-chlorobenzyl)-2-[4-cyclohexyl-4(1*H-1*,2,4-triazol-1-ylmethyl)piperidin-1-yl]-2-oxoethyl}amino)-*N*-(2,4-difluorophenyl)piperidine-1-carboxamide
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(3,4-difluorophenyl)piperidine-1-carboxamide
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(2-fluorophenyl)piperidine-1-carboxamide
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-(2-methoxyphenyl)piperidine-1-carboxamide
4-({(1*R*)-1-(4-chlorobenzyl)-2-[4-cyclohexyl-4-(1*H*-1,2,4-triazol-1-ylmethyl)piperidin-1-yl]-2-oxoethyl}amino)-*N*-[4-(dimethylamino)phenyl]piperidine-1-carboxamide
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(5-fluoro-1*H*-indol-2-yl)carbonyl]piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(pyrazin-2-ylcarbonyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(isoxazol-5-ylcarbonyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(quinolin-2-ylcarbonyl)piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(3-methylpyridin-2-yl)carbonyl]piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(1*H*-1,2,4-triazol-3-ylcarbonyl)piperidin-4-amine
1-(1,3-benzothiazol-2-ylcarbonyl)-*N*-{(1*R*)-1-(4-chlorobenzyl)-2-[4-cyclohexyl-4-(1*H*-1,2,4-triazol-1-ylmethyl)piperidin-1-yl]-2-oxoethyl}piperidin-4-amine
*N*-{(1*R*)-1-(4-chlorobenzyl)-2-[4-cyclohexyl-4-(1*H*-1,2,4-triazol-1-ylmethyl)pipeidin-1-yl]-2-oxoethyl}-1-[(6-methylpyridin-2-yl)carbonyl]piperidin-4-amine
1-(1-benzofuran-2-ylcarbonyl)-*N*-{(1*R*)-1-(4-chlorobenzyl)-2-[4-cyclohexyl-4-(1*H-*1,2,4-triazol-1-ylmethyl)piperidin-1-yl]-2-oxoethyl}piperidin-4-amine
*N*-{(1*R*)1-(4-chlorobenzyl)-2-[4-cyclohexyl-4-(1*H*-1,2,4-triazol-1-ylmethyl)piperidin-1-yl]-2-oxoethyl}-1-[(6-fluoropyridin-2-yl)carbonyl]piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-(2,4-difluorobenzoyl)piperidin-4-amine

29. Compounds having the following names:
1-[4-({(1*R*)-1-(4-chlorobenzyl)-2-[4-cyclohexyl-4-(1*H*-1,2,4-triazol-1-ylmethyl)piperidin-1-yl]-2-oxoethyl}amino)piperidin-1-yl]butan-2-one
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(5-phenyl-1,3-oxazol-4-yl)carbonyl]piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(1-phenyl-1*H*-pyrazol-5-yl)carbonyl]piperidin-4-amine
*N*-{(1*R*)-1-(4-chlorobenzyl)-2-[4-cyclohexyl-4-(1*H*-1,2,4-triazol-1-ylmethyl)piperidin-1-yl]-2-oxoethyl}-1-[(6-phenylpyridin-2-yl)carbonyl]piperidin-4-amine

30. Medicament, **characterized in that** it comprises a compound of formula (I) according to any one of Claims 1 to 29, or an addition salt of this compound with a pharmaceutically acceptable acid, or else a hydrate or a solvate of the compound of formula (I).

31. Pharmaceutical composition, **characterized in that** it comprises a compound of formula (I) according to any one of Claims 1 to 29, or a pharmaceutically acceptable salt, a hydrate or a solvate of this compound, and also at least one pharmaceutically acceptable excipient.

32. Use of a compound of formula (I) according to any one of Claims 1 to 29, in the manufacture of a medicament for use in the treatment and in the prevention of obesity, diabetes and sexual dysfunctions that can affect both sexes, in the treatment of cardiovascular diseases and also in antiinflammatory applications or in the treatment of alcohol dependence.

33. Use according to Claim 32, **characterized in that** said sexual dysfunctions consist of erectile dysfunctions.

34. Method for preparing a compound of formula (I) according to any one of Claims 1 to 22, **characterized in that** a reductive amination of a compound of formula (V): 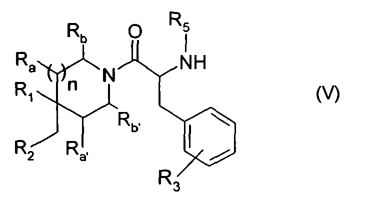 is carried out in the presence of a derivative of the group R₄ of ketone type, R₁, R₂, R₃, R₄, R₅, Rₐ, R_{a'}, R_{b}, R_{b'}, and n being as defined in any one of Claims 1 to 22.

35. Compounds of formulae (IV) and (V): 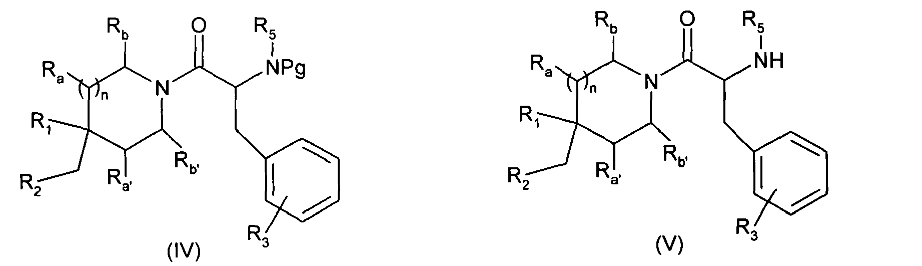 in which R₁, Rₐ, R_{a'}, R_{b} and R_{b'} are as defined in any one of Claims 1 to 22, Pg represents a protective group, and:
n = 1, Rₐ and R_{a'}, which may be identical to or different from one another, represent a hydrogen atom, or an alkyl or cycloalkyl group, and R_{b} and R_{b'} form, together with the carbon atoms of the ring to which they are attached, a carbon bridge comprising 4 or 5 members.

36. Compounds of formulae (VI), (XXVIII) and (XXIX), in which R₁, R₂, R₃, R₅, Rₐ, R_{a'}, R_{b}, R_{b'}, and n are as defined in any one of Claims 1 to 22, and in which R₄ represents a group of formula (a) or (b) as defined in any one of Claims 1 to 17 and Pg represents an amine-protecting or hydroxyl-protecting group:

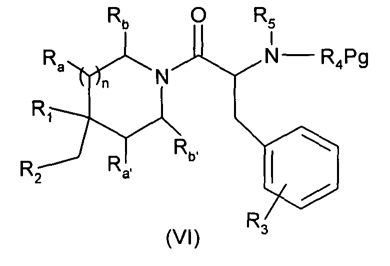

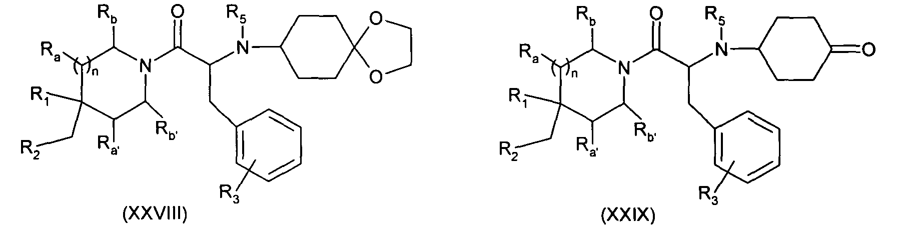

37. Compounds of formula (II): 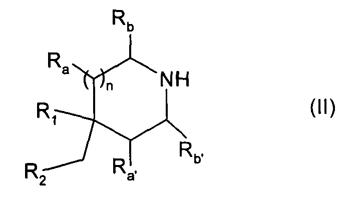 in which R₁, Rₐ, R_{a'}, R_{b} and R_{b'} are as defined in any one of Claims 1 to 22, Pg represents a protective group, and:
n = 1, Rₐ and R_{a'}, which may be identical to or different from one another, represent a hydrogen atom, or an alkyl or cycloalkyl group, and R_{b} and R_{b'} form, together with the carbon atoms of the ring to which they are attached, a carbon bridge comprising 4 or 5 members.

## Patentansprüche

1. Verbindung der Formel (I): 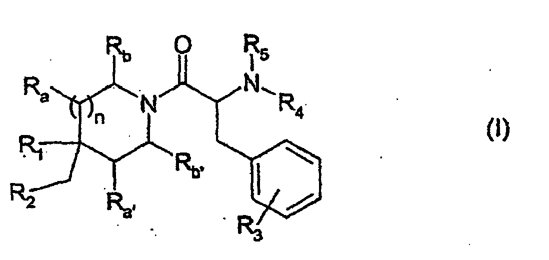 worin:
n gleich 1 ist,
Rₐ, R_{a'}, R_{b} und R_{b'} gleich oder voneinander verschieden sind und für ein Wasserstoffatom oder eine Alkyl- oder Cycloalkylgruppe stehen, wobei R_{b} und R_{b'}, zusammen mit den Kohlenstoffatomen des Rings, an die sie gebunden sind, eine Kohlenstoffbrücke mit 4 bis 5 Gliedern bilden können,
R₁ für eine Alkyl- oder Cycloalkylgruppe steht,
R₂ für eine Heteroarylgruppe steht,
R₃ für 1 bis 3 Gruppen steht, die gleich oder voneinander verschieden sind, in beliebigen Stellungen des Rings, an den sie gebunden sind, vorliegen und unter Halogenatomen und Alkyl-, Cycloalkyl-, -OR-, -NRR'-, -CO-NRR'-, -NR-CO-R'-, -NR-CO-NRR'-, -NR-COOR'-, -NO₂-, -CN- und -COOR-Gruppen ausgewählt sind,
R₅ für ein Wasserstoffatom oder eine Alkyl- oder Cycloalkylgruppe steht,
R₄ unter den nachstehenden Gruppen der Formeln (a), (b) und (c), die gegebenenfalls durch eine Oxogruppe substituiert oder ein- oder mehrfach durch eine Aryl- oder Heteroarylgruppe substituiert sein können, steht: 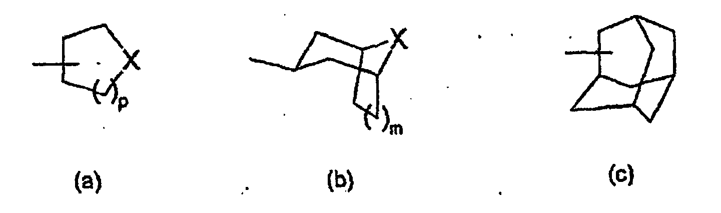
worin:
p = 0, 1, 2 oder 3,
m = 0, 1 oder 2,
und entweder
a) X für ein -N(R₁₀)-Glied steht, worin
R₁₀ unter:
einer -(CH₂)ₓ-OR₈-, -(CH₂)ₓ-COOR₈-, -(CH₂)ₓ-NR₈R₉-, -(CH₂)ₓ-CO-NR₈R₉-, - (CH₂)ₓ-NR₈COR₉- oder -(CH₂)ₓ-COR₈-Gruppe, worin x = 1, 2, 3 oder 4, • einer Cycloalkyl- oder Heterocycloalkylgruppe, die mit einer Aryl- oder Heteroarylgruppe anelliert ist,
einer Cycloalkyl- Heterocycloalkyl- Aryl-, Heteroaryl-, Alkylaryl-, Alkylheteroaryl-, -CO-Alkyl-, -CO-Cycloalkyl-, -CO-Heterocycloalkyl-, -CO-Aryl-, -CO-Heteroaryl-, -CO-Alkylaryl-, -CO-Alkylheteroaryl-, -CS-Alkyl, -CS-Cycloalkyl-, -CS-Heterocycloalkyl-, -CS-Aryl-, -CS-Heteroaryl-, -CS-Alkylaryl-, -CS-Alkylheteroaryl-, -CS-NR₈R₉-, -C(=NH)-NR₈R₉-, -SO₂-Alkyl-, -SO₂-Cycloalkyl-, -SO₂-Heterocycloalkyl-, -SO₂-Aryl-, -SO₂-Heteroaryl-, -SO₂-Alkylaryl-, -SO₂-Alkylheteroaryl- oder -SO₂-NR₈R₉-Gruppe, ausgewählt ist, wobei die Alkyl-, Cycloalkyl-, Heterocycloalkyl-, Aryl- oder Heteroarylgruppen gegebenenfalls durch eine oder mehrere unter Halogenatomen und R-, R'-, OR-, NRR'-, -CO-NRR'-, -NRCOR'-, NRCONRR'-, -NO₂-, CN-, -COOR-, OCOR-, COR-, OCONRR'- und NRCOOR'-Gruppen ausgewählte Gruppen substituiert sind;
wobei die Cycloalkyl- oder Heterocycloalkylgruppen gegebenenfalls mit einer Aryl- oder Heteroarylgruppe anelliert sind;
oder R₁₀ mit dem Stickstoffatom, an das es gebunden ist, und einem Kohlenstoffatom in einer beliebigen Stellung der cyclischen Struktur der Formel (a), jedoch nicht benachbart zu dem genannten Stickstoffatom, eine Brücke mit 3 bis 5 Gliedern bildet,
R₈ und R₉ unabhängig voneinander unter einem Wasserstoffatom und Alkyl-, Cycloalkyl-, Heterocycloalkyl-, Aryl-, Heteroaryl-, Alkylaryl-, Alkylheteroaryl-, -CO-Alkyl-, -CO-Cycloalkyl-, -CO-Heterocycloalkyl-, -CO-Aryl-, -CO-Heteroaryl-, -CO-Alkylaryl-, -CO-Alkylheteroaryl-, -SO₂-Alkyl-, -SO₂-Cycloalkyl-, -SO₂-Heterocycloalkyl-, -SO₂-Aryl-, -SO₂-Heteroaryl-, -SO₂-Alkylaryl-, -SO₂-Alkylheteroaryl-, -C(=NH)-NRR'-, -COOR-, -CO-NRR'-, -CS-NRR'- und -(CH₂)ₓ-OR-Gruppen, worin x = 0, 1, 2, 3 oder 4, ausgewählt sind, wobei die Alkyl-, Cycloalkyl-, Heterocycloalkyl-, Aryl- und Heteroarylgruppen gegebenenfalls durch eine oder mehrere unter Halogenatomen und R-, R'-, OR-, NRR'-, -CO-NRR'-, -NRCOR'-, NRCONRR'-, -NO₂-, CN-, -COOR-, OCOR-, COR-, OCONRR'- und NRCOOR'-Gruppen ausgewählte Gruppen substituiert sind;
oder R₈ und R₉ zusammen ein Cycloalkyl oder ein Heterocycloalkyl bilden;
R und R' unabhängig voneinander für ein Wasserstoffatom oder eine Alkyl-, Cycloalkyl-, Heterocycloalkyl-, Aryl-, Heteroaryl-, Alkylaryl-oder Alkylheteroarylgruppe stehen oder zusammen ein Cycloalkyl oder ein Heterocycloalkyl bilden können;
oder
b) X für ein -C(R₆) (R₇)-Glied steht, worin
R₆ unter:
• einem Wasserstoffatom, einem Halogenatom,
• einer-(CH₂)ₓ-OR₈-, -(CH₂)ₓ-COOR₈-, -(CH₂)ₓ-NR₈R₉-, - (CH₂)ₓ-CO-NR₈R₉- oder - (CH₂)ₓ-NR₈COR₉-Gruppe, worin x = 0, 1, 2, 3 oder 4,
• einer Alkyl-, Cycloalkyl-, Heterocycloalkyl, Aryl-, Heteroaryl-, Alkylaryl-, Alkylheteroaryl-, -CO-Alkyl-, -CO-Cycloalkyl-, -CO-Heterocycloalkyl-, -CO-Aryl-, -CO-Heteroaryl-, -CO-Alkylaryl-, -CO-Alkylheteroaryl-, -CS-Alkyl, -CS-Cycloalkyl-, -CS-Heterocycloalkyl-, -CS-Aryl-, -CS-Heteroaryl-, -CS-Alkylaryl-, -CS-Alkylheteroaryl-, -CS-NR₈R₉- oder -C(=NH)-NR₈R₉-Gruppe
• einer Cycloalkyl- oder Heterocycloalkylgruppe, die gegebenenfalls anelliert ist und an dem Ring der Formel (a), an den sie gebunden ist, in spiro-Stellung steht,
• einer Cycloalkyl- oder Heterocycloalkylgruppe, die mit einer Aryl- oder Heteroarylgruppe anelliert ist,
ausgewählt ist, wobei die Alkyl-, Cycloalkyl-, Heterocycloalkyl-, Aryl- oder Heteroarylgruppen gegebenenfalls durch eine oder mehrere unter Halogenatomen und R-, R'-, OR-, NRR'-, -CO-NRR'-, -NRCOR'-, NRCONRR'-, -NO₂-, CN-, -COOR-, OCOR-, COR-, OCONRR'- und NRCOOR'-Gruppen ausgewählte Gruppen substituiert sind;
wobei die Cycloalkyl- oder Heterocycloalkylgruppen gegebenenfalls mit einer Aryl- oder Heteroarylgruppe anelliert sind,
R₇ unter Wasserstoff- und Halogenatomen und Alkyl-, Cycloalkyl-, Aryl-, Heteroaryl-, Alkylaryl-, Alkylheteroaryl-, -OR-, -O-Aryl-, -O-Heteroaryl-, -O-Alkylaryl-, -O-Alkylheteroaryl-, -NRR'-, -CO-NRR' -, -NR-CO-R'-, -NR-CO-NRR' -, -NR-COOR'-, -NO₂-, -CN- und -COOR-Gruppen ausgewählt ist,
R₈ und R₉ unabhängig voneinander unter einem Wasserstoffatom und Alkyl-, Cycloalkyl-, Heterocycloalkyl-, Aryl-, Heteroaryl-, Alkylaryl-, Alkylheteroaryl-, -CO-Alkyl-, -CO-Cycloalkyl-, -CO-Heterocycloalkyl-, -CO-Aryl-, -CO-Heteroaryl-, -CO-Alkylaryl-, -CO-Alkylheteroaryl-, -SO₂-Alkyl-, -SO₂-Cycloalkyl-, -SO₂-Heterocycloalkyl-, -SO₂-Aryl-, -SO₂-Heteroaryl-, -SO₂-Alkylaryl-, -SO₂-Alkylheteroaryl-, -C(=NH)-NRR'-, -COOR-, -CO-NRR' -, -CS-NRR'- und -(CH₂)ₓ-OR-Gruppen, worin x = 0, 1, 2, 3 oder 4, ausgewählt sind, wobei die Alkyl-, Cycloalkyl-, Heterocycloalkyl-, Aryl- und Heteroarylgruppen gegebenenfalls durch eine oder mehrere unter Halogenatomen und R-, R'-, OR-, NRR'-, -CO-NRR' -, -NRCOR'-, NRCONRR'-, -NO₂-, CN-, -COOR-, OCOR-, COR-, OCONRR'- und NRCOOR'-Gruppen ausgewählte Gruppen substituiert sind;
oder R₈ und R₉ zusammen ein Cycloalkyl oder ein Heterocycloalkyl bilden;
R und R' unabhängig voneinander für ein Wasserstoffatom oder eine Alkyl-, Cycloalkyl-, Heterocycloalkyl-, Aryl-, Heteroaryl-, Alkylaryl-oder Alkylheteroarylgruppe stehen oder zusammen ein Cycloalkyl oder ein Heterocycloalkyl bilden;
in Basen- oder Säureadditionssalzform sowie in Hydrat- oder Solvatform.

2. Verbindung der Formel (I) nach Anspruch 1, **dadurch gekennzeichnet, daß** R₄ unter Gruppen der Formeln (a), (b) und (c), die gegebenenfalls ein-oder mehrfach durch eine Aryl- oder Heteroarylgruppe substituiert sein können, ausgewählt ist, wobei X für ein -C(R₆)(R₇)-Glied steht, worin
R₆ unter:
• einem Wasserstoffatom,
• einer -(CH₂)ₓ-OR₈-, -(CH₂)ₓ-COOR₈-, -(CH₂)ₓ-NR₈R₉-, - (CH₂)ₓ-CO-NR₈R₉- oder -(CH₂)ₓ-NR₈COR₉-Gruppe, worin x = 0, 1, 2, 3 oder 4,
• einer Alkyl-, Cycloalkyl-, Heterocycloalkyl-, Aryl-, Heteroaryl-, Alkylaryl-, Alkylheteroaryl-, -CO-Alkyl-, -CO-Cycloalkyl-, -CO-Heterocycloalkyl-, -CO-Aryl-, -CO-Heteroaryl-, -CO-Alkylaryl- oder -CO-Alkylheteroarylgruppe,
• einer Cycloalkyl- oder Heterocycloalkylgruppe, die an dem Ring der Formel (a), an den sie gebunden ist, in spiro-Stellung vorliegt,
• einer Cycloalkyl- oder Heterocycloalkylgruppe, die mit einer Aryl- oder Heteroarylgruppe fusioniert ist,
ausgewählt ist,
R₇ unter Wasserstoff- und Halogenatomen und Alkyl-, Cycloalkyl-, Aryl-, Heteroarylgruppen, Alkylaryl-, Alkylheteroaryl-, -OR-, -O-Aryl-, -O-Heteroaryl-, -0-Alkylaryl-, -O-Alkylheteroaryl-, -NRR'-, -CO-NRR' -, -NR-CO-R'-, -NR-CO-NRR'-, -NR-COOR'-, -NO₂-, -CN- und -COOR-Gruppen ausgewählt ist,
R₈ und R₉ unabhängig voneinander unter einem Wasserstoffatom und Alkyl-, Cycloalkyl-, Heterocycloalkyl-, Aryl-, Heteroaryl-, Alkylaryl-, Alkylheteroaryl-, -CO-Alkyl-, -CO-Cycloalkyl-, -CO-Heterocycloalkyl-, -CO-Aryl-, -CO-Heteroaryl-, -CO-Alkylaryl-, -CO-Alkylheteroaryl-, -SO₂-Alkyl-, -SO₂-Cycloalkyl-, -SO₂-Heterocycloalkyl-, -SO₂-Aryl-, -SO₂-Heteroaryl-, -SO₂-Alkylaryl-, -SO₂-Alkylheteroaryl-, -C(=NH)-NRR'-, -COOR-, -CO-NRR'-, -CS-NRR'- und -(CH₂)ₓ-OR-Gruppen, worin x = 0, 1, 2, 3 oder 4, ausgewählt sind;
R und R' unabhängig voneinander für ein Wasserstoffatom oder eine Alkyl-, Cycloalkyl-, Heterocycloalkyl-, Aryl-, Heteroaryl-, Alkylaryl-oder Alkylheteroarylgruppe stehen.

3. Verbindung der Formel (I) nach Anspruch 1, **dadurch gekennzeichnet, daß** R₄ unter Gruppen der Formeln (a), (b) und (c) ausgewählt ist, wobei X für ein -C(R₆) (R₇) -Glied steht, worin R₆ unter einem Halogenatom oder einer Cycloalkyl- oder Heterocycloalkylgruppe, die gegebenenfalls anelliert ist, und an dem Ring der Formel (a), an den sie gebunden ist, in spiro-Stellung vorliegt, ausgewählt ist.

4. Verbindung der Formel (I) nach Anspruch 1, **dadurch gekennzeichnet, daß** R₄ unter Gruppen der Formeln (a), (b) und (c) ausgewählt ist, wobei X für ein -C(R₆) (R₇) -Glied steht, worin R₆ unter -CS-Alkyl-, -CS-Cycloalkyl-, -CS-Heterocycloalkyl-, -CS-Aryl-, -CS-Heteroaryl-, -CS-Alkylaryl-, -CS-Alkylheteroaryl-, -CS-NR₈R₉ und -C(=NH)-NR₈R₉ ausgewählt ist.

5. Verbindung der Formel (I) nach Anspruch 1, **dadurch gekennzeichnet, daß** R₄ unter Gruppen der Formeln (a), (b) und (c) ausgewählt ist, wobei X für ein -C(R₆) (R₇)-Glied steht, worin die Alkyl-, Cycloalkyl-, Heterocycloalkyl-, Aryl- oder Heteroarylgruppen gegebenenfalls durch eine oder mehrere unter R oder R', OCOR, COR, OCONRR', NRCOOR' ausgewählte Gruppen substituiert sind.

6. Verbindung der Formel (I) nach Anspruch 1, **dadurch gekennzeichnet, daß** R₄ unter Gruppen der Formeln (a), (b) und (c) ausgewählt ist, wobei X für ein -C(R₆) (R₇)-Glied steht, worin die Cycloalkyl- oder Heterocycloalkylgruppen gegebenenfalls mit einer Aryl- oder Heteroarylgruppe anelliert sein können.

7. Verbindung der Formel (I) nach Anspruch 1, **dadurch gekennzeichnet, daß** R₄ unter Gruppen der Formeln (a), (b) und (c) ausgewählt ist, wobei X für ein -C(R₆) (R₇) -Glied steht, worin R₈ und R₉ unabhängig voneinander für Alkyl-, Cycloalkyl-, Heterocycloalkyl-, Aryl- und Heteroarylgruppen stehen, die gegebenenfalls durch eine oder mehrere unter R-, R'-, COR, OCOR-, OCONRR', NRCOOR'-Gruppen ausgewählte Gruppen substituiert sind; oder R₈ und R₉ zusammen ein Cycloalkyl oder ein Heterocycloalkyl bilden.

8. Verbindung der Formel (I) nach Anspruch 1, **dadurch gekennzeichnet, daß** R₄ unter Gruppen der Formeln (a), (b) und (c) ausgewählt ist, wobei X für ein -C(R₆) (R₇) -Glied steht, worin R und R' zusammen ein Cycloalkyl oder ein Heterocycloalkyl bilden können.

9. Verbindungen der Formel (I) nach einem der Ansprüche 1 bis 8, **dadurch gekennzeichnet, daß** R₇ für Wasserstoff steht.

10. Verbindungen der Formel (I) nach einem der Ansprüche 1 bis 9, **dadurch gekennzeichnet, daß** R₄ für die Gruppe der Formel a) mit p = 2 gemäß der nachstehenden Definition steht:

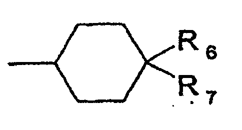

11. Verbindungen der Formel (I) nach Anspruch 1, **dadurch gekennzeichnet, daß**
R₄ unter Gruppen der Formeln (a), (b) und (c), die gegebenenfalls ein- oder mehrfach durch eine Aryl-oder Heteroarylgruppe substituiert sein können, ausgewählt ist, wobei X für ein -N(R₁₀)-Glied steht, worin
R₁₀ unter:
einer -CO-NR₈R₉- oder -COOR₈-Gruppe,
einer -(CH₂)ₓ-OR₈-, - (CH₂)ₓ-COOR₈-, -(CH₂)ₓ-NR₈R₉-, - (CH₂)ₓ-CO-NR₈R₉- oder -(CH₂)ₓ-NR₈COR₉, worin x = 1, 2, 3 oder 4,
• einer Cycloalkyl- oder Heterocycloalkylgruppe, die mit einer Aryl- oder Heteroarylgruppe anelliert ist,
einer Cycloalkyl-, Heterocycloalkyl-, Aryl-, Heteroaryl-, Alkylaryl-, Alkylheteroaryl-, -CO-Cycloalkyl-, -CO-Heterocycloalkyl-, -CO-Heteroaryl-, -CO-Alkylaryl-, -CO-Alkylheteroaryl-, -CS-Alkyl-, -CS-Cycloalkyl-, -CS-Heterocycloalkyl-, -CS-Aryl-, -CS-Heteroaryl-, -CS-Alkylaryl-, -CS-Alkylheteroaryl-, -CS-NR₈R₉-, -C(=NH)-NR₈R₉-, -SO₂-Cycloalkyl-, -SO₂-Heterocycloalkyl-, -SO₂-Heteroaryl-, -SO₂-Alkylaryl-, -SO₂-Alkylheteroaryl- oder -SO₂-NR₈R₉-Gruppe
ausgewählt ist;
oder R₁₀ mit dem Stickstoffatom, an das es gebunden ist, und einem Kohlenstoffatom in beliebiger Stellung der cyclischen Struktur der Formel (a), jedoch nicht benachbart zu dem genannten Stickstoffatom, eine Brücke mit 3 bis 5 Gliedern bildet;
R₈ und R₉ unabhängig voneinander unter einem Wasserstoffatom und Alkyl-, Cycloalkyl-, Heterocycloalkyl-, Aryl-, Heteroaryl-, Alkylaryl-, Alkylheteroaryl-, -CO-Alkyl-, -CO-Cycloalkyl-, -CO-Heterocycloalkyl-, -CO-Aryl-, -CO-Heteroaryl-, -CO-Alkylaryl-, -CO-Alkylheteroaryl-, -SO₂-Alkyl-, -SO₂-Cycloalkyl-, -SO₂-Heterocycloalkyl-, -SO₂-Aryl-, -SO₂-Heteroaryl-, -SO₂-Alkylaryl-, -SO₂-Alkylheteroaryl-, -C(=NH)-NRR'-, -COOR-, -CO-NRR'-, -CS-NRR'- und -(CH₂)ₓ-OR-Gruppen, worin X = 0, 1, 2, 3 oder 4, ausgewählt sind;
R und R' unabhängig voneinander für ein Wasserstoffatom oder eine Alkyl-, Cycloalkyl-, Heterocycloalkyl-, Aryl-, Heteroaryl-, Alkylaryl-oder Alkylheteroarylgruppe stehen.

12. Verbindung der Formel (I) nach Anspruch 1, **dadurch gekennzeichnet, daß** R₄ unter Gruppen der Formeln (a), (b) und (c), die gegebenenfalls durch eine Oxogruppe substituiert sind, ausgewählt sind, wobei X für ein -N(R₁₀)-Glied steht.

13. Verbindung der Formel (I) nach Anspruch 1, **dadurch gekennzeichnet, daß** R₄ unter Gruppen der Formeln (a), (b) und (c) ausgewählt ist, wobei X für ein -N(R₁₀)-Glied steht, worin R₈ und R₉ unabhängig voneinander für Alkyl-, Cycloalkyl-, Heterocycloalkyl-, Aryl- und Heteroarylgruppen stehen, die gegebenenfalls durch eine oder mehrere unter Halogenatomen und R-, R'-, OR-, NRR'-, -CO-NRR'-, -OCOR, NRCOR'-, NRCONRR'-, COR-, -NO₂-, CN-, -COOR-, OCONRR'- und NRCOOR'-Gruppen ausgewählte Gruppen substituiert sind;
oder R₈ und R₉ zusammen ein Cycloalkyl oder ein Heterocycloalkyl bilden.

14. Verbindung der Formel (I) nach Anspruch 1, **dadurch gekennzeichnet, daß** R₄ unter Gruppen der Formeln (a), (b) und (c) ausgewählt ist, wobei X für ein -N (R₁₀)-Glied steht, worin R₁₀ für -(CH₂)ₓ-COR₈ mit x = 1, 2, 3 oder 4 steht.

15. Verbindung der Formel (I) nach Anspruch 1, **dadurch gekennzeichnet, daß** R₄ unter Gruppen der Formeln (a), (b) und (c) ausgewählt ist, wobei X für ein -N(R₁₀)-Glied steht, worin die Alkyl-, Cycloalkyl-, Heterocycloalkyl-, Aryl- oder Heteroarylgruppen gegebenenfalls durch ein oder mehrere unter R, R', OCOR, COR, OCONRR' oder NRCOOR' ausgewählte Gruppen substituiert sind.

16. Verbindung der Formel (I) nach Anspruch 1, **dadurch gekennzeichnet, daß** R₄ unter Gruppen der Formeln (a), (b) und (c) ausgewählt ist, wobei X für ein -N(R₁₀)-Glied steht, worin die Cycloalkyl-oder Heterocycloalkylgruppen gegebenenfalls mit einer Aryl- oder Heteroarylgruppe anelliert sind.

17. Verbindung der Formel (I) nach einem der Ansprüche 1 und 11 bis 16, **dadurch gekennzeichnet, daß** R₄ für die Gruppe der Formeln (a) mit p = 2 gemäß der nachstehenden Definition steht:

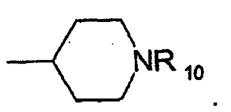

18. Verbindung der Formel (I) nach einem der Ansprüche 1 bis 17, **dadurch gekennzeichnet, daß** R₁ für eine Cycloalkylgruppe steht,
in Basen- oder Säureadditionssalzform sowie in Hydrat- oder Solvatform.

19. Verbindung der Formel (I) nach einem der Ansprüche 1 bis 18, **dadurch gekennzeichnet, daß** R₂ für eine Triazolylgruppe steht,
in Basen- oder Säureadditionssalzform sowie in Hydrat- oder Solvatform.

20. Verbindung der Formel (I) nach einem der Ansprüche 1 bis 19, **dadurch gekennzeichnet, daß** R₃ für 1 bis 3 Gruppen steht, die gleich oder voneinander verschieden sind und unter Halogenatomen ausgewählt sind,
in Basen- oder Säureadditionssalzform sowie in Hydrat- oder Solvatform.

21. Verbindung der Formel (I) nach einem der Ansprüche 1 bis 20, **dadurch gekennzeichnet, daß** R₅ für ein Wasserstoffatom steht
in Basen- oder Säureadditionssalzform sowie in Hydrat- oder Solvatform.

22. Verbindung der Formel (I) nach einem der Ansprüche 1 bis 21, **dadurch gekennzeichnet, daß**:
- n = 1 und Rₐ = R_{a'},= R_{b} = R_{b'} = H, oder
- n = 1, Rₐ = R_{a'} = H und R_{b} und R_{b'} zusammen mit den Kohlenstoffatomen des Rings, an die sie gebunden sind, eine Kohlenstoffbrücke mit 4 Gliedern bilden,
in Basen- oder Säureadditionssalzform sowie in Hydrat- oder Solvatform.

23. Verbindungen mit den folgenden Namen:
N-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-(2-phenylethyl)piperidin-4-amin 4-[4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexyl]phenol
*cis*-*N*-{(1*R*)-1-(4-Chlorbenzyl)-2-[4-cyclohexyl-4-(1*H*-1,2,4-triazol-1-ylmethyl)piperidin-1-yl]-2-oxoethyl}cyclohexan-1,4-diamin
*trans*-*N*-{(1*R*)-1-(4-Chlorbenzyl)-2-[4-cyclohexyl-4-(1*H*-1,2,4-triazol-1-ylmethyl)piperidin-1-yl]-2-oxoethyl}cyclohexan-1,4-diamin
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-8-methyl-8-azabicyclo[3.2.1]octan-3-amin
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-9-methyl-9-azabicyclo[3.3.1]nonan-3-amin
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-1-phenylpiperidin-4-amin
1-Benzoyl-*N*-{(1*R*)-1-(4-chlorbenzyl)-2-[4-cyclohexyl-4-(*1H*-1,2,4-triazol-1-ylmethyl)piperidin-1-yl]-2-oxoethyl}piperidin-4-amin
1-Acetyl-*N*-{(1*R*)-1-(4-chlorbenzyl)-2-[4-cyclohexyl-4-(*1H*,2,4-triazol-1-ylmethyl)piperidin-1-yl]-2-oxoethyl}piperidin-4-amin
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-1,4-dioxyspiro[4.5]decan-8-amin
*N'*-{(1*R*)-1-(4-Chlorbenzyl)-2-[4-cyclohexyl-4-(*1H-*1,2,4-triazol-1-ylmethyl)piperidin-1-yl]-2-oxo-ethyl}-*N*,*N*-dimethylcyclohexan-1,4-diamin
4-(Aminomethyl)-*N*-{(1*R*)-1-(4-chlorbenzyl)-2-[4-cyclohexyl-4-(*1H*-1,2,4-triazol-1-ylmethyl)-piperidin-1-yl]-2-oxoethyl}cyclohexanamin
3-({(1*R*)-1-(4-Chlorbenzyl)-2-[4-cyclohexyl-4-(*1H-*1,2,4-triazol-1-ylmethyl)piperidin-1-yl]-2-oxo-ethyl}amino)-8-methyl-8-azabicyclo[3.2.1]octan-6-ol
*cis*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexanol
*trans*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexanol
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-1-(trifluoracetyl)piperidin-4-amin
4-({(1*R*)-1-(4-Chlorbenzyl)-2-[4-cyclohexyl-4-(*1H-*1,2,4-triazol-1-ylmethyl)piperidin-1-yl]-2-oxo-ethyl}amino)piperidin-1-carboxamid
*cis*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)-1-phenylcyclohexanol
*trans*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)-1-phenylcyclohexanol
*cis*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-*N'*-(4-fluorphenyl)cyclohexan-1,4-diamin
*trans*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-*N'*-(4-fluorphenyl)cyclohexan-1,4-diamin
*N*-[*cis*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]-2,2,2-trifluoracetamid
*N*-[*trans*-4-({(1*R*)-1-(4-Chlorbenzyl)-2-[4-cyclohexyl-4-(*1H*-1,2,4-triazol-1-ylmethyl)-piperidin-1-yl]-2-oxoethyl}amino)cyclohexyl]-2,2,2-trifluoracetamid
*N*-[*cis*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]acetamid
*N*-[*trans*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]acetamid
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-1-(4-fluorbenzoyl)piperidin-4-amin
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-1-(cyclopentylcarbonyl)piperidin-4-amin
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-1-(cyclobutylcarbonyl)piperidin-4-amin
*cis*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-*N'*-methylcyclohexan-1,4-diamin
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-1-(pyridin-2-ylcarbonyl)piperidin-4-amin
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-1-(phenylacetyl)piperidin-4-amin
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-1-(methylsulfonyl)piperidin-4-amin
*N*-[*cis*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]-*N*-methylacetamid
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-4-(1,3-dihydro-2*H*-isoindol-2-yl)cyclo-hexanamin
*N*-[*cis*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]-*N*-methylbenzamid
*cis*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexancarbonsäureethylester
*trans*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexancarbonsäureethylester
*trans*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-*N'*-phenylcyclohexan-1,4-diamin
*cis*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-*N'-*phenylcyclohexan-1,4-diamin
*N'*-{(1*R*)-1-(4-Chlorbenzyl)-2-[4-cyclohexyl-4-(*1H-*1,2,4-triazol-1-ylmethyl)piperidin-1-yl]-2-oxo-ethyl}-*N*-methyl-*N*-phenylcyclohexan-1,4-diamin
*N'*-{(1*R*)-1-(4-Chlorbenzyl)-2-[4-cyclohexyl-4-(*1H-*1,2,4-triazol-1-ylmethyl)piperidin-1-yl]-2-oxo-ethyl}-*N*-(4-fluorphenyl)-*N*-methylcyclohexan-1,4-diamin
*cis-* oder *trans*-4-({(1*R*)-1-(4-Chlorbenzyl)-2-[4-cyclohexyl-4-(*1H*-1,2,4-triazol-1-ylmethyl)-piperidin-1-yl]-2-oxoethyl}amino)-*N*,*N*-diethyl-cyclohexancarboxamid
*cis-* oder *trans*-4-({(1*R*)-1-(4-Chlorbenzyl)-2-[4-cyclohexyl-4-(*1H*-1,2,4-triazol-1-ylmethyl)-piperidin-1-yl]-2-oxoethyl}amino)-*N*,*N*-diethyl-cyclohexancarboxamid
*cis-* oder *trans*-4-({(1*R*)-1-(4-Chlorbenzyl)-2-[4-cyclohexyl-4-(*1H*-1,2,4-triazol-1-ylmethyl)-piperidin-1-yl]-2-oxoethyl}amino)-*N*,*N*-dimethyl-cyclohexancarboxamid
*cis-* oder *trans*-4-({(1*R*)-1-(4-Chlorbenzyl)-2-[4-cyclohexyl-4-(*1H*-1,2,4-triazol-1-ylmethyl)-piperidin-1-yl]-2-oxoethyl}amino)-*N*,*N*-dimethyl-cyclohexancarboxamid
*cis*-*N*-Benzyl-4-({(1*R*)-1-(4-chlorbenzyl)-2-[4-cyclohexyl-4-(*1H*-1,2,4-triazol-1-ylmethyl)-piperidin-1-yl]-2-oxoethyl}amino)-*N*-methylcyclo-hexancarboxamid
*trans*-*N*-Benzyl-4-({(1*R*)-1-(4-chlorbenzyl)-2-[4-cyclohexyl-4-(*1H*-1,2,4-triazol-1-ylmethyl)-piperidin-1-yl]-2-oxoethyl}amino)-*N*-methylcyclo-hexancarboxamid
*cis*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-4-(3-methyl-1,2,4-oxadiazol-5-yl)cyclo-hexanamin
*trans*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-4-(3-methyl-1,2,4-oxadiazol-5-yl)cyclo-hexanamin
*cis*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexancarboxamid
*trans*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexancarboxamid
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-1-isonicotinoylpiperidin-4-amin
*cis*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)-1-(4-fluorphenyl)cyclohexanol
*trans*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)-1-(4-fluorphenyl)cyclohexanol *N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-1-[(1-methyl-*1H*-imidazol-2-yl)carbonyl]-piperidin-4-amin
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-1-[(5-methylisoxazol-3-yl)Carbonyl]-piperidin-4-amin
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-1-(3,4-difluorbenzoyl)piperidin-4-amin
1-[(1-tert.-Butyl-5-methyl-*1H*-pyrazol-3-yl)-carbonyl]-*N*-{(1*R*)-1-(4-chlorbenzyl)-2-[4-cyclohexyl-4-(*1H*-1,2,4-triazol-1-ylmethyl)piperidin-1-yl]-2-oxoethyl}piperidin-4-amin
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-1-[(3,5-dimethylisoxazol-4-yl)carbonyl]-piperidin-4-amin
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-1-[(3-thienylcarbonyl]piperidin-4-amin
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-1-[(pyrrolidin-1-ylcarbonyl]piperidin-4-amin
4-({(1*R*)-1-(4-Chlorbenzyl)-2-[4-cyclohexyl-4-(*1H-*1,2,4-triazol-1-ylmethyl)piperidin-1-yl]-2-oxo-ethyl}amino)-*N*-phenylpiperidin-1-carboxamid
4-({(1*R*)-1-(4-Chlorbenzyl)-2-[4-cyclohexyl-4-(*1H-*1,2,4-triazol-1-ylmethyl)piperidin-1-yl]-2-oxo-ethyl}amino)-*N*,*N*-dimethylpiperidin-1-carboxamid
4-({(1*R*)-1-(4-Chlorbenzyl)-2-[4-cyclohexyl-4-(*1H-*1,2,4-triazol-1-ylmethyl)piperidin-1-yl]-2-oxo-ethyl}amino)-*N*,*N*-diethylpiperidin-1-carboxamid
*N*-({(1*R*)-1-(4-Chlorbenzyl)-2-[4-cyclohexyl-4-(*1H-*1,2,4-triazol-1-ylmethyl)piperidin-1-yl]-2-oxo-ethyl}-1-(piperidin-1-ylcarbonyl)piperidin-4-amin
*N*-({(1*R*)-1-(4-Chlorbenzyl)-2-[4-cyclohexyl-4-(*1H-*1,2,4-triazol-1-ylmethyl)piperidin-1-yl]-2-oxo-ethyl}-1-(morpholin-4-ylcarbonyl)piperidin-4-amin 4-({(1*R*)-1-(4-Chlorbenzyl)-2-[4-cyclohexyl-4-(*1H-*1,2,4-triazol-1-ylmethyl)piperidin-1-yl]-2-oxo-ethyl}amino)-*N*-methyl-*N*-phenylpiperidin-1-carboxamid
*N*-Benzyl-4-({(1*R*)-1-(4-chlorbenzyl)-2-[4-cyclohexyl-4-(*1H*-1,2,4-triazol-1-ylmethyl)piperidin-1-yl]-2-oxoethyl}amino)-*N*-methylpiperidin-1-carboxamid
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-4-methoxycyclohexanamin
4-[4-(Benzyloxy)phenyl]-*N*-{(1*R*)-1-(4-chlorbenzyl)-2-[4-cyclohexyl-4-(*1H*-1,2,4-triazol-1-ylmethyl)-piperidin-1-yl]-2-oxoethyl}cyclohexanamin
*N*-[*cis*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]-2-methoxyacetamid
Essigsäure-2-{[*cis*-4-({(1*R*)-1-(4-Chlorbenzyl)-2-[4-cyclohexyl-4-(*1H*-1,2,4-triazol-1-ylmethyl)-piperidin-1-yl]-2-oxoethyl}amino)cyclohexyl]-amino}-2-oxoethylester
2-(Benzyloxy)-*N*-[*cis*-4-({(1*R*)-1-(4-Chlorbenzyl)-2-[4-cyclohexyl-4-(*1H*-1,2,4-triazol-1-ylmethyl)-piperidin-1-yl]-2-oxoethyl}amino)cyclohexyl]-acetamid
3-[*cis*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]-1,3-oxazolidin-2-on
3-[*trans*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]-1,3-oxazolidin-2-on
*cis*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-*N'*-(2-methoxyethyl)cyclohexan-1,4-diamin
*trans*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-*N'*-(2-methoxyethyl)cyclohexan-1,4-diamin
*cis*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-4-morpholin-4-ylcyclohexanamin
*trans*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-4-morpholin-4-ylcyclohexanamin
1-[*cis*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]pyrrolidin-2-on
1-[*trans*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]pyrrolidin-2-on *N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-4-(2-methoxyethoxy)cyclohexanamin
4-({(1*R*)-1-(4-Chlorbenzyl)-2-[4-cyclohexyl-4-(*1H-*1,2,4-triazol-1-ylmethyl)piperidin-1-yl]-2-oxo-ethyl}amino)piperidin-1-carbonsäureethylester
4-({(1*R*)-1-(4-Chlorbenzyl)-2-[4-cyclohexyl-4-(*1H-*1,2,4-triazol-1-ylmethyl)piperidin-1-yl]-2-oxo-ethyl}amino)piperidin-1-carbonsäuremethylester
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-1-[(2*S*)piperidin-2-ylcarbonyl]piperidin-4-amin
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-1-[(2*R*)piperidin-2-ylcarbonyl]piperidin-4-amin
*cis*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexancarbonitril
*trans*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexancarbonitril
1-[*cis*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]piperidin-2-on
1-[*trans*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]piperidin-2-on
*N*-[4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]propanamid
(2-{[*cis*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]amino)-2-oxoethyl)carbamidsäure-tert.-butylester
*N*-[*cis*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]glycinamid *N*-[*cis*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]-2-hydroxyacetamid
*N*-[*cis*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]-2,2-dimethylpropanamid
*N*-[*trans*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]-2,2-dimethyl-propanamid
*cis*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-*N'*-(4-methoxyphenyl)cyclohexan-1,4-diamin
*trans*-*N*-{(1*R*)-1-(4-Chlorbenzyl)-2-[4-cyclohexyl-4-(2*H*-1,2,4-triazol-1-ylmethyl)piperidin-1-yl]-2-oxoethyl}-*N'*-(4-methoxyphenyl)cyclohexan-1,4-diamin
*cis*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-4-(2*H*-tetrazol-5-yl)cyclohexanamin
*trans*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-4-(2*H*-tetrazol-5-yl)cyclohexanamin
2-{[*cis*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]amino}phenol
2-{[*trans*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]amino}phenol Essigsäure-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexylester
*N²*-[*cis*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]-*N*,*N*-dimethylglycinamid
*N²*-[*trans*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]-*N*,*N*-dimethyl-glycinamid
*N²*-[*cis*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]glycinamid
*N²*-[*trans*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]glycinamid
4-[*cis*-4-(({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]piperazin-2-on
4-[*trans*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]piperazin-2-on
*cis*-4-(4-Acetylpiperazin-1-yl)-*N*-{(1*R*)-1-(4-chlorbenzyl)-2-[4-cyclohexyl-4-(*1H*-1,2,4-triazol-1-ylmethyl)piperidin-1-yl]-2-oxoethyl}cyclohexanamin
*trans*-4-(4-Acetylpiperazin-1-yl)-*N*-{(1*R*)-1-(4-chlorbenzyl)-2-[4-cyclohexyl-4-(*1H*-1,2,4-triazol-1-ylmethyl)piperidin-1-yl]-2-oxoethyl}cyclohexanamin
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-1-pyridin-2-ylpiperidin-4-amin
*N*-[4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]glycinmethylester
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-1-(2,2-difluorethyl)piperidin-4-amin
*cis*-*N*-{(1*R*)-1-(4-Chlorbenzyl)-2-[4-cyclohexyl-4-(2*H*-1,2,4-triazol-1-ylmethyl)piperidin-1-yl]-2-oxoethyl}-*N'*-(4-chlorphenyl)cyclohexan-1,4-diamin
*trans*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-*N*'-(4-chlorphenyl)cyclohexan-1,4-diamin
Pivalinsäure-4-({(1*R*)-1-(4-Chlorbenzyl)-2-[4-cyclohexyl-4-(*1H*-1,2,4-triazol-1-ylmethyl)-piperidin-1-yl]-2-oxoethyl}amino)cyclohexylester
*cis*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-*N'*-(4-fluor-2-methylphenyl)cyclohexan-1,4-diamin *trans*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-*N'*-(4-fluor-2-methylphenyl)cyclohexan-1,4-diamin
4-{[4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]amino}benzonitril
*N*-[4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]cyclopropancarboxamid
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-1-(6-methylpyridazin-3-yl)piperidin-4-amin
[4-({(1*R*)-1-(4-Chlorbenzyl)-2-[4-cyclohexyl-4-(*1H-*1,2,4-triazol-1-ylmethyl)piperidin-1-yl]-2-oxo-ethyl}amino)piperidin-1-yl]essigsäuremethylester
*cis*- oder *trans*-*N*-{(1*R*)-1-(4-Chlorbenzyl)-2-[4-cyclohexyl-4-(*1H*-1,2,4-triazol-1-ylmethyl)-piperidin-1-yl]-2-oxoethyl}-*N'*-(4-morpholin-4-ylphenyl)cyclohexan-1,4-diamin
*cis*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-*N'*-(2,4-difluorphenyl)cyclohexan-1,4-diamin
*trans*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-*N'*-(2,4-difluorphenyl)cyclohexan-1,4-diamin
*cis*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-*N'*-(5-fluorpyridin-2-yl)cyclohexan-1,4-diamin
*trans*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-*N'*-(5-fluorpyridin-2-yl)cyclohexan-1,4-diamin
*N*-*1H*-1,2,3-Benzotriazol-5-yl-*N'*-{(1*R*)-1-(4-chlorbenzyl)-2-[4-cyclohexyl-4-(*1H*-1,2,4-triazol-1-ylmethyl)piperidin-1-yl]-2-oxoethyl}cyclohexan-1,4-diamin
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-1-pyrimidin-2-ylpiperidin-4-amin
1-[*cis*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]imidazolidin-2-on
1-[*trans*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]imidazolidin-2-on
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-1-cyclopropylpiperidin-4-amin
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-1-{[(2*S*)-4,4-difluorpiperidin-2-yl]-carbonyl}piperidin-4-amin
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-4,4-difluorcyclohexanamin
*cis*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-*N'*-(3,4-difluorphenyl)cyclohexan-1,4-diamin
*trans*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-*N'*-(3,4-difluorphenyl)cyclohexan-1,4-diamin
*cis*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-*N'*-(4-fluor-3-methoxyphenyl)cyclohexan-1,4-diamin
*trans*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-*N'*-(4-fluor-3-methoxyphenyl)cyclohexan-1,4-diamin
*cis*-*N'*-{(1*R*)-1-(4-Chlorbenzyl)-2-[4-cyclohexyl-4-(*1H*-1,2,4-triazol-1-ylmethyl)piperidin-1-yl]-2-oxoethyl}-*N*-ethyl-*N*-(4-fluor-3-methoxyphenyl)-cyclohexan-1,4-diamin *trans-N'-{(1R)-1-(4-*Chlorbenzyl)-2-[4-cyclohexyl-4-(*1H*-1,2,4-triazol-1-ylmethyl)piperidin-1-yl]-2-oxoethyl}-*N*-ethyl-*N-*(4-fluor-3-methoxyphenyl)cyclohexan-1,4-diamin
*cis*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-*N'*-[4-(trifluormethyl)phenyl]cyclohexan-1,4-diamin
*trans*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-*N'*-[4-(trifluormethyl)phenyl]cyclohexan-1,4-diamin
*cis*-*N*-{(1*R*)-1-(4-Chlorbenzyl)-2-[4-cyclohexyl-4-(2*H*-1,2,4-triazol-1-ylmethyl)piperidin-1-yl]-2-oxoethyl}-*N'*-(4-fluor-3-methylphenyl)cyclohexan-1,4-diamin
*trans*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-*N*'-(4-fluor-3-methylphenyl)cyclohexan-1,4-diamin
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-1-(4,4-difluor-L-prolyl)piperidin-4-amin 1-(*1H*-Benzimidazol-2-yl)-*N*-{(1*R*)-1-(4-chlorbenzyl)-2-[4-cyclohexyl-4-(*1H*-1,2,4-triazol-1-ylmethyl)piperidin-1-yl]-2-oxoethyl}piperidin-4-amin
1-(2,1-Benzisoxazol-3-ylcarbonyl)-*N*-{(1*R*)-1-(4-chlorbenzyl)-2-[4-cyclohexyl-4-(*1H*-1,2,4-triazol-1-ylmethyl)piperidin-1-yl]-2-oxoethyl}piperidin-4-amin
1-[4-({(1*R*)-1-(4-chlorbenzyl)-2-[4-cyclohexyl-4-(*1H*-1,2,4-triazol-1-ylmethyl)piperidin-1-yl]-2-oxoethyl}amino)piperidin-1-yl]butan-2-on
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-1-(2,2,2-trifluorethyl)piperidin-4-amin
4-({(1*R*)-1-(4-Chlorbenzyl)-2-[4-cyclohexyl-4-(*1H-*1,2,4-triazol-1-ylmethyl)piperidin-1-yl]-2-oxo-ethyl}amino)-*N*-(4-methoxyphenyl)piperidin-1-carboxamid
4-({(1*R*)-1-(4-Chlorbenzyl)-2-[4-cyclohexyl-4-(*1H-*1,2,4-triazol-1-ylmethyl)piperidin-1-yl]-2-oxo-ethyl}amino)-*N*-(4-fluorphenyl)piperidin-1-carboxamid
4-({(1*R*)-1-(4-Chlorbenzyl)-2-[4-cyclohexyl-4-(*1H-*1,2,4-triazol-1-ylmethyl)piperidin-1-yl]-2-oxo-ethyl}amino)-*N*-(2,4-difluorphenyl)piperidin-1-carboxamid
4-({(1*R*)-1-(4-Chlorbenzyl)-2-[4-cyclohexyl-4-(*1H-*1,2,4-triazol-1-ylmethyl)piperidin-1-yl]-2-oxo-ethyl}amino)-*N*-(3,4-difluorphenyl)piperidin-1-carboxamid
4-({(1*R*)-1-(4-Chlorbenzyl)-2-[4-cyclohexyl-4-(*1H-*1,2,4-triazol-1-ylmethyl)piperidin-1-yl]-2-oxo-ethyl}amino)-*N*-(2-fluorphenyl)piperidin-1-carboxamid
4-({(1*R*)-1-(4-Chlorbenzyl)-2-[4-cyclohexyl-4-(*1H-*1,2,4-triazol-1-ylmethyl)piperidin-1-yl]-2-oxo-ethyl}amino)-*N*-(2-methoxyphenyl)piperidin-1-carboxamid
4-({(1*R*)-1-(4-Chlorbenzyl)-2-[4-cyclohexyl-4-(*1H-*1,2,4-triazol-1-ylmethyl)piperidin-1-yl]-2-oxo-ethyl}amino)-*N*-[4-(dimethylamino)phenyl}piperidin-1-carboxamid
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-1-[(5-fluor-1*H*-indol-2-yl)carbonyl]piperidin-4-amin
*N*-{(1*R*)-1-(4-Chlorbenzyl)-2-[4-cyclohexyl-4-(*1H-*1,2,4-triazol-1-ylmethyl)piperidin-1-yl]-2-oxo-ethyl}-1-(pyrazin-2-ylcarbonyl)piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-[(5-phenyl-1,3-oxazol-4-yl)carbonyl]piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-(isoxazol-5-ylcarbonyl)piperidin-4-amin
3-[*trans*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexyl]-6-fluor-1,3-benzoxazol-2(3*H*)-on
3-[*cis*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexyl]-6-fluor-1,3-benzoxazol-2(3*H*)-on
*N*-[*cis*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexyl]pyridin-2-carboxamid
2-{[*cis*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexyl]amino}-5-fluorphenyl oder
2-{[*trans*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexyl]amino}-5-fluorphenol
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-(chinolin-2-ylcarbonyl)]piperidin-4-amin
*N*-1(1*R*)-1-(4-Chlorbenzyl)-2-[4-cyclohexyl-4-(1*H-*1,2,4-triazol-1-ylmethyl)piperidin-1-yl]-2-oxo-ethyl}-1-[(3-methylpyridin-2-yl)carbonyl]-piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-(1*H*-1,2,4-triazol-3-ylcarbonyl)piperidin-4-amin
*N*-[*cis*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexyl]-2,1-benzisoxazol-3-carboxamid
1-(1,3-Benzothiazol-2-ylcarbonyl)-*N*-{(1*R*)-1-(4-chlorbenzyl)-2-[4-cyclohexyl-4-(1*H*-1,2,4-triazol-1-ylmethyl)piperidin-1-yl]-2-oxoethyl}piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-[(6-methylpyridin-2-yl)carbonyl]piperidin-4-amin
1-(1-Benzofuran-2-ylcarbonyl)-*N*-{(1*R*)-1-(4-chlorbenzyl)-2-[4-cyclohexyl-4-(1*H*-1,2,4-triazol-1-ylmethyl)piperidin-1-yl]-2-oxoethyl}piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-[(6-fluorpyridin-2-yl)carbonyl]piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-[(1-phenyl-1*H*-pyrazol-5-yl)carbonyl]-piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-(2,4-difluorbenzoyl)piperidin-4-amin *cis*-*N*-(1,3-Benzothiazol-2-ylmethyl)-*N*'-{(1*R*)-1-(4-chlorbenzyl)-2-[4-cyclohexyl-4-(1*H*-1,2,4-triazol-1-ylmethyl)piperidin-1-yl]-2-oxoethyl}cyclohexan-1,4-diamin
*cis*-*N*-{(1*R*)-1-(4-Chlorbenzyl)-2-[4-cyclohexyl-4-(1*H*-1,2,4-triazol-1-ylmethyl)piperidin-1-yl]-2-oxoethyl}-*N*'-(1,3-thiazol-2-ylmethyl)cyclohexan-1,4-diamin
2-{[*cis*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexyl]amino}-5-fluor-*N*,*N*-dimethylbenzamid
2-{[*trans*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexyl]amino}-5-fluor-*N*,*N*-dimethylbenzamid
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-[(6-phenylpyridin-2-yl)carbonyl]-piperidin-4-amin
*trans*-*N*-(tert.-Butyl)-4-({(1*R*)-1-(4-chlorbenzyl)-2-[4-cyclohexyl-4-(1*H*-1,2,4-triazol-1-ylmethyl)-piperidin-1-yl]-2-oxoethyl}amino)cyclohexan-carboxamid
*cis*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)-*N*-(3,4-difluorphenyl)cyclohexancarboxamid
*cis*-*N*-{(1*S*)-1-(4-Chlorbenzyl)-2-[4-cyclohexyl-4-(1*H*-1,2,4-triazol-1-ylmethyl)piperidin-1-yl]-2-oxoethyl}cyclohexan-1,4-diamin *trans*-*N*-{(1*S*)-1-(4-Chlorbenzyl)-2-[4-cyclohexyl-4-(1*H*-1,2,4-triazol-1-ylmethyl)piperidin-1-yl]-2-oxoethyl}cyclohexan-1,4-diamin.

24. Verbindungen mit den folgenden Namen:
4-[4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexyl]phenol
*cis*-*N*-{(1*R*)-1-(4-Chlorbenzyl)-2-[4-cyclohexyl-4-(1*H*-1,2,4-triazol-1-ylmethyl)piperidin-1-yl]-2-oxoethyl}cyclohexan-1,4-diamin
*trans*-*N*-{(1*R*)-1-(4-Chlorbenzyl)-2-[4-cyclohexyl-4-(1*H*-1,2,4-triazol-1-ylmethyl)piperidin-1-yl]-2-oxoethyl}cyclohexan-1,4-diamin
*cis*-*N*-{(1*R*)-1-(4-Chlorbenzyl)-2-[3-cyclohexyl-3-(1*H*-1,2,4-triazol-1-ylmethyl)-8-azabicyclo[3.2.1]oct-8-yl]-2-oxoethyl}cyclohexan-1,4-diamin
*trans*-*N*-{(1*R*)-1-(4-Chlorbenzyl)-2-[3-cyclohexyl-3-(1*H*-1,2,4-triazol-1-ylmethyl)-8-azabicyclo[3.2.1]oct-8-yl]-2-oxoethyl}cyclohexan-1,4-diamin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1,4-dioxaspiro[4.5]decan-8-amin
*N*'-{(1*R*)-1-(4-Chlorbenzyl)-2-[4-cyclohexyl-4-(1*H-*1,2,4-triazol-1-ylmethyl)piperidin-1-yl]-2-oxo-ethyl}-*N*,*N*-dimethylcyclohexan-1,4-diamin
4-(Aminomethyl)-*N*-{(1*R*)-1-(4-chlorbenzyl)-2-[4-cyclohexyl-4-(1*H*-1,2,4-triazol-1-ylmethyl)-piperidin-1-yl]-2-oxoethyl}cyclohexanamin
*cis*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexanol
*trans*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexanol
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-4-phenylcyclohexanamin
*cis*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)-1-phenylcyclohexanol
*trans*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)-1-phenylcyclohexanol
*cis*-4-({(1*R*)-1-(4-Chlorbenzyl)-2-[4-cyclohexyl-4-1*H*-1,2,4-triazol-1-ylmethyl)piperidin-1-yl]-2-oxoethyl}amino)-1-phenylcyclohexancarbonitril
*trans*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)-1-phenylcyclohexancarbonitril
*cis*-*N*-{(1*R*)-1-(4-Chlorbenzyl)-2-[4-cyclohexyl-4-(1*H*-1,2,4-triazol-1-ylmethyl)piperidin-1-yl]-2-oxoethyl}-*N'*-(4-fluorphenyl)cyclohexan-1,4-diamin *trans*-*N*-{(1*R*)-1-(4-Chlorbenzyl)-2-[4-cyclohexyl-4-(1*H*-1,2,4-triazol-1-ylmethyl)piperidin-1-yl]-2-oxoethyl}-*N'*-(4-fluorphenyl)cyclohexan-1,4-diamin
*N*-[*cis*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexyl]-2,2,2-trifluoracetamid
*N*-[*trans*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]-2,2,2-trifluor-acetamid
*N*-[*cis*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]acetamid
*N*-[*trans*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]acetamid
*cis*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-*N*'-methylcyclohexan-1,4-diamin
*N*-[*cis*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]-*N*-methylacetamid
N-{(1*R*)-1-(4-Chlorbenzyl)-2-[4-cyclohexyl-4-(*1H-*1,2,4-triazol-1-ylmethyl)piperidin-1-yl]-2-oxo-ethyl}-4-(1,3-dihydro-2H-isoindol-2-yl)cyclo-hexanamin
*N*-[*cis*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexyl]-*N*-methylbenzamid
*cis*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexancarbonsäureethylester
*trans*-4-({(1*R*)-1-(4-Chlorbenzyl)-2-[4-cyclohexyl-4-(*1H*-1,2,4-triazol-1-ylmethyl)piperidin-1-yl]-2-oxoethyl}amino)cyclohexancarbonsäureethylester
*cis*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-4-(trifluormethyl)cyclohexanamin
*trans*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-4-(trifluormethyl)cyclohexanamin
*trans*-*N*-{(1*R*)-1-(4-Chlorbenzyl)-2-[4-cyclohexyl-4-(*1H*-1,2,4-triazol-1-ylmethyl)piperidin-1-yl]-2-oxoethyl}-*N*'-phenylcyclohexan-1,4-diamin
*cis*-*N*-{(1*R*)-1-(4-Chlorbenzyl)-2-[4-cyclohexyl-4-(1*H*-1,2,4-triazol-1-ylmethyl)piperidin-1-yl]-2-oxoethyl}-*N*'-phenylcyclohexan-1,4-diamin
*N*-1(1*R*)-1-(4-Chlorbenzyl)-2-[4-cyclohexyl-4-(1*H-*1,2,4-triazol-1-ylmethyl)piperidin-1-yl]-2-oxo-ethyl}-4-(2,5-dimethyl-2,5-dihydro-1*H*-pyrrol-1-yl)cyclohexanamin
*N*-Benzyl-*N*'-{(1*R*)-1-(4-chlorbenzyl)-2-[4-cyclohexyl-4-(1*H-*1,2,4-triazol-1-ylmethyl)piperidin-1-yl]-2-oxoethyll-*N*-methylcyclohexan-1,4-diamin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-4-pyrrolidin-1-cyclohexanamin
2-{[4-({(1*R*)-1-(4-Chlorbenzyl)-2-[4-cyclohexyl-4-(1*H-*1,2,4-triazol-1-ylmethyl)piperidin-1-yl]-2-oxoethyl}amino)cyclohexyl]amino}ethanol
2-{Benzyl[4-({(1*R*)-1-(4-Chlorbenzyl)-2-[4-cyclohexyl-4-(1*H-*1,2,4-triazol-1-ylmethyl)piperidin-1-yl]-2-oxoethyl}amino)cyclohexyl]amino}ethanol
*N*'-{(1*R*)-1-(4-Chlorbenzyl)-2-[4-cyclohexyl-4-(1*H-*1,2,4-triazol-1-ylmethyl)piperidin-1-yl]-2-oxo-ethyl}-*N*-methyl-*N*-phenylcyclohexan-1,4-diamin *N*'-{(1*R*)-1-(4-Chlorbenzyl)-2-[4-cyclohexyl-4-(1*H-*1,2,4-triazol-1-ylmethyl)piperidin-1-yl]-2-oxo-ethyl}-*N*-(4-fluorphenyl)-*N*-methylcyclohexan-1,4-diamin
*cis- oder trans*-4-({(1*R*)-1-(4-Chlorbenzyl)-2-[4-cyclohexyl-4-(1*H-*1,2,4-triazol-1-ylmethyl)-piperidin-1-yl]-2-oxoethyl}amino)cyclohexancarbonsäure
*cis- oder trans*-4-({(1*R*)-1-(4-Chlorbenzyl)-2-[4-cyclohexyl-4-(1*H*-1,2,4-triazol-1-ylmethyl)-piperidin-1-yl]-2-oxoethyl}amino)cyclohexancarbonsäure
*cis- oder trans*-4-({(1*R*)-1-(4-Chlorbenzyl)-2-[4-cyclohexyl-4-(1*H*-1,2,4-triazol-1-ylmethyl)-piperidin-1-yl]-2-oxoethyl}amino)-*N*,*N*-diethyl-cyclohexancarboxamid
*cis- oder trans*-4-({(1*R*)-1-(4-Chlorbenzyl)-2-[4-cyclohexyl-4-(1*H*-1,2,4-triazol-1-ylmethyl)-piperidin-1-yl]-2-oxoethyl}amino)-*N*,*N*-diethyl-cyclohexancarboxamid
*cis- oder trans*-4-({(1*R*)-1-(4-Chlorbenzyl)-2-[4-cyclohexyl-4-(1*H*-1,2,4-triazol-1-ylmethyl)-piperidin-1-yl]-2-oxoethyl}amino)-*N*,*N*-dimethyl-cyclohexancarboxamid
*cis- oder trans*-4-({(1*R*)-1-(4-Chlorbenzyl)-2-[4-cyclohexyl-4-(1*H-*1,2,4-triazol-1-ylmethyl)-piperidin-1-yl]-2-oxoethyl}amino)-*N*,*N*-dimethyl-cyclohexancarboxamid.

25. Verbindungen mit den folgenden Namen:
*cis*-*N*-Benzyl-4-({(1*R*)-1-(4-chlorbenzyl)-2-[4-cyclohexyl-4-(1*H*-1,2,4-triazol-1-ylmethyl)-piperidin-1-yl]-2-oxoethyl}amino)-*N*-methylcyclo-hexancarboxamid
*trans*-*N*-Benzyl-4-({(1*R*)-1-(4-chlorbenzyl)-2-[4-cyclohexyl-4-(1*H*-1,2,4-triazol-1-ylmethyl)-piperidin-1-yl]-2-oxoethyl}amino)-*N*-methylcyclo-hexancarboxamid
*cis*-*N*-{(1*R*)-1-(4-Chlorbenzyl)-2-[4-cyclohexyl-4-(1*H*-1,2,4-triazol-1-ylmethyl)piperidin-1-yl]-2-oxoethyl}-4-(3-methyl-1,2,4-oxadiaziol-5-yl)cyclo-hexanamin
*trans*-*N*-{(1*R*)-1-(4-Chlorbenzyl)-2-[4-cyclohexyl-4-(1*H-*1,2,4-triazol-1-ylmethyl)piperidin-1-yl]-2-oxoethyl}-4-(3-methyl-1,2,4-oxadiaziol-5-yl)cyclo-hexanamin
*cis*-4-({(1*R*)-1-(4-Chlorbenzyl)-2-[4-cyclohexyl-4-(1*H-*1,2,4-triazol-1-ylmethyl)piperidin-1-yl]-2-oxoethyl}amino)cyclohexancarboxamid
*trans*-4-({(1*R*)-1-(4-Chlorbenzyl)-2-[4-cyclohexyl-4-(1*H-*1,2,4-triazol-1-ylmethyl)piperidin-1-yl]-2-oxoethyl}amino)cyclohexancarboxamid *cis*-4-({(1*R*)-1-(4-Chlorbenzyl)-2-[4-cyclohexyl-4-(1*H-*1,2,4-triazol-1-ylmethyl)piperidin-1-yl]-2-oxoethyl}amino)-1-(4-fluorphenyl)cyclohexanol
*trans*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)-1-(4-fluorphenyl)cyclohexanol
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-4-methoxycyclohexanamin
4-[4-(Benzyloxy)phenyl]-*N*-{(1*R*)-1-(4-chlorbenzyl)-2-[4-cyclohexyl-4-(1*H*-1,2,4-triazol-1-ylmethyl)-piperidin-1-yl]-2-oxoethyl}cyclohexanamin
4-(Benzyloxy)-*N*-{(1*R*)-1-(4-chlorbenzyl)-2-[4-cyclohexyl-4-(1*H*-1,2,4-triazol-1-ylmethyl)-piperidin-1-yl]-2-oxoethyl}cyclohexanamin
*N*-[*cis*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexyl]-2-methoxyacetamid
2-(Benzyloxy)-*N*-[*cis*-4-({(1*R*)-1-(4-chlorbenzyl)-2-[4-cyclohexyl-4-(1*H-*1,2,4-triazol-1-ylmethyl)-piperidin-1-yl]-2-oxoethyl}amino)cyclohexyl]-acetamid
3-[*cis*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexyl]-1,3-oxazolidin-2-on
3-[*trans*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexyl]-1,3-oxazolidin-2-on
*cis*-*N*-{(1*R*)-1-(4-Chlorbenzyl)-2-[4-cyclohexyl-4-(1*H-*1,2,4-triazol-1-ylmethyl)piperidin-1-yl]-2-oxoethyl}-*N*'-(2-methoxyethyl)cyclohexan-1,4-diamin
*trans*-*N*-{(1*R*)-1-(4-Chlorbenzyl)-2-[4-cyclohexyl-4-(1*H-*1,2,4-triazol-1-ylmethyl)piperidin-1-yl]-2-oxoethyl}-*N*'-(2-methoxyethyl)cyclohexan-1,4-diamin
*cis*-*N*-{(1*R*)-1-(4-Chlorbenzyl)-2-[4-cyclohexyl-4-(1*H-*1,2,4-triazol-1-ylmethyl)piperidin-1-yl]-2-oxoethyl}-4-morpholin-4-ylcyclohexanamin *trans*-*N*-{(1*R*)-1-(4-Chlorbenzyl)-2-[4-cyclohexyl-4-(1*H-*1,2,4-triazol-1-ylmethyl)piperidin-1-yl]-2-oxoethyl}-4-morpholin-4-ylcyclohexanamin
1-[*cis*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexyl]pyrrolidin-2-on
1-[*trans*-4-({(1*R*)-1-(4-Chlorbenzyl)-2-[4-cyclohexyl-4-(1*H-*1,2,4-triazol-1-ylmethyl)piperidin-1-yl]-2-oxoethyl}amino)cyclohexyl]pyrrolidin-2-on
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-4-(2-methoxyethoxy)cyclohexanamin
*cis*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexancarbonitril
*trans*-4-({(1*R*)-1-(4-Chlorbenzyl)-2-[4-cyclohexyl-4-(1*H-*1,2,4-triazol-1-ylmethyl)piperidin-1-yl]-2-oxoethyl}amino)cyclohexancarbonitril
1-[*cis*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexyl]piperidin-2-on
1-[*trans*-4-({(1*R*)-1-(4-Chlorbenzyl)-2-[4-cyclohexyl-4-(1*H-*1,2,4-triazol-1-ylmethyl)piperidin-1-yl]-2-oxoethyl}amino)cyclohexyl]piperidin-2-on
*N*-[4-({(1*R*)-1-(4-Chlorbenzyl)-2-[4-cyclohexyl-4-(1*H-*1,2,4-triazol-1-ylmethyl)piperidin-1-yl]-2-oxoethyl}amino)cyclohexyl]propanamid
*N*-[*cis*-4-({(1*R*)-1-(4-Chlorbenzyl)-2-[4-cyclohexyl-4-(1*H-*1,2,4-triazol-1-ylmethyl)piperidin-1-yl]-2-oxoethyl}amino)cyclohexyl]glycinamid
*N*-[*cis*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexyl]-2-hydroxyacetamid
*N*-[*cis*-4-({(1*R*)-1-(4-Chlorbenzyl)-2-[4-cyclohexyl-4-(1*H-*1,2,4-triazol-1-ylmethyl)piperidin-1-yl]-2-oxoethyl}amino)cyclohexyl]-2,2-dimethylpropanamid
*N*-[*trans*-4-({(1*R*)-1-(4-Chlorbenzyl)-2-[4-cyclohexyl-4-(1*H-*1,2,4-triazol-1-ylmethyl)piperidin-1-yl]-2-oxoethyl}amino)cyclohexyl]-2,2-dimethyl-propanamid
*cis*-*N*-{(1*R*)-1-(4-Chlorbenzyl)-2-[4-cyclohexyl-4-(1*H-*1,2,4-triazol-1-ylmethyl)piperidin-1-yl]-2-oxoethyl}-*N*'-(4-methoxyphenyl)cyclohexan-1,4-diamin
*trans*-*N*-{(1*R*)-1-(4-Chlorbenzyl)-2-[4-cyclohexyl-4-(1*H*-1,2,4-triazol-1-ylmethyl)piperidin-1-yl]-2-oxoethyl}-*N'*-(4-methoxyphenyl)cyclohexan-1,4-diamin
*cis*-*N*-{(1*R*)-1-(4-Chlorbenzyl)-2-[4-cyclohexyl-4-(1*H-*1,2,4-triazol-1-ylmethyl)piperidin-1-yl]-2-oxoethyl}-4-(2*H-*tetrazol-5-yl)cyclohexanamin
*trans*-*N*-{(1*R*)-1-(4-Chlorbenzyl)-2-[4-cyclohexyl-4-(1*H-*1,2,4-triazol-1-ylmethyl)piperidin-1-yl]-2-oxoethyl}-4-(2*H-*tetrazol-5-yl)cyclohexanamin
2-{[*cis*-4-({(1*R*)-1-(4-Chlorbenzyl)-2-[4-cyclohexyl-4-(1*H-*1,2,4-triazol-1-ylmethyl)piperidin-1-yl]-2-oxoethyl}amino)cyclohexyl]amino}phenol
2-{[*trans*-4-({(1*R*)-1-(4-Chlorbenzyl)-2-[4-cyclohexyl-4-(1*H-*1,2,4-triazol-1-ylmethyl)piperidin-1-yl]-2-oxoethyl}amino)cyclohexyl]amino}phenol
Essigsäure-4-({(1*R*)-1-(4-chlorbenzyl)-2-[4-cyclohexyl-4-(1*H-*1,2,4-triazol-1-ylmethyl)piperidin-1-yl]-2-oxoethyl}amino)cyclohexylester
H²-[*cis*-4-({(1*R*)-1-(4-Chlorbenzyl)-2-[4-cyclohexyl-4-(1*H-*1,2,4-triazol-1-ylmethyl)piperidin-1-yl]-2-oxoethyl}amino)cyclohexyl]-*N*,*N*-dimethylglycinamid
*N*²-[*trans*-4-({(1*R*)-1-(4-Chlorbenzyl)-2-[4-cyclohexyl-4-(1*H-*1,2,4-triazol-1-ylmethyl)piperidin-1-yl]-2-oxoethyl}amino)cyclohexyl]-*N*,*N*-dimethyl-glycinamid
*H*²-[*cis*-4-({(1*R*)-1-(4-Chlorbenzyl)-2-[4-cyclohexyl-4-(1*H-*1,2,4-triazol-1-ylmethyl)piperidin-1-yl]-2-oxoethyl}amino)cyclohexyl]glycinamid
*N*²-[*trans*-4-({(1*R*)-1-(4-Chlorbenzyl)-2-[4-cyclohexyl-4-(1*H-*1,2,4-triazol-1-ylmethyl)piperidin-1-yl]-2-oxoethyl}amino)cyclohexyl]glycinamid
[*cis*-4-({(1*R*)-1-(4-Chlorbenzyl)-2-[4-cyclohexyl-4-(1*H-*1,2,4-triazol-1-ylmethyl)piperidin-1-yl]-2-oxoethyl}amino)cyclohexyl]piperazin-2-on
4-[*trans*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexyl]piperazin-2-on
*cis*-4-(Acetylpiperazin-1-yl)-*N*-{(1*R*)-1-(4-chlorbenzyl)-2-[4-cyclohexyl-4-(1*H-*1,2,4-triazol-1-ylmethyl)piperidin-1-yl]-2-oxoethyl}cyclohexanamin
*trans*-4-(Acetylpiperazin-1-yl)-*N*-{(1*R*)-1-(4-chlorbenzyl)-2-[4-cyclohexyl-4-(1*H-*1,2,4-triazol-1-ylmethyl)piperidin-1-yl]-2-oxoethyl}cyclohexanamin
*cis*-*N*-{(1*R*)-1-(4-Chlorbenzyl)-2-[4-cyclohexyl-4-(1*H-*1,2,4-triazol-1-ylmethyl)piperidin-1-yl]-2-oxoethyl}-*N'*-(4-chlorphenyl)cyclohexan-1,4-diamin
*trans*-*N*-{(1*R*)-1-(4-Chlorbenzyl)-2-[4-cyclohexyl-4-(1*H-*1,2,4-triazol-1-ylmethyl)piperidin-1-yl]-2-oxoethyl}-*N'*-(4-chlorphenyl)cyclohexan-1,4-diamin
Pivalinsäure-4-({(1*R*)-1-(4-Chlorbenzyl)-2-[4-cyclohexyl-4-(1*H*-1,2,4-triazol-1-ylmethyl)-piperidin-1-yl]-2-oxoethyl}amino)cyclohexylester
*cis*-*N*-{(1*R*)-1-(4-Chlorbenzyl)-2-[4-cyclohexyl-4-(1*H-*1,2,4-triazol-1-ylmethyl)piperidin-1-yl]-2-oxoethyl}-*N'*-(4-fluor-2-methylphenyl)cyclohexan-1,4-diamin
*trans*-*N*-{(1*R*)-1-(4-Chlorbenzyl)-2-[4-cyclohexyl-4-(1*H-*1,2,4-triazol-1-ylmethyl)piperidin-1-yl]-2-oxoethyl}-*N'*-(4-fluor-2-methylphenyl)cyclohexan-1,4-diamin
4-{[4-({(1*R*)-1-(4-Chlorbenzyl)-2-[4-cyclohexyl-4-(1*H-*1,2,4-triazol-1-ylmethyl)piperidin-1-yl]-2-oxoethyl}amino)cyclohexyl]amino}benzonitril
*N*-[4-({(1*R*)-1-(4-Chlorbenzyl)-2-[4-cyclohexyl-4-(1*H-*1,2,4-triazol-1-ylmethyl)piperidin-1-yl]-2-oxoethyl}amino)cyclohexyl]cyclopropancarboxamid *cis*-*N*-{(1*R*)-1-(4-Chlorbenzyl)-2-[4-cyclohexyl-4-(1*H-*1,2,4-triazol-1-ylmethyl)piperidin-1-yl]-2-oxoethyl}-*N'*-(2,4-difluorphenyl)cyclohexan-1,4-diamin
*trans*-*N*-{(1*R*)-1-(4-Chlorbenzyl)-2-[4-cyclohexyl-4-(1*H*-1,2,4-triazol-1-ylmethyl)piperidin-1-yl]-2-oxoethyl}-*N'*-(2,4-difluorphenyl)cyclohexan-1,4-diamin
*cis*-*N*-{(1*R*)-1-(4-Chlorbenzyl)-2-[4-cyclohexyl-4-(1*H-*1,2,4-triazol-1-ylmethyl)piperidin-1-yl]-2-oxoethyl}-*N'*-(5-fluorpyridin-2-yl)cyclohexan-1,4-diamin
*trans*-*N*-{(1*R*)-1-(4-Chlorbenzyl)-2-[4-cyclohexyl-4-(1*H-*1,2,4-triazol-1-ylmethyl)piperidin-1-yl]-2-oxoethyl}-*N'*-(5-fluorpyridin-2-yl)cyclohexan-1,4-diamin
*N*-1*H-*1,2,3-Benzotriazol-5-yl-*N'*-{(1*R*)-1-(4-chlorbenzyl)-2-[4-cyclohexyl-4-(1*H-*1,2,4-triazol-1-ylmethyl)piperidin-1-yl]-2-oxoethyl}cyclohexan-1,4-diamin
1-[*cis*-4-({(1*R*)-1-(4-Chlorbenzyl)-2-[4-cyclohexyl-4-(1*H-*1,2,4-triazol-1-ylmethyl)piperidin-1-yl]-2-oxoethyl}amino)cyclohexyl]imidazolidin-2-on
1-[*trans*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexyl]imidazolidin-2-on *cis*-*N*-{(1*R*)-1-(4-Chlorbenzyl)-2-[4-cyclohexyl-4-(1*H-*1,2,4-triazol-1-ylmethyl)piperidin-1-yl]-2-oxoethyl}-*N'*-(3,4-difluorphenyl)cyclohexan-1,4-diamin
*trans*-*N*-{(1*R*)-1-(4-Chlorbenzyl)-2-[4-cyclohexyl-4-(1*H-*1,2,4-triazol-1-ylmethyl)piperidin-1-yl]-2-oxoethyl}-*N'*-(3,4-difluorphenyl)cyclohexan-1,4-diamin
*cis*-*N*-{(1*R*)-1-(4-Chlorbenzyl)-2-[4-cyclohexyl-4-(1*H-*1,2,4-triazol-1-ylmethyl)piperidin-1-yl]-2-oxoethyl}-*N'*-(4-fluor-3-methoxyphenyl)cyclohexan-1,4-diamin
*trans*-*N*-{(1*R*)-1-(4-Chlorbenzyl)-2-[4-cyclohexyl-4-(1*H-*1,2,4-triazol-1-ylmethyl)piperidin-1-yl]-2-oxoethyl}-*N'*-(4-fluor-3-methoxyphenyl)cyclohexan-1,4-diamin
*cis*-*N*'-{(1*R*)-1-(4-Chlorbenzyl)-2-[4-cyclohexyl-4-(1*H-*1,2,4-triazol-1-ylmethyl)piperidin-1-yl]-2-oxoethyl}-*N*-ethyl-*N*-(4-fluor-3-methoxyphenyl)-cyclohexan-1,4-diamin
*trans*-*N*'-{(1*R*)-1-(4-Chlorbenzyl)-2-[4-cyclohexyl-4-(1*H-*1,2,4-triazol-1-ylmethyl)piperidin-1-yl]-2-oxoethyl}-*N*-ethyl-*N*-(4-fluor-3-methoxyphenyl)-cyclohexan-1,4-diamin
*cis*-*N*-{(1*R*)-1-(4-Chlorbenzyl)-2-[4-cyclohexyl-4-(1*H*-1,2,4-triazol-1-ylmethyl)piperidin-1-yl]-2-oxoethyl}-*N*'-[4-(trifluormethyl)phenyl]cyclohexan-1,4-diamin
*trans*-*N*-{(1*R*)-1-(4-Chlorbenzyl)-2-[4-cyclohexyl-4-(1*H*-1,2,4-triazol-1-ylmethyl)piperidin-1-yl]-2-oxoethyl}-*N*'-[4-(trifluormethyl)phenyl]cyclohexan-1,4-diamin
*cis*-*N*-{(1*R*)-1-(4-Chlorbenzyl)-2-[4-cyclohexyl-4-(1*H*-1,2,4-triazol-1-ylmethyl)piperidin-1-yl]-2-oxoethyl}-*N*'-[4-fluor-3-methylphenyl)cyclohexan-1,4-diamin
*trans*-*N*-{(1*R*)-1-(4-Chlorbenzyl)-2-[4-cyclohexyl-4-(1*H*-1,2,4-triazol-1-ylmethyl)piperidin-1-yl]-2-oxoethyl}-*N*'-[4-fluor-3-methylphenyl)cyclohexan-1,4-diamin
3-[*trans*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexyl]-6-fluor-1,3-benzoxazol-2-(3*H*)-on
3-[*cis*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexyl]-6-fluor-1,3-benzoxazol-2-(3*H*)-on
*N*-[*cis*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexyl]pyridin-2-carboxamid
2-{[*cis*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexyl]amino}-5-fluorphenol oder
2-{[*trans*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexyl]amino}-5-fluorphenol
*N*-[*cis*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexyl]-2,1-benzisoxazol-3-carboxamid
*cis*-*N*-(1,3-Benzothiozol-2-ylmethyl)-*N*'-{(1*R*)-1-(4-chlorbenzyl)-2-[4-cyclohexyl-4-(1*H*-1,2,4-triazol-1-ylmethyl)piperidin-1-yl]-2-oxoethyl}cyclohexan-1,4-diamin
*cis*-*N*-{(1*R*)-1-(4-Chlorbenzyl)-2-[4-cyclohexyl-4-(1*H*-1,2,4-triazol-1-ylmethyl)piperidin-1-yl]-2-oxoethyl}-*N*'-(1,3-thiazol-2-ylmethyl)cyclohexan-1,4-diamin
2-{[*cis*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexyl]amino}-5-fluor-*N*,*N*-dimethylbenzamid
2-{[*trans*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexyl]amino}-5-fluor-*N*,*N*-dimethylbenzamid
*trans*-*N*-(*tert*.-Butyl)-4-({(1*R*)-1-(4-chlorbenzyl)-2-[4-cyclohexyl-4-(1*H*-1,2,4-triazol-1-ylmethyl)-piperidin-1-yl]-2-oxoethyl}amino)cyclohexan-carboxamid
*cis*-4-({(1*R*)-1-(4-Chlorbenzyl)-2-[4-cyclohexyl-4-(1*H*-1,2,4-triazol-1-ylmethyl)piperidin-1-yl]-2-oxoethyl}amino)-*N*-(3,4-difluorphenyl)cyclohexan-carboxamid
*cis*-*N*-({(1*S*)-1-(4-Chlorbenzyl)-2-[4-cyclohexyl-4-(1*H*-1,2,4-triazol-1-ylmethyl)piperidin-1-yl]-2-oxoethyl}cyclohexan-1,4-diamin
*trans*-*N*-({(1*S*)-1-(4-Chlorbenzyl)-2-[4-cyclohexyl-4-(1*H*-1,2,4-triazol-1-ylmethyl)piperidin-1-yl]-2-oxoethyl}cyclohexan-1,4-diamin.

26. Verbindungen mit den folgenden Namen:
Essigsäure-2-{[*cis*-4-({(1*R*)-1-(4-Chlorbenzyl)-2-[4-cyclohexyl-4-(1*H*-1,2,4-triazol-1-ylmethyl)-piperidin-1-yl]-2-oxoethyl}amino)cyclohexyl]-amino}-2-oxoethylester
:(2-{[*cis*-4-({(1*R*)-1-(4-Chlorbenzyl)-2-[4-cyclohexyl-4-(1*H-*1,2,4-triazol-1-ylmethyl)piperidin-1-yl]-2-oxoethyl}amino)cyclohexyl]amino}-2-oxoethyl)carbamidsäure-tert.-butylester
*cis-* oder *trans*-*N*-({(1*R*)-1-(4-Chlorbenzyl)-2-[4-cyclohexyl-4-(1*H-*1,2,4-triazol-1-ylmethyl)-piperidin-1-yl]-2-oxoethyl}-*N'*-(4-morpholin-4-ylphenyl)cyclohexan-1,4-diamin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-4,4-difluorcyclohexanamin.

27. Verbindungen mit den folgenden Namen:
1-Benzyl-*N*-{(1*R*)-1-(4-chlorbenzyl)-2-[4-cyclohexyl-4-(1*H-*1,2,4-triazol-1-ylmethyl)piperidin-1-yl]-2-oxoethyl}piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-(2-phenylethyl)piperidin-4-amin
2-[4-({(1*R*)-1-(4-Chlorbenzyl)-2-[4-cyclohexyl-4-(1*H-*1,2,4-triazol-1-ylmethyl)piperidin-1-yl]-2-oxoethyl}amino)piperidin-1-yl]ethanol
3-[4-({(1*R*)-1-(4-Chlorbenzyl)-2-[4-cyclohexyl-4-(1*H-*1,2,4-triazol-1-ylmethyl)piperidin-1-yl]-2-oxoethyl}amino)piperidin-1-yl]propan-1-ol 4-[4-({(1*R*)-1-(4-Chlorbenzyl)-2-[4-cyclohexyl-4-(1*H-*1,2,4-triazol-1-ylmethyl)piperidin-1-yl]-2-oxoethyl}amino)piperidin-1-yl]butan-1-ol
4-({(1*R*)-1-(4-Chlorbenzyl)-2-[4-cyclohexyl-4-(1*H-*1,2,4-triazol-1-ylmethyl)piperidin-1-yl]-2-oxo-ethyl}amino)piperidin-1-carbonsäure-tert.-butylester
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-8-azabicyclo[3.2.1]octan-3-amin *N*-1(1*R*)-1-(4-Chlorbenzyl)-2-[4-cyclohexy1-4-(1*H-*1,2,4-triazol-1-ylmethyl)piperidin-1-yl]-2-oxo-ethyl}-8-methyl-8-azabicyclo[3.2.1]octan-3-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-9-methyl-9-azabicyclo[3.2.1]nonan-3-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}chinolidin-3-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}azepan-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}piperidin-3-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-phenylpiperidin-4-amin
*N*-{(1*R*)-1-(4-Chlorbenzyl)-2-[3-cyclohexyl-3-(1*H-*1,2,4-triazol-1-ylmethyl)-8-azabicyclo[3.2.1]oct-8-yl]-2-oxoethyl}piperidin-4-amin
1-Benzyl-*N*-{(1*R*)-1-(4-chlorbenzyl)-2-[4-cyclohexyl-4-(1*H*-1,2,4-triazol-1-ylmethyl)piperidin-1-yl]-2-oxoethyl}pyrrolidin-3-amin
1-Benzoyl-*N*-{(1*R*)-1-(4-chlorbenzyl)-2-[4-cyclohexyl-4-(1*H*-1,2,4-triazol-1-ylmethyl)piperidin-1-yl]-2-oxoethyl}piperidin-4-amin
1-Acetyl-*N*-{(1*R*)-1-(4-chlorbenzyl)-2-[4-cyclohexyl-4-(1*H*-1,2,4-triazol-1-ylmethyl)piperidin-1-yl]-2-oxoethyl}piperidin-4-amin
3-({(1*R*)-1-(4-Chlorbenzyl)-2-[4-cyclohexyl-4-(1*H-*1,2,4-triazol-1-ylmethyl)piperidin-1-yl]-2-oxo-ethyl}amino)-8-methyl-8-azabicyclo[3.2.1]octan-6-ol
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-(trifluoracetyl)piperidin-4-amin
4-({(1*R*)-1-(4-Chlorbenzyl)-2-[4-cyclohexyl-4-(1*H-*1,2,4-triazol-1-ylmethyl)piperidin-1-yl]-2-oxo-ethyl}amino)piperidin-1-carboxamid
*N*-{(1R)-1-(4-Chlorbenzyl)-2-[4-cyclohexyl-4-(1*H-*1,2,4-triazol-1-ylmethyl)piperidin-1-yl]-2-oxo-ethyl}-1-(4-fluorbenzoyl)piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-(cyclopentylcarbonyl)piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-(cyclobutylcarbonyl)piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-[(4-methylphenyl)sulfonyl]piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-(pyridin-2-ylcarbonyl)piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-(phenylacetyl)piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-(methylsulfonyl)piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-2-phenylpiperidin-4-amin
(1*S*, 3*R*, 5*S*, 7*S*)-4-({(1*R*)-1-(4-Chlorbenzyl)-2-[4-cyclohexyl-4-(1*H-*1,2,4-triazol-1-ylmethyl)-piperidin-1-yl]-2-oxoethyl}amino)adamantan-1-ol.

28. Verbindungen mit den foglenden Bezeichnungen:
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-isonicotinoylpiperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-[(1-methyl-1*H-*imidazol-2-yl)carbonyl]-piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-[(5-methylisoxazol-3-yl)carbonyl]-piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-(3,4-difluorbenzoyl)piperidin-4-amin
1-[(1-tert.-Butyl-5-methyl-1*H-*pyrazol-3-yl)-carbonyl]-*N*-{(1*R*)-1-(4-chlorbenzyl)-2-[4-cyclohexyl-4-(1*H-*1,2,4-triazol-1-ylmethyl)piperidin-1-yl]-2-oxoethyl}piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-[(3,5-dimethylisoxazol-4-yl)carbonyl]-piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-(3-thienylcarbonyl)piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-(1-pyrrolidin-1-ylcarbonyl)piperidin-4-amin
4-({(1*R*)-1-(4-Chlorbenzyl)-2-[4-cyclohexyl-4-(1*H-*1,2,4-triazol-1-ylmethyl)piperidin-1-yl]-2-oxo-ethyl}amino)-*N*-phenylpiperidin-1-carboxamid
4-({(1*R*)-1-(4-Chlorbenzyl)-2-[4-cyclohexyl-4-(1*H-*1,2,4-triazol-1-ylmethyl)piperidin-1-yl]-2-oxo-ethyl}amino)-*N,N*-dimethylpiperidin-1-carboxamid
4-({(1*R*)-1-(4-Chlorbenzyl)-2-[4-cyclohexyl-4-(1*H-*1,2,4-triazol-1-ylmethyl)piperidin-1-yl]-2-oxo-ethyl}amino)-*N,N*-diethylpiperidin-1-carboxamid
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-(piperidin-1-ylcarbonyl)piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-(morpholin-4-ylcarbonyl)piperidin-4-amin
4-({(1*R*)-1-(4-Chlorbenzyl)-2-[4-cyclohexyl-4-(1*H-*1,2,4-triazol-1-ylmethyl)piperidin-1-yl]-2-oxo-ethyl}amino)-*N*-methyl-*N*-phenylpiperidin-1-carboxamid
*N*-Benzyl-4-({(1*R*)-1-(4-chlorbenzyl)-2-[4-cyclohexyl-4-(1*H-*1,2,4-triazol-1-ylmethyl)piperidin-1-yl]-2-oxoethyl}amino)-*N*-methylpiperidin-1-carboxamid
4-({(1*R*)-1-(4-Chlorbenzyl)-2-[4-cyclohexyl-4-(1*H-*1,2,4-triazol-1-ylmethyl)piperidin-1-yl]-2-oxo-ethyl}amino)piperidin-1-carbonsäureethylester
4-({(1*R*)-1-(4-Chlorbenzyl)-2-[4-cyclohexyl-4-(1*H-*1,2,4-triazol-1-ylmethyl)piperidin-1-yl]-2-oxo-ethyl}amino)piperidin-1-carbonsäuremethylester
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-[(2*S*)piperidin-2-ylcarbonyl]piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-[(2*R*)piperidin-2-ylcarbonyl]piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-pyridin-2-ylpiperidin-4-amin
*N*-[4-({(1*R*)-1-(4-Chlorbenzyl)-2-[4-cyclohexyl-4-(1*H*-1,2,4-triazol-1-ylmethyl)piperidin-1-yl]-2-oxoethyl}amino)cyclohexyl]glycinmethylester *N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-(2,2-difluorethyl)piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-(6-methylpyridazin-3-yl)piperidin-4-amin
[4-({(1*R*)-1-(4-Chlorbenzyl)-2-[4-cyclohexyl-4-(1*H-*1,2,4-triazol-1-ylmethyl)piperidin-1-yl]-2-oxo-ethyl}amino)piperidin-1-yl]essigsäuremethylester
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-pyrimidin-2-ylpiperidin-4-amin *N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-cyclopropylpiperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-{[(2*S*)-4,4-difluorpiperidin-2-yl]carbonyl}piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-(4,4-difluor-L-prolyl)piperidin-4-amin
1-(1*H-*Benzimidazol-2-yl)-*N*-{(1*R*)-1-(4-chlorbenzyl)-2-[4-cyclohexyl-4-(1*H*-1,2,4-triazol-1-ylmethyl)piperidin-1-yl]-2-oxoethyl}piperidin-4-amin
1-(2,1-Benzisoxazol-3-ylcarbonyl)-*N*-{(1*R*)-1-(4-chlorbenzyl)-2-[4-cyclohexyl-4-(1*H-*1,2,4-triazol-1-ylmethyl)piperidin-1-yl]-2-oxoethyl}piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-(2,2,2-trifluorethyl)piperidin-4-amin
4-({(1*R*)-1-(4-Chlorbenzyl)-2-[4-cyclohexyl-4-(1*H-*1,2,4-triazol-1-ylmethyl)piperidin-1-yl]-2-oxo-ethyl}amino)-*N*-(4-methoxyphenyl)piperidin-1-carboxamid
4-({(1*R*)-1-(4-Chlorbenzyl)-2-[4-cyclohexyl-4-(1*H-*1,2,4-triazol-1-ylmethyl)piperidin-1-yl]-2-oxo-ethyl}amino)-*N*-(4-fluorphenyl)piperidin-1-carboxamid
4-({(1*R*)-1-(4-Chlorbenzyl)-2-[4-cyclohexyl-4-(1*H-*1,2,4-triazol-1-ylmethyl)piperidin-1-yl]-2-oxo-ethyl}amino)-*N*-(2,4-difluorphenyl)piperidin-1-carboxamid
4-({(1*R*)-1-(4-Chlorbenzyl)-2-[4-cyclohexyl-4-(1*H-*1,2,4-triazol-1-ylmethyl)piperidin-1-yl]-2-oxo-ethyl}amino)-*N*-(3,4-difluorphenyl)piperidin-1-carboxamid
4-({(1*R*)-1-(4-Chlorbenzyl)-2-[4-cyclohexyl-4-(1*H-*1,2,4-triazol-1-ylmethyl)piperidin-1-yl]-2-oxo-ethyl}amino)-*N*-(2-fluorphenyl)piperidin-1-carboxamid
4-({(1*R*)-1-(4-Chlorbenzyl)-2-[4-cyclohexyl-4-(1*H-*1,2,4-triazol-1-ylmethyl)piperidin-1-yl]-2-oxo-ethyl}amino)-*N*-(2-methoxyphenyl)piperidin-1-carboxamid
4-({(1*R*)-1-(4-Chlorbenzyl)-2-[4-cyclohexyl-4-(1*H-*1,2,4-triazol-1-ylmethyl)piperidin-1-yl]-2-oxo-ethyl}amino)-*N*-[4-(dimethylamino)phenyl]piperidin-1-carboxamid
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-[(5-fluor-1*H*-indol-2-yl)carbonyl)-piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-(pyrazin-2-ylcarbonyl)piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-(isoxazol-5-ylcarbonyl)piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-(chinolin-2-ylcarbonyl)piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-[(3-methylpyridin-2-yl)carbonyl]-piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-(1*H-*1,2,4-triazol-3-ylcarbonyl)piperidin-4-amin
: 1-(1,3-Benzothiazol-2-ylcarbonyl)-*N*-{(1*R*)-1-(4-chlorbenzyl)-2-[4-cyclohexyl-4-(1*H-*1,2,4-triazol-1-ylmethyl)piperidin-1-yl]-2-oxoethyl}piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-[(6-methylpyridin-2-yl)carbonyl]-piperidin-4-amin
1-(1-Benzofuran-2-ylcarbonyl)-*N*-{(1*R*)-1-(4-chlorbenzyl)-2-[4-cyclohexyl-4-(1*H-*1,2,4-triazol-1-ylmethyl)piperidin-1-yl]-2-oxoethyl}piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-[(6-fluorpyridin-2-yl)carbonyl]piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-(2,4-difluorbenzoyl)piperidin-4-amin.

29. Verbindungen mit den folgenden Namen:
1-[4-({(1*R*)-1-(4-Chlorbenzyl)-2-[4-cyclohexyl-4-(1*H*-1,2,4-triazol-1-ylmethyl)piperidin-1-yl]-2-oxoethyl}amino)piperidin-1-yl]butan-2-on *N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-[(5-phenyl-1,3-oxazol-4-yl)carbonyl]-piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-[(1-phenyl-1*H*-pyrazol-5-yl)carbonyl]-piperidin-4-amin
*N*-{(1*R*)-1-(4-Chlorbenzyl)-2-[4-cyclohexyl-4-(1*H-*1,2,4-triazol-1-ylmethyl)piperidin-1-yl]-2-oxo-ethyl}-1-[(6-phenylpyridin-2-yl)carbonyl]-piperidin-4-amin.

30. Arzneimittel, **dadurch gekennzeichnet, daß** es eine Verbindung der Formel (I) nach einem der Ansprüche 1 bis 29 oder ein Additionssalz dieser Verbindung mit einer pharmazeutisch unbedenklichen Säure oder ein Hydrat oder ein Solvat der Verbindung der Formel (I) enthält.

31. pharmazeutische Zusammensetzungen, **dadurch gekennzeichnet, daß** sie eine Verbindung der Formel (I) nach einem der Ansprüche 1 bis 29 oder ein pharmazeutisch unbedenkliches Salz, ein Hydrat oder ein Solvat dieser Verbindung sowie mindestens einen pharmazeutisch unbedenklichen Hilfsstoff enthält.

32. Verwendung einer Verbindung der Formel (I) nach einem der Ansprüche 1 bis 29 zur Herstellung eines Arzneimittels zur Behandlung und Prävention von Fettleibigkeit, Diabetes und Sexualdysfunktionen, die beide Geschlechter betreffen können, zur Behandlung von Herz-Kreislauf-Krankheiten sowie bei entzündungshemmenden Anwendungen oder bei der Behandlung von Alkoholismus.

33. Verwendung nach Anspruch 32, **dadurch gekennzeichnet, daß** die Sexualdysfunktionen aus erektilen Dysfunktionen bestehen.

34. Verfahren zur Herstellung einer Verbindungen der Formel (I) nach einem der Ansprüche 1 bis 22, **dadurch gekennzeichnet, daß** man eine Verbindung der Formel (V): 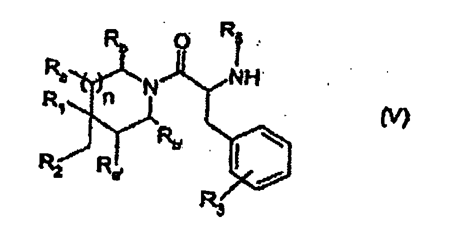 in Gegenwart eines Derivats der Gruppe R₄ vom Keton-Typ reduktiv aminiert, wobei R₁, R₂, R₃, R₄, R₅, Rₐ, P_{a'}, R_{b}, R_{b'} und n die in einem der Ansprüche 1 bis 22 angegebene Bedeutung besitzen.

35. Verbindungen der Formel (IV) und (V): 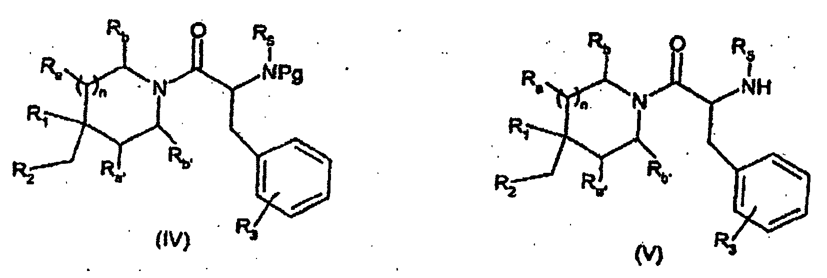 worin R₁, Rₐ, R_{a'}, R_{b} und R_{b'} die in einem der Ansprüche 1 bis 22 angegebene Bedeutung besitzen, Pg für eine Schutzgruppe steht und:
n = 1, Rₐ und R_{a'} gleich oder voneinander verschieden sind und für ein Wasserstoffatom oder eine Alkyl- oder Cycloalkylgruppe stehen und R_{b} und R_{b'} zusammen mit den Kohlenstoffatomen des Rings, an den sie gebunden sind, eine Kohlenstoffbrücke mit 4 bis 5 Gliedern bilden.

36. Verbindungen der Formeln (VI), (XXVIII) und (XXIX), worin R₁, R₂, R₃, R₅, Rₐ, R_{a'}, R_{b}, R_{b'} und n die in einem der Ansprüche 1 bis 22 angegebene Bedeutung besitzen und R₄ für eine Gruppe der Formel (a) oder (b) gemäß einem der Ansprüche 1 bis 17 steht und Pg für eine Amin- oder Hydroxylschutzgruppe steht:

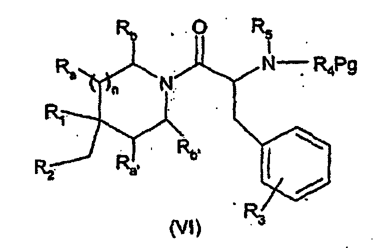

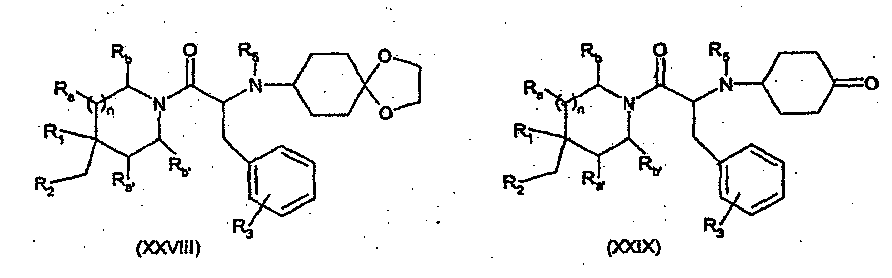

37. Verbindungen der Formel (II): 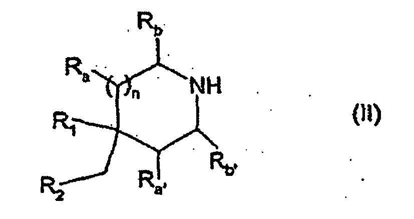 worin R₁, Rₐ, R_{a'}, R_{b} und R_{b'} die in einem der Ansprüche 1 bis 22 angegebene Bedeutung besitzen, Pg für eine Schutzgruppe steht und:
n = 1, Rₐ und R_{a'} gleich oder voneinander verschieden sind und für ein Wasserstoffatom oder eine Alkyl- oder Cycloalkylgruppe stehen und R_{b} und R_{b'} zusammen mit den Kohlenstoffatomen des Rings, an den sie gebunden sind, eine Kohlenstoffbrücke mit 4 bis 5 Gliedern bilden.
